# EUROPÄISCHE PATENTANMELDUNG

(11) **EP 2 371 849 A1**
(43) Veröffentlichungstag der Anmeldung: **05.10.2011**
(21) Anmeldenummer: 11002014.6
(22) Anmeldetag: 18.05.2005
(51) Int. Cl.: C07K 14/47, C07K 16/30, A61K 39/395, A61P 35/00

(54) **Differentiell in Tumoren exprimierte Genprodukte und deren Verwendung**

(30) Priorität: 18.05.2004 DE 102004024617
(62) Teilanmeldung aus: 05745559.4
(71) Anmelder: GANYMED Pharmaceuticals AG, 55131 Mainz (DE)
(72) Erfinder: Türeci, Özlem, 55116 Mainz (DE); Sahin, Ugur, 55116 Mainz (DE); Koslowski, Michael, 65933 Frankfurt (DE); Fritz, Stefan, 55237 Flonheim (DE); Geppert, Harald-Gerhard, 42697 Solingen (DE)
(74) Vertreter: Schnappauf, Georg

(57) **Zusammenfassung**

Erfindungsgemäss wurden Tumor-assoziiert exprimierte Genprodukte und die dafür kodierenden Nukleinsäuren identifiziert. Die vorliegende Erfindung betrifft die Therapie und Diagnose von Erkrankungen, bei denen diese Tumor-assoziiert exprimierten Genprodukte aberrant exprimiert werden. Des weiteren betrifft die Erfindung Proteine, Polypeptide und Peptide, die Tumor-assoziiert exprimiert werden und die dafür kodierenden Nukleinsäuren.

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen. An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = Histokompatibilitätsantigene) Klassen II bzw. I präsentiert werden, während B-Lymphozyten zirkulierende Antikörperinoleküle produzieren, die direkt an unprozessierte Antigene binden. Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster oder zirkulierende Autoantikörper, wurden genutzt, um die respektiven Antigene zu klonieren.

Durch diese Ansätze sind in den letzten Jahren eine Vielzahl von Antigenen in verschiedenen Neoplasien definiert worden. Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumor-assoziiert exprimierter Antigene und der dafür kodierenden Nukleinsäuren verfolgt. Diese Strategie beruht auf der Tatsache, dass bestimmte Gene, die Organ-spezifisch, z.B. ausschließlich im Kolon-, Lungen- oder Nieren-Gewebe, exprimiert werden, in den entsprechenden Organen auch von Tumorzellen und darüber hinaus in anderen Geweben in Tumorzellen ektop und unerlaubt reaktiviert werden. Durch Datamining wird zunächst eine möglichst komplette Liste aller bekannten Organ-spezifischen Gene aufgestellt und diese sodann durch Expressionsanalysen mittels spezifischer RT-PCR auf ihre aberrante Aktivierung in unterschiedlichen Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumorspezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Organ-spezifischer Gene zu nutzen und diese sodann auf Expression in Tumoren zu evaluieren.

Somit betrifft die Erfindung in einem Aspekt eine Strategie zur Identifizierung von Gewebespezifischen und differentiell in Tumoren exprimierten Genen. Diese Strategie kombiniert Datamining von öffentlichen Sequenzbanken ("*in silico*") mit darauf folgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Tumor-Gene. Diese sind bisher als rein Organ-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser Tumor-assoziierten Gene und der dadurch kodierten Genprodukte erfolgte erfindungsgemäß unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: **1-8, 41-44, 51-59, 84, 117, 119 und 138,** einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NO: **1-8, 41-44, 51-59, 84, 117, 119 und 138** ausgewählt ist In einer weiteren bevorzugten Ausführungsform umfasst ein erfindungsgemäß identifiziertes Tumor-assoziiertes Antigen eine Aminosäuresequenz, die aus der Gruppe bestehend aus SEQ ID NO: **9-19, 45-48, 60-66, 85, 90-97, 100-102, 105,106, 111-116, 118, 120, 123,124, 135-137, 139 und 142-150,** einem Teil oder Derivat davon ausgewählt ist.

Die vorliegende Erfindung betrifft allgemein die Verwendung von erfindungsgemäß identifizierten Tumor-assoziierten Antigenen oder von Teilen oder Derivaten davon, von dafür kodierenden Nukleinsäuren oder von Nukleinsäuren, die gegen die kodierenden Nukleinsäuren gerichtet sind, oder von Antikörpern, die gegen die erfindungsgemäß identifizierten Tumor-assoziierten Antigene oder Teile oder Derivate davon gerichtet sind, für die Therapie und Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigenen vorliegt.

Die Erfindung betrifft auch Nukleinsäuren und Genprodukte, die tumorzellassoziiert exprimiert werden.

Desweiteren betrifft die Erfindung Genprodukte, d.h. Nukleinsäuren und Proteine bzw. Peptide, die durch verändertes Spleißen (Spleißvarianten) bekannter Gene bzw. durch veränderte Translation unter Nutzung alternativer offener Leserahmen entstehen. In diesem Aspekt betrifft die Erfindung Nukleinsäuren, die eine Nukleinsäuresequenz ausgewählt aus der Gruppe bestehend aus den Sequenzen gemäß SEQ ID NO: 3-5 des Sequenzprotokolls umfassen. Außerdem betrifft die Erfindung in diesem Aspekt Proteine bzw. Peptide, die eine Aminosäuresequenz ausgewählt aus der Gruppe bestehend aus den Sequenzen gemäß SEQ ID NO: **10 und 12-14** des Sequenzprotokolls umfassen. Die erfindungsgemäßen Spleißvarianten sind erfindungsgemäß als Targets für die Diagnostik und Therapie von Tumorerkrankungen verwendbar.

Insbesondere betrifft die Erfindung die Aminosäuresequenz gemäß SEQ ID NO: 10 des Sequenzprotokolls, die durch einen erfindungsgemäß identifizierten alternativen offenen Leseraster kodiert wird und sich von der vorbeschriebenen Protein-Sequenz (SEQ ID NO: 9) durch 85 zusätzliche Aminosäuren am N-Terminus des Proteins unterscheidet.

Für die Entstehung von Spleißvarianten können verschiedenste Mechanismen ursächlich sein, beispielsweise
- die Nutzung variabler Transkriptionsinitiationsstellen,
- die Nutzung zusätzlicher Exons,
- das vollständige oder unvollständige Ausspleißen von einzelnen oder mehreren Exons,
- durch Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei Tumor-assoziierten Spleißvarianten können Tumor-assoziierte Transkripte und Tumor-assoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvarianten-spezifischen Oligonukleotiden erfolgen. Als Oligonukleotide eignen sich insbesondere Paare von Primern, von denen mindestens einer unter stringenten Bedingungen an die Region der Spleißvariante bindet, die Tumor-assoziiert ist. Erfindungsgemäß geeignet sind die in den Beispielen für diesen Zweck beschriebenen Oligonukleotide, insbesondere Oligonukleotide, die eine Sequenz ausgewählt aus SEQ ID NO: **34-36, 39, 40 und 107-110** des Sequenzprotokolls aufweisen bzw. umfassen. Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. In diesem Aspekt betrifft die Erfindung insbesondere Peptide, die eine Sequenz ausgewählt aus SEQ ID NO: **17-19, 111-115, 120 und 137** des Sequenzprotokolls aufweisen bzw. umfassen und dagegen gerichtete spezifische Antikörper. Die Detektion von Tumorzellen kann auch durch Antikörper erfolgen, die tumorspezifisch veränderte Glykosylierungsvarianten erkennen. Für die Generierung von derartigen Antikörpern können Peptidregionen genutzt werden, die sich in Tumorzellen und gesunden Zellen glykosylierungsbedingt unterscheiden. In diesem Aspekt betrifft die Erfindung insbesondere Peptide, die eine Sequenz ausgewählt aus SEQ ID NO: **17-19, 111-115, 120, 137 und 142-145,** des Sequenzprotokolls aufweisen bzw. umfassen und dagegen gerichtete spezifische Antikörper. Durch endogene Deglykosylierung von N-gekoppelten Zuckerresten wird Asparagin in Asparaginsäure transformiert. Erfindungsgemäß können daher die hier beschriebenen Proteine tumorspezifisch sequenzverändert sein und damit andere biochemische und Antikörper-Bindungseigenschaften aufweisen. In diesem Aspekt betrifft die Erfindung insbesondere Peptide, die eine Sequenz ausgewählt aus SEQ ID NO: **146-150** des Sequenzprotokolls aufweisen bzw. umfassen und dagegen gerichtete spezifische Antikörper. Für die Immunisierung eignen sich besonders die Aminosäuren, die deutliche Epitopunterschiede zu der (den) Variante(n) des Genprodukts aufweisen, welche(s) bevorzugt in gesunden Zellen gebildet wird (werden). Der Nachweis der Tumorzellen mit Antikörpern kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben (vgl. z.B. Kessler JH, et al. 2001, Sahin et al., 1997) und basieren ebenfalls auf der Nutzung von Oligo-oder Polypeptiden, die die Spleißvarianten-spezifischen Epitope beinhalten oder Nukleinsäuren, die für diese kodieren. Oligo- oder Polypeptiden, die die Spleißvarianten-spezifischen Epitope beinhalten, oder Nukleinsäuren, die für diese Polypeptide kodieren, sind auch als pharmazeutisch wirksame Substanzen bei der aktiven Immuntherapie (Vakzinierung, Vakzintherapie) verwendbar.

Erfindungsgemäß werden auch Proteine beschrieben, die sich durch Art und Menge ihrer sekundären Modifikationen in Normal- und Tumorgewebe unterscheiden (z.B. Durand & Seta, 2000; Clin. Chem. 46: 795-805; Hakomori, 1996; Cancer Res. 56: 5309-18).

Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western-Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

Erfindungsgemäß konnte für Claudin-18 eine differentielle Glykosylierung in Tumoren nachgewiesen werden. Gastrointestinale Karzinome, Pankreaskarzinome, Ösophagustumoren, Prostatatumoren als auch Lungentumoren weisen eine weniger glykosylierte Form von Claudin-18 auf. Die Glykosylierung in gesunden Geweben maskiert Proteinepitope von Claudin-18, die auf Tumorzellen aufgrund fehlender Glykosylierung freigelegt sind. Entsprechend lassen sich erfindungsgemäß Liganden und Antikörper selektieren, die an diese Domänen binden. Derartige Liganden und Antikörper binden erfindungsgemäß nicht an das Claudin-18 auf gesunden Zellen, da hier die Epitope durch die Glykosylierung verdeckt sind.

Ähnlich wie oben für von Tumor-assoziierten Spleißvarianten abgeleitete Proteinepitope beschrieben kann somit die differenzielle Glycosylierung zur Unterscheidung von Normal-und Tumorzellen mit diagnostischer wie auch therapeutischer Intention genutzt werden.

In einem Aspekt betrifft die Erfindung eine pharmazeutische Zusammensetzung umfassend ein Mittel, das das erfindungsgemäß identifizierte Tumor-assoziierte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter oder Toxin-konjugierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt der Erfindung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zellen lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die Erfindung eine pharmazeutische Zusammensetzung umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein erfindungsgemäß identifiziertes Tumor-assoziiertes Antigen ist.

Des weiteren betrifft die Erfindung eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die Erfindung eine pharmazeutische Zusammensetzung, die einen oder mehrer Bestandteile umfasst, die aus der Gruppe ausgewählt sind, bestehend aus (i) einem erfindungsgemäß identifizierten Tumor-assoziierten Antigen oder einem Teil davon, (ii) einer Nukleinsäure, die für ein erfmdungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfmdungsgemäß identifiziertes Tumor-assoziiertes Antigen oder einen Teil davon exprimiert, und (vi) isolierten Komplexen zwischen einem erfindungsgemäB identifizierten Tumor-assoziierten Antigen oder einem Teil davon und einem HLA-Molekül.

Eine Nukleinsäure, die für ein erfindungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer erfindungsgemäßen pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer erfindungsgemäßen pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die alle durch kombinatorische Techniken hergestellt werden können. Der Antikörper kann mit einem therapeutisch oder diagnostisch nützlichen Mittel oder Stoff gekoppelt sein.

Eine in einer erfindungsgemäßen pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch eine erfindungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine erfindungsgemäße pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Cytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren betrifft die Erfindung Verfahren zur Behandlung, Diagnose und/oder Überwachung einer Erkrankung, die sich durch die Expression oder abnormale Expression eines oder mehrerer Tumor-assoziierter Antigene auszeichnet. In einer Ausführungsform umfasst die Behandlung die Verabreichung einer erfindungsgemäßen pharmazeutischen Zusammensetzung.

Vorzugsweise ist die Erkrankung Krebs, wobei der Begriff "Krebs" in nicht-begrenzender Weise Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

In einem Aspekt betrifft die Erfindung ein Verfahren zur Diagnose einer Erkrankung, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (üi) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

In einem weiteren Aspekt betrifft die Erfindung ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, wodurch spezifische T-Lymphozyten identifiziert werden können.

In einem weiteren Aspekt betrifft die Erfindung ein Verfahren zur Behandlung, Diagnose oder Überwachung einer Erkrankung, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel oder Stoff gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

In manchen Ausführungsformen erfolgen die erfindungsgemäßen Verfahren zur Diagnose oder Überwachung einer Erkrankung, die sich durch die Expression oder abnormale Expression eines erfindungsgemäß identifizierten Tumor-assoziierten Antigens auszeichnet, mit Hilfe von oder durch Nachweis von disseminierenden Tumorzellen oder von Tumormetastasen. Ein Nachweis disseminierender Tumorzellen kann beispielsweise im Blut, Serum, Knochenmark, Sputum, Bronchialaspirat und/oder Bronchiallavage erfolgen.

Die Erfindung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die Erfindung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Erfindung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Erfindung betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assozüerten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die erfindungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Erfindung eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NO: **3-5,** einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. Des weiteren betrifft die Erfindung eine Nukleinsäure, die für ein Protein oder Polypeptid kodiert, das eine Aminosäuresequenz umfasst, ausgewählt aus der Gruppe bestehend aus SEQ ID NO: **10, 12-14 und 146-150,** einem Teil oder Derivat davon.

In einem weiteren Aspekt betrifft die Erfindung Promotorsequenzen von erfindungsgemäßen Nukleinsäuren. Diese können funktionell mit einem anderen Gen vorzugsweise in einem Expressionsvektor verbunden werden und somit die selektive Expression dieses Gens in entsprechenden Zellen gewährleisten.

In einem weiteren Aspekt betrifft die Erfindung ein rekombinantes Nukleinsäuremolekül, insbesondere DNA- oder RNA-Molekül, das eine erfindungsgemäße Nukleinsäure umfasst.

Die Erfindung betrifft auch Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Erfindung Oligonukleotide, die mit einer erfindungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northem-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Erfindung ein Protein, Polypeptid oder Peptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: **3-5,** einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform betrifft die Erfindung ein Protein oder Polypeptid oder Peptid, das eine Aminosäuresequenz umfasst, ausgewählt aus der Gruppe bestehend aus SEQ ID NO: **10,12-14 und 146-150,** einem Teil oder Derivat davon.

In einem weiteren Aspekt betrifft die Erfindung ein immunogenes Fragment eines erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, insbesondere mindestens **8,** mindestens 10, mindestens 12, mindestens 15, mindestens 20, mindestens 30 oder mindestens 50 Aminosäuren.

In diesem Aspekt betrifft die Erfindung insbesondere ein Peptid, das eine Sequenz, ausgewählt aus der Gruppe bestehend aus SEQ ID NO: **17-19, 90-97, 100-102, 105, 106, 111-116, 120, 123, 124, 135-137, 139 und 142-150,** einem Teil oder Derivat davon aufweist oder umfasst.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellter Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Insbesondere betrifft die Erfindung ein solches Mittel, insbesondere einen Antikörper, das/der spezifisch an ein Peptid bindet, das eine Sequenz ausgewählt aus der Gruppe bestehend aus SEQ ID NO: **17-19, 90-97, 100-102, 105, 106, 111-116, 120, 123, 124, 135-137, 139 und 142-150,** einem Teil oder Derivat davon aufweist oder umfasst.

Hinsichtlich Claudin-18 betrifft die Erfindung auch Mittel, insbesondere Antikörper, die spezifisch an eine Variante von Claudin-18 binden. In einer Ausführungsform bindet das Mittel, insbesondere ein Antikörper, spezifisch an die Variante Claudin-18A1 (SEQ ID NO: 118). In einer anderen Ausführungsform bindet das Mittel, insbesondere ein Antikörper, spezifisch an die Variante Claudin-18A2 (SEQ ID NO: 16). Solche spezifischen Antikörper können beispielweise durch Immunisieren mit den in Beispiel 4 beschriebenen Peptiden erhalten werden.

Des weiteren betrifft die Erfindung hinsichtlich Claudin-18 Mittel, insbesondere Antikörper, die spezifisch an eine Form von Claudin-18A2 binden, die ein bestimmtes Glykosylierungmuster aufweist. In einer Ausführungsform bindet das Mittel, insbesondere ein Antikörper, spezifisch an eine Form von Claudin-18A2, die an einer oder mehreren möglichen Glykosylierungsstellen nicht-glykosyliert ist. In einer anderen Ausführungsform bindet das Mittel, insbesondere ein Antikörper, spezifisch an eine Form von Claudin-18A2, die an einer oder mehreren möglichen Glykosylierungsstellen glykosyliert ist. Vorzugsweise betrifft eine derartige mögliche Glykosylierungsstelle eine oder mehrere Positionen, die aus der Gruppe ausgewählt sind, bestehend aus den Aminösäurepositionen 37, 38, 45, 116, 141, 146 und 205 von Claudin-18A2. Des weiteren betrifft eine derartige mögliche Glykosylierung vorzugsweise eine N-Glykosylierung.

Ein für eine Variante bzw. Form von Claudin-18 spezifisches Mittel, insbesondere ein für eine Variante bzw. Form von Claudin-18 spezifischer Antikörper, meint in diesem Zusammenhang, dass das Mittel bzw. der Antikörper stärker an die Variante bzw. Form für die es/er spezifisch ist, bindet als an eine andere Variante bzw. Form. Ein Mittel, insbesondere Antikörper, bindet stärker an eine erste Variante bzw. Form oder an ein erstes Epitop im Vergleich zu einer zweiten Variante bzw. Form oder einem zweiten Epitop, wenn es/er an die erste Variante bzw. Form oder an das erste Epitop mit einer Dissoziationskonstante (K_{D}) bindet, die geringer ist als die Dissoziationskonstante für die zweite Variante bzw. Form oder das zweite Epitop. Vorzugsweise ist die Dissoziationskonstante (K_{D}) für die Variante bzw. Form oder das Epitop, an die/das das Mittel, insbesondere ein Antikörper, spezifisch bindet mehr als 10-fach, vorzugsweise mehr als 20-fach, mehr bevorzugt mehr als 50-fach, noch mehr bevorzugt mehr als 100-fach und insbesondere mehr als 200-fach, 500-fach oder 1000-fach geringer als die Dissoziationskonstante (K_{D}) für die Variante bzw. Form oder das Epitop, an die/das das Mittel, insbesondere ein Antikörper, nicht spezifisch bindet. Vorzugsweise bindet ein Mittel, insbesondere ein Antikörper, nicht oder im wesentlichen nicht an die Variante bzw. Form oder das Epitop für die/das das Mittel, insbesondere der Antikörper, nicht spezifisch ist.

Die vorstehend beschriebenen Mittel, insbesondere Antikörper und Derivate davon wie hierin beschrieben, die an eine Variante bzw. Form von Claudin-18 spezifisch binden, sind auch für eine Verwendung in den erfindungsgemäßen Zusammensetzungen und Verfahren vorgesehen.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel oder Stoff. In einer Ausführungsform ist das therapeutische oder diagnostische Mittel ein Toxin.

In einem weiteren Aspekt betrifft die Erfindung einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

Die Erfindung betrifft insbesondere folgendes:
1. Pharmazeutische Zusammensetzung, umfassend ein Mittel, das die Expression oder Aktivität eines Tumor-assoziierten Antigens hemmt, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
2. Pharmazeutische Zusammensetzung, umfassend ein Mittel mit tumorhemmender Aktivität, das selektiv ist für Zellen, die eine Expression oder abnormale Expression eines tumorassoziierten Antigens aufweisen, wobei das Tumor-assoziierte Antigen eine Sequenz aufweist, die von einer Nukleinsäure kodiert wird, die aus der Gruppe ausgewählt ist, bestehend aus
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
3. Pharmazeutische Zusammensetzung nach Ziffer 2, wobei das Mittel die Induktion des Zelltods, die Reduktion des Zellwachstums, eine Schädigung der Zellmembran oder eine Sekretion von Zytokinen bewirkt.
4. Pharmazeutische Zusammensetzung nach Ziffer 1 oder 2, wobei das Mittel eine Antisense-Nukleinsäure ist, die selektiv mit der Nukleinsäure hybridisiert, die für das Tumor-assoziierte Antigen kodiert.
5. Pharmazeutische Zusammensetzung nach Ziffer 1 oder 2, wobei das Mittel ein Antikörper ist, der selektiv an das Tumor-assoziierte Antigen bindet.
6. Pharmazeutische Zusammensetzung nach Ziffer 2, wobei das Mittel ein komplementaktivierender Antikörper ist, der selektiv an das Tumor-assoziierter Antigen bindet.
7. Pharmazeutische Zusammensetzung, umfassend ein Mittel, das bei einer Verabreichung selektiv die Menge an Komplexen zwischen einem HLA-Molekül und einem Tumor-assoziierten Antigen oder einem Teil davon erhöht, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist
8. Pharmazeutische Zusammensetzung nach Ziffer 7, wobei das Mittel einen oder mehrere Bestandteile umfasst, die aus der Gruppe ausgewählt sind, bestehend aus:
   (i) dem Tumor-assoziierten Antigen oder einem Teil davon,
   (ii) einer Nukleinsäure, die für das Tumor-assoziierte Antigen oder einen Teil davon kodiert,
   (iii) einer Wirtszelle, die das Tumor-assoziierte Antigen oder einen Teil davon exprimiert, und
   (iv) isolierten Komplexen zwischen dem Tumor-assoziierten Antigen oder einem Teil davon und einem HLA-Molekül.
9. Pharmazeutische Zusammensetzung nach Ziffer 1, 2 oder 7, wobei das Mittel mehrere Mittel umfasst, die jeweils selektiv die Expression oder Aktivität verschiedener Tumor-assoziierter Antigene hemmen, jeweils selektiv für Zellen sind, die verschiedene Tumor-assoziierte Antigene exprimieren oder die Menge an Komplexen zwischen HLA-Molekülen und verschiedenen Tumor-assoziierten Antigenen oder Teilen davon erhöhen, wobei mindestens eines der Tumor-assoziierten Antigene eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
10. Pharmazeutische Zusammensetzung umfassend einen oder mehrer Bestandteile, die aus der Gruppe ausgewählt sind, bestehend aus:
   (i) einem Tumor-assoziierten Antigen oder einem Teil davon,
   (ii) einer Nukleinsäure, die für ein Tumor-assoziiertes Antigen oder einen Teil davon kodiert,
   (iii) einem Antikörper, der an ein Tumor-assoziiertes Antigen oder einen Teil davon bindet,
   (iv) einer Antisense-Nukleinsäure, die spezifisch mit einer Nukleinsäure, die für ein Tumor-assoziiertes Antigen kodiert, hybridisiert,
   (v) einer Wirtszelle, die ein Tumor-assoziiertes Antigen oder einen Teil davon exprimiert, und
   (vi) isolierten Komplexen zwischen einem Tumor-assoziierten Antigen oder einem Teil davon und einem HLA-Molekül,
   wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
11. Pharmazeutische Zusammensetzung nach Ziffer 8 oder 10, wobei die Nukleinsäure unter (ii) in einem Expressionsvektor vorliegt.
12. Pharmazeutische Zusammensetzung nach Ziffer 8 oder 10, wobei die Nukleinsäure unter (ii) funktionell mit einem Promotor verbunden ist
13. Pharmazeutische Zusammensetzung nach Ziffer 8 oder 10, wobei die Wirtszelle das Tumor-assoziierte Antigen oder den Teil davon sekretiert.
14. Pharmazeutische Zusammensetzung nach Ziffer 8 oder 10, wobei die Wirtszelle zusätzlich ein HLA-Molekül exprimiert, das an das Tumor-assoziierte Antigen oder den Teil davon bindet.
15. Pharmazeutische Zusammensetzung nach Ziffer 14, wobei die Wirtszelle das HLA-Molekül und/oder das Tumor-assoziierte Antigen oder den Teil davon rekombinant exprimiert.
16. Pharmazeutische Zusammensetzung nach Ziffer 14, wobei die Wirtszelle das HLA-Molekül endogen exprimiert.
17. Pharmazeutische Zusammensetzung nach Ziffer 8, 10, 14 oder 16, wobei die Wirtszelle eine Antigen-präsentierende Zelle ist.
18. Pharmazeutische Zusammensetzung nach Ziffer 17, wobei die Antigen-präsentierende Zelle eine dendritische Zelle oder ein Makrophage ist.
19. Pharmazeutische Zusammensetzung nach einer der Ziffern 8, 10 und 13-18, wobei die Wirtszelle nicht-proliferativ ist.
20. Pharmazeutische Zusammensetzung nach Ziffer 5 oder 10, wobei der Antikörper ein monoklonaler Antikörper ist.
21. Pharmazeutische Zusammensetzung nach Ziffer 5 oder 10, wobei der Antikörper ein chimärer oder humanisierten Antikörper ist.
22. Pharmazeutische Zusammensetzung nach Ziffer 5 oder 10, wobei der Antikörper ein Fragment eines natürlichen Antikörpers ist.
23. Pharmazeutische Zusammensetzung nach Ziffer 5 oder 10, wobei der Antikörper mit einem therapeutischen oder diagnostischen Mittel gekoppelt ist.
24. Pharmazeutische Zusammensetzung nach Ziffer 4 oder 10, wobei die Antisense-Nukleinsäure eine Sequenz von 6-50 zusammenhängenden Nukleotiden aus der Nukleinsäure, die für das Tumor-assoziierte Antigen kodiert, umfasst.
25. Pharmazeutische Zusammensetzung nach einer der Ziffern 8 und 10-13, wobei das durch die pharmazeutische Zusammensetzung bereitgestellte Tumor-assoziierte Antigen oder der Teil davon an MHC-Moleküle auf der Oberfläche von Zellen bindet, die eine abnormale Menge des Tumor-assoziierten Antigens oder eines Teils davon exprimieren.
26. Pharmazeutische Zusammensetzung nach Ziffer 25, wobei die Bindung eine cytolytische Reaktion hervorruft und/ oder eine Cytokinausschüttung induziert
27. Pharmazeutische Zusammensetzung nach einer der Ziffern 1-26, ferner umfassend einen pharmazeutisch verträglichen Träger und/oder ein Adjuvans.
28. Pharmazeutische Zusammensetzung nach Ziffer 27, wobei das Adjuvans Saponin, GM-CSF, CpG, Zytokin oder ein Chemokin ist.
29. Pharmazeutische Zusammensetzung nach einer der Ziffern 1-28, die zur Behandlung einer Erkrankung eingesetzt werden kann, die sich durch die Expression oder abnormale Expression eines Tumor-assoziierten Antigens auszeichnet.
30. Pharmazeutische Zusammensetzung nach Ziffer 29, wobei die Erkrankung Krebs ist.
31. Pharmazeutische Zusammensetzung nach Ziffer 29, wobei die Erkrankung ein Lungentumor, ein Brusttumor, ein Prostatatumor, ein Melanom, ein Kolontumor, ein Magentumor, ein Pankreastumor, ein HNO-Tumor, Nierenzellkarzinom oder ein Zervixkarzinom, ein Kolonkarzinom oder ein Mammakarzinom ist
32. Pharmazeutische Zusammensetzung nach einer der Ziffern 1-31, wobei das Tumor-assoziierte Antigen eine Aminosäuresequenz umfasst, die aus der Gruppe bestehend aus SEQ ID NOs: 9-19, 45-48, 60-66, 85, 90-97, 100-102, 105, 106, 111-116, 118, 120, 123, 124, 135-137, 139 und 142-150, einem Teil oder Derivat davon ausgewählt ist.
33. Verfahren zur Diagnose einer Erkrankung, die sich durch die Expression oder abnormale Expression eines Tumor-assoziierten-Antigens auszeichnet, umfassend
   (i) den Nachweis einer Nukleinsäure, die für das Tumor-assoziierte Antigen kodiert, oder eines Teils davon, und/oder
   (ii) den Nachweis des Tumor-assoziierten Antigens oder eines Teils davon, und/oder
   (iii) den Nachweis eines Antikörpers gegen das Tumor-assoziierte Antigen oder eines Teils davon und/oder
   (iv) den Nachweis von cytotoxischen oder Helfer-T-Lymphozyten, die für das Tumor-assoziierte. Antigen oder einen Teil davon spezifisch sind in einer aus einem Patienten isolierten biologischen Probe, wobei
   das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
34. Verfahren nach. Ziffer 33, wobei der Nachweis
   (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an die cytotoxischen oder Helfer-T-Lymphozyten bindet, und
   (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten umfasst.
35. Verfahren nach Ziffer 33 oder 34, wobei der Nachweis mit dem Nachweis in einer vergleichbaren normalen biologischen Probe verglichen wird.
36. Verfahren nach einer der Ziffern 33-35, wobei sich die Erkrankung durch die Expression oder abnormale Expression mehrerer verschiedener Tumor-assoziierter Antigene auszeichnet und der Nachweis einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind, umfasst.
37. Verfahren nach einer der Ziffern 33-36, wobei der Nachweis der Nukleinsäure oder des Teils davon mit einer Polynukleotid-Sonde erfolgt, die spezifisch mit der Nukleinsäure oder dem Teil davon hybridisiert.
38. Verfahren nach Ziffer 37, wobei die Polynukleotid-Sonde eine Sequenz von 6-50 zusammenhängenden Nukleotiden aus der Nukleinsäure, die für das Tumor-assoziierte Antigen kodiert, umfasst.
39. Verfahren nach einer der Ziffern 33-36, wobei der Nachweis der Nukleinsäure oder des Teils davon durch selektive Amplifikation der Nukleinsäure oder des Teils davon erfolgt.
40. Verfahren nach einer der Ziffern 33-36, wobei das nachzuweisende Tumor-assoziierte Antigen oder der Teil davon in einem Komplex mit einem MHC-Molekül vorliegt.
41. Verfahren nach Ziffer 40, wobei das MHC-Molekül ein HLA-Molekül ist.
42. Verfahren nach einer der Ziffern 33-36 und 40-41, wobei der Nachweis des Tumor-assoziierten Antigens oder des Teils davon mit einem Antikörper erfolgt, der spezifisch an das Tumor-assoziierte Antigen oder den Teil davon bindet.
43. Verfahren nach einer der Ziffern 33-36, wobei der Nachweis des Antikörpers mit einem Protein oder Peptid erfolgt, das spezifisch an den Antikörper bindet
44. Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, ausgewählt aus der Gruppe, bestehend aus:
   (i) der Menge der Nukleinsäure, die für das Tumor-assoziierte Antigen kodiert, oder eines Teil davon,
   (ii) der Menge des Tumor-assoziierten Antigens oder eines Teils davon,
   (iii) der Menge an Antikörpern, die an das Tumor-assoziierte Antigen oder einen Teil davon binden, und
   (iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
45. Verfahren nach Ziffer 44, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Erkrankung ermittelt wird.
46. Verfahren nach Ziffer 44 oder 45, wobei die Erkrankung sich durch die Expression oder abnormale Expression mehrerer verschiedener Tumor-assoziierter Antigene auszeichnet und die Überwachung eine Überwachung
   (i) der Menge mehrerer Nukleinsäuren, die für die mehreren verschiedenen Tumor-assoziierten Antigene kodieren, oder von Teilen davon,
   (ii) der Menge der mehreren verschiedenen Tumor-assoziierten Antigene oder von Teilen davon,
   (iii) der Menge mehrerer Antikörper, die an die mehreren verschiedenen Tumor-assoziierten Antigene oder an Teile davon binden, und/oder
   (iv) der Menge mehrerer cytolytischer oder Cytokine-ausschüttender T-Zellen, die für Komplexe zwischen den mehreren verschiedenen Tumor-assoziierten Antigenen oder von Teilen davon und MHC-Molekülen spezifisch sind, umfasst.
47. Verfahren nach einer der Ziffern 44-46, wobei die Überwachung der Menge der Nukleinsäure oder des Teils davon mit einer Polynukleotid-Sonde erfolgt, die spezifisch mit der Nukleinsäure oder dem Teil davon hybridisiert.
48. Verfahren nach Ziffer 47, wobei die Polynukleotid-Sonde eine Sequenz von 6-50 zusammenhängenden Nukleotiden aus der Nukleinsäure, die für das Tumor-assoziierte Antigen kodiert, umfasst.
49. Verfahren nach einer der Ziffern 44-46, wobei die Überwachung der Menge der Nukleinsäure oder des Teils davon durch selektive Amplifikation der Nukleinsäure oder des Teils davon erfolgt.
50. Verfahren nach einer der Ziffern 44-46, wobei die Überwachung der Menge des Tumor-assoziierten Antigens oder des Teils davon mit einem Antikörper erfolgt, der spezifisch an das Tumor-assoziierte Antigen oder den Teil davon bindet.
51. Verfahren nach einer der Ziffern 44-46, wobei die Überwachung der Menge an Antikörpern mit einem Protein oder Peptid erfolgt, das spezifisch an den Antikörper bindet.
52. Verfahren nach einer der Ziffern 44-46, wobei die Überwachung der Menge an cytolytischen oder Cytokin-ausschüttenden T-Zellen mit einer Zelle erfolgt, die den Komplex zwischen dem Tumor-assoziierten Antigen oder dem Teil davon und einem MHC-Molekül präsentiert.
53. Verfahren nach einer der Ziffern 37-38, 42-43, 47-48 und 50-52, wobei die Polynukleotid-Sonde, der Antikörper, das Protein oder Peptid oder die Zelle nachweisbar markiert sind.
54. Verfahren nach Ziffer 53, wobei der nachweisbare Marker ein radioaktiver Marker oder ein Enzymmarker ist.
55. Verfahren nach einer der Ziffern 33-54, wobei die Probe Körperflüssigkeit und/oder Körpergewebe umfasst.
56. Verfahren zur Behandlung einer Erkrankung, die sich durch die Expression oder abnormale Expression eines Tumor-assoziierten Antigens auszeichnet, umfassend die Verabreichung einer pharmazeutischen Zusammensetzung nach einer der Ziffern 1-32, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
57. Verfahren zur Behandlung, Diagnose oder Überwachung einer Erkrankung, die sich durch die Expression oder abnormale Expression eines Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
58. Verfahren nach Ziffer 42, 50 oder 57, wobei der Antikörper ein monoklonaler Antikörper ist.
59. Verfahren nach Ziffer 42, 50 oder 57, wobei der Antikörper ein chimärer oder humanisierter Antikörper ist.
60. Verfahren nach Ziffer 42, 50 oder 57, wobei der Antikörper ein Fragment eines natürlichen Antikörpers ist.
61. Verfahren zur Behandlung eines Patienten mit einer Erkrankung, die sich durch die Expression oder abnormale Expression eines Tumor-assoziierten Antigens auszeichnet, umfassend:
   (i) die Entfernung einer Probe mit immunreaktiver Zellen aus dem Patienten,
   (ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
   (iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
62. Verfahren nach Ziffer 61, wobei die Wirtszelle ein HLA-Molekül rekombinant exprimiert, das an das Tumor-assoziierte Antigen oder einen Teil davon bindet.
63. Verfahren nach Ziffer 62, wobei die Wirtszelle ein HLA-Molekül endogen exprimiert, das an das Tumor-assoziierte Antigen oder einen Teil davon bindet.
64. Verfahren zur Behandlung eines Patienten mit einer Erkrankung, die sich durch die Expression oder abnormale Expression eines Tumor-assoziierten Antigens auszeichnet, umfassend:
   (i) die Identifizierung einer Nukleinsäure, die von Zellen exprimiert wird, die mit der Erkrankung assoziiert sind, wobei die Nukleinsäure aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist,
   (ii) die Transfektion einer Wirtszelle mit der Nukleinsäure oder einem Teil davon,
   (iii) die Kultivierung der transfizierten Wirtszelle für eine Expression der Nukleinsäure, und
   (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen.
65. Verfahren nach Ziffer 64, ferner umfassend die Identifizierung eines MHC-Moleküls, das das Tumor-assoziierte Antigen oder einen Teil davon präsentiert, wobei die Wirtszelle das identifizierte MHC-Molekül exprimiert und das Tumor-assoziierte Antigen oder einen Teil davon präsentiert.
66. Verfahren nach Ziffer 64 oder 65, wobei die Immunreaktion eine B-Zellen-Reaktion oder eine T-Zellen-Reaktion umfasst.
67. Verfahren nach Ziffer 66, wobei die Immunreaktion eine T-Zellen-Reaktion ist, umfassend die Produktion cytolytischer oder Cytokine-ausschüttenden T-Zellen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.
68. Verfahren nach einer der Ziffern 61-67, wobei die Wirtszellen nicht-proliferativ sind.
69. Verfahren zur Behandlung einer Erkrankung, die sich durch die Expression oder abnormale Expression eines Tumor-assoziierten Antigens auszeichnet, umfassend:
   (i) die Identifikation von Zellen aus dem Patienten, die abnormale Mengen des Tumor-assoziierten Antigens exprimieren,
   (ii) die Isolierung einer Probe der Zellen,
   (iii) die Kultivierung der Zellen, und
   (iv) das Einbringen der Zellen in den Patienten in einer Menge, die geeignet ist, eine Immunreaktion gegen die Zellen auszulösen, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
70. Verfahren nach einer der Ziffern 33-69, wobei die Erkrankung Krebs ist.
71. Verfahren zur Hemmung der Entwicklung von Krebs bei einem Patienten, umfassend die Verabreichung einer wirksamen Menge einer pharmazeutischen Zusammensetzung nach einer der Ziffern 1-32.
72. Verfahren nach einer der Ziffern 33-71, wobei das Tumor-assoziierte Antigen eine Aminosäuresequenz umfasst, die aus der Gruppe bestehend aus SEQ ID NOs: 9-19, 45-48, 60-66, 85, 90-97, 100-102, 105, 106, 111-116, 118, 120, 123, 124 , 135-137, 139 und 142-150, einem Teil oder Derivat davon ausgewählt ist.
73. Nukleinsäure, die aus der Gruppe ausgewählt ist, bestehend aus:
   (a) einer Nukleinsäure, die eine Nukleinsäuresequenz umfasst, die aus der Gruppe bestehend aus SEQ ID NOs: 3-5, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
74. Nukleinsäure, die für ein Protein oder Polypeptid kodiert, das eine Aminosäuresequenz umfasst, ausgewählt aus der Gruppe bestehend aus SEQ ID NOs: 10, 12-14, und 146-150, einem Teil oder Derivat davon.
75. Rekombinantes DNA- oder RNA-Molekül, das eine Nukleinsäure nach Ziffer 73 oder 74 umfasst.
76. Rekombinantes DNA-Molekül nach Ziffer 75, wobei das rekombinante DNA-Molekül ein Vektor ist.
77. Rekombinantes DNA-Molekül nach Ziffer 76, wobei der Vektor ein viraler Vektor oder ein Bakteriophage ist.
78. Rekombinantes DNA-Molekül nach einer der Ziffern 75-77, das ferner Expressionskontrollsequenzen umfasst, die die Expression der Nukleinsäure steuern.
79. Rekombinantes DNA-Molekül nach Ziffer 78, wobei die Expressionskontrollsequenzen homo- oder heterolog zu der Nukleinsäure sind.
80. Wirtszelle, die eine Nukleinsäure nach Ziffer 73 oder 74 oder ein rekombinantes DNA-Molekül nach einer der Ziffern 75-79 umfasst.
81. Wirtszelle nach Ziffer 80, die ferner eine Nukleinsäure umfasst, die für ein HLA-Molekül kodiert.
82. Protein oder Polypeptid, das von einer Nukleinsäure nach Ziffer 73 kodiert wird.
83. Protein oder Polypeptid, das eine Aminosäuresequenz umfasst, ausgewählt aus der Gruppe bestehend aus SEQ ID NOs: 10, 12-14, 17-19, 90-97, 100-102, 105, 106, 111-116, 120, 123, 124, 135-137 und 142-150, einem Teil oder Derivat davon.
84. Immunogenes Fragment des Proteins oder Polypeptids nach Ziffer 82 oder 83.
85. Fragment des Proteins oder Polypeptids nach Ziffer 82 oder 83, das an menschlichen HLA-Rezeptor oder menschlichen Antikörper bindet.
86. Mittel, das spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
87. Mittel nach Ziffer 86, wobei das Protein oder Polypeptid eine Aminosäuresequenz umfasst, die aus der Gruppe ausgewählt ist, bestehend aus SEQ ID NOs: 9-19, 45-48, 60-66, 85, 90-97, 100-102, 105, 106, 111-116, 118, 120, 123, 124, 135-137, 139 und 142-150, einem Teil oder Derivat davon.
88. Mittel nach Ziffer 86 oder 87, wobei das Mittel ein Antikörper ist.
89. Mittel nach Ziffer 88, wobei der Antikörper ein monoklonaler, chimärer oder humanisierter Antikörper oder ein Fragment eines Antikörpers ist.
90. Antikörper, der selektiv an einen Komplex aus:
   (i) einem Protein oder Polypeptid oder einem Teil davon und
   (ii) einem MHC-Molekül bindet, an das das Protein oder Polypeptid oder der Teil davon bindet, wobei der Antiköper nicht alleine an (i) oder (ii) bindet und das Protein oder Polypeptid von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
91. Antikörper nach Ziffer 90, wobei das Protein oder Polypeptid eine Aminosäuresequenz umfasst, die aus der Gruppe ausgewählt ist, bestehend aus SEQ ID NOs: 9-19, 45-48, 60-66, 85, 90-97, 100-102, 105, 106, 111-116,118, 120, 123, 124, 135-137, 139 und 142-150, einem Teil oder Derivat davon.
92. Antikörper nach Ziffer 90 oder 91, wobei der Antikörper ein monoklonaler, chimärer oder humanisierter Antikörper oder ein Fragment eines Antikörpers ist.
93. Konjugat zwischen einem Mittel nach einer der Ziffern 86-89 oder einem Antikörper nach einer der Ziffern 90-92 und einem therapeutischen oder diagnostischen Mittel.
94. Konjugat nach Ziffer 93, wobei das therapeutische oder diagnostische Mittel ein Toxin ist.
95. Kit zum Nachweis der Expression oder abnormalen Expression eines Tumor-assoziierten Antigens, umfassend Mittel zum Nachweis
   (i) der Nukleinsäure, die für das Tumor-assoziierte Antigen kodiert, oder eines Teils davon,
   (ii) des Tumor-assoziierten Antigens oder eines Teils davon,
   (iii) von Antikörpern, die an das Tumor-assoziierte Antigen oder einen Teil davon binden, und/oder
   (iv) von T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1-8, 41-44, 51-59, 84, 117, 119 und 138, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
96. Kit nach Ziffer 95, wobei die Mittel zum Nachweis der Nukleinsäure, die für das Tumor-assoziierte Antigen kodiert, oder eines Teils davon Nukleinsäuremoleküle für die selektive Amplifikation der Nukleinsäure sind.
97. Kit nach Ziffer 96, wobei die Nukleinsäuremoleküle für die selektive Ainplifikation der Nukleinsäure eine Sequenz von 6-50 zusammenhängenden Nukleotiden aus der Nukleinsäure, die für das Tumor-assoziierte Antigen kodiert, umfassen.
98. Rekombinantes DNA-Molekül, umfassend eine Promotorregion, die von einer Nukleinsäuresequenz abgeleitet ist, die aus der Gruppe bestehend aus SEQ ID NOs: 1-8, 41-44,51-59, 84, 117, 119 und 138 ausgewählt ist.
99. Pharmazeutische Zusammensetzung nach einem der Ansprüche 5, 10 und 20-23, oder Verfahren nach einer der Ziffern 42, 50, 53 und 57-60, wobei der Antikörper durch Immunisieren mit einem Protein, Peptid oder Polypeptid erhältlich ist, das eine Aminosäuresequenz umfasst, die aus der Gruppe bestehend aus SEQ ID NOs: 9-19, 45-48, 60-66, 85, 90-97, 100-102, 105, 106, 111-116, 118, 120, 123, 124, 135-137, 139 und 142-150, einem Teil oder Derivat davon ausgewählt ist.
100. Pharmazeutische Zusammensetzung nach einem der Ansprüche 5, 10 und 20-23, oder Verfahren nach einer der Ziffern 42, 50, 53 und 57-60, wobei die selektive Bindung des Antikörpers an das Tumor-assoziierte Antigen die Erkennung einer Glykosylierung an dem Tumor-assoziierten Antigen einbezieht.
101. Pharmazeutische Zusammensetzung nach einer der Ziffern 5, 10 und 20-23, oder Verfahren nach einem der Ansprüche 42, 50, 53 und 57-60, wobei der Antikörper selektiv an eine bestimmte Glykosylierungsform des Tumor-assoziierten Antigens, vorzugsweise die nicht glykosylierte Form, bindet.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene und/oder deren Genprodukte und/oder ihre Derivate und/oder Teile bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleirisäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können erfindungsgemäß die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist ein rekombinantes DNA-Molekül erfmdungsgemäß ein Vektor, gegebenenfalls mit einem Promotor, der die Expression einer Nukleinsäure, z.B. einer Nukleinsäure, die für eine erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte erfindungsgemäße Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein erfindungsgemäßes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein erfindungsgemäßes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist Ein isoliertes Protein oder Polypeptid kann in einem im Wesentlichen aufgereinigten Zustand vorliegen. Der Begriff "im Wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen, Polypeptiden oder Peptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine, Polypeptide oder Peptide. Ferner erstreckt sich der Begriff "Derivat" auch auf alle funktionellen chemischen Äquivalente der Proteine, Polypeptide oder Peptide.

Ein Teil oder Fragment eines Tumor-assoziierten Antigens weist erfindungsgemäß eine funktionelle Eigenschaft des Polypeptids auf, aus dem es abgeleitet ist Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Erfindungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Erfindungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindungan solche Antigene Verwendung finden.

Erfindungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Solche Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow; David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären Teilsequenzen, wie auch mit Zellen, die das Zielmolekül in physiologisch gefalteter Form exprimieren, immunisiert werden. Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2-und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Erfindungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluorin und Gadolinium. Der Begriff "therapeutisch nützlicher Stoff' meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, wobei der Begriff "Krebs" erfindungsgemäß Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Sputum, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung.

Die klonal expandierten autologen cytotoxischen T-Lymphozyten werden sodann an den Patienten verabreicht

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse 1-Molekül bindet, gefaltet Nach Aufreinigung der MHC/Peptid-Komplexe werden diese mit Biotin markiert. Tetramere werden durch Mischen der biotinylierten Peptid-MHC-Komplexe mit -markiertem Avidin (z.B. Phycoerythrin) bei einem molaren Verhältnis von 4:1 gebildet Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von gesunden Personen oder von einer anderen Spezies (z.B. Maus) kombiniert. Dies führt zu einer Vermehrung hochaffiner spezifischer cytotoxischer T-Lymphozyten, wenn die T-Lymphozyten aus einem Spenderorganismus kommen, der mit dem spezifischen Komplex bisher keinen Kontakt hatte. Der hochaffine T-Zell-Rezeptor aus diesen vermehrten spezifischen T-Lymphozyten wird kloniert. Wurden die hochaffinen T-Zellrezeptoren aus einer anderen Spezies kloniert, können diese in unterschiedlichem Ausmaß humanisiert werden. Durch Gentransfer z.B. mit retroviralen Vektoren werden solche T-Zell-Rezeptoren dann beliebig in T-Zellen von Patienten transduziert. Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61,2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer T-Zell-Antworten entstehen können. Eine effektive Immunantwort benötigt diese (Ossendorp et al., Immunol Lett. 74:75-9, 2000; Ossendorp et al., J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc Natl Acad Sci USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J Immunol. 167:787-96, 2001; Appella et al., Biomed Pept Proteins Nucleic Acids 1:177-84, 1995; Wentworth et al., Mol Immunol. 32:603-12, 1995).

Die erfindungsgemäß beschriebenen pharmazeutischen Zusammensetzungen können auch als Vakzinen für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können zum Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vakzinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Cytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch ex vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägem, ergänzenden immunitätssteigernden Stoffen wie Adjuvanzien, CpG und Cytokinen und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen:

### Abb. 1. GPR35-mRNA-Expression in Kolontumor-Biopsien

RT-PCR-Untersuchungen mit DNA-freier RNA zeigen GPR35-Expression in der Mehrzahl der Kolontumor-Biopsien. Hingegen ist eine Expression in Normalgeweben nicht nachweisbar. (1: Brust, 2: Lunge, 3: Lymphknoten, 4: Thymus, 5: Kolon, 6-15: Kolontumore, 16: Negativ-Kontrolle).

### Abb. 2. Quantitative PCR-Analyse der GUCY2C-mRNA-Expression in Normal- und Tumor-Geweben

Real-Time-PCR-Untersuchungen mit GUCY2C-spezifischen Primern (SEQ ID NO: 22-23) zeigen eine selektive mRNA-Expression in Ileum- und Kolon-Normalgeweben sowie in allen Kolontumor-Biopsien. Deutliche GUCY2C-Transkriptmengen wurden auch in einer Kolontumor-Metastase in der Leber detektiert.

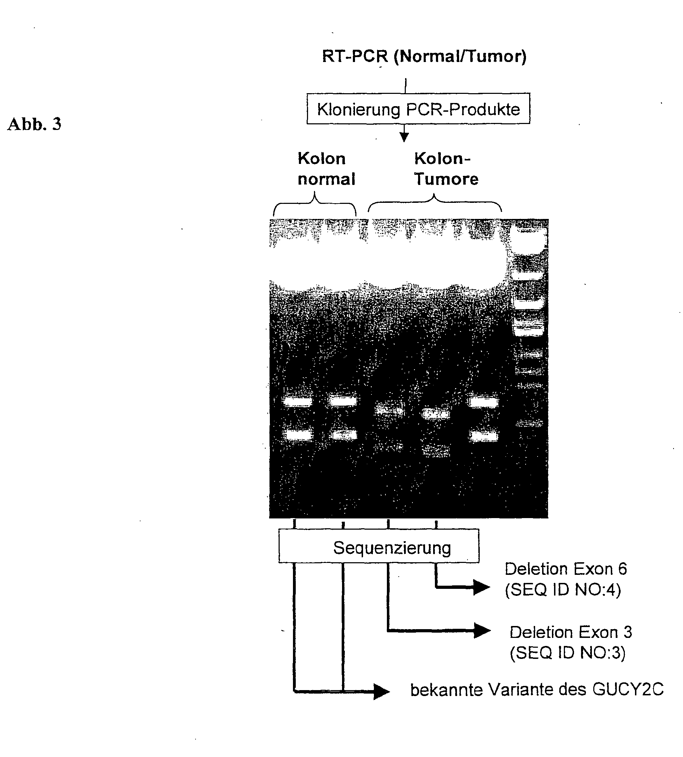

### Abb. 3. Identifikation von tumorspezifischen GUCY2C-Spleißvarianten

PCR-Produkte von normalen Kolongeweben und Kolontumoren wurden kloniert und Klone aus beiden Gruppen durch Restriktionsanalyse (EcoR I) überprüft und sequenziert.

### Abb. 4. Selektive SCGB3A-Expression in normaler Lunge und Lungentumoren

Die RT-PCR-Analyse mit Gen-spezifischen SCGB3A2-Primern (SEQ ID NO: 37, 38) zeigt eine cDNA-Amplifikation ausschließlich in normaler Lunge (Spuren 8, 14-15) und in Lungentumor-Biopsien (Spuren 16-24). (1-15: Normalgewebe, 1: Leber, 2: PBMC, 3: Lymphknoten, 4: Magen, 5: Testis, 6: Brust, 7: Niere, 8: Lunge, 9: Thymus, 10: Ovar, 11: Nebenniere, 12: Milz, 14-15: Lunge, 16-24: Lungentumore, 25: Negativ-Kontrolle).

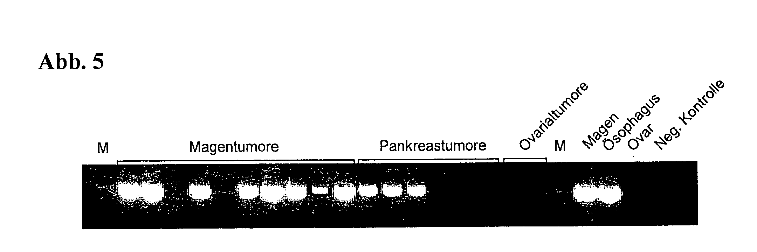

### Abb. 5. Claudin-18A2.1-Expression in Magen und Ösophagus sowie Magen- und Pankreastumoren

RT-PCR-Analyse mit Claudin-18A2.1-spezifischen Primern (SEQ ID NO: 39, 40) zeigte erfindungsgemäß in 8/10 Magentumor-Biopsien sowie in 3/6 Pankreastumor-Biopsien eine ausgeprägte Claudin-18A2.1-Expression. In Magen- und Ösophagus-Normalgeweben wurde ebenfalls eine deutliche Expression nachgewiesen. Im Gegensatz dazu wurde im Ovar und in Ovarialtumoren keine Expression detektiert.

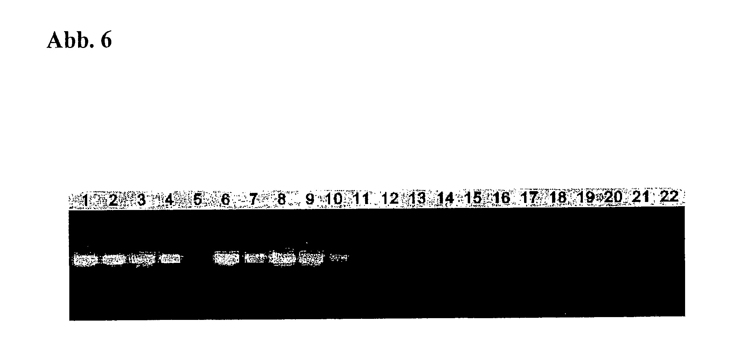

### Abb. 6. SLC13A1-Expression in der Niere und Nierentumoren

RT-PCR-Analyse mit SLC13A1-spezifischen Primern (SEQ ID NO: 49, 50) zeigte in 7/8 Nierentumor-Proben eine Expression. Ansonsten wurden Transkripte innerhalb der Normalgewebe ausschließlich in der Niere detektiert. (1-2: Nieren-Normalgewebe, 3-10: Nierentumore, 11: Brust-, 12: Lungen-, 13: Leber-, 14: Kolon-, 15: Lymphknoten-, 16: Milz-, 17: Ösophagus-, 18: Thymus-, 19: Schilddrüsen-, 20: PBMC-, 21: Ovar-, 22: Testis-Normalgewebe).

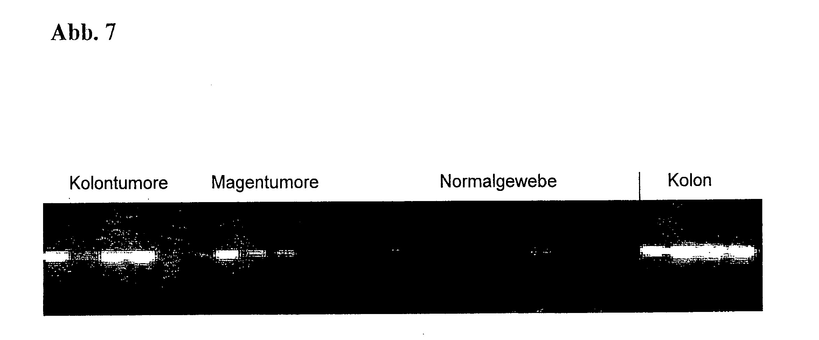

### Abb. 7. CLCA1-Expression in Kolon-Normalgewebe sowie in Kolon- und Magentumoren

RT-PCR-Untersuchungen mit CLCA1-spezifischen Primern (SEQ ID NO: 67, 68) bestätigten eine selektive Expression im Kolon und zeigten eine hohe Expression in 3/7 untersuchten Kolon- und 1/3 untersuchten Magentumor-Proben. Die übrigen Normalgewebe zeigten keine oder nur eine sehr schwache Expression.

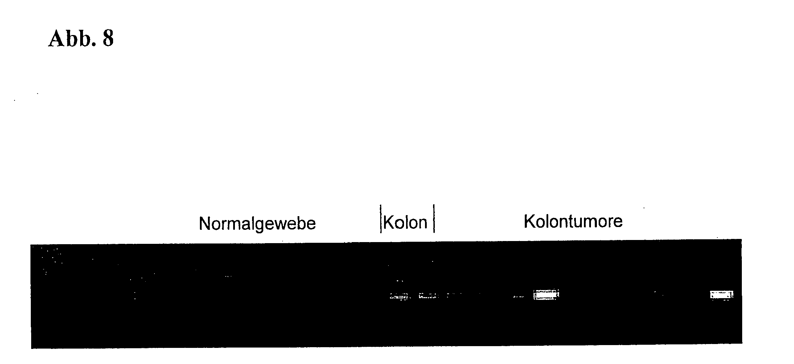

### Abb. 8. FLJ21477-Expression in Kolon-Normalgewebe und Kolontumoren

RT-PCR-Untersuchungen mit FLJ21477-spezifischen Primern (SEQ ID NO: 69, 70) zeigten eine selektive Expression im Kolon und darüber hinaus unterschiedlich stark ausgeprägte Expression in 7/12 untersuchten Kolontumor-Proben. Die übrigen Normalgewebe zeigten keine Expression.

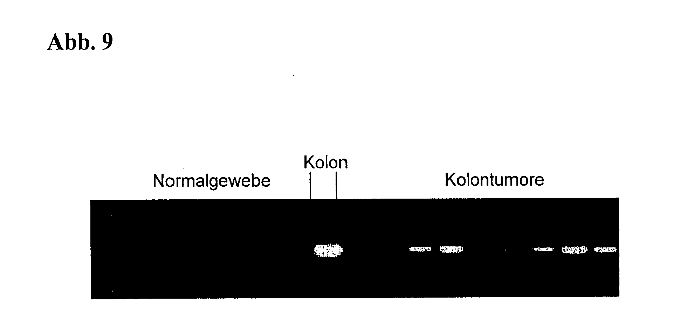

### Abb. 9. FLJ20694-Expression in Kolon-Normalgewebe und Kolontumoren

RT-PCR-Untersuchungen mit FLJ20694-spezifischen Primern (SEQ ID NO: 71, 72) zeigten eine selektive Expression im Kolon und darüber hinaus unterschiedlich stark ausgeprägte Expression in 5/9 untersuchten Kolontumor-Proben. Die übrigen Normalgewebe zeigten keine Expression.

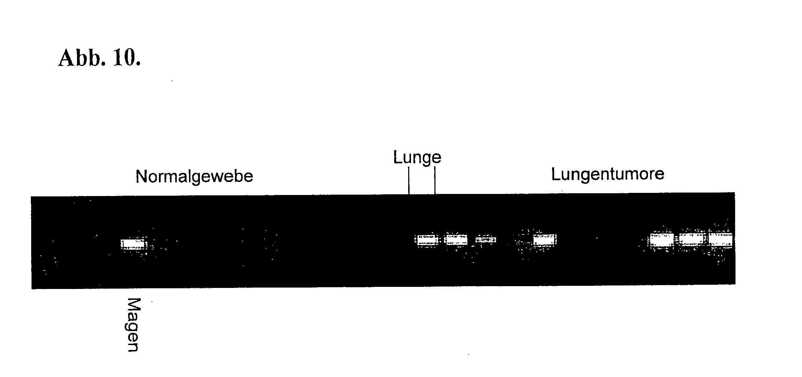

### Abb. 10. von Ebner-Expression in Magen- und Lunge-Normalgeweben sowie Lungentumoren

RT-PCR-Untersuchungen mit von Ebner-spezifischen Primern (SEQ ID NO: 73, 74) zeigten eine selektive Expression im Magen, in der Lunge und in 5/10 untersuchten Lungentumor-Proben. Die übrigen Normalgewebe zeigten keine Expression.

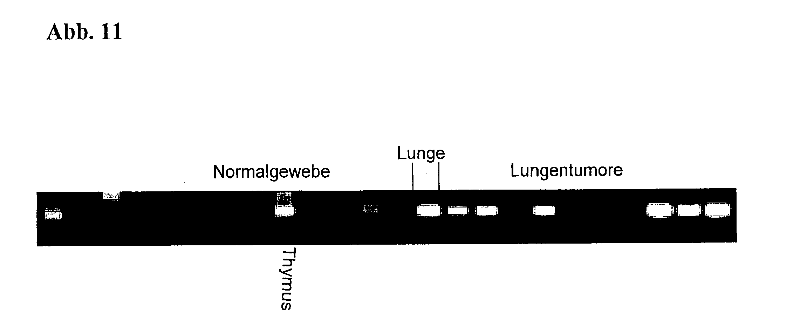

### Abb. 11. Plunc-Expression in Thymus- und Lunge-Normalgewebe sowie Lungentumoren

RT-PCR-Untersuchungen mit Plunc-spezifischen Primern (SEQ ID NO: 75, 76) zeigten eine selektive Expression im Thymus, in der Lunge und in 6/10 untersuchten Lungentumor-Proben. Die übrigen Normalgewebe zeigten keine Expression.

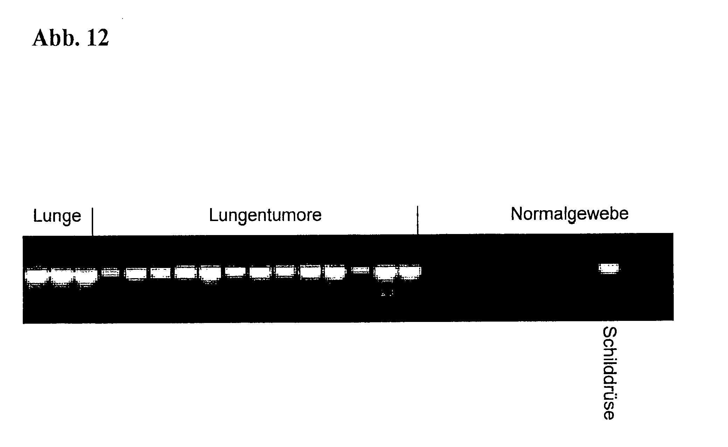

### Abb. 12. SLC26A9-Expression in Lunge, Lungentumoren und Schilddrüse

RT-PCR-Untersuchungen mit SLC26A9-spezifischen Primern (SEQ ID NO: 77, 78) zeigten eine selektive Expression in der Lunge und in allen (13/13) untersuchten Lungentumor-Proben. Die übrigen Normalgewebe zeigten mit Ausnahme der Schilddrüse keine Expression.

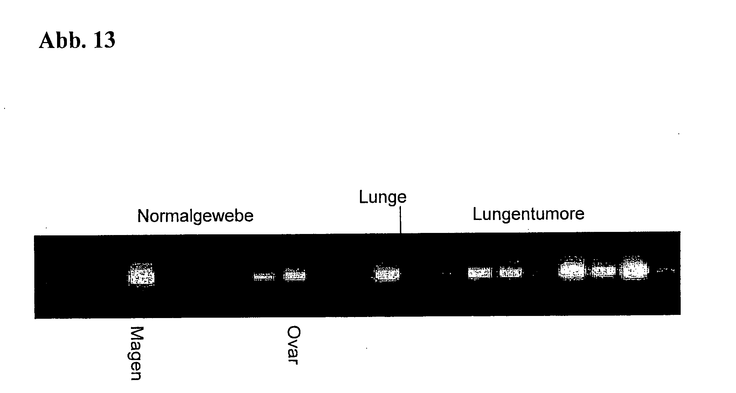

### Abb. 13. THC1005163-Expression in Magen, Ovar, Lunge und Lungentumoren

RT-PCR-Untersuchungen mit einem THC1005163-spezifischen Primer (SEQ ID NO: 79) und einem unspezifischen Oligo dT-Tag-Primer zeigten eine Expression in Magen, Ovar, Lunge und in 5/9 Lungentumor-Biopsien. Die übrigen Normalgewebe zeigten keine Expression.

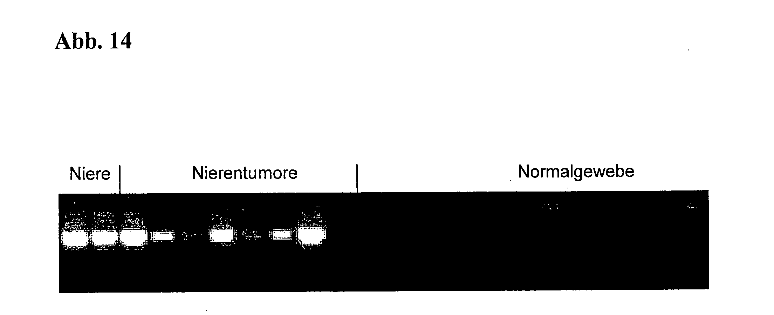

### Abb.14. LOC134288-Expression in Niere und Nierentumoren

RT-PCR-Untersuchungen mit LOC134288-spezifischen Primern (SEQ ID NO: 80, 81) zeigten eine selektive Expression in der Niere und in 5/8 untersuchten Nierentumor-Biopsien.

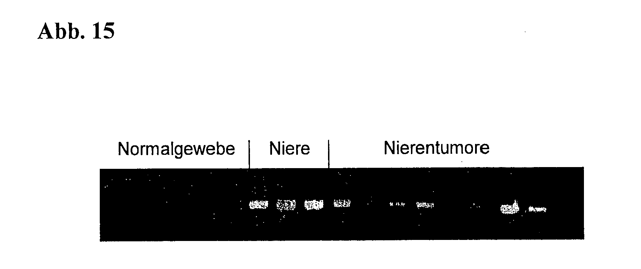

### Abb. 15. THC943866-Expression in Niere und Nierentumoren

RT-PCR-Untersuchungen mit THC943866-spezifischen Primern (SEQ ID NO: 82, 83) zeigten eine selektive Expression in der Niere und in 4/8 untersuchten Nierentumor-Biopsien.

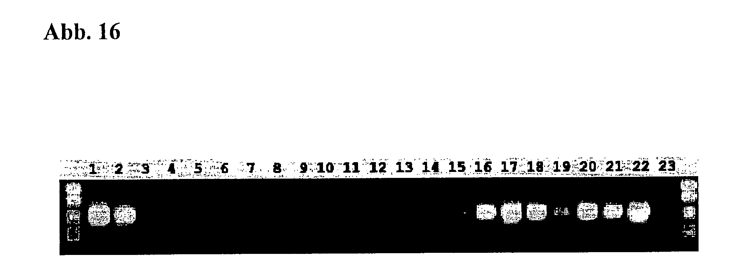

### Abb. 16. FLJ21458-Expression in Kolon und Kolontumoren

RT-PCR-Untersuchungen mit FLJ21458-spezifischen Primern (SEQ ID NO: 86, 87) zeigten eine selektive Expression im Kolon und in 7/10 untersuchten Kolontumor-Biopsien. (1-2: Kolon-, 3: Leber-, 4: PBMC-, 5: Milz-, 6: Prostata-, 7: Niere- , 8: Ovar-, 9: Haut-, 10: Ileum-, 11: Lunge-, 12: Testis-Normalgewebe, 13-22: Kolontumore, 23: Negativ- Kontrolle).

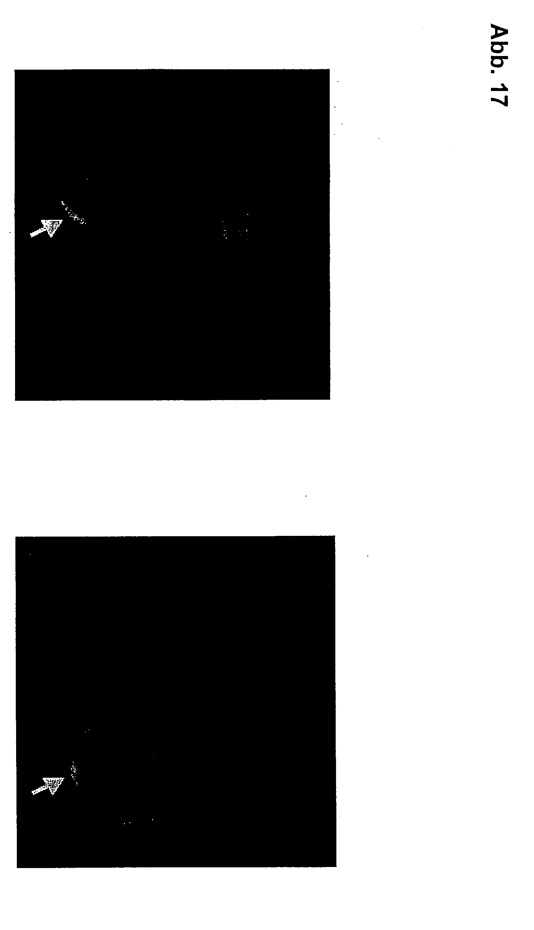

### Abb. 17. Zelluläre Lokalisation von GPR35

Immunfluoreszenz zum Nachweis der zellulären Lokalisation von GPR35 nach Transfektion eines Plasmides, dass ein GPR35-GFP Fusionsprotein exprimiert. Die Pfeile kennzeichnen die membranständige Fluoreszenz des fluoreszierenden GFP.

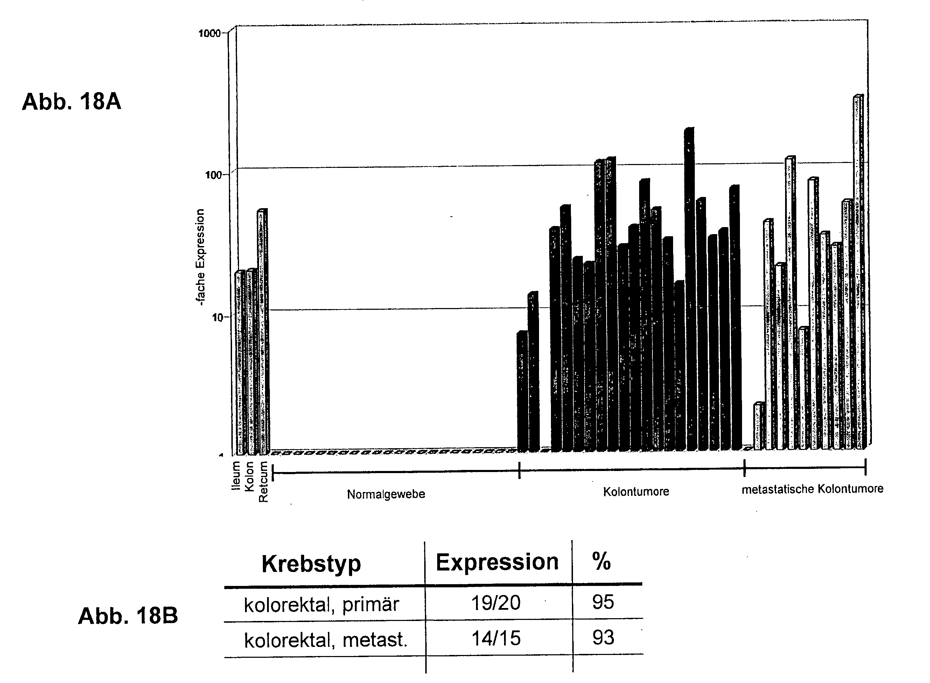

### Abb. 18. Quantitative Expression von GPR35

A. Quantitative RT-PCR-Untersuchungen mit GPR35-spezifischen Primern (SEQ ID NO: 88, 89) zeigen die selektive Expression in verschiedenen Darmbereichen, in Kolontumorproben und in Metastasen aus Kolontumoren. Folgende Normalgewebe wurden analysiert: Leber, Lunge, Lymphknoten, Magen, Milz, Nebenniere, Niere, Ösophagus, Ovar, Testis, Thymus, Haut, Brust, Pankreas, Lymphozyten, aktivierte Lymphozyten, Prostata, Schilddrüse, Ovar, Endometrium, Kleinhirn, Gehirn.
B. Prävalenz von GPR35 in Kolontumoren und deren Metastasen. In über 90% der Fälle ist GPR35 sowohl in Tumoren als auch in Metastasen exprimiert.

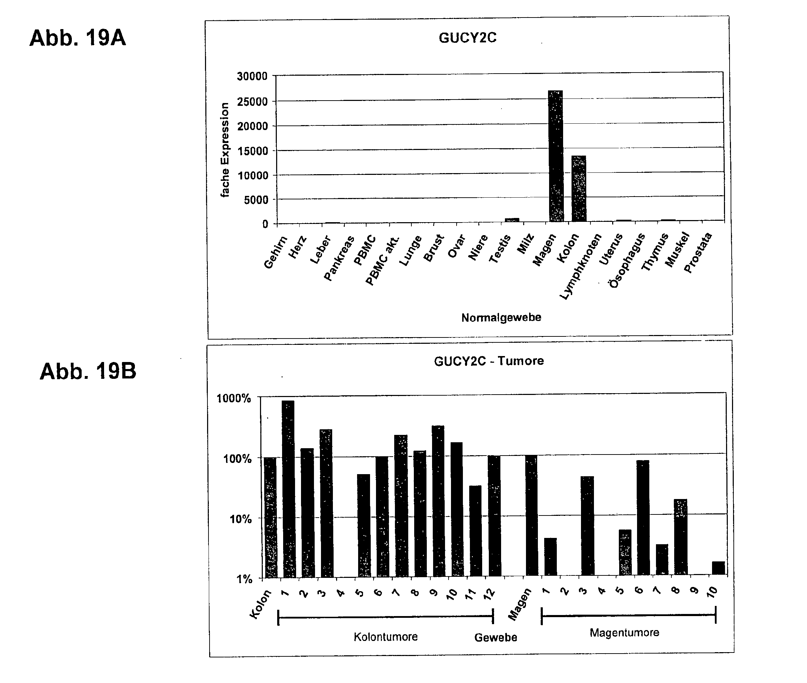

### Abb. 19. Quantitative Expression von GUCY2C

Quantitative RT-PCR Untersuchungen mit GUCY2C-spezifischen Primern (SEQ ID NO: 98, 99) zeigen die hohe und selektive Expression im normalen Kolon- und Magengewebe (A) sowie die GUCY2C-spezifische Expression in Kolon- und Magentumor-Proben (B). GUCY2C ist nachweisbar in 11/12 Kolontumoren und in 7/10 Magentumoren.

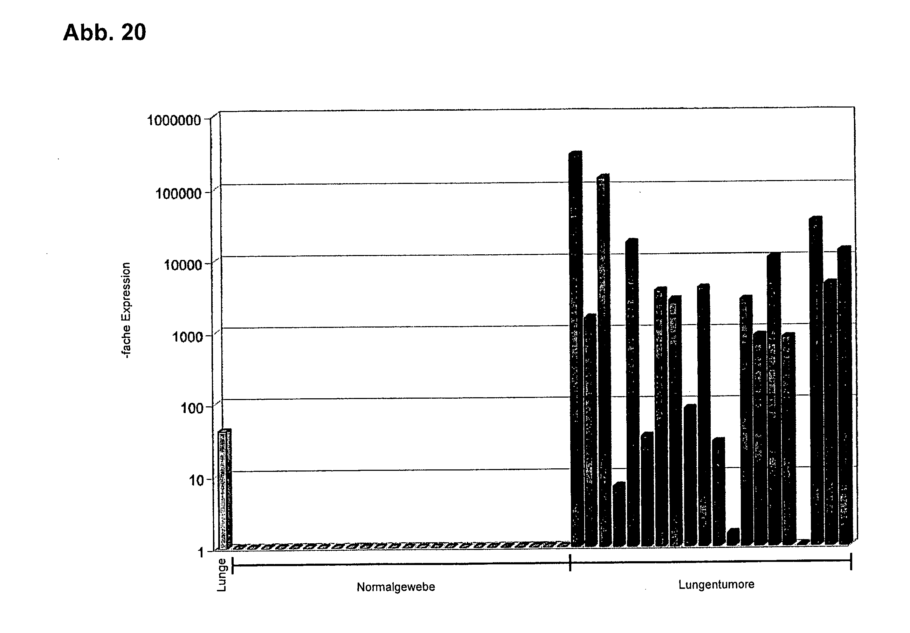

### Abb. 20. Quantitative Expression von SCGB3A2

Quantitative RT-PCR-Untersuchungen mit SCGB3A2-spezifischen Primern (SEQ ID NO: 103, 104) zeigen die selektive Expression in Lungen- und Lungentumor-Proben. 19/20 Lungentumor-Proben sind SCGB3A2-positiv, in mehr als 50 % der Proben ist SCGB3A2 um mindestens den Faktor 10 überexprimiert. Folgende Normalgewebe wurden analysiert: Leber, Lunge, Lymphknoten, Magen, Milz, Nebenniere, Niere, Ösophagus, Ovar, Testis, Thymus, Haut, Brust, Pankreas, Lymphozyten, aktivierte Lymphozyten, Prostata, Schilddrüse, Ovar, Endometrium, Kleinhirn, Gehirn.

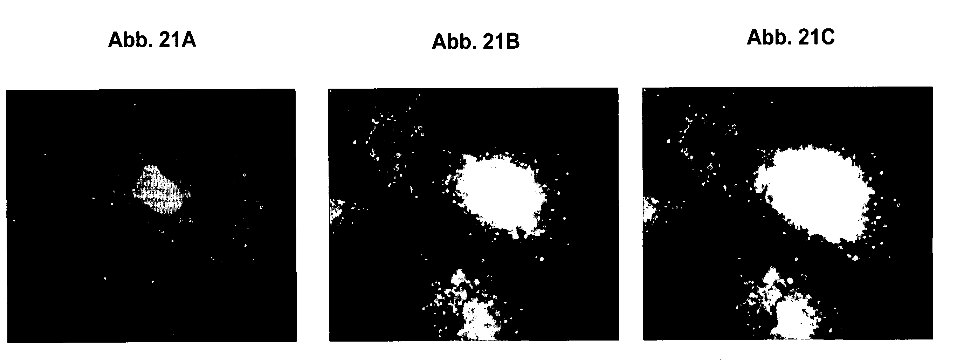

### Abb. 21. Immunfluoreszenz mit SCGB3A2-spezifischen Antikörpern

COS7-Zellen wurden mit einem Plasmid transfiziert, das für ein SCGB3A2-GFP-Fusionsprotein kodiert.
**A.** Nachweis des transfizierten Fusionsproteins mit einem SCGB3A2-spezifischen Kaninchen-Antiserum (Immunisierung mit SEQ ID NO: 105).
**B.** Nachweis des transfizierten Fusionsproteins durch GFP-Fluoreszenz.
**C.** Überlagerung der beiden Fluoreszenzen aus A und B. Die gelbe Färbung entsteht an den Stellen, an denen sich beide Fluoreszenzen überlagern und weist damit die Spezifität des SCGB3A2-Antiserums nach.

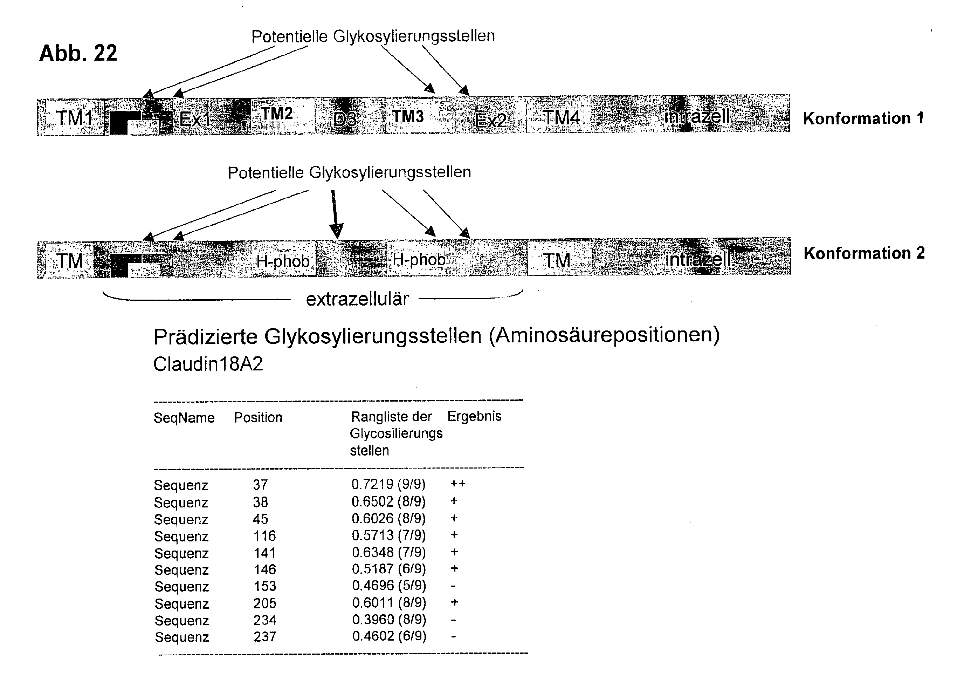

### Abb. 22. Schematische Darstellung von Claudin-18-Konformationen

erfindungsgemäß kann das Claudin-18A2-Polypeptid in zwei Konformationen auf der Zelle vorliegen. In der Konformation 1 liegt das Protein als Membranmolekül mit 4 Transmembrandomänen (TM) vor und weist zwei separate, extrazellulär gelegene Domänen auf. In der Konformation 2 üben die beiden mittleren hyrophoben Bereiche (h-phob) keine Transmembrandomänen-Funktion aus. Dadurch sind in dieser Konformation im Vergleich zu der Konformation 1 zusätzliche Peptidregionen extrazellulär gelegen. Außerdem resultiert aus dieser Konformation eine zusätzliche N-Glykosylierungsstelle an Position 116 (dickerer Pfeil). Alle prädizierten Glykosylierungsdomänen sind im unteren Teil der Abbildung aufgeführt. Ex1: extrazelluläre Domäne 1, Ex2: extrazelluläre Domäne 2, TM: Transmembrandomäne, H-phob: extrazelluläre hydrophobe Region.

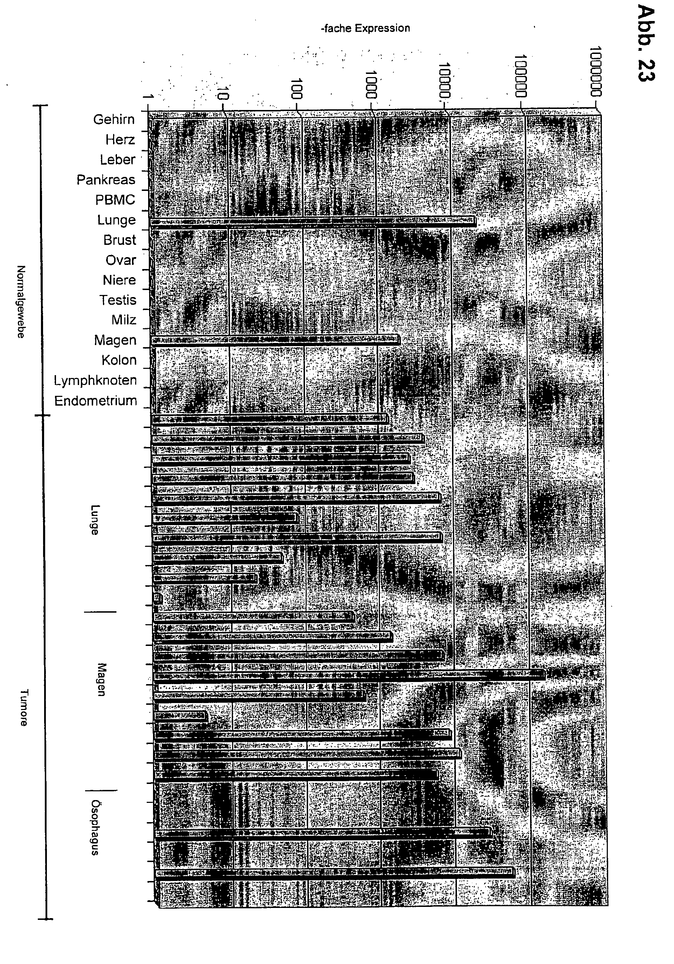

### Abb. 23. Quantitative Expression von Claudin-18, Variante A1

Claudin-18A1 ist in keinem Normalgewebe außer Lungen- und Magengewebe detektierbar. Claudin-18A1 ist in einer Vielzahl von Tumorgeweben stark exprimiert. Eine besonders starke Expression findet sich in Magentumoren, Lungentumoren, Pankreastumoren und Ösophagustumoren.

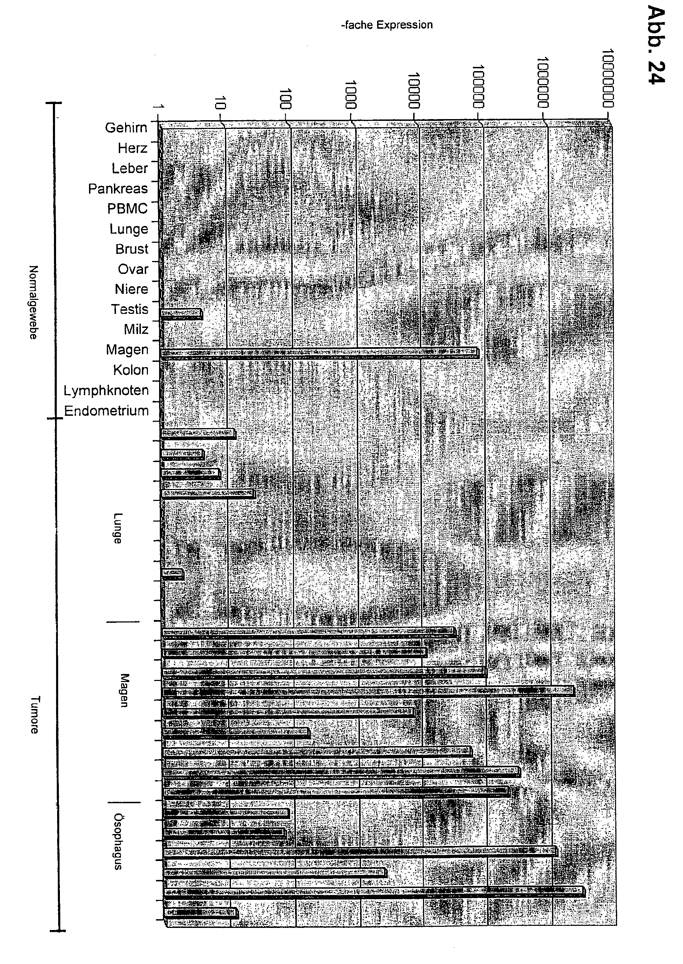

### Abb. 24. Quantitative Expression von Claudin-18, Variante A2

Claudin-18A2 ist in keinem Normalgewebe ausser Magengewebe detektierbar. Claudin-18A2 ist in einer Vielzahl von Tumorgeweben insbesondere Magentumoren, Lungentumoren, Pankreastumore und Ösophagustumoren exprimiert.

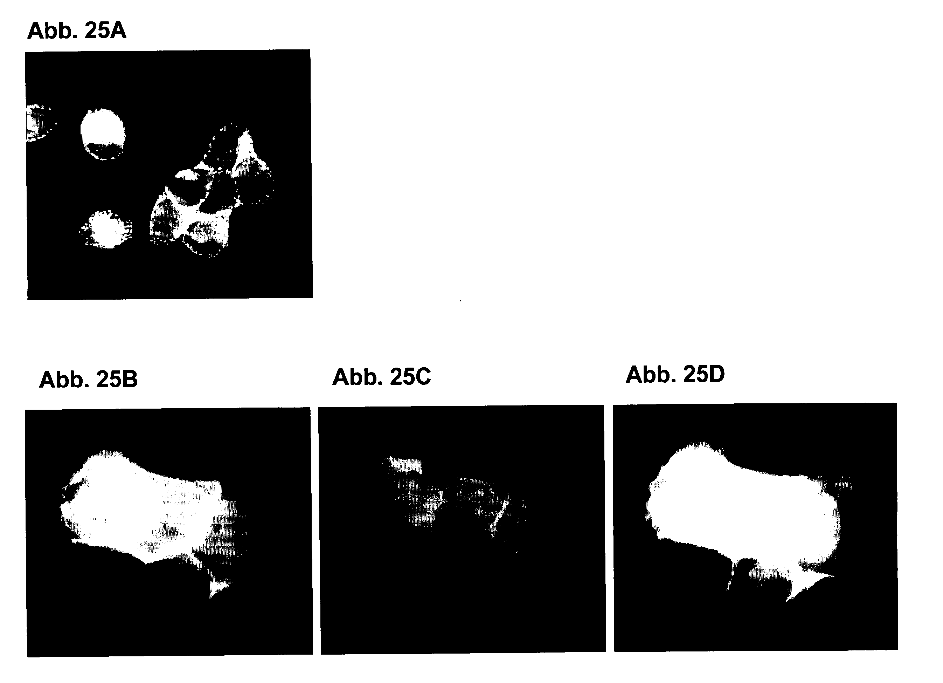

### Abb. 25. Verwendung Claudin-18A2-spezifischer Antikörper (extrazelluläre Domäne)

**A:** Färbung von Claudin-18A2-positiven Magentumorzellen (SNU-16, Methanol-fixiert) mit einem Antikörper, der durch Immunisierung mit einem Peptid (SEQ ID NO: 17) hergestellt wurde. Eine Membranfärbung tritt besonders stark in den Zell/Zell-Interaktionsbereichen auf. Das Protein aggregiert in fokalen Membranbereichen.
**B, C, D:** Nachweis der Spezifität des Antikörpes durch Kolokalisationsanalyse in Claudin-18A2-GFP-transfizierten 293T-Zellen. B: GFP-Fluoreszenz; C: anti-Claudin-18A2; D: Überlagerung.

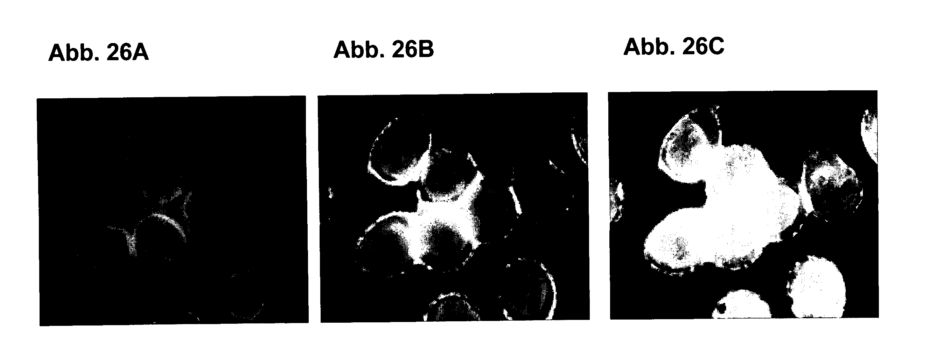

### Abb. 26. Verwendung Claudin-18A2-spezifischer Antikörper (extrazelluläre Domäne)

Membranfärbung von Claudin-18A2-positiven Magentumorzellen (SNU-16) mit einem Antikörper, der durch Immunisierung mit einem Peptid (SEQ ID NO: 113, N-terminal gelegene extrazelluläre Domäne) hergestellt wurde. Zur Gegenfärbung wurde ein monoklonaler Antikörper verwendet, der gegen E-Cadherin gerichtet ist. A: Claudin-18A2-Antikörper; B: anti E-Cadherin-Gegenfärbung; C: Überlagerung.

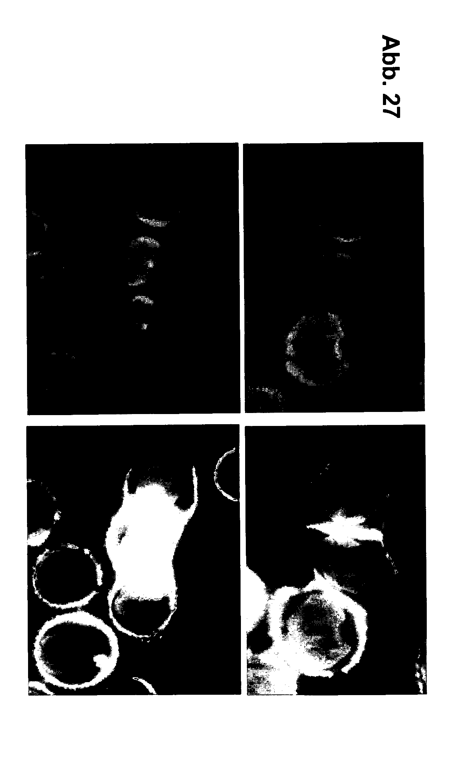

### Abb. 27. Verwendung von Antikörpern gegen die C-terminale extrazelluläre Domäne von Claudin-18

Linke Abbildungen: Membranfärbung von Claudin-18A2-positiven Magentumorzellen (SNU-16) mit einem Antikörper, der durch Immunisierung mit einem Peptid (SEQ ID NO: 116, C-terminal gelegene extrazelluläre Domäne) hergestellt wurde. Zur Gegenfärbung wurde ein monoklonaler Antikörper verwendet, der gegen E-Cadherin gerichtet ist (rechte Abbildungen).

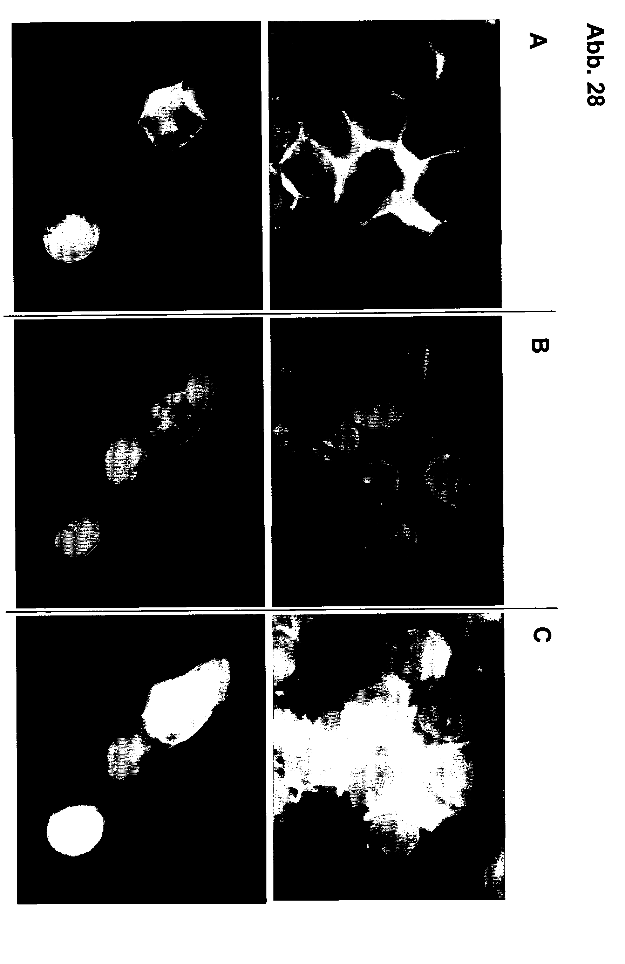

### Abb. 28. Verwendung Claudin-18A1-spezifischer Antikörper

Oben: schwache bis fehlende Färbung von Magentumorzellen (SNU-16; Claudin18A2-positiv) mit einem Antikörper, der durch Immunisierung mit einem Claudin-18A1-spezifischen Peptid (SEQ ID NO: 115) hergestellt wurde. A: anti-E-Cadherin; B: anti-Claudin-18A1; C: Überlagerung.

Unten: Nachweis der Spezifität des Antikörpes durch Kolokalisationsanalyse in Claudin-18A1-GFP-transfizierten 293T-Zellen. A: GFP-Fluoreszenz; B: anti-Claudin-18A1; C: Überlagerung.

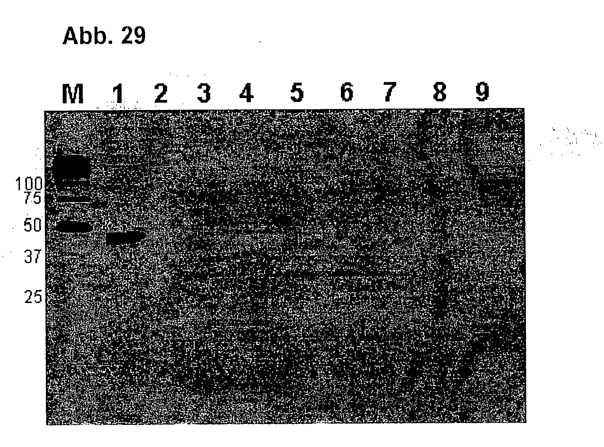

### Abb. 29. Nachweis von Claudin-18A2 im Western-Blot.

Western-Blot mit Lysaten aus verschiedenen gesunden Geweben mit einem Claudin-18A2-spezifischen Antikörper, gerichtet gegen das Epitop mit SEQ ID NO: 17. 1: Magen-; 2: Testis-; 3: Haut-; 4: Brust-; 5: Leber-; 6: Kolon-; 7: Lunge-; 8: Niere-; 9: Lymphknoten-Normalgewebe.

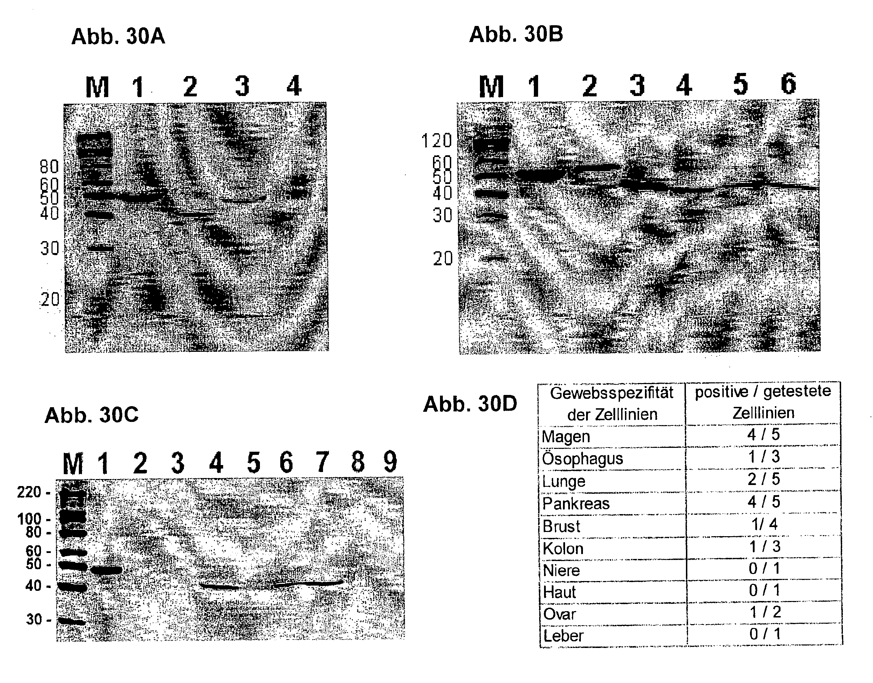

### Abb. 30. Western-Blot von Claudin-18A2 mit Proben aus Magen und Magentumoren sowie verschiedenen Tumorzelllinien.

Lysate aus Magen und Magentumoren (A, B) und Tumorzelllinien (C, D) wurden geblottet und mit einem Claudin-18A2-spezifischen Antikörper gegen das Epitop mit SEQ ID NO: 17 getestet. Magentumore weisen eine geringer glykosylierte Form von Claudin-18A2 auf. Eine PNGase F-Behandlung von Magenlysaten führt zur Bildung der niedrigglykosylierten Form.
**A:** 1: Magen-Normalgewebe #A; 2: Magentumor #A; 3: Magen-Normalgewebe #B; 4: Magentumor #B
**B:** 1: Magen-Normalgewebe #A; 2: Magen-Normalgewebe #B; 3: Magen-Normalgewebe #B + PNGase F; 4: Magentumor #C; 5: Magentumor #D; 6: Magentumor #D + PNGase F
**C:** 1: Magen-Normalgewebe; 2: MDA-MB-231; 3: SK-MEL-37; 4: AGS; 5: SNU-1; 6: SNU-16; 7: EFO27; 8: TOV-112D; 9: OVCAR. Zu beachten ist, dass die Tumorzelllinien die deglykosylierte Variante von Claudin-18A2 exprimieren.
**D:** Tabellarische Zusammenfassung der Western-Blot-Daten für eine Auswahl von Zelllinien, die mit dem Claudin-18A2-spezifischen Antikörper getestet wurden.

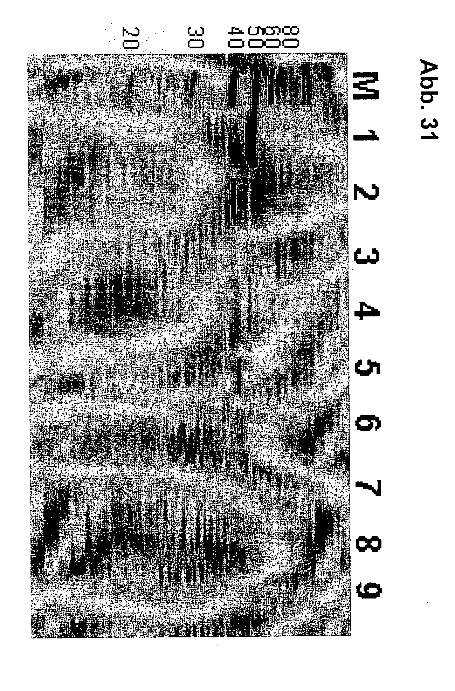

### Abb. 31. Expression von Claudin-18 in Lungentumoren

Entsprechend zu Abb. 30 erfolgte ein Nachweis von niedrigglykosylierten Claudin-18A2-Varianten in Lungentumoren. 1: Magen-Normalgewebe; 2: Magentumor; 3-9: Lungentumore.

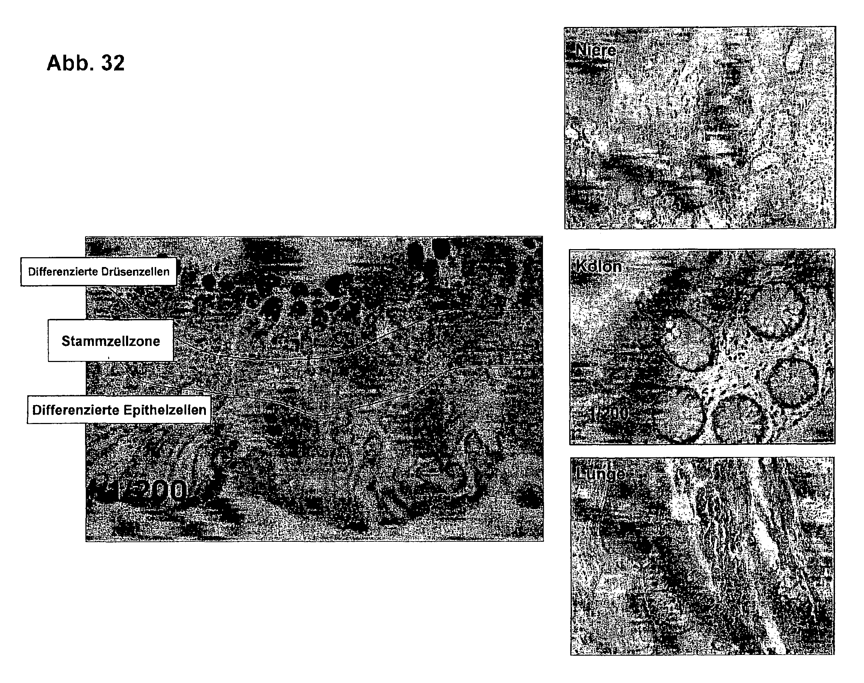

### Abb. 32. Immunhistochemische Analyse von Claudin-18 mit Claudin-18A2-spezifischen Antikörpern in Normalgeweben

In der Magenschleimhaut werden lediglich differenzierte epitheliale Zellen an der Öffnung wie auch am Boden der Drüsen gefärbt. Claudin-18A2 ist in Stammzellen des Magens nicht nachweisbar. Alle anderen von uns untersuchten Normalgewebe exprimieren dieses Gen ebenfalls nicht, wie beispielhaft für Niere, Lunge und Kolon dargestellt.

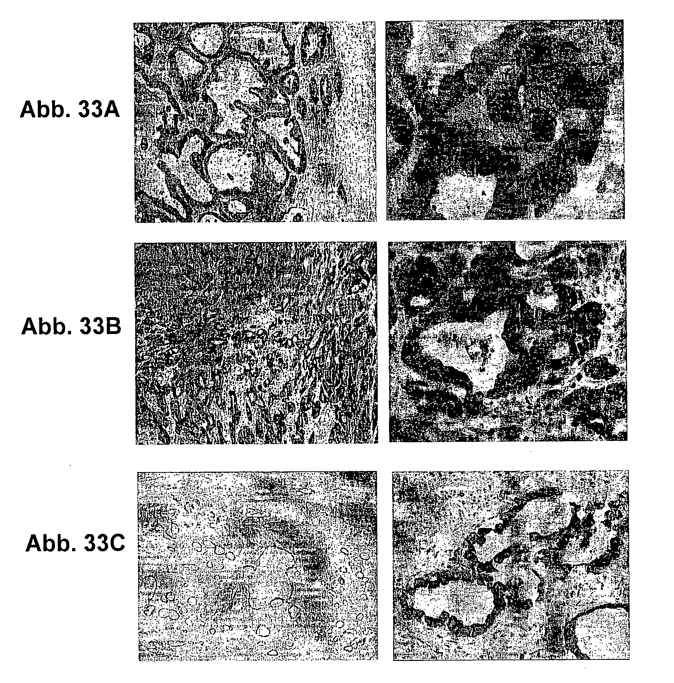

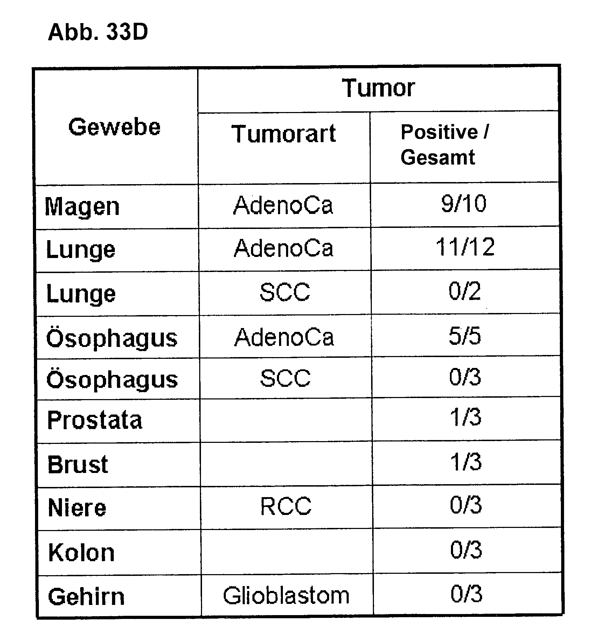

### Abb. 33. Resultate der Immunhistologie mit Claudin-18A2-spezifischem polyklonalen Antiserum

**A:** Beispiele für spezifische Färbungen von Lungentumor-Geweben. Zu beachten ist, dass das normale Lungengewebe, welches die Variante Claudin-18A1 exprimiert, durch das Claudin-18A2-spezifische Antiserum nicht erkannt wird.
**B**: Beispiele für spezifische Tumorfärbung von Ösophagustumoren. Zu beachten ist, dass gesunde Zellen in der Umgebung nicht gefärbt werden.
**C:** Beispiele für spezifische Tumorfärbung von Magentumorepithelien. Auch hier werden gesunde Zellen in der Umgebung nicht gefärbt.
**D:** Beispeilhafte tabellarische Zusammenfassung immunhistochemischer Färbedaten mit Claudin-18A2-spezifischen Antikörpern. AdenoCa: Adenokarzinom; SCC: Plattenepithelkarzinom; RCC: Nierenzellkarzinom.

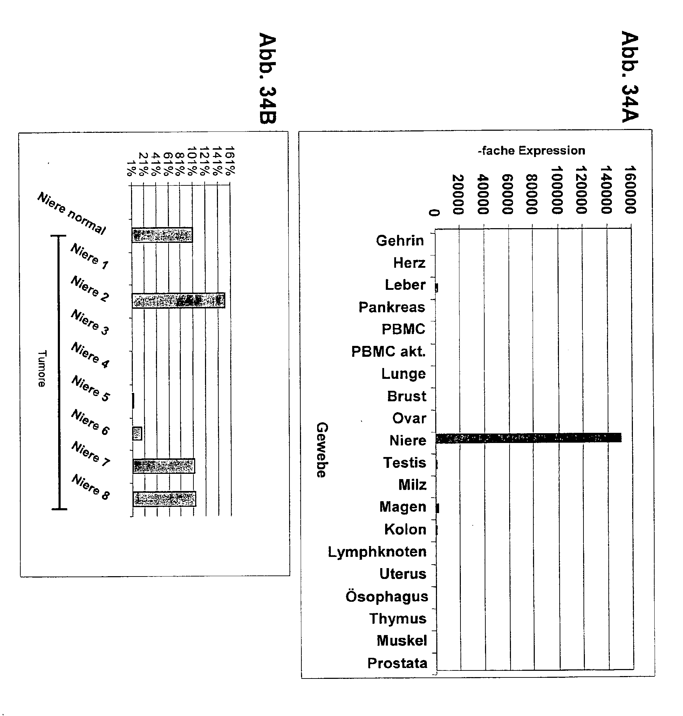

### Abb. 34. Quantitative Expression von SLC13A1

Quantitative RT-PCR-Untersuchungen mit SLC13A1-spezifischen Primern (SEQ ID NO: 121, 122) zeigen die hohe und selektive Expression in normalem Nierengewebe (A) sowie die SLC13A1-spezifische Expression in Nierentumoren (B). SLC13A1-Transkription ist in 5/8 Nierentumoren nachweisbar.

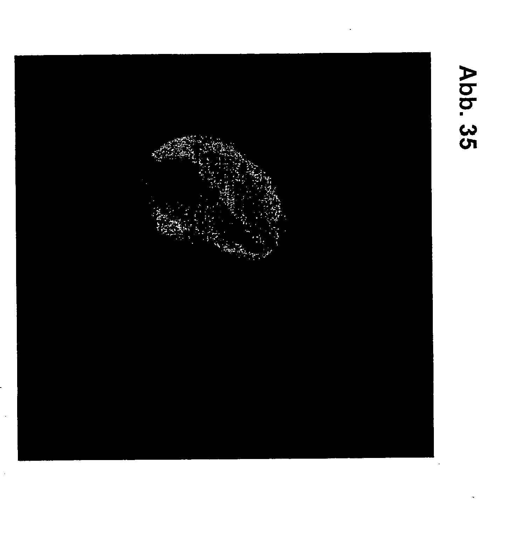

### Abb. 35. Zelluläre Lokalisation von SLC13A1

Immunfluoreszenz zum Nachweis der zellulären Lokalisation von SLC13A1 nach Transfektion eines Plasmides, das ein SLC13A1-GFP-Fusionsprotein bereitstellt. Deutlich zu sehen ist die membranständige Fluoreszenz (als Ring um die transfizierte Zelle) des SLC 13A1-Fusionsproteins.

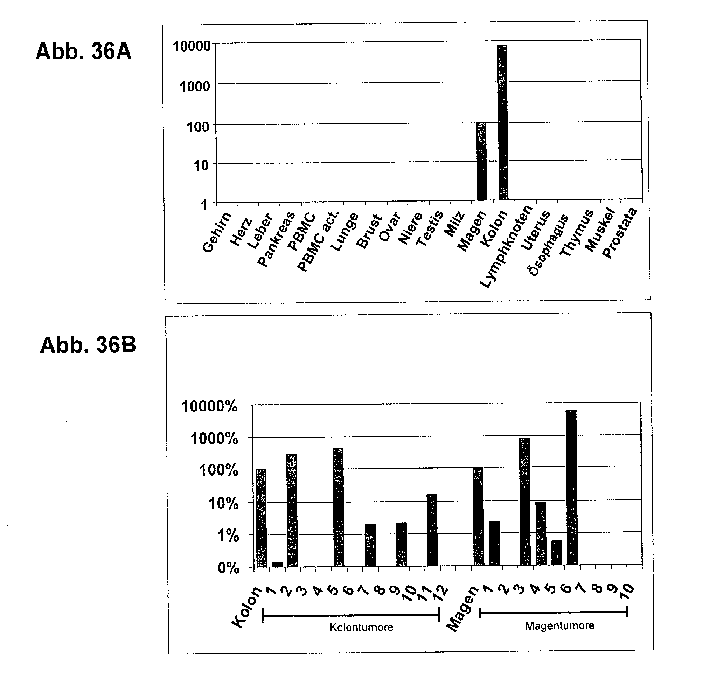

### Abb. 36. Quantitative Expression von CLCA1

Quantitative RT-PCR-Untersuchungen mit CLCA1-spezifischen Primern (SEQ ID NO: 125, 126) zeigen die hohe und selektive Expression im normalen Kolon- und Magengewebe (A) sowie die CLCA1-spezifische Expression in Kolon- und Magentumorproben (B). CLCA1 ist nachweisbar in 6/12 Kolontumoren und in 7/10 Magentumoren.

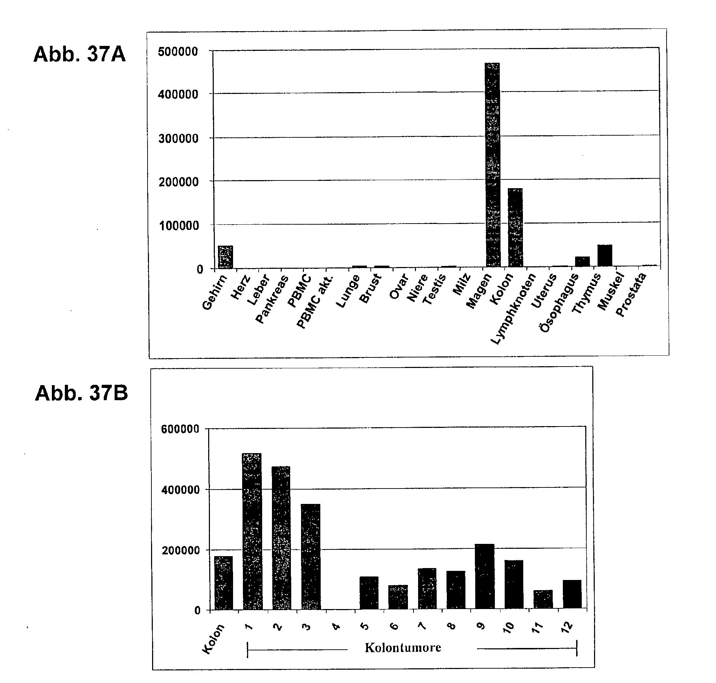

### Abb. 37. Quantitative Expression von FLJ21477

Quantitative RT-PCR-Untersuchungen mit FLJ21477-spezifischen Primern (SEQ ID NO: 127, 128) zeigen die hohe und selektive Expression im normalen Kolon- und Magengewebe sowie eine schwache Expression in Thymus, Ösophagus und Gehirn (A) sowie die FLJ21477-spezifische Expression in Kolontumor-Proben (B). FLJ21477 ist nachweisbar in 11/12 Kolontumoren.

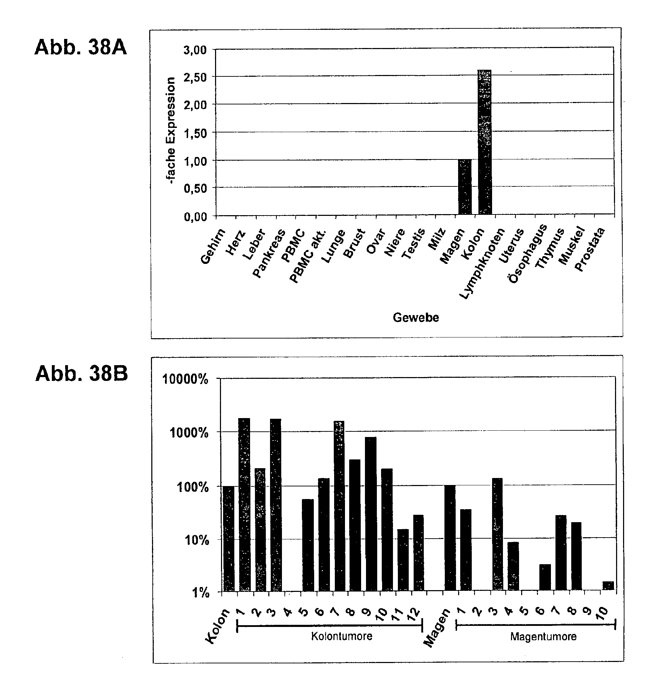

### Abb. 38. Quantitative Expression von FLJ20694

quantitative RT-PCR-Untersuchungen mit FLJ20694-spezifischen Primern (SEQ ID NO: 129, 130) zeigen die hohe und selektive Expression im normalen Kolon- und Magengewebe (A) sowie die FLJ20694-spezifische Überexpression in Kolon- und Magentumor-Proben (B). FLJ20694 ist nachweisbar in 11/12 Kolontumoren und in 7/10 Magentumoren.

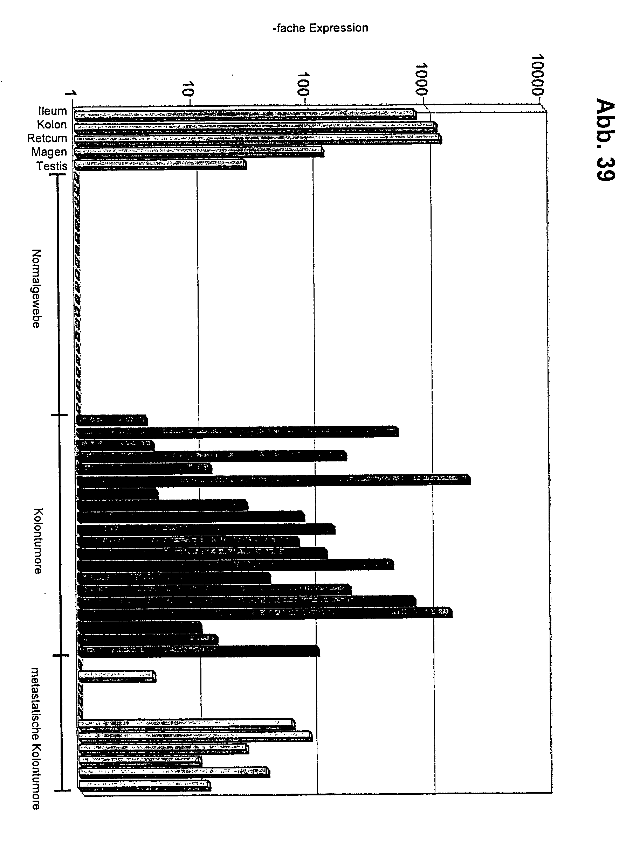

### Abb. 39. Quantitative Expression von FLJ21458

Quantitative RT-PCR-Untersuchungen mit FLJ21458-spezifischen Primern (SEQ ID NO: 133, 134) zeigen die selektive Expression in Testis, Magengewebe und verschiedenen Darmarealen. Außerdem konnten FLJ21458-spezifische Transkripte in 20/20 Kolontumoren und in 7/11 Kolonmetastasen nachgewiesen werden. Folgende Normalgewebe wurden analysiert: Leber, Lunge, Lymphknoten, Milz, Nebenniere, Niere, Ösophagus, Ovar, Testis, Thymus, Haut, Brust, Pankreas, Lymphozyten, aktivierte Lymphozyten, Prostata, Schilddrüse, Ovar, Endometrium, Kleinhirn, Gehirn.

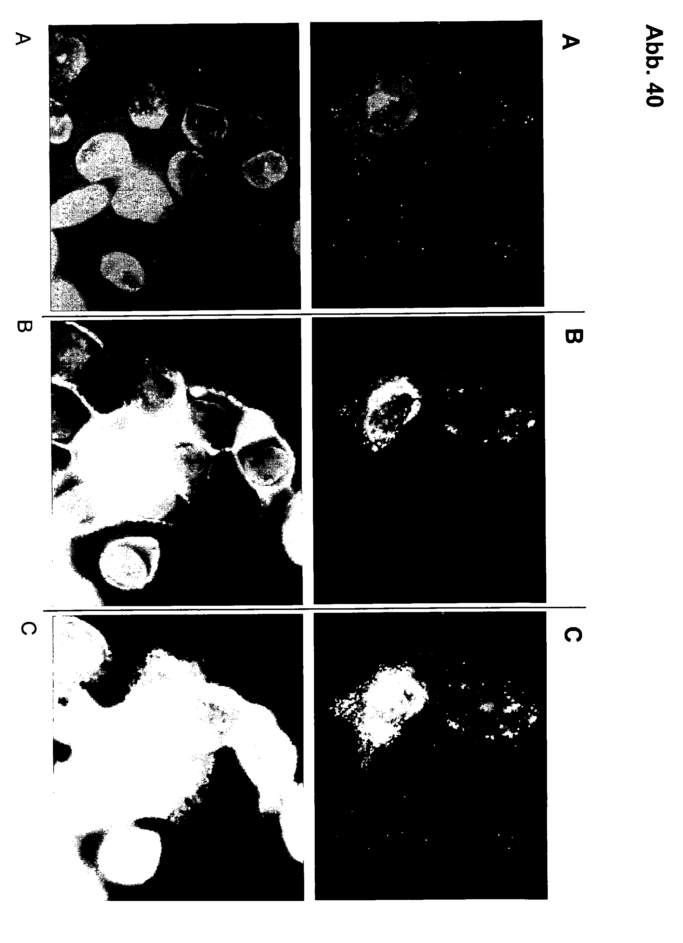

### Abb. 40. Immunfluoreszenz mit FLJ21458-spezifischen Antikörpern

Oben: 293-Zellen wurden mit einem Plasmid transfiziert, das für ein FLJ21458-GFP-Fusionsprotein kodiert. A: Nachweis des transfizierten Fusionsproteins mit einem FLJ21458-spezifischen Kaninchen-Antiserum (Immunisierung mit SEQ ID NO: 136). B: Nachweis des transfizierten Fusionsproteins durch GFP-Fluoreszenz. C: Überlagerung der beiden Fluoreszenzen aus A und B. Die gelbe Färbung entsteht an den Stellen, an denen sich beide Fluoreszenzen überlagern und weist damit die Spezifität des FLJ21458-Antiserums nach. Unten: Analyse von Snu16-Zellen, die endogen FLJ21458 synthetisieren. A: Proteinnachweis mit einem FLJ21458-spezifischen Kaninchen-Antiserum (Immunisierung mit SEQ ID NO: 136). B: Nachweis des Membranproteins E-Cadherin. C: Überlagerung der beiden Flureszenzen aus A und B. Die gelbe Färbung entsteht an den Stellen, an denen sich beide Fluoreszenzen überlagern, und weist die Membranlokalisation von FLJ21458 nach.

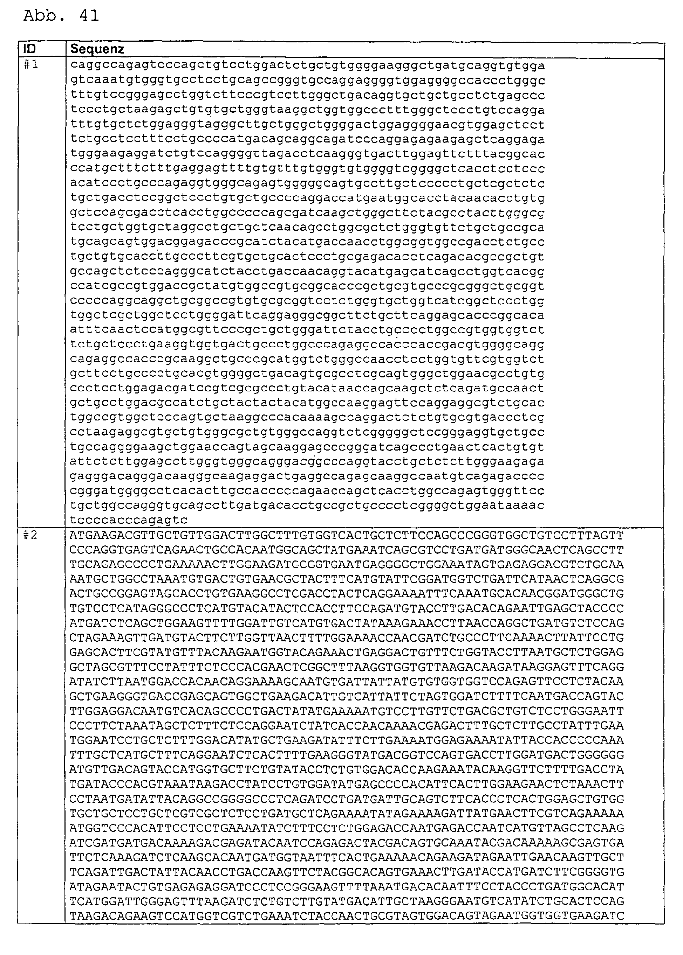

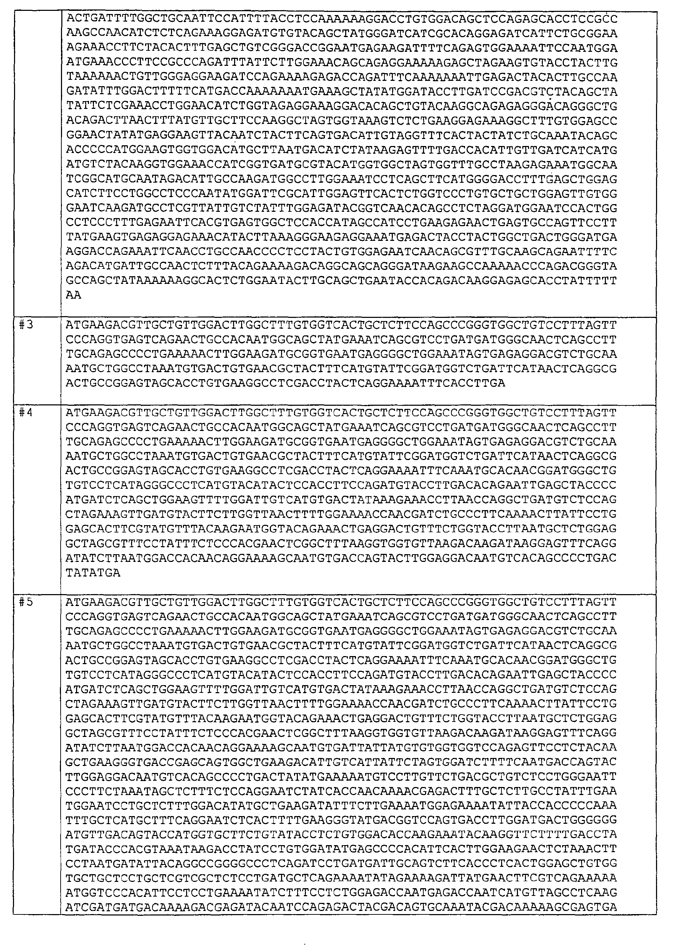

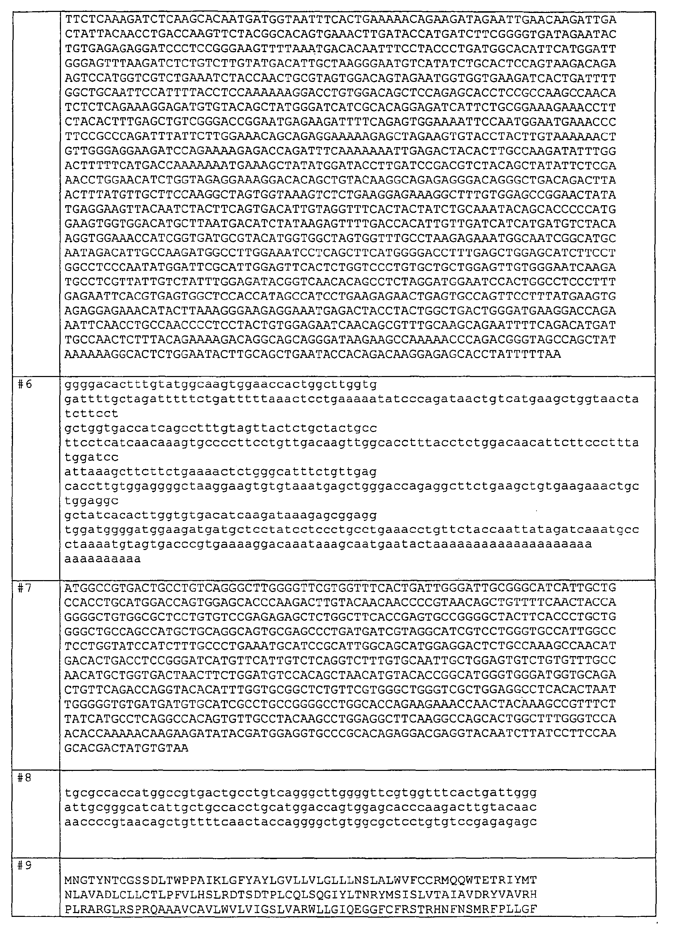

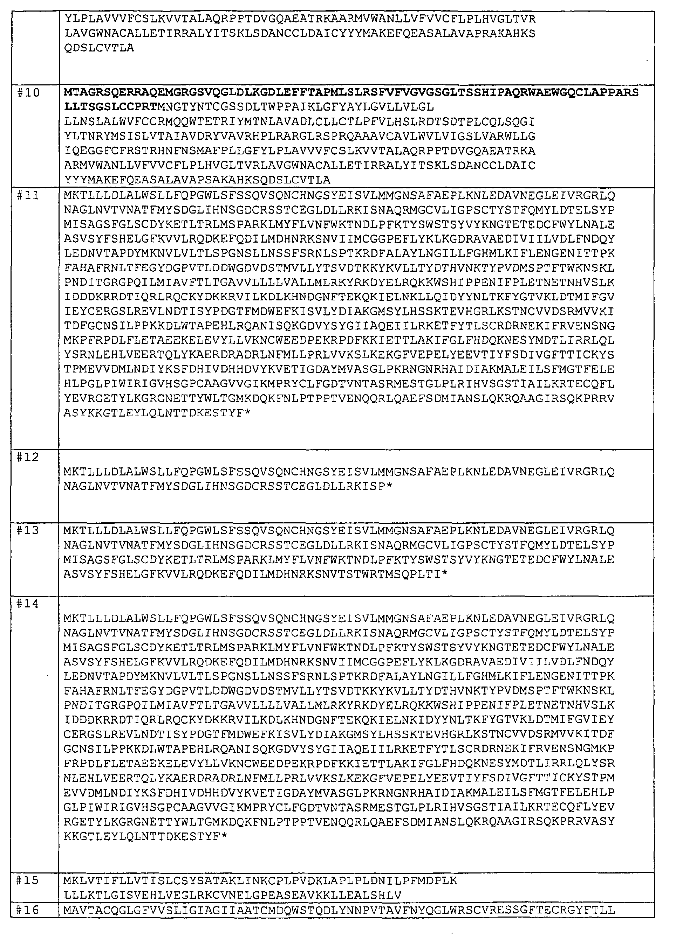

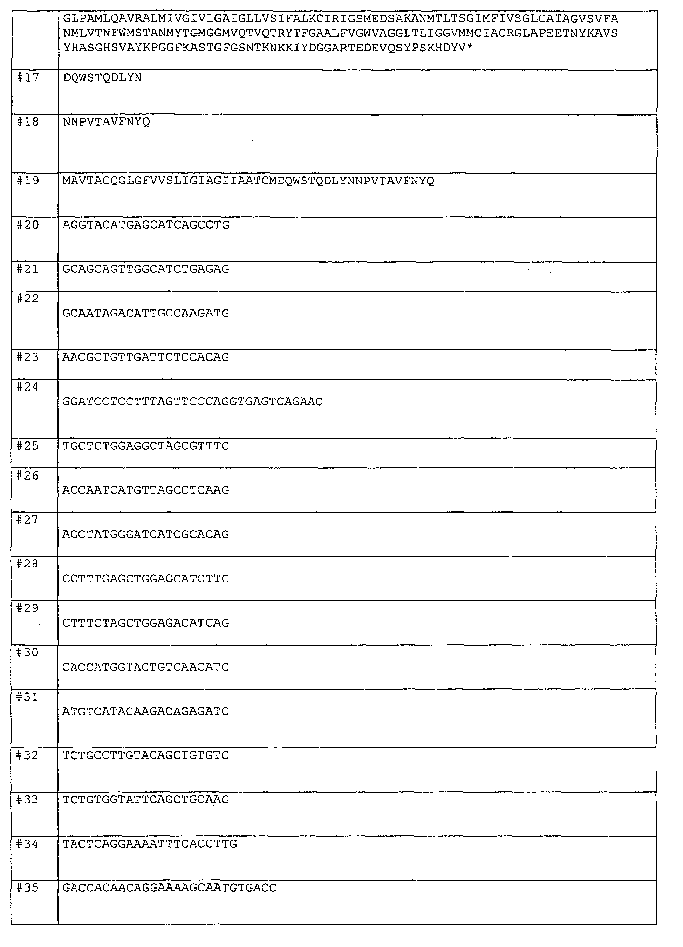

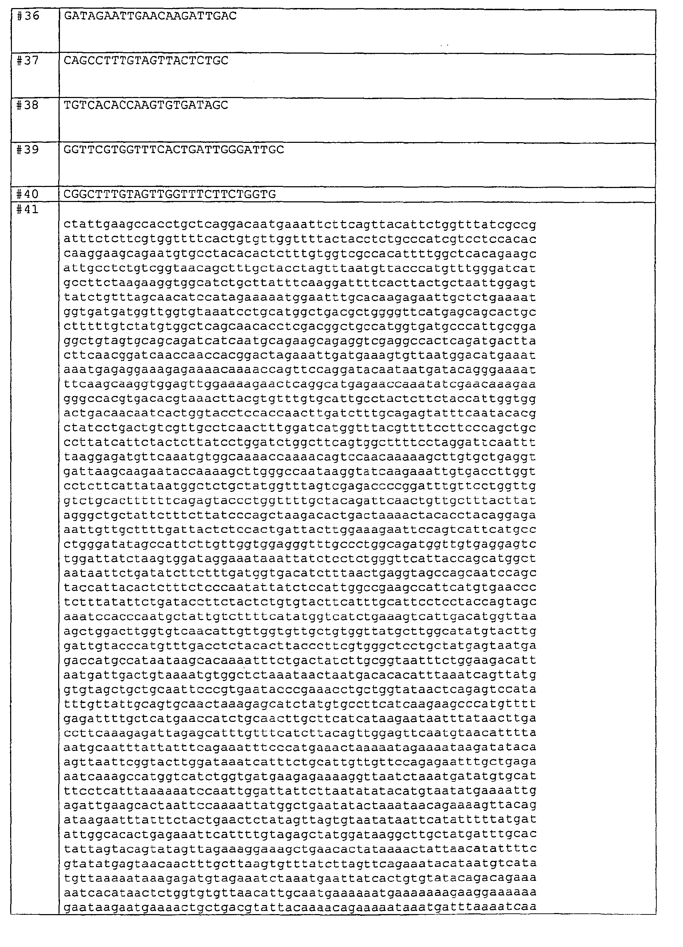

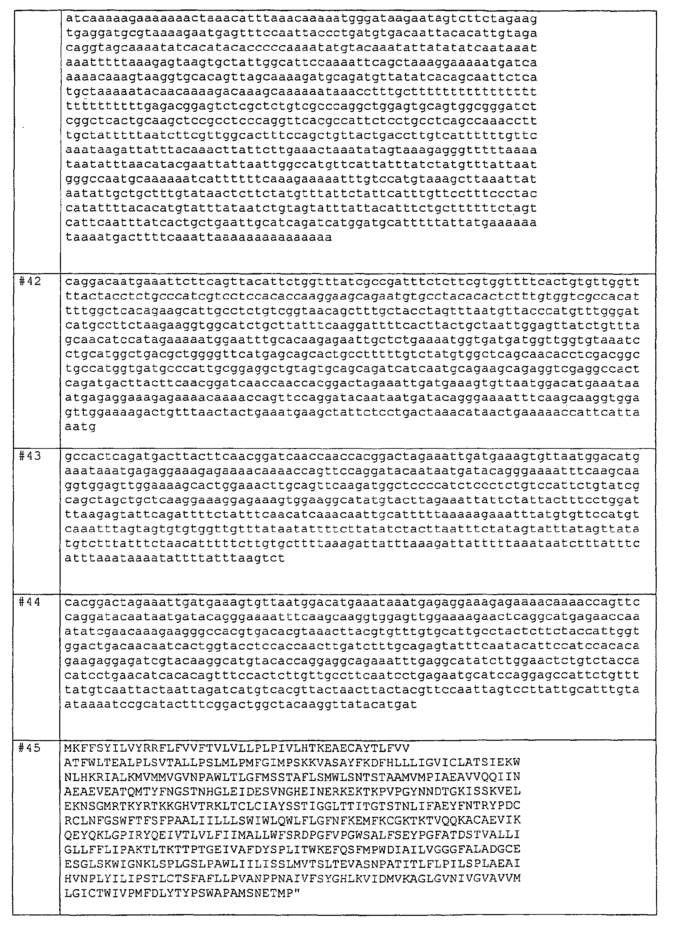

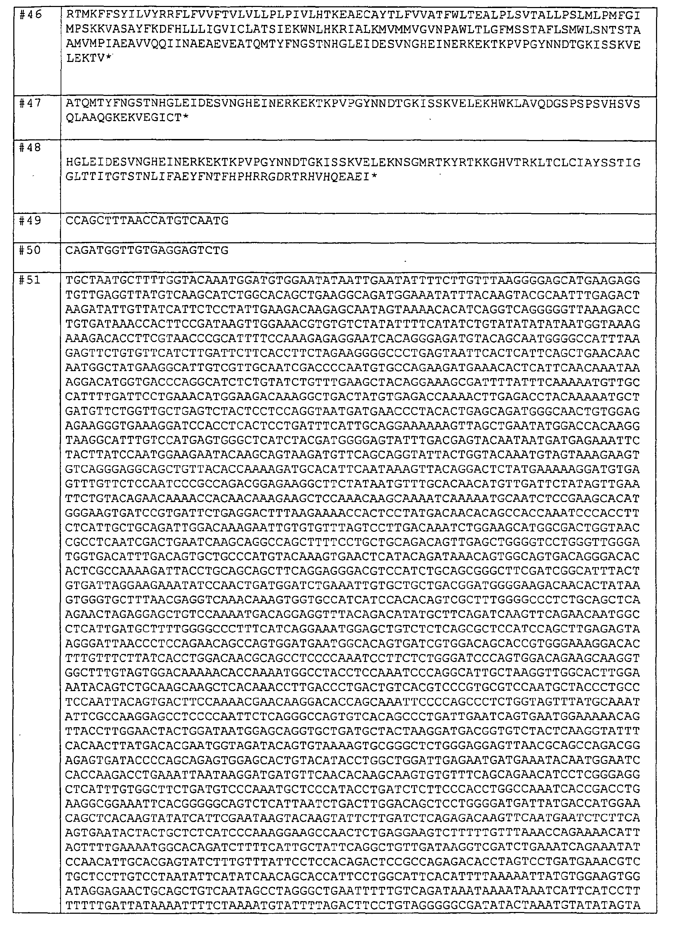

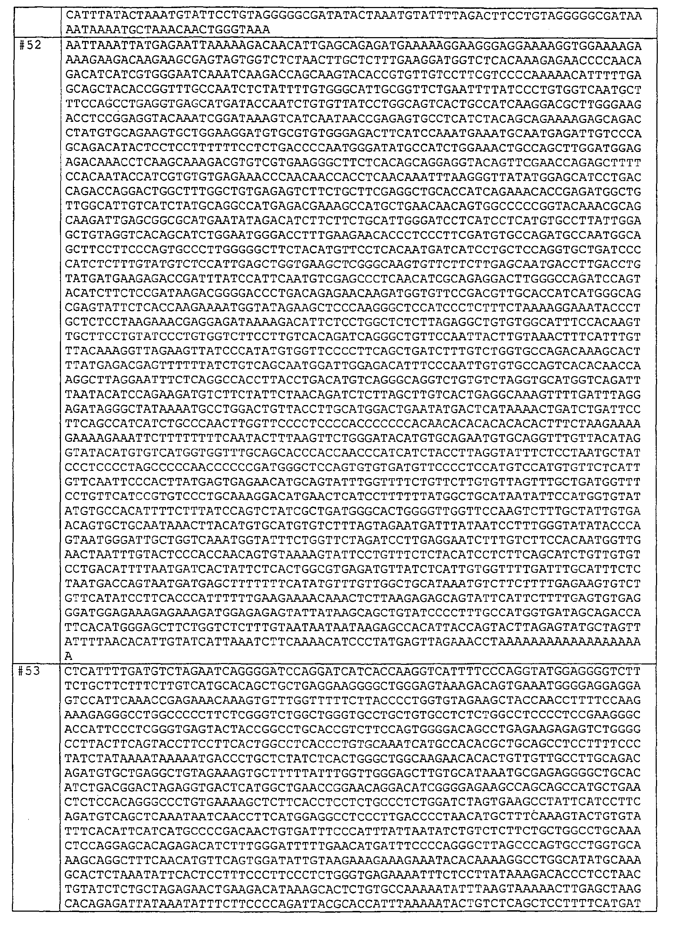

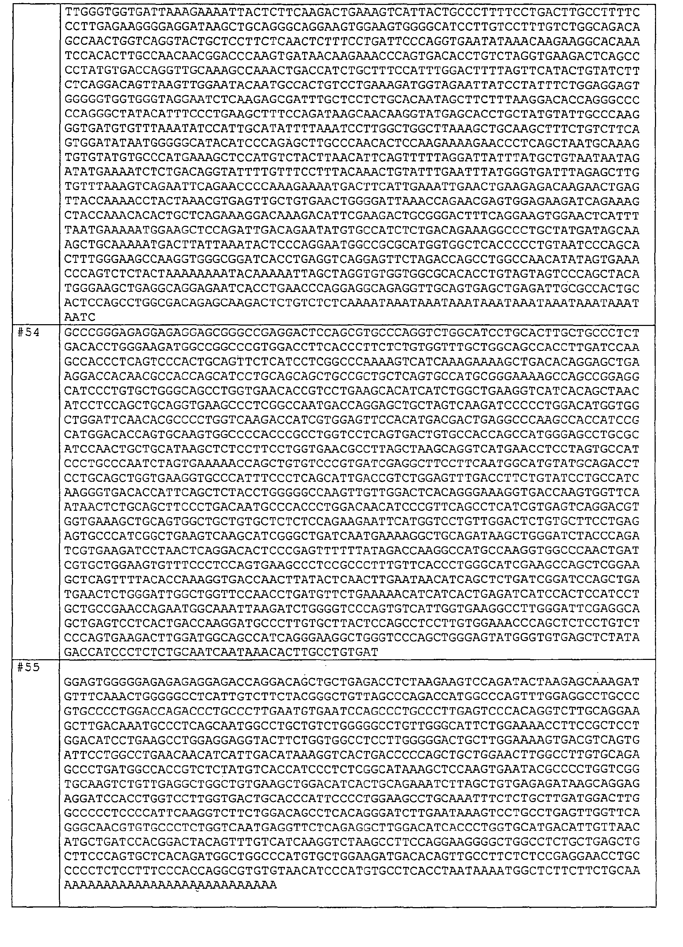

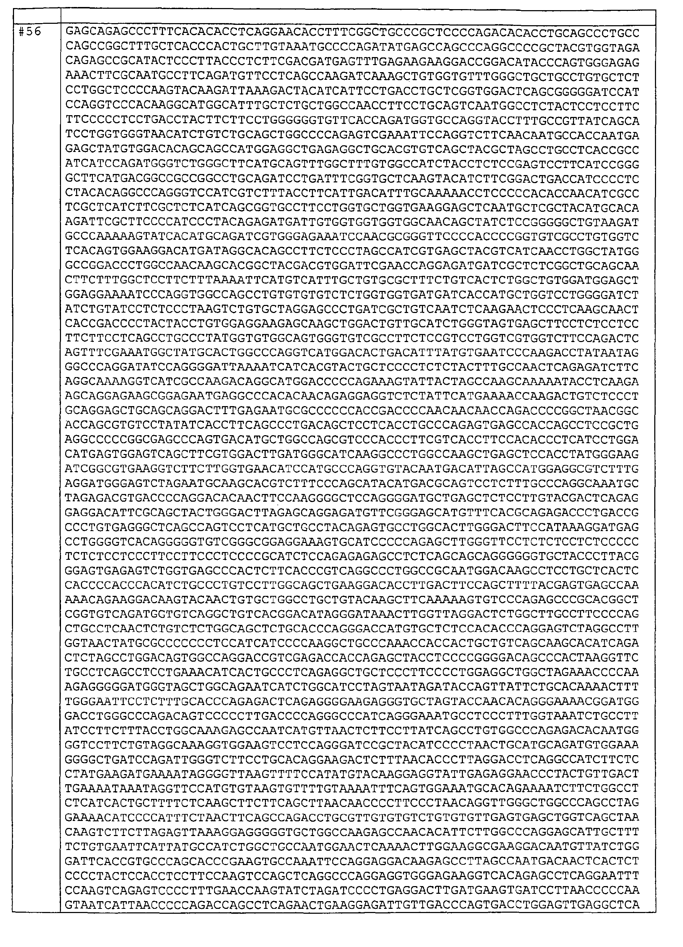

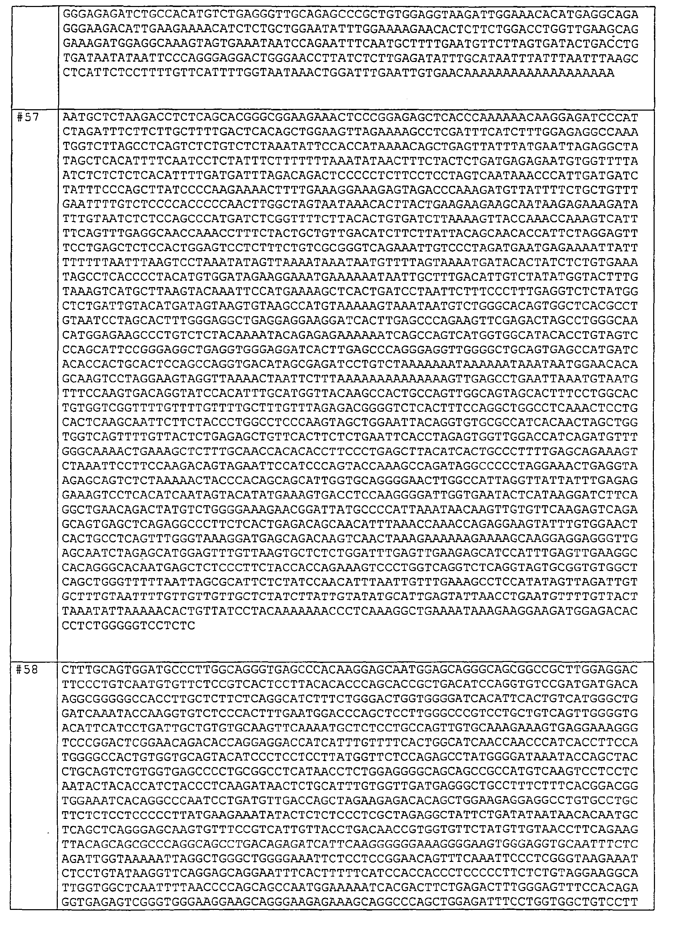

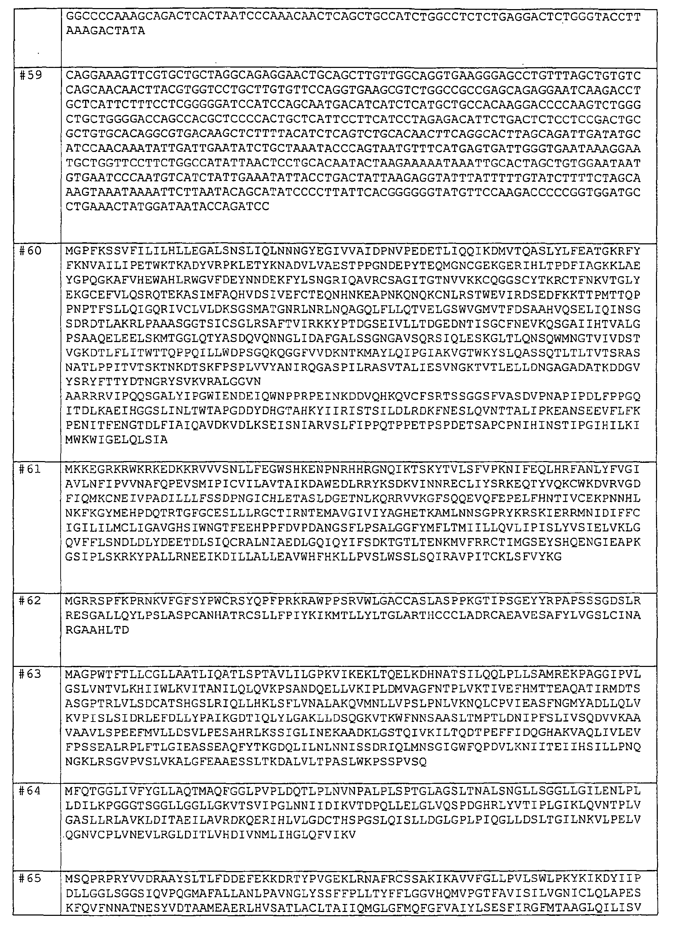

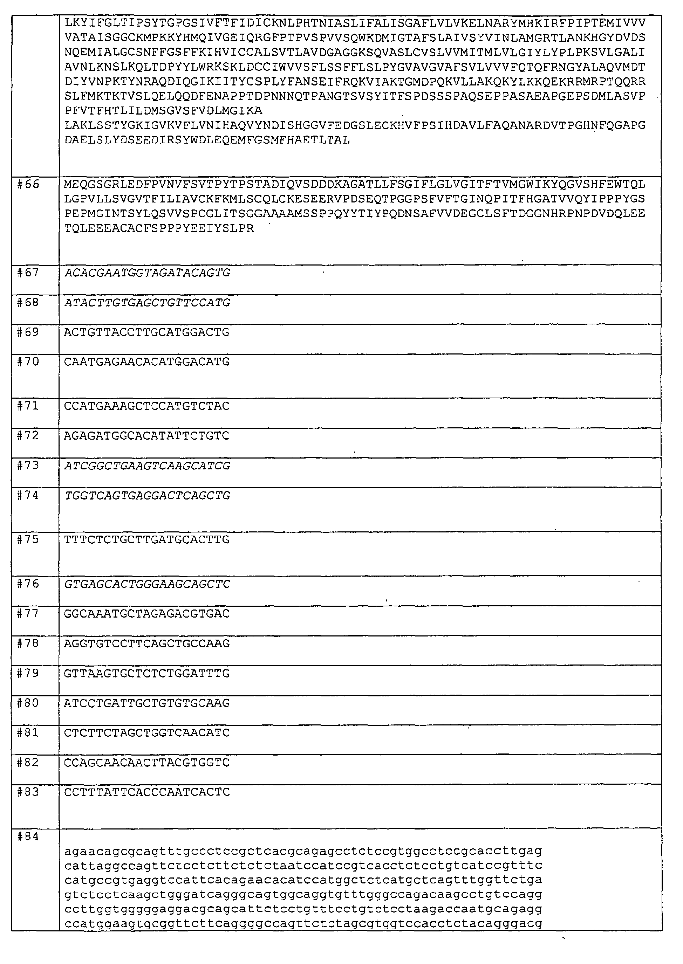

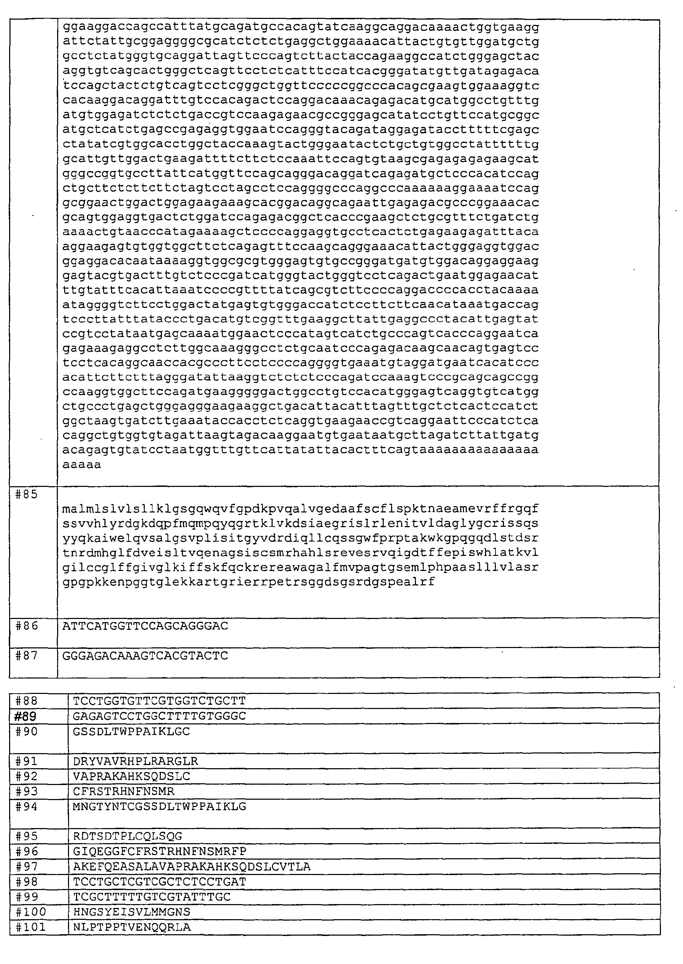

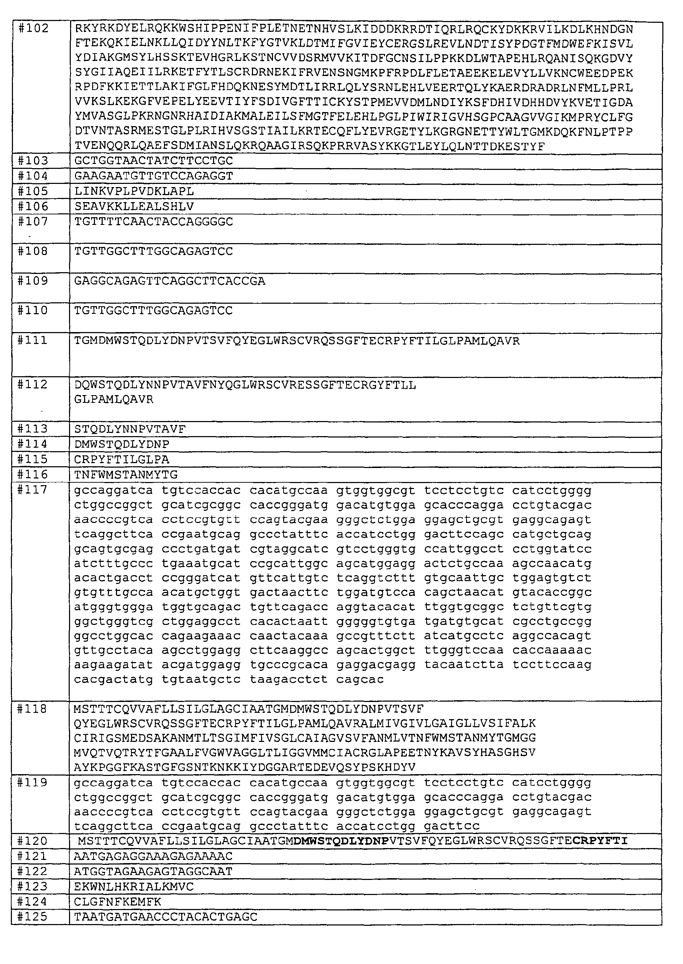

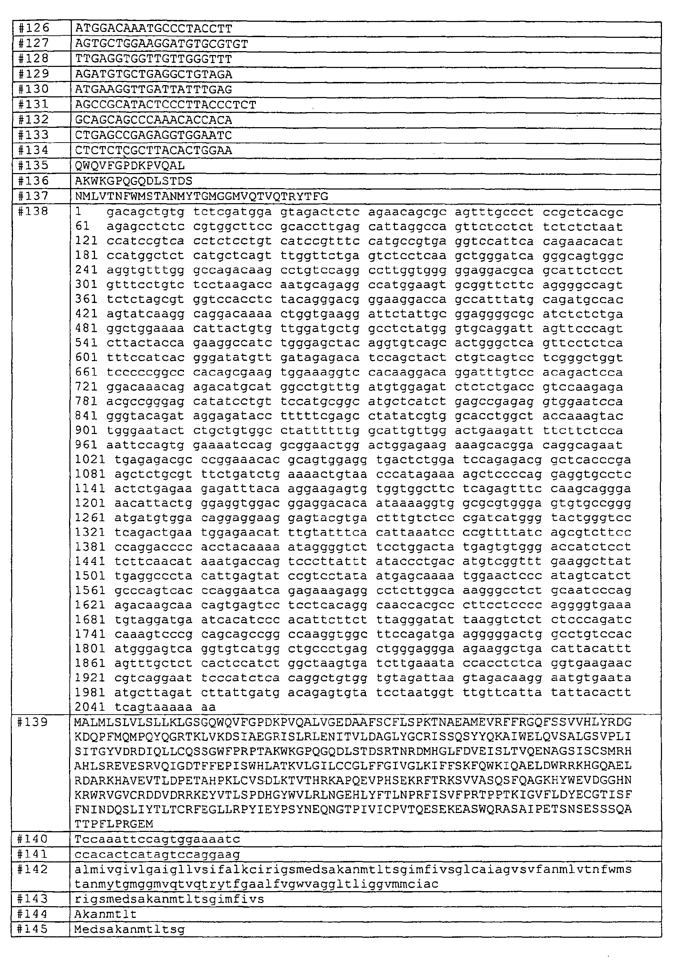

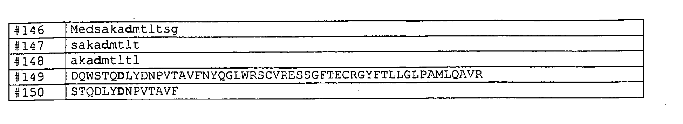

### Abb. 41. Sequenzen

Gezeigt sind die Sequenzen, auf die hierin verwiesen wird.

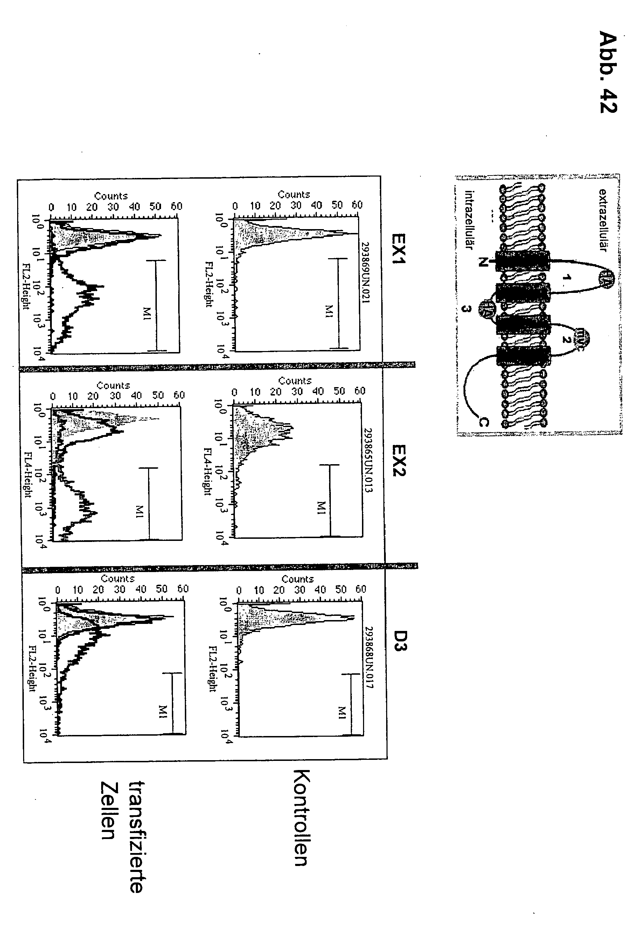

### Abb. 42. Ermittlung extrazellulärer Bereiche von Claudin-18A2

Drei Konstrukte wurden hergestellt, die jeweils eine Marker-Sequenz (myc- oder HA-Tag) in einer der Domänen EX1 (= extrazelluläre Domäne 1), EX2 (= extrazelluläre Domäne 2), oder D3 (= Domäne 3) trugen (oben). Diese wurden in Zelllinien transfiziert und daraufhin getestet, ob ein gegen diese Markersequenzen gerichteter Antikörper an nichtpermeabilisierte Zellen bindet. Dies erfordert, dass der entsprechende Bereich des Proteins topologisch extrazellulär ist. In der Durchflusszytometrie erwies sich, dass alle drei Bereiche des Moleküls für die Antikörper zugänglich sind (unten).

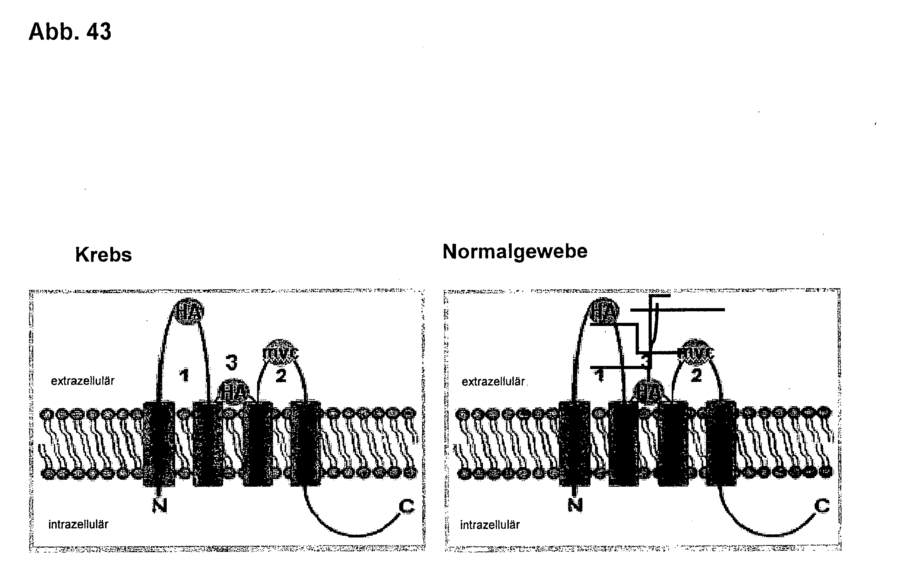

### Abb. 43. Claudin-18A2-Membrantopologie

Entsprechend unserer Daten kann Claudin-18A2 in der Konformation 2 vorliegen, wobei die inneren beiden hydrophoben Domänen nicht integral durch die Zellmembran hindurchführen. Dadurch sind größere Bereiche dieses Moleküls extrazellulär. Hier befinden sich auch Glykosylierungsdomänen, die nach unseren Daten in Magennormalgewebe glykosyliert sind, in Tumoren aber nicht Es entstehen somit rein tumorgewebsspezifische Epitope.

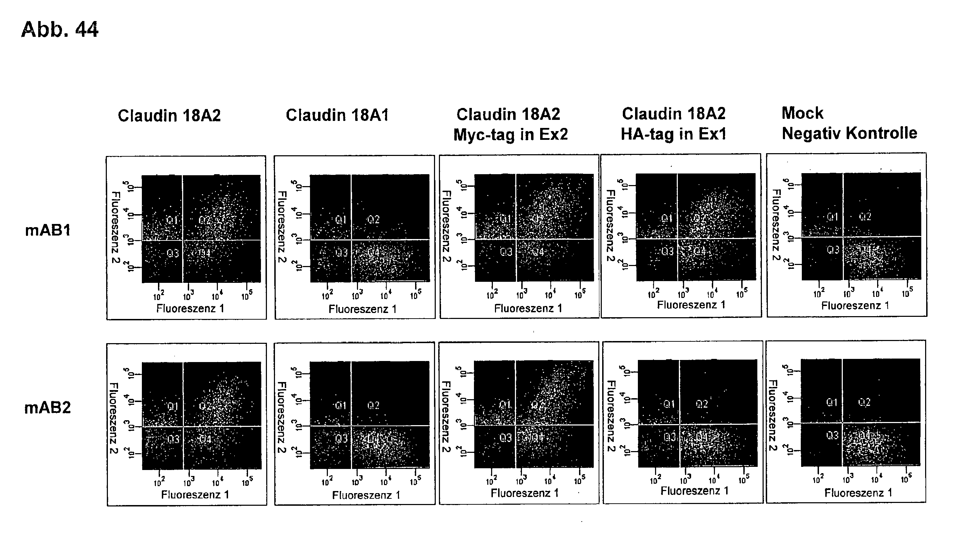

### Abb. 44. FACS-Analyse zur Bestimmung der extrazellulären Lokalisation von Claudin 18

Die Abbildung zeigt durchflusszytometrische Analysen an nichtpermeabilisierten Zellen, die mit Voll-Längen Claudin-18A1, Claudin-18A2 und Mock sowie Abschnitten aus Claudin-18A2 transfiziert wurden. Erkennbar ist, dass die Antikörper mAB1 und mAB2 spezifisch Claudin-18A2 (linke Spalte) und die extrazelluläre Domäne 2 (Ex2, 3. Spalte) an der Zelloberfläche erkennen, während Claudin-18A1 (2. Spalte) und die Negativ-Kontrolle (letzte Spalte) negativ sind. Der Antikörper mAB1 bindet, im Gegensatz zu mAB2, auch spezifisch die extrazelluläre Domäne 1 (Ex1, 4. Spalte).

### Beispiele:

### Material und Methoden

Die Begriffe "*in silico*", "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini werden, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von neuen Tumor-assoziierten Genen

Zwei *in silico* Strategien nämlich GenBank-Schlagwort-Suche und der cDNAxProfiler wurden kombiniert. Es wurde unter Nutzung des ENTREZ Search and Retrieval Systems des NCBI. (http://www.ncbi.nlm.nih.gov/Entrez) eine Suche nach Kandidaten-Genen in der GenBank durchgeführt, die annotiert sind als spezifisch exprimiert in bestimmten Geweben (Wheeler et al., Nucleic Acids Research 28:10-14, 2000).

Durch Suchabfragen mit Schlagworten wie beispielsweise "colon-specific gene", " stomachspecific gene", oder "kidney-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.

Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht Organ-spezifischen Normalgeweben hatten. Für dies Beurteilung wurde auch berücksichtigt, dass es falsch-annotierte cDNA-Banken in der öffentlichen Domäne gibt (Scheurle et al., Cancer Res. 60: 4037-4043, 2000) (www.fau.edu/cmbb/publications/cancergenes6.htm). Als zweites Datamining-Verfahren wurde der **cDNA xProfiler** des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt, Pools von in Datenbanken abgelegten Transkriptomen durch logische Operatoren in Beziehung zueinander zu setzen. Wir haben einen Pool A definiert, dem beispielsweise alle aus Colon hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Colon hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthem-Studien unterzogen, sowie durch eine Literaturrecherche abgesichert.

Dieses kombinierte Datamining schließt alle etwa 13 000 Volllänge-Gene in der öffentlichen Domäne ein und prädiziert aus diesen Gene mit potentieller Organ-spezifischer Expression.

Alle anderen Gene wurden zunächst durch spezifische RT-PCR in Normalgeweben evaluiert. Alle GOI, die sich als in nicht Organ-spezifischen Normalgeweben exprimiert erwiesen, hatten als Falsch-Positive zu gelten und wurden aus weiteren. Untersuchungen ausgeschlossen. Die verbliebenen wurden in einem großen Panel an verschiedensten Tumorgeweben untersucht. Die unten dargestellten Antigene erwiesen sich dabei als in Tumorzellen aktiviert.

### RNA-Extraktion, Herstellung von poly-d(T) geprimter cDNA und konventionelle RT-PCR Analyse

Gesamt-RNA aus nativem Gewebematerial wurde unter Verwendung von Guanidiumisothiocyanat als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst

Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.

Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.

Die Primer wurden so ausgewählt, dass sie in 2 verschiedenen Exons liegen, und die Beseitigung der Interferenz durch kontaminierende genomische DNA als Grund für falsch positive Resultate wurde durch Testen von nicht revers transkribierter DNA als Matrize bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min).

20µl dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet.
GPR35 (65°C)
Sense: 5'-AGGTACATGAGCATCAGCCTG-3'
Antisense: 5'-GCAGCAGTTGGCATCTGAGAG-3'
GUCY2C (62°C)
Sense: 5'-GCAATAGACATTGCCAAGATG-3'
Antisense: 5'-AACGCTGTTGATTCTCCACAG-3'
SCGB3A2 (66°C)
Sense: 5'-CAGCCTTTGTAGTTACTCTGC-3'
Antisense: 5'-TGTCACACCAAGTGTGATAGC-3'
Claudin18A2 (68°C)
Sense1: 5'-GGTTCGTGGTTTCACTGATTGGGATTGC-3'
Antisense1: 5'-CGGCTTTGTAGTTGGTTTCTTCTGGTG-3'
Sense2: 5'- TGTTTTCAACTACCAGGGGC-3'
Antisense2: 5'- TGTTGGCTTTGGCAGAGTCC-3'
Claudin18A1 (64°C)
Sense: 5'-GAGGCAGAGTTCAGGCTTCACCGA-3'
Antisense: 5'- TGTTGGCTTTGGCAGAGTCC-3'
SLC13A1 (64°C)
Sense: 5'-CAGATGGTTGTGAGGAGTCTG-3'
Antisense: 5'-CCAGCTTTAACCATGTCAATG-3'
CLCA1 (62°C)
Sense: 5'-ACACGAATGGTAGATACAGTG-3'
Antisense: 5'-ATACTTGTGAGCTGTTCCATG-3'
FLJ21477 (68°C)
Sense: 5'- ACTGTTACCTTGCATGGACTG-3'
Antisense: 5'- CAATGAGAACACATGGACATG-3'
FLJ20694 (64°C)
Sense: 5'- CCATGAAAGCTCCATGTCTA-3'
Antisense: 5'- AGAGATGGCACATATTCTGTC
Ebner (70°C)
Sense: 5'-ATCGGCTGAAGTCAAGCATCG-3'
Antisense: 5'-TGGTCAGTGAGGACTCAGCTG-3'
Plunc (55°C)
Sense: 5'-TTTCTCTGCTTGATGCACTTG-3'
Antisense: 5'-GTGAGCACTGGGAAGCAGCTC-3'
SLC26A9 (67°C)
Sense: 5'-GGCAAATGCTAGAGACGTGA-3'
Antisense: 5'-AGGTGTCCTTCAGCTGCCAAG-3'
THC1005163 (60°C)
Sense: 5'- GTTAAGTGCTCTCTGGATTTG-3'
LOC134288 (64°C)
Sense. 5'-ATCCTGATTGCTGTGTGCAAG-3'
Antisense: 5'-CTCTTCTAGCTGGTCAACATC-3'
THC943866 (59°C)
Sense: 5'-CCAGCAACAACTTACGTGGTC-3'
Antisense: 5'-CCTTTATTCACCCAATCACTC-3'
FLJ21458 (62°C)
Sense: 5'-ATTCATGGTTCCAGCAGGGAC-3'
Antisense: 5'-GGGAGACAAAGTCACGTACTC-3'

### Herstellung von Random-Hexamer-geprimter cDNA und quantitative Real-Time-PCR

Die Expression mehrere Gene wurde mittels Real-Time-PCR quantifiziert. Dabei wurden die PCR-Produkte mit SYBR-Green als interkalierendem Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green-Fluoreszenz als Folge der spezifischen Amplifikation mittels GOI-spezifischen Primern nach jedem PCR-Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression wurde nach Normalisierung der Proben gegen 18s RNA als sog. Housekeeping-Gen mittels der ΔΔ-Cₜ Methode (PE Biosystems, USA) ermittelt. Die Reaktionen wurden in Duplex-Ansätzen durchgeführt und in Triplikaten bestimmt. Verwendet wurde der QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer 300nM, antisense-Primer 300nM; initiale Denaturierung 95°C 15 min; 95°C 30 sec; Annealing 30 sec; 72°C 30 sec; 40 Zyklen. Die Sequenzen der verwendeten Primer sind in den jeweiligen Beispielen aufgeführt.

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA-Vektor zu klonieren. Die Sequenzierung wurde durch einen kommerziellen Service durchgeführt. Die Sequenzen wurden mittels gängiger Prädiktionsprogramme und Algorithmen analysiert.

### Western-Blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%igen SDS Lösung lysiert. Das SDS denaturiert dabei die im Lysat enthaltenen Proteine. Die Lysate eines experimentellen Ansatzes werden abhängig von der erwarteten Proteingröße auf 8-15 %igen denaturierenden Polyacrylamidgelen (enthalten 1% SDS) der Größe nach elektrophoretisch aufgetrennt (SDS-Polyacrylamid-Gelelektrophorese, SDS-PAGE). Anschließend werden die Proteine durch das semi-dry Elektroblot Verfahren (Biorad) auf Nitrozellulose-Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

Die Analyse von Proteinmodifikationen erfolgt in der Regel im Western-Blot. Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Pröteinlysate aus Geweben oder Zellen vor der Denaturierung durch SDS mit O- oder N-Glykosidasen inkubiert (nach Angaben des jeweiligen Herstellers, z.B. PNgase, Endoglykosidase F, Endoglykosidase H, Roche Diagnostics). Anschließend erfolgt ein Western-Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Mit Algorithmen und Prädiktionsprogrammen kann die genaue Position der glykosylierten Aminosäure prädiziert werden.

### Immunfluoreszenz

Es werden Zellen etablierter Zelllinien benutzt, die entweder das Zielprotein endogen synthetisieren (Nachweis der RNA in der RT-PCR oder des Proteins im Western-Blot) oder aber vor der IF mit Plasmid-DNA transfiziert worden sind. Zur Transfektion von Zelllinien mit DNA sind die verschiedensten Methoden (z.B. Elektroporation, Liposomen-basierte Transfektion, Calciumphosphatpräzipitation) gut etabliert (z.B. Lemoine et al. Methods Mol. Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen und kurze Peptidsequenzen von 6-12 Aminosäuren, für die hoch affine und spezifische Antikörper zur Verfügung stehen. Zellen, die das Zielprotein synthetisieren, werden mit Paraformaldehyd, Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden; deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und/oder (3) den Zelltyp in einem Gewebe (Tumor, gesunde Zellen) zu definieren, in dem das Zielprotein nachweisbar ist

Je nach dem individuellen Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Die Immunhistochemie (IHC) an spezifischen Gewebeproben dient dem Proteinnachweis im entsprechenden Gewebe. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR-Analysen bekannt ist. Eine Hintergrundkontrolle ist immer mitzuführen.

Fixierte Gewebe (z.B. Fixation mit aldehydhaltigen Substanzen, Formaldehyd, Paraformaldehyd oder in alkoholischen Lösungen) oder schockgefrorene Gewebestücke mit einer Dicke von 1-10µm werden auf einem Glasträger aufgebracht. Paraffineingebettete Proben werden z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der von Farbsubstraten, die von dem gebundenen Enyzmen umgesetzt werden (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Immunisierung

(Siehe auch Monoclonal Antibodies: A Practical Approach by Philip Shepherd, Christopher Dean isbn 0-19-963722-9; Antibodies: A Laboratory Manual by Ed Harlow, David Lane ISBN: 0879693142; Using Antibodies : A Laboratory Manual : Portable Protocol NO. by Edward Harlow, David Lane, Ed Harlow ISBN: 0879695447).

Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.

Die Immunisierung der Tiere erfolgt in der Regel über eines von vier gut etablierten Verfahren, wobei auch andere Verfahren verfügbar sind. Immunisiert werden kann dabei mit Peptiden, die für das Zielprotein spezifisch sind, dem gesamten Protein oder mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro-Verfahren synthetisiert und diese Peptide zur Immunisierung verwendet. In der Regel erfolgen 3 Immunisierungen mit einer Konzentration von 5-1000 µg/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, wobei die Aufreinigung dabei in der Regel über standardisierte chromatografische Methoden erfolgt. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst-Fusionsproteine). Eine Vielzahl von Protokollen finden sich z.B. in den "Current Protocols in Molecular Biology", John Wiley & Sons Ltd., Wiley InterScience.
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend werden 5-100 µg DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et al., Hybrid Hybridomics 21: 287-293,2002).

### Qualitätskontrolle des polyklonalen Serums bzw. Antikörpers

Zum Spezifitätsnachweis eignen sich am besten auf Zellkultur-basierende Tests mit anschließendem Western-Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter der Kontrolle eines starken eukaryontischen Promotors steht (z.B. Cytomegalovirus-Promotor). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgenspezifische RT-PCR). Zur Kontrolle werden im Experiment im Idealfall homologe Gene mit transfiziert, um im folgenden Western-Blot die Spezifität des analysierten Antikörpers nachweisen zu können.

Im anschließenden Western-Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid-Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

Zur Bestätigung der im in silico-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western-Blot) oder aber mit Plasmid-DNA transfiziert worden sind. Zur Transfektion von Zelllinien mit DNA sind die verschiedensten Vefahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz den Nachweis, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zur Immunfluoreszenz kann durch Verwendung von z.B. Propidiumiodid oder Trypanblau zwischen toten und lebenden Zellen unterschieden werden und damit falsch positive Ergebnisse vermieden werden.

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin, pH 2,7 eluiert und das Eluat sogleich in 2 M TRIS, pH 8 neutralisiert. Die so gereinigten Antikörper konnten dann zur spezifischen Detektion der Zielproteine sowohl durch Westernblotting als auch durch Immunfluoreszenz eingesetzt werden.

### Herstellung von GFP-Transfektanfen

Für die Immunfluoreszenz-Mikroskopie von heterolog exprimierten Tumor-assoziierten Antigenen wurde der komplette ORF der Antigene in pGFP-C1- und pGFP-N3-Vektoren (Clontech) kloniert. Auf Objekträgern kultivierte CHO- und NIH3T3-Zellen wurden mit den entsprechenden Plasmidkonstrukten unter Verwendung von Fugene-Transfektionsreagenz (Roche) nach Herstellerangaben transfiziert und nach 12-24h mittels Immunfluoreszenz-Mikroskopie analysiert.

### Durchflusszytometrie

Durchflusszytometrische Messungen wurden gemäß bekannter Verfahren durchgeführt (z. B. Robinson (Editor) Handbook of flow cytometry methods. Wiley-Liss, New York, 1993).

### Beispiel 1: Identifizierung von GPR35 als diagnostisches und therapeutisches Krebs-Target

GPR35 (SEQ ID NO: 1) und sein Translationsprodukt (SEQ ID NO: 9) wurden als putativer G-Protein-gekoppelter Rezeptor beschrieben. Die Sequenz ist in der Genbank unter der Zugangs-Nr. AF089087 veröffentlicht. Dieses Transkript kodiert für ein Protein von 309 Aminosäuren mit einem Molekulargewicht von 34 kDa. Es wurde prädiziert, dass GPR35 zur Super-Familie der G-Protein-gekoppelten Rezeptoren mit 7 Transmembran-Domänen gehört (O'Dowd et al., Genomics 47:310-13, 1998). Um die prädizierte Lokalisation von GPR35 in der Zelle zu bestätigen, wurde das Protein mit GFP als Reportermolekül fusioniert und nach Transfektion des entsprechenden Plasmids heterolog in 293-Zellen exprimiert. Anschließend wurde die Lokalisation im Fluoreszensmikroskop analysiert. Erfindungsgemäß wurde bestätigt, dass GPR35 ein integrales Transmembranmolekül ist (Abb. 17). Bisherige Untersuchung zu humanem GPR35 (sua Horikawa Y, Oda N, Cox NJ, Li X, Orho-Melander M, Hara M, Hinokio Y, Lindner TH, Mashima H, Schwarz PE, del Bosque-Plata L, Horikawa Y, Oda Y, Yoshiuchi I, Colilla S, Polonsky KS, Wei S, Concannon P, Iwasaki N, Schulze J, Baier LJ, Bogardus C, Groop L, Boerwinkle E, Hanis CL, Bell GI Nat Genet. 2000 Oct;26(2):163-75) legten nahe, dass GPR35 in vielen gesunden Geweben aktiviert ist. Das Leseraster des Gens enthält ein einzelnes Exon. Erfindungsgemäß wurde mit einem Gen-spezifischen Primerpaar (SEQ ID NO: 20, 21) für GPR35 in RT-PCR-Analysen cDNA in Kolon-Normalgewebe und in Kolontumoren (13/26) amplifiziert. Dagegen ist eine signifikante Expression in anderen Normalgeweben nicht nachweisbar. Aufgrund der Besonderheit, dass GPR35 aus einem einzelnen Exon besteht, können genomische DNA-Verunreinigungen nicht mit Intron-überspannenden Primern nachgewiesen werden. Um eine genomische Verunreinigung der RNA-Proben auszuschließen, wurden deshalb alle RNAs mit DNAse behandelt. Erfindungsgemäß wurden mit DNA-freier RNA GPR35-Transkripte nur im Kolon, im Enddarm, in Testis und in Kolontumoren nachgewiesen.

**Tab. 1. GPR35 -Expression in Normalgeweben**

| **Normalgewebe** | **Expression** |
|---|---|
| Gehirn | - |
| Cerebellum (Kleinhirn) | - |
| Herzmuskel | - |
| Muskel | - |
| Rektum | ++ |
| Magen | - |
| Kolon | ++ |
| Pankreas | - |
| Niere | - |
| Testis | - |
| Thymus | - |
| Brustdrüse | - |
| Ovar | - |
| Uterus | nd |
| Haut | - |
| Lunge | - |
| Schilddrüse | - |
| Lymphknoten | - |
| Milz | - |
| PBMC | - |
| Nebenniere | - |
| Ösophagus | - |
| Dünndarm | + |
| Prostata | - |

| | |
|---|---|
| **(nd = nicht bestimmt)** | |

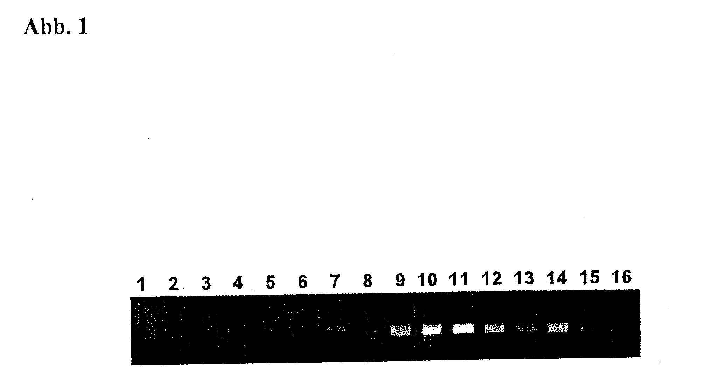

Die selektive und hohe Expression von GPR35-Transkripten im normalen Kolon-Gewebe, sowie in Kolontumor-Biopsien (Abb. 1) war bisher nicht bekannt und kann erfindungsgemäß für molekulare diagnostische Verfahren wie RT-PCR zum Nachweis disseminierender Tumorzellen im Serum und Knochenmark und zum Nachweis von Metastasen in anderen Geweben genutzt werden. Auch die quantitative RT-PCR mit spezifischen Primern (SEQ ID NO: 88 und 89) bestätigt, dass GPR35 ein hochselektives kolonspezifisches und auch in Kolontumoren und in Kolontumor-Metastasen enthaltenes Differenzierungsantigen ist. In einigen Kolontumoren ist es im Vergleich zum normalen Kolon sogar um ca. den Faktor 10 überexprimiert (Abb. 18). Zum Nachweis von GPR35-Protein wurden Antikörper durch Immunisieren von Kaninchen hergestellt. Folgende Peptide wurden zur Propagierung dieser Antikörper genutzt:
SEQ ID NO: 90: GSSDLTWPPAIKLGC (AS 9-23)
SEQ ID NO: 91: DRYVAVRHPLRARGLR (AS 112-127)
SEQ ID NO: 92: VAPRAKAHKSQDSLC (C-Terminus)
SEQ ID NO: 93: CFRSTRHNFNSMR (extrazelluläre Domäne 2)

Färbungen mit diesen Antikörpern z.B. im Western-Blot bestätigen die Expression in Tumoren. Alle 4 extrazellulären Domänen von GPR35 (Position der prädizierten extrazellulären Domänen in der Sequenz von SEQ ID NO: 9: AS 1-22 (SEQ ID NO: 94); AS 81-94 (SEQ ID NO: 95); AS 156-176 (SEQ ID NO: 96); AS 280-309 (SEQ ID NO: 97)) können erfindungsgemäß als Zielstrukturen von monoklonalen Antikörpern genutzt werden. Diese Antikörper binden spezifisch an die Zelloberfläche von Tumorzellen und können sowohl für diagnostische als auch für therapeutische Verfahren genutzt werden. Die Überexpression von GPR35 in Tumoren unterstützt einen solchen Einsatz noch zusätzlich. Des weiteren können die für Proteine kodierenden Sequenzen erfindungsgemäß als Vakzine (RNA, DNA, Peptid, Protein) zur Induktion von Tumor-spezifischen Immun-Antworten (T-Zell- und B-Zell-vermittelte Immun-Reaktionen) genutzt werden. Darüberhinaus wurde überraschenderweise festgestellt, dass 5' vor dem allgemein bekannten Startcodon ein weiteres Startcodon existiert, welches ein N-terminal verlängertes Protein exprimiert.

Somit wurde erfindungsgemäß festgestellt, dass ein zuvor als ubiquitär exprimiert beschriebenes Protein, GPR35, selektiv in gastrointestinalen Tumoren, insbesondere in Tumoren des Kolons Tumor-assoziiert überexprimiert wird. GPR35 eignet sich daher insbesondere als molekulare Zielstruktur für die Diagnose und Behandlung dieser Tumoren. Bisherige Untersuchung zu humanem GPR35, vgl. z.B. Horikawa Y, Oda N, Cox NJ, Li X, Orho-Melander M, Hara M, Hinokio Y, Lindner TH, Mashima H, Schwarz PE, del Bosque-Plata L, Horikawa Y, Oda Y, Yoshiuchi I, Colilla S, Polonsky KS, Wei S, Concannon P, Iwasaki N, Schulze J, Baier LJ, Bogardus C, Groop L, Boerwinkle E, Hanis CL, Bell GI Nat Genet. 2000 Oct;26(2):163-75, legten nahe, dass GPR35 in vielen gesunden Geweben exprimiert ist. Die erfindungsgemäßen Untersuchungen zeigten dagegen, dass GPR35 in den meisten Normalgeweben überraschenderweise nicht signifikant nachweisbar ist und im Gegensatz dazu stark in primären und metastasierenden Kolontumoren aktiviert ist. Ferner wurde erfindungsgemäß neben der beschriebenen GPR35-Sequenz eine neue Translationsvariante gefunden, die von einem alternativen Startcodon Gebrauch macht (SEQ ID NO:10).

GPR35 ist ein Mitglied der Gruppe von G-gekoppelten Rezeptoren (GPCR), eine sehr große Proteinfamilie, die strukturell und funktionell sehr gut untersucht ist. GPCR eignen sich hervorragend als Zielstrukturen für die Entwicklung pharmazeutisch wirksamer Substanzen, da die dafür notwendigen Verfahren (z.B. Rezeptorexpression, Aufreinigung, Ligandenscreening, Mutagenisierung, funktionelle Inhibition, Auswahl agonistischer und antagonistischer Liganden, radioaktive Markierung von Liganden) sehr gut entwickelt und ausführlich beschrieben sind, vgl. z.B. "G Protein-Coupled Receptors" von Tatsuya Haga, Gabriel Berstein und Gabriel Bernstein ISBN: 0849333849 bzw. in "Identification and Expression of G-Protein Coupled Receptors Receptor Biochemistry and Methodology" von Kevin R. Lynch ASIN: 0471183105. Die erfindungsgemäße Erkenntnis, dass GPR35 in den meisten gesunden Geweben nicht nachweisbar ist, jedoch Tumor-assoziiert an der Zelloberfläche exprimiert wird, ermöglicht dessen Verwendung als Tumor-assoziierte Zielstruktur z.B. für pharmazeutisch aktive Liganden, insbesondere in Konjugation z,B. mit radioaktiven Molekülen als pharmazeutische Substanzen. In einer besonderen Ausführungsform können radioaktiv markierte Liganden, die an GPR35 binden, zum Nachweis von Tumorzellen oder zur Behandlung von Kolontumoren in vivo verwendet werden.

### Beispiel 2: Identifizierung von GUCY2C in Leber- und Ovarialtumoren und neuen GUCY2C-Spleißvarianten als diagnostische und therapeutische Krebs-Targets

Die Guanylatcyclase 2C (GUCY2C; SEQ ID NO: 2; Translationsprodukt: SEQ ID NO: 11) - ein Typ I Transmembranprotein - gehört zur Familie der natriuretischen Peptidrezeptoren. Die Sequenz ist in der Genbank unter der Zugangsnummer NM_004963 veröffentlicht. Durch Bindung der Peptide Guanylin bzw. Uroguanylin oder auch hitzestabiler Enterotoxine (STa) wird die intrazelluläre cGMP-Konzentration erhöht, wodurch Signalbansduktionsprozesse innerhalb der Zelle induziert werden.

Neuere Untersuchungen weisen darauf hin, dass sich die Expression von GUCY2C auch auf extraintestinale Bereiche, wie beispielsweise primäre und metastasierende Adenotumore des Magens und des Ösophagus erstreckt (Park et al., Cancer Epidemiol Biomarkers Prev. 11: 739-44, 2002). Eine Spleißvariante des GUCY2C, die sowohl in normalem als auch transformiertem Gewebe des Intestinums gefunden wird, beinhaltet eine 142 bp Deletion im Exon 1, wodurch die Translation eines GUCY2C-ähnlichen Produktes verhindert wird (Pearlman et al., Dig. Dis. Sci. 45:298-05, 2000). Die bisher beschriebene einzige Spleißvariante führt zu keinem Translationsprodukt.

Ein erfindungsgemäßes Ziel war, Tumor-assoziierte Spleißvarianten für GUCY2C zu identifizieren, die sowohl diagnostisch als auch therapeutisch nutzbar sind.

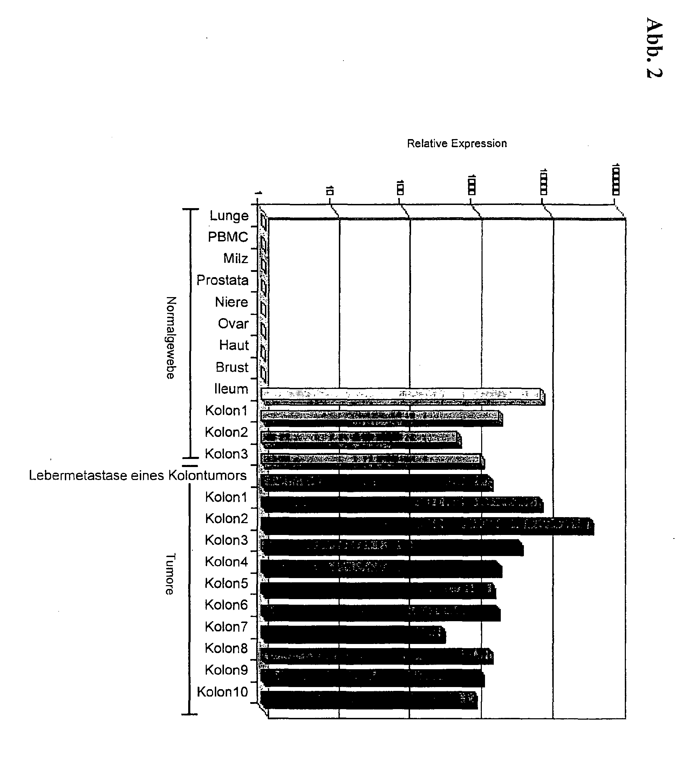

RT-PCR-Untersuchungen mit einem GUCY2C-spezifischen Primerpaar (SEQ ID NO: 22, 23, 98, 99) zeigen eine ausgeprägte Expression von GUCY2C-Transkripten im normalen Kolon und Magen, sowie eine schwache Expression in Leber, Testis, Ovar, Thymus, Milz, Gehirn und Lunge (Tab. 2, Abb. 19). Die Expression in Kolon und Magen war dabei mindestens 50-fach höher als in allen anderen Normalgeweben. Ausgeprägte GUCY2C-Transkript-Spiegel wurden in Kolon- und Magen-Tumoren nachgewiesen (Tab. 2). Diese Ergebnisse wurden durch eine quantitative PCR-Analyse präzisiert und zeigten eine ausgeprägte GUCY2C-Expression im normalen Kolon, Ileum sowie in fast allen untersuchten Kolontumor-Proben (Abb. 2, 19B). In manchen Kolontumor-Proben war eine massive Überexpression nachweisbar. Weiterhin findet sich eine Expression in 7/10 Magentumoren. Darüberhinaus stellten wir überraschenderweise fest, dass das Gen in vielen anderen bisher nicht beschriebenen Tumoren, u.a. Ovarial-, Brust-, Leber- und Prostatatumoren, aktiviert ist (Abb. 19B, Tab. 2).

**Tabelle 2: GUCY2C-Expression in Normal- und Tumorgeweben**

| **Normalgewebe** | **Expression** | | **Tumortyp** | **Expression** |
|---|---|---|---|---|
| Gehirn | + | | Kolon | +++ |
| Cerebellum | | | Pankreas | - |
| Myokard | | | Ösophagus | - |
| Muskel | - | | Magen | +++ |
| Herzmuskel | | | Lunge | - |
| Magen | +++ | | Brust | -+ |
| Kolon (Dickdarm) | +++ | | Ovar | + |
| Pankreas | - | | Endometrium | |
| Niere | - | | HNO | |
| Leber | + | | Niere | |
| Testis | ++ | | Prostata | + |
| Thvmus | + | | Leber | + |
| Brust | - | | | |
| Ovar | + | | | |
| Uterus | + | | | |
| Haut | | | | |
| Lunge | + | | | |
| Schilddrüse | | | | |
| Lymphknoten | - | | | |
| Milz | + | | | |
| PBMC | - | | | |
| Prostata | - | | | |
| | | | | |

Für die Detektion von Spleißvarianten in Kolon- und Kolontumor-Gewebe wurden folgende Primerpaare verwendet: GUCY2C-118s/GUCY2C-498as (SEQ ID NO: 24, 29); GUCY2C-621s/GUCY2C-1140as (SEQ ID NO: 25, 30); GUCY2C-1450s/GUCY2C-1790as (SEQ ID NO: 26, 31); GUCY2C-1993s/GUCY2C-2366as (SEQ ID NO: 27, 32); GUCY2C-2717s/GUCY2C-3200as (SEQ ID NO: 28, 33); GUCY2C-118s/GUCY2C-1140as (SEQ ID NO: 24, 30); GUCY2C-621s/GUCY2C-1790as (SEQ ID NO: 25, 31); GUCY2C-1450s/GUCY2C-2366as (SEQ ID NO: 26, 32); GUCY2C-1993s/GUCY2C-3200as (SEQ ID NO: 27, 33).

Bei der Untersuchung von Spleißvarianten im Kolontumor-Gewebe wurden erfindungsgemäß drei bisher unbekannte Formen identifiziert.
a) Eine Deletion von Exon 3 (SEQ ID NO: 3), die zu einer nur 111 Aminosäuren langen Variante der GUCY2C führt, bei der das Asparagin an Position 111 durch ein Prolin ersetzt ist.
b) Eine Deletion von Exon 6 (SEQ ID NO: 4), die in einem 258 Aminosäuren langen Expressionprodukt resultiert. C-terminal entstünde hierbei ein 13 Aminosäuren umfassendes Neoepitop.
c) Eine Variante, bei der die Nukleotide an den Positionen 1606-1614 bzw. die korespondierenden Aminosäuren L(536), L(537) und Q(538) deletiert sind (SEQ ID NO: 5).

Die erfindungsgemäßen Spleißvarianten mit Deletionen im Exon 3 bzw. Exon 6 (SEQ ID NO: 3, 4) zeichnen sich vor allem dadurch aus, dass die Translationsprodukte (SEQ ID NO: 12, 13) über keine Transmembrandomäne verfügen. Im Fall der Exon 6-Deletion entsteht C-terminal ein Neoepitop von 13 Aminosäuren, welches keinerlei Homologie zu bisher bekannten Proteinen aufweist. Dadurch ist dieses Neoepitop als Zielstruktur für eine Immuntherapie prädestiniert. Die erfindungsgemäße Spleißvariante mit Basendeletionen an den Positionen 1606-1614 (SEQ ID NO: 5) und ihr Translationsprodukt (SEQ ID NO: 14) beinhaltet ebenfalls ein Neopitop. Zum Nachweis des GUCY2C-Proteins wurden Antikörper durch Immunisieren von Kaninchen hergestellt. Folgende Peptide wurden zur Propagierung dieser Antikörper genutzt:
SEQ ID NO: 100: HNGSYEISVLMMGNS (AS 31-45)
SEQ ID NO: 101: NLPTPPTVENQQRLA (AS 1009-1023)

Solche Antikörper können prinzipiell für diagnostische wie auch therapeutische Zwecke genutzt werden.

Insbesondere die extrazelluläre Domäne von GUCY2C (Position der prädizierten extrazellulären Domäne aus der Sequenz von SEQ ID NO:11: AS 454-1073 (SEQ ID NO: 102) kann erfindungsgemäß als Zielstruktur von monoklonalen Antikörpern genutzt werden. Allerdings ist die Strukturvorhersage nicht ganz eindeutig und experimentell noch nicht belegt, so dass auch eine alternative Membranorientierung denkbar ist. In diesem Fall würden die Aminosäuren 1-431 extrazellulär sein und sich als Ansatzpunkt für monoklonale Antikörper eignen. Diese Antikörper binden spezifisch an die Zelloberfläche von Tumorzellen und können sowohl für diagnostische als auch für therapeutische Verfahren genutzt werden. Die Überexpression von GUCY2C, insbesondere in den Kolontumoren, unterstützt einen solchen Einsatz noch zusätzlich. Des weiteren können die für Proteine kodierenden Sequenzen erfindungsgemäß als Vakzine (RNA, DNA, Peptide, Protein) zur Induktion von Tumor-spezifischen Immun-Antworten (T-Zell- und B-Zell-vermittelte Immun-Reaktionen) genutzt werden.

Des weiteren können entsprechend der zellulären Funktion des GUCY2C-Moleküls erfindungsgemäß Substanzen, insbesondere kleine Moleküle entwickelt werden, die die Funktion des Enzyms auf Tumorzellen modulieren. Das Produkt der Enzymreaktion, cGMP, ist ein bekanntes zelluläres Signalmolekül mit unterschiedlichsten Funktionen (Tremblay et al. Mol Cell Biochem 230, 31, 2002).

### Beispiel 3: Identifizierung von SCGB3A2 als diagnostisches und therapeutisches Krebs-Target

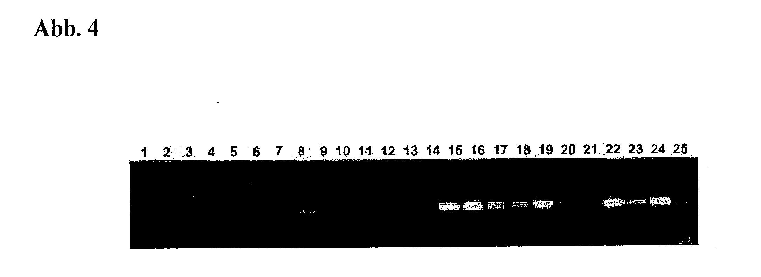

SCGB3A2 (SEQ ID NO: 6) (Translationsprodukt: SEQ ID NO: 15) gehört zur Genfamilie der Sekretoglobine. Die Sequenz ist in der GenBank unter der Zugangsnummer NM_054023 veröffentlicht. SCGB3A2 (UGRP1) ist ein homodimerisches sekretorisches Protein von 17 kDa Größe, das ausschließlich in der Lunge und in den Tracheen exprimiert wird (Niimi et al., Am J Hum Genet 70:718-25, 2002). RT-PCR-Untersuchungen mit einem Primerpaar (SEQ ID NO: 37, 38) bestätigten eine selektive Expression in normalem Lungen-Gewebe. Lungen- und luftröhrenspezifische Gene, z.B. für Surfactant-Proteine, werden in malignen Tumoren im Rahmen der Dedifferenzierung stark herunterreguliert und lassen sich üblicherweise nicht in Lungentumoren nachweisen. Überraschenderweise wurde festgestellt, dass SCGB3A2 in primären und metastasierenden Lungentumoren aktiv ist. Die erfindungsgemäßen Untersuchungen zeigten, dass SCGB3A2 in Lungentumoren stark und frequent exprimiert wird (Abb. 4). Alle anderen getesteten 23 Normalgewebe weisen bis auf Lunge und Trachea keine Expression auf (vgl. Abb. 20).

Dies wurde mit einer spezifischen quantitativen RT-PCR (SEQ ID NO:103, 104) zusätzlich bestätigt (Abb. 20), die zusätzlich in mehr als 50 % der Lungentumoren eine Überexpression von mindestens Faktor 10 aufweist.

Die selektive und hohe Expression von SCGB3A2 im normalen Lungen-Gewebe sowie in Lungentumor-Biopsien kann erfindungsgemäß für molekulare diagnostische Verfahren wie RT-PCR zum Nachweis disseminierender Tumorzellen im Blut und Knochenmark, Sputum, Bronchial-Aspirat oder Lavage und zum Nachweis von Metastasen in anderen Geweben, z.B. in lokalen Lymphknoten, genutzt werden. In der gesunden Lunge wird SCGB3A2 von spezialisierten Zellen ausschliesslich in die Bronchien ausgeschüttet. Dementsprechend ist nicht zu erwarten, dass sich bei gesunden Individuen SCGB3A2-Protein in Körperflüssigkeiten ausserhalb der Atemwege nachweisen lässt. Dagegen sekretieren insbesondere metastasierende Tumorzellen ihre Proteinprodukte direkt in die Blutbahn. Ein Aspekt der Erfindung betrifft daher die Detektion von SCGB3A2-Produkten im Serum oder Plasma von Patienten über einen spezifischen Antikörpertest als diagnostischer Befund für Lungentumoren.

Zum Nachweis von SCGB3A2-Protein wurden Antikörper durch Immunisieren von Kaninchen hergestellt. Folgende Peptide wurden zur Propagierung dieser Antikörper genutzt:
SEQ ID NO: 105: LINKVPLPVDKLAPL
SEQ ID NO: 106: SEAVKKLLEALSHLV

Eine SCGB3A2-spezifische Reaktion konnte in der Immunfluoreszenz nachgewiesen werden (Abb. 21). Wie für ein sezerniertes Protein erwartet, ergab sich eine Verteilung von SCGB3A2 in der Zelle, die dem endoplasmatischem Retikulum und Sekretionsgranula zugeordnet werden konnte (Abb. 21A). Zur Spezifitätskontrolle wurden die Zellen parallel mit einem Plasmid transfiziert, dass ein SCGB3A2-GFP-Fusionsprotein synthetisiert. Der Proteinnachweis erfolgte hier über das autofluoreszierende GFP (grünes fluoreszierendes Protein) (Abb. 21B). Eine Überlagerung beider Fluoreszenzbilder zeigt eindeutig, dass das Immunserum spezifisch SCGB3A2-Protein erkennt (Abb. 21 C).

Solche Antikörper können erfindungsgemäß z.B. in Form von Immuntests für diagnostische wie auch therapeutische Zwecke genutzt werden.

### Beispiel 4: Identifizierung von Claudin-18A1- und Claudin-18A2-Spleißvarianten als diagnostische und therapeutische Krebs-Targets

Das" Claudin-18-Gen kodiert für ein Oberflächenmembranmolekül mit 4 hydrophoben Bereichen. Entsprechend der Prädiktionsprogramme (TMHMM, TMPred) und der für viele andere Mitglieder dieser Familie beschriebenen Topologie zufolge besitzt Claudin-18 vier Transmembran- und somit zwei extrazelluläre Domänen EX1 und EX2, deren extrazelluläre Lokalisation (Konformation 1) in Abb. 22 dargestellt ist. Die Domäne D3, die zwischen den beiden extrazellulären Epitopen lokalisiert ist, ist in der Literatur für Claudin-18 und andere Mitglieder dieser Familie als intrazellulär beschrieben und wird mit herkömmlichen Prädiktionsprogrammen auch so prädiziert. Der N- wie auch C-Terminus sind intrazellulär. Niimi und Kollegen (Mol. Cell. Biol. 21:7380-90, 2001) beschrieben zwei Spleißvarianten des Maus- und humanen Claudin-18, die als selektiv in Lungengewebe (Claudin-18A1) bzw. in Magengewebe (Claudin-18A2) exprimiert beschrieben wurden. Diese Varianten unterscheiden sich im N-Terminus.

Erfindungsgemäß wurde untersucht, inwieweit die Spleißvarianten Claudin-18A2 (SEQ ID NO: 7) und Claudin-18A1 (SEQ ID NO: 117) sowie ihre jeweiligen Translationsprodukte (SEQ ID NO: 16 und 118) als Marker bzw. therapeutische Zielstrukturen für Tumoren genutzt werden können. Es wurde eine quantitative PCR etabliert, die zwischen beiden Varianten unterscheiden kann, indem A1-spezifische (SEQ ID NO: 109, 110) bzw. A2-spezifische (SEQ ID NO: 107, 108) Primerpaare ausgewählt wurden. Die Spleißvariante A2 wurde zusätzlich mit einem zweiten Primerpaar in einer konventionellen PCR getestet (SEQ ID NO: 39, 40). Für die Variante A1 ist beschrieben, dass sie nur in gesundem Lungengewebe aktiv ist. Jedoch stellten wir erfindungsgemäß überraschenderweise fest, dass die Variante A1 auch in der Magenschleimhaut aktiv ist (Abb. 23). Magen und Lunge sind die einzigen Normalgeweben, die eine signifikante Aktivierung aufweisen. Alle anderen Normalgewebe sind negativ für Claudin-A1. Bei der Untersuchung von Tumoren wurde überraschend festgestellt, dass Claudin-A1 in einer Vielzahl von Tumorgeweben stark aktiviert ist. Ein besonders starke Expression findet sich in Magentumoren, Lungentumoren, Pankreastumoren, Ösophagustumoren (Abb. 23), HNO-Tumoren und Prostatatumoren. Die Expressionsspiegel von Claudin-A1 in HNO-, Prostata-, Pankreas- und Ösophagustumoren sind 100-10000 höher als die Spiegel in den korrespondierenden Normalgeweben. Für die Untersuchung der Claudin-A2-Spleißvariante wurden Oligonukleotide verwendet, die spezifisch die Amplifikation dieses Transkripts ermöglichen (SEQ ID NO: 39, 40 bzw. 107, 108). Die Untersuchung ergab, dass die Spleißvariante A2 in keinem der mehr als 20 untersuchten Normalgewebe außer in Magenschleimhaut und in geringem Ausmaß auch Testisgewebe exprimiert wird (Abb. 24). Wir stellten fest, dass wie die Variante A1 auch die Variante A2 in , vielen Tumoren aktiviert ist (Abb. 24). Hierzu zählen Magentumore, Pankreastumore, Ösophagustumore und Lebertumore. Obwohl sich in gesunder Lunge keine Aktivierung von Claudin-18A2 nachweisen lässt, wurde überraschend festgestellt, dass ein Teil der Lungentumoren die Spleißvariante A2 exprimieren.

**Tabelle 3A. Expression von Claudin-18A2 in Normal- und Tumor-Geweben**

| **Normalgewebe** | **Expression** | | **Tumortyp** | **Expression** |
|---|---|---|---|---|
| Gehirn | - | | Kolon | - |
| Cerebellum | - | | Pankreas ++ | ++ |
| Myokard | - | | Ösophagus ++ | ++ |
| Skelettmuskel | - | | Magen +++ | +++ |
| Endometrium | - | | Lunge ++ | ++ |
| Magen | +++ | | Brust - | - |
| Kolon | - | | Ovar - | - |
| Pankreas | - | | Endometrium n.u. | n.u. |
| Niere | - | | HNO ++ | ++ |
| Leber | - | | Niere - | - |
| Testis (Hoden) | + | | Prostata - | - |
| Thymus | - | | | |
| Brust | - | | | |
| Ovar | - | | | |
| Uterus | - | | | |
| Haut | - | | | |
| Lunge | - | | | |
| Schilddrüse | - | | | |
| Lymphknoten | - | | | |
| Milz | - | | | |
| TPBMC | - | | | |
| Ösophagus | - | | | |
| | | | | |

**Tabelle 3B. Expression von Claudin-18A1 in Normal- und Tumor-Geweben**

| **Normalgewebe** | **Expression** | | **Tumortyp** | **Expression** |
|---|---|---|---|---|
| Gehirn | - | | Kolon | - |
| Cerebellum | - | | Pankreas | ++ |
| Herzmuskel | - | | Ösophagus | ++ |
| Muskel | - | | Magen | +++ |
| Endometrium | - | | Lunge | ++ |
| Magen | +++ | | Brust | + |
| Kolon | - | | Ovar | n.u |
| Pankreas | - | | Endometrium | n.u. |
| Niere | - | | HNO | ++ |
| Leber | - | | Niere | - |
| Testis | + | | Prostata | ++ |
| Thymus | - | | | |
| Brust | - | | | |
| Ovar | - | | | |
| Uterus | - | | | |
| Haut | - | | | |
| Lunge | +++ | | | |
| Schilddrüse | - | | | |
| Lymphknoten | - | | | |
| Milz | - | | | |
| PBMC | - | | | |
| Ösophagus | - | | | |
| | | | | |

Auch die konventionelle PCR als unabhängige Kontrolluntersuchung bestätigte die Resultate der quantitativen PCR. Hierzu wurden Oligonukleotide (SEQ ID NO: 39, 40) verwendet, die eine spezifische Amplifikation der Spleißvariante A2 erlauben. Erfindungsgemäß wurde gezeigt, dass die meisten Magentumore und die Hälfte der getesteten Pankreastumore eine starke Expression dieser Spleißvariante aufweisen (Abb. 5). Dagegen ist eine Expression mit konventioneller PCR in anderen Geweben nicht nachweisbar. Insbesondere findet sich in wichtigen Normalgeweben wie Lunge, Leber, Blut, Lymphknoten, Brust und Niere keine Expression (Tab. 3). Hiermit stellen die Spleißvarianten erfindungsgemäß hochspezifische molekulare Marker für Tumoren des oberen Magendarmtraktes wie auch Lungentumoren, HNO-Tumoren, Prostatatumoren und ihre Metastasen dar. Diese molekulare Marker können erfindungsgemäß zum Nachweis von Tumorzellen genutzt werden. Die Detektion der Tumoren kann erfindungsgemäß mit den genannten Oligonukleotiden (SEQ ID NO: 39, 40, 107-110) erfolgen. Als Oligonukleotide eignen sich insbesondere Primerpaare, von denen mindestens einer unter stringenten Bedingungen an einen 180 Basenpaare langen Abschnitt des Transkripts bindet, der spezifisch für die eine (SEQ ID NO: 8) oder andere Spleißvariante (SEQ ID NO: 119) ist.

Diese Genprodukte sind attraktive therapeutische Zielstukturen, da durch ihr Fehlen in den meisten toxizitätsrelevanten Organen an diesen keine Nebenwirkungen zu erwarten sind, während die starke Aktivierung in Zellen der genannten Krebsarten eine gute Bindung an diese und Vermittlung ensprechender zellschädigender Effekte erwarten lässt.

Um diese Daten auf Proteinebene zu bestätigen, wurden Claudin-spezifische Antikörper bzw. Immunseren durch Immunisierung von Tieren generiert.

Die N-terminale extrazelluläre Domäne EX1 unterscheidet sich in der Sequenz bei den beiden Spleißvarianten A1 und A2 (SEQ ID NO: 111 für A1 und SEQ ID NO: 112 für A2). Die C-terminale extrazelluläre Domäne EX2 ist für beide Varianten identisch (SEQ ID NO: 137). Bisher sind noch keine Antikörper beschrieben, die an die extrazelluläre Domänen von Claudin-18 binden. Auch wurden bisher keine Antikörper beschrieben, die spezifisch zwischen A1- und A2-Varianten unterscheiden können. Erfindungsgemäß wurden für die Immunisierung zur Generierung von Antikörpern extrazellulär gelegene Peptidepitope und Proteinfragemente ausgewählt, die spezifisch für die Variante A1 oder A2 sind bzw. in beiden Varianten vorkommen. Unter anderem wurden zur Immunisierung folgende Peptide für die Herstellung von Antikörpern ausgewählt:
SEQ ID NO: 17: DQWSTQDLYN (N-terminal-extrazelluläre Domäne, A2-spezifisch, Bindung unabhängig von Glykosylierung)
SEQ ID NO: 18: NNPVTAVFNYQ (N-terminal-extrazelluläre Domäne, A2-spezifisch, Bindung hauptsächlich an unglykosylierte Form, N37)
SEQ ID NO: 113: STQDLYNNPVTAVF (N-terminal-extrazelluläre Domäne, A2-spezifisch, Bindung nur an nicht glykosylierte Form, N37)
SEQ ID NO: 114: DMWSTQDLYDNP (N-terminal-extrazelluläre Domäne, A1-spezifisch)
SEQ ID NO: 115: CRPYFTILGLPA (N-terminal-extrazelluläre Domäne, hauptsächlich spezifisch für A1)
SEQ ID NO: 116: TNFWMSTANMYTG (C-terminal-extrazelluläre Domäne, erkennt sowohl A1 als auch A2).

Wir konnten unter anderem Antikörper generieren, die selektiv die N-terminale Domäne der Spleißvariante Claudin-18-A1 erkennen, aber nicht die A2-Variante (Abb. 28). Durch Nutzung von Epitopen für Immunisierungen, welche in der C-terminal-extrazellulären Domäne liegen, die identisch in beiden Spleißvarianten ist, konnten wir Antikörper herstellen, die beide Varianten erkennen (Abb. 27).

Beispielhaft werden die Daten für einen A2-spezifischen Antikörper, der durch Immunisierung mit SEQ ID NO: 17 hergestellt wurde, dargestellt. Der spezifische Antikörper lässt sich unter verschiedenen Fixierungsbedingungen für Immunfluoreszenz-Untersuchungen nutzen. Bei vergleichenden Färbungen von RT-PCR-positiven wie auch negativen-Zelllinien ist das entsprechende Protein in gut nachweisbarer Menge spezifisch u.a. in den als positiv typisierten Magentumor-, Ösophagustumor- und Pankreastumor-Zelllinien detektierbar (Abb. 25). Das endogene Protein ist membranlokalisiert und bildet größere fokale Aggregate an der Membran (Abb. 25). Mit diesem Antikörper wurden immunhistochemische Färbungen an menschlichen Geweben durchgeführt. Wir bestätigten die selektive Gewebeverteilung dieses Proteins. Ein umfangreiches Sortiment verschiedender Normalgewebe wurde untersucht, von denen in fast allen Claudin-18A2-Protein nicht nachweisbar ist, wie beispielhaft für Leber, Lunge, Niere und Kolon dargestellt wurde. Wir fanden lediglich eine Aktivierung dieses Proteins in normalem Magengewebe (Abb. 32). Überraschenderweise sahen wir, dass die A2-Variante von Claudin-18 zwar in den differenzierten Zellen der Magenschleimhaut nachweisbar ist, aber nicht in den Stammzellen. Differenzierte Magenschleimhautzellen sind einer ständigen Erneuerung unterworfen. Physiologischerweise wird das gesamte Magenepithel ausgehend von den Stammzellen des Magens kontinuierlich ersetzt. Dies unterstützt die Nutzbarkeit der A2-Variante als therapeutische Zielstuktur, da wir erfindungsgemäß zeigen, dass Stammzellen des Magens als die indispensible Zellpopulation der Magenschleimhaut genauso wie alle anderen gesunden Organe die A2-Variante nicht tragen und somit von einer spezifisch gegen die A2-Variante gerichteten Substanz nicht angegriffen werden können. Wir wiesen in einer Reihe humaner Tumoren mittels dieses Antikörpers die A2-Variante von Claudin-18 nach (Abb. 33), insbesondere in Tumoren von Magen, Ösophagus und Lunge, die uns bereits in RT-PCR-Untersuchungen aufgefallen waren. Erfindungsgemäß sind diese Tumoren therapeutisch zugänglich.

Der oben beschriebene Antikörper wurde des Weiteren für einen Proteinnachweis im Western-Blot eingesetzt. Erwartungsgemäß wird Protein lediglich in Magen und keinem anderen Normalgewebe, auch nicht Lunge, in der lediglich die A1-Variante aktiviert ist, detektiert (Abb. 29). Bei der vergleichenden Färbung von Magentumoren und adjazentem normalem Magengewebe aus Patienten fiel überraschend auf, dass in allen Magentumoren, in denen Claudin-18A2 detektiert wird, dieses Protein ein kleineres Massengewicht hat (Abb. 30, links). In einer Serie von Experimenten wurde erfindungsgemäß festgestellt, dass eine Bande auf dieser Höhe sich dadurch ergibt, dass man Lysat normalen Magengewebes mit dem deglykosylierenden Agens PNGase F behandelt (Abb. 30, rechts). Während in allen normalen Magengeweben ausschließlich die glykosylierte Form der Variante A2 nachweisbar ist, ist A2 als solches in über 60% der untersuchten Magentumore, und zwar ausschließlich in der deglykosylierten Form, nachweisbar. Obwohl die A2-Variante von Claudin-18 in normaler Lunge auch auf Proteinebene nicht detektiert wird, ist sie wie auch schon in der quantitativen RT-PCR in Lungentumoren zu finden. Auch hier liegt-lediglich die deglykosylierte Variante vor (Abb. 31).

Claudin-18 selbst ist ein hochselektives Differenzierungsantigen von Magen (Variante A2) bzw. von Lunge und Magen (Variante A1). Unsere Daten weisen daraufhin, dass es offensichtlich von Tumor-assoziierten Veränderungen der Glykosylierungmaschinerie betroffen ist und in Tumoren eine besondere Form der Variante A2 entsteht, die deglykosyliert ist. Die Resultate der PNGaseF-Behandlung zeigen, dass Claudin-18A2 sich in Tumor-und Normalgewebe hinsichtlich seiner N-Glykosylierung unterscheidet.

Die Glykosylierung eines Epitops kann die Bindung eines für dieses Epitop-spezifischen Antikörpers verhindern und im vorliegenden Fall dazu beitragen, dass ein solcher Antikörper nicht an Claudin-18A2 in Normalgeweben binden kann, sondern exklusiv an die nicht-glykosylierte Form in Krebszellen. Um erfindungsgemäß Antikörper herzustellen, die exklusiv an nicht-glykosylierte Epitope binden, wurde dies bei der Auswahl der Immunogene berücksichtigt. Erfindungsgemäß haben wir verschiedene Regionen von Claudin-18A2 identifiziert, die in Tumor- und Normalgewebe unterschiedlich glykosyliert vorliegen können. Als potenzielle Glykosylierungsstellen für Claudin-18A2 haben wir u.a. die Regionen, die die Aminosäuren 37, 38, 45, 116, 141, 146, 205 von Claudin-18A2 umfassen, identifiziert (Abb. 22 unten). Erfindungsgemäß unterscheiden sich Tumorzellen und Normalgewebe durch eine unterschiedliche Glykosylierung in einer oder mehrerer dieser Positionen. Die meisten dieser Bereiche stellen keine klassische Glykosylierungsstelle dar, aber enthalten Asparagin, Serin und Threonin, die in seltenen Fällen auch glykosyliert werden können (Prädiktion Abb. 22 unten). Beide Varianten von Claudin-18 haben ein einziges klassisches Glykosylierungsmotiv in der D3-Domäne, die in der Literatur und entsprechend gängigen Prädiktionsalgorithmen als intrazellulär gilt

Allerdings konnte bei einem strukturell dem Claudin-18 ähnlichen Tetraspanin, dem PMP 22, gezeigt werden, dass die hydrophoben Membrandomänen 2 und 3 von PMP 22 nicht vollständig durch die Zellmembran laufen, sondern nur partiell in die Plasmamembran interkalieren (Taylor et al., J Neurosc. Res. 62:15-27, 2000). Aus diesem Grund ist der gesamte Bereich zwischen den zwei äusseren Transmembrandomänen bei PMP22 extrazellulär lokalisiert. Wir haben die Möglichkeit einer solchen Topologie für Claudin-18A2 hypothetisiert und überprüft. Hierzu haben wir 3 Konstrukte hergestellt, die jeweils eine Marker-Sequenz (His- oder HA-Tag) in einer der Domänen EX1, EX2, oder D3 trugen (Abb. 42 oben). Diese wurden in Zelllinien transfiziert und daraufhin getestet, ob ein gegen diese Markersequenzen gerichteter Antikörper an nichtpermeabilisierte Zellen bindet, was erfordert, dass der entsprechende Bereich des Proteins topologisch extrazellulär ist. Da alle drei Bereiche des Moleküls durchflusszytometrisch als extrazellulär gemessen wurden (Abb. 42 unten), konnten wir bestätigen, dass Claudin-18A2 in einer Konformation mit zwei Transmembrandomänen und einer großen extrazellulär gelegenen Domäne vorliegen kann (Abb. 22, Konformation 2). Diese Konformation ist biochemisch und therapeutisch relevant, da sie zusätzliche Bindungsstellen für therapeutische Antikörper (SEQ ID NO: 142, 143) beinhaltet.

Erfindungsgemäß und vorzugsweise können Antikörper hergestellt werden, die zwischen glykosylierten und nicht glykosylierten Varianten von Claudin-18A2 unterscheiden. Diese haben eine besonders hohe Spezifität für Tumorzellen. Bei der Herstellung glykosilierungsspezifischer Antikörper haben wir neben den Glykosylierungsdomänen auch diese unterschiedlichen Konformationsoptionen berücksichtigt.

Vorzugsweise, aber nicht ausschließlich, eignen sich Proteinfragmente aus dem D3-Bereich von Claudin-18A2 zur Immunisierung von Tieren. Beispielhaft ist dies für zwei Antikörper mAB1 und mAB2 dargestellt (Abb. 44). Wir untersuchten die Bindeeigenschaften dieser Antikörper an Zelllinien, die entweder die A1- oder die A2-Variante des Claudin-18 exprimieren. Wir konnten zeigen, dass Claudin-18A2 für Antikörper auf der Zelloberfläche zugänglich ist. Erfindungsgemäss sind solche Antikörper spezifisch für die A2-Variante und binden nicht an die A1-Variante (Abb. 44). Wir haben kurze Fremdsequenzen (myc-tag) jeweils in den Bereich der Extrazellulärdomänen Ex1 und Ex2 eingeführt. Am Beispiel von mAB1 ist dargestellt, dass die Bindeeigenschaften des Antikörpers dadurch nicht beeinträchtigt werden und das eigentliche Epitop sich in der D3-Domäne befindet.

Die generierten Antikörper können diagnostisch wie auch therapeutisch genutzt werden. Immunseren wie das hier beschriebene (gegen Peptid SEQ ID NO: 17) können z.B. im Western-Blot diagnostisch genutzt werden. Erfindungsgemäß können durch Immunisierung mit Peptiden, die mindestens eine diese Regionen beinhalten (z.B. Peptid SEQ ID NO: 113 (Abbildung 26), Peptide SEQ ID NO: 142-145), Antikörper hergestellt werden, die an das glykosylierte Epitop gar nicht binden können. Erfindungsgemäß binden derartige Antikörper spezifisch an die deglykosylierten Epitope auf Tumorzellen. Die im Vergleich zu Normalgeweben fehlende Glykosylierung an einer der genannten Positionen könnte auch durch eine sekundäre, endogene Deglykosylierung in Tumorzellen bedingt sein. Eine derartige Deglykosylierung ist mit einer Asn (N)→ Asp (D) Transformation der betreffenden Aminosäure assoziiert. Für die Herstellung von Antikörpern gegen derartig veränderte tumorasssoziierte Varianten können daher erfindunggemäß Claudin-18A2-derivierte Peptide genutzt werden, in denen die Aminosäure Asn (N) an mindestens einer der Positionen 37, 38, 45, 116, 141, 146, 205 des Claudin-18A2-Peptids durch ein Asp (D) ersetzt ist (z.B. SEQ ID NO: 146-150). Insbesondere können solche Antikörper therapeutisch eingesetzt werden, da sie hochselektiv für Tumorzellen sind. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigenspezifischen T-Lymphozyten stellen die Aminosäuren 1-47 (SEQ ID NO: 19 und 120) besonders gute Epitope dar.

### Beispiel 5: Identifizierung von SLC13A1 als diagnostisches und therapeutisches Krebs-Target

SLC13A1 gehört zur Familie der Natrium-Sulfat-Cotransporter. Das humane Gen ist im Gegensatz zum Maus-Homolog dieses Gens selektiv in der Niere exprimiert (Lee et al., Genomics 70:354-63, 2000). SLC13A1 kodiert für ein Protein von 595 Aminosäuren und enthält 13 putative Transmembran-Domänen. Durch alternatives Spleißen entstehen 4 verschiedene Transkripte (SEQ ID NO: 41-44) und seine entsprechenden Translationsprodukte (SEQ ID NO: 45-48). Es wurde untersucht, ob SLC13A1 als Marker für Nierentumore genutzt werden kann. Hierzu wurden Oligonukleotide (SEQ ID NO: 49, 50) verwendet, die eine spezifische Amplifikation von SLC13A1 ermöglichen.

**Tabelle 4. Expression von SLC13A1 in Normal- und Tumorgeweben**

| Normalgewebe | Expression | | Tumortyp | Expression |
|---|---|---|---|---|
| Gehirn | - | | Kolon | nd |
| Cerebellum | nd | | Pankreas | nd |
| Myokard | nd | | Ösophagusr | nd |
| Muskel | nd | | Magen | nd |
| Herzmuskel | - | | Lunge | nd |
| Magen | - | | Brust | nd |
| Kolon | - | | Ovar | nd |
| Pankreas | nd | | Endometrium | nd |
| Niere | +++ | | HNO | nd |
| Leber | - | | Niere | +++ |
| Testis | + | | Prostata | nd |
| Thymus | - | | | |
| Brust | - | | | |
| Ovar | - | | | |
| Uterus | nd | | | |
| Haut | nd | | | |
| Lunge | - | | | |
| Schilddrüse | - | | | |
| Lymphknoten | - | | | |
| Milz | - | | | |
| PBMC | - | | | |
| Sigma | - | | | |
| Ösophagus | - | | | |

RT-PCR-Untersuchungen mit einem SLC13A1-spezifischen Primerpaar (SEQ ID NO: 49, 50) bestätigten eine nahezu selektive Expression in der Niere, und zeigten erfindungsgemäß eine hohe Expression in nahezu allen (7/8) untersuchten Nierentumor-Biopsien (Tab. 4, Abb. 6). Auch quantitative RT-PCR-Untersuchungen mit spezifischen Primern (SEQ ID NO: 121, 122) bestätigen diese Daten (Abb. 34). Schwache Signale waren in folgenden Normalgeweben nachweisbar: Kolon, Magen, Testis, Brust, Leber und Gehirn. Die Expression in Nierentumoren war aber mindestens 100-fach höher als in allen anderen Normalgeweben,

Um die subzelluläre Lokalisation von SLC13A1 in der Zelle zu analysieren, wurde das Protein mit GFP als Reportermolekül fusioniert und nach Transfektion des entsprechenden Plasmids heterolog in 293-Zellen exprimiert. Anschließend wurde die Lokalisation im Fluoreszenzmikroskop analysiert. Unsere Daten bestätigen nachdrücklich, dass SLC13A1 ein integrales Transmembranmolekül ist (Abb. 35).

Zum Nachweis des SLC13A1-Proteins wurden Antikörper durch Immunisieren von Kaninchen hergestellt. Zur Propagierung dieser Antikörper wurden die Peptide der SEQ ID NO: 123 und 124 genutzt. Solche Antikörper können prinzipiell für diagnostische wie auch therapeutische Zwecke genutzt werden.

Das SLC13A1-Protein hat 13 Transmembrandomänen und 7 extrazelluläre Regionen. Insbesondere diese extrazellulären Domänen von SLC13A1 können erfindungsgemäß als Zielstrukturen von monoklonalen Antikörpern genutzt werden. SLC13A1 ist als Kanalprotein an dem Transport von Ionen beteiligt. Die extrazellulären Domänen von SLC13A1 in der gesunden Niere sind polar in Richtung Harnwege (luminal) gerichtet. Therapeutisch eingesetzte hochmolekulare monoklonale Antikörper werden jedoch nicht in die Harnwege ausgeschieden, so dass keine Bindung an SLC13A1 in der gesunden Niere stattfindet. Dagegen ist die Polarität von SLC13A1 in Tumorzellen aufgehoben und das Protein direkt über den Blutkreislauf für Antikörper zugänglich. Die ausgeprägte Expression und hohe Inzidenz von SLC13A1 in Nierentumoren machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark, Urin, sowie die Detektion von Metastasen in anderen Organen mittels RT-PCR. Des weiteren können die extrazellulären Domänen von SLC13A1 erfindungsgemäß als Zielstruktur zur Immun-Diagnostik und Therapie mittels monoklonaler Antikörper verwendet werden. Des weiteren kann SLC13A1 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compounds", die die biologische Aktivität von SLC13A1 modulieren und zur Therapie von rehalen Tumoren eingesetzt werden können.

### Beispiel 6: Identifizierung von CLCA1 als diagnostisches und therapeutisches Krebs-Target

CLCA1 (SEQ ID NO: 51; Translationsprodukt: SEQ ID NO: 60) gehört zur Familie der Ca⁺⁺-aktivierten Cl⁻-Kanäle. Die Sequenz ist in der Genbank unter der Zugangs-Nr. NM_001285 veröffentlicht. CLCA1 ist ausschließlich im intestinalen Kryptenepithel und in den Becherzellen exprimiert (Gruber et al., Genomics 54:200-14, 1998). Es wurde untersucht, ob CLCA1 als Marker für Kolon- und Magentumoren genutzt werden kann. Hierzu wurden Oligonukleotide (SEQ ID NO: 67, 68) verwendet, die eine spezifische Amplifikation von CLCA1 ermöglichen. RT-PCR-Untersuchungen mit diesem Primer-Set bestätigten eine selektive Expression im Kolon, und zeigten erfindungsgemäß eine hohe Expression in 3/7 untersuchten Kolon- und 1/3 untersuchten Magentumor-Proben (Abb. 7). Die übrigen Normalgewebe zeigten keine oder nur eine sehr schwache Expression. Dies wurde mit einer spezifischen quantitativen RT-PCR (SEQ ID NO: 125, 126) zusätzlich bestätigt, wobei in den analysierten Normalgeweben keine Expression nachgewiesen werden konnte (Abb. 36). Bei den in diesem Experiment untersuchten Tumorproben waren 6/12 Kolontumor-Proben und 5/10 Magentumor-Proben positiv für CLCA1. Insgesamt scheint die Expression des Genes in Tumoren dysreguliert zu sein. Neben sehr stark exprimierenden Proben war CLCA1 in anderen Proben deutlich herunterreguliert.

Für das Protein sind 4 Transmembrandomänen mit insgesamt 2 extrazellulären Regionen prädiziert. Insbesondere diese extrazellulären Domänen von CLCA1 können erfindungsgemäß als Zielstrukturen von monoklonalen Antikörpern genutzt werden.

Die ausgeprägte Expression und die hohe Inzidenz von CLCA1 für Magen- und Kolontumore machen dieses Protein erfindungsgemäß zu einem interessanten diagnostischen und therapeutischen Marker. Dies umfasst erfindungsgemäß den Nachweis disseminierter Tumorzellen im Serum, Knochenmark, Urin, sowie die Detektion von Metastasen in anderen Organen mittels RT-PCR. Des weiteren können die extrazellulären Domänen von CLCA1 erfindungsgemäß als Zielstruktur zur Immun-Diagnostik und Therapie mittels monoklonaler Antikörper verwendet werden. Des weiteren kann CLCA1 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compounds", die die biologische Aktivität als Transportprotein von CLCA1 modulieren und zur Therapie von gastrointestinalen Tumoren eingesetzt werden können.

### Beispiel 7: Identifizierung von FLJ21477 als diagnostisches und therapeutisches Krebs-Target

FLJ21477 (SEQ ID NO: 52) und sein prädiziertes Translationsprodukt (SEQ ID NO: 61) wurde als hypothetisches Protein in der Genbank unter der Zugangs-Nr. NM_025153 veröffentlicht. Es handelt es sich um ein integrales Membranprotein mit ATPase-Aktivität und 4 Transmembrandomänen, das entsprechend für die Therapie mit spezifischen Antikörpern geeignet ist. RT-PCR-Untersuchungen mit FLJ21477-spezifischen Primern (SEQ ID NO: 69, 70) zeigten eine selektive Expression im Kolon, und darüber hinaus unterschiedlich stark ausgeprägte Expression in 7/12 untersuchten Kolontumor-Proben (Abb. 8). Die übrigen Normalgewebe zeigten keine Expression. Dies wurde mit einer spezifischen quantitativen RT-PCR (SEQ ID NO: 127, 128) zusätzlich bestätigt. Sowohl in Kolon (Abb. 37A) als auch in 11/12 Kolontumoren war eine FLJ21477-spezifische Expression nachweisbar. Neben der Expression in Kolongewebe konnte zusätzlich eine Expression in Magengewebe nachgewiesen werden. Außerdem war unter den Bedingungen der quantitativen RT-PCR eine im Vergleich mit Kolon und Magen deutlich schwächere Expression in Gehirn, Thymus und Ösophagus nachweisbar (Abb. 37A). Zusätzlich konnte außerdem in den folgenden Tumorproben eine FLJ21477-spezifische Expression nachgewiesen werden: Magen, Pankreas, Ösophagus und Leber.

Für das Protein sind 4 Transmembrandomänen mit insgesamt 2 extrazellulären Regionen prädiziert. Insbesondere diese extrazellulären Domänen von FLJ21477 können erfindungsgemäß als Zielstrukturen von monoklonalen Antikörpern genutzt werden.

Die Expression und hohe Inzidenz von FLJ21477 für Magen- und Kolontumore machen dieses Protein erfindungsgemäß zu einem wertvollen diagnostischen und therapeutischen Marker. Dies umfasst erfindungsgemäß den Nachweis disseminierter Tumorzellen im Serum, Knochenmark, Urin, sowie die Detektion von Metastasen in anderen Organen mittels RT-PCR. Des Weiteren können die extrazellulären Domänen von FLJ21477 erfindungsgemäß als Zielstruktur zur Immun-Diagnostik und Therapie mittels monoklonaler Antikörper verwendet werden. Des weiteren kann FLJ21477 erfindungsgemäß als Vakzine (RNA, DNA, Protein, Peptide) zur Induktion Tumor-spezischer Immunantworten (T-und B-Zell vermittelte Immunreaktionen) eingesetzt werden.

### Beispiel 8: Identifizierung von FLJ20694 als diagnostisches und therapeutisches Krebs-Target

FLJ20694 (SEQ ID NO: 53) und sein Translationsprodukt (SEQ ID NO: 62) wurden als hypothetisches Protein in der Genbank unter der Zugangs-Nr. NM_017928 veröffentlicht. Bei diesem Protein handelt es sich um ein integrales Transmembranmolekül (Transmembrandomäne AS 33-54), höchstwahrscheinlich mit Thioredoxinfunktion. RT-PCR-Untersuchungen mit FLJ20694-spezifischen Primern (SEQ ID NO: 71, 72) zeigten eine selektive Expression im Kolon, und darüber hinaus unterschiedlich stark ausgeprägte Expression in 5/9 untersuchten Kolontumor-Proben (Abb. 9). Die übrigen Normalgewebe zeigten keine Expression. Dies wurde mit einer spezifischen quantitativen RT-PCR (SEQ ID NO: 129, 130) zusätzlich bestätigt (Abb. 38). In keinem anderen Normalgewebe außer in Kolon und Magen (im ersten Experiment nicht analysiert) konnte eine FLJ20694-Expression nachgewiesen werden.

Für das Protein ist eine Transmembrandomäne mit einer extrazellulären Region prädiziert. Insbesondere diese extrazellulären Domänen von FLJ20694 können erfindungsgemäß als Zielstrukturen von monoklonalen Antikörpern genutzt werden.

Des weiteren kann FLJ20694 erfindungsgemäß als Vakzine (RNA, DNA, Protein, Peptide) zur Induktion Tumor-spezifischer Immunantworten (T-und B-Zell vermittelte Immunreaktionen) eingesetzt werden. Dies umfasst erfindungsgemäß auch die Entwicklung von sogenannten "small compounds", die die biologische Aktivität von FLJ20694 modulieren und zur Therapie von gastrointestinalen Tumoren eingesetzt werden können.

### Beispiel 9: Identifizierung des von Ebner-Proteins (c20orf114) als diagnostisches und therapeutisches Krebs-Target

Die von Ebner-Protein-kodierende mRNA (SEQ ID NO: 54) und sein Translationsprodukt (SEQ ID NO: 63) wurden als Plunc-verwandtes Protein der oberen Luftwege und des Nasen-Rachen-Epithels in der Genbank unter der Zugangs-Nr. AF364078 veröffentlicht. Erfindungsgemäß wurde untersucht, ob das von Ebner-Protein als Marker von Lungentumoren genutzt werden kann. Hierzu wurden Oligonukleotide (SEQ ID NO: 73, 74) verwendet, die eine spezifische Amplifikation des Ebner-Proteins-kodierender cDNA ermöglichen. RT-PCR-Untersuchungen mit diesem Primer-Set zeigten eine selektive Expression in der Lunge und in 5/10 untersuchten Lungentumor-Proben (Abb. 10). Innerhalb der Gruppe der Normalgewebe zeigte sich auch eine Expression im Magen. Die übrigen Normalgewebe zeigten keine Expression.

### Beispiel 10: Identifizierung von Plunc als diagnostisches und therapeutisches Krebs-Target

Plunc (SEQ ID NO: 55) und sein Translationsprodukt (SEQ ID NO: 64) wurden in der Genbank unter der Zugangs-Nr. NM_016583 veröffentlicht. Die humane Plunc-mRNA kodiert für ein Protein von 256 Aminosäuren und weist eine 72%ige Homologie mit dem murinen Plunc-Protein auf (Bingle und Bingle, Biochim Biophys Acta 1493:363-7, 2000). Die Exression von Plunc beschränkt sich auf die Trachea, die oberen Luftwege, Nasen-Rachen-Epithel und Speicheldrüse.

Erfindungsgemäß wurde untersucht, ob Plunc als Marker von Lungentumoren genutzt werden kann. Hierzu wurden Oligonukleotide (SEQ ID NO: 75, 76) verwendet, die eine spezifische Amplifikation von Plunc ermöglichen.

RT-PCR-Untersuchungen mit diesem Primer-Set zeigten eine selektive Expression im Thymus, in der Lunge und in 6/10 untersuchten Lungentumor-Proben (Abb. 11). Die übrigen Normalgewebe zeigten keine Expression.

### Beispiel 11: Identifizierung von SLC26A9 als diagnostisches und therapeutisches Krebs-Target

SLC26A9 (SEQ ID NO: 56) und sein Translationsprodukt (SEQ ID NO: 65) wurden in der Genbank unter der Zugangs-Nr. NM_134325 veröffentlicht. SLC26A9 gehört zur Familie der Anionen-Austauscher. Die Expression von SLC26A9 beschränkt sich auf das bronchioläre und alveoläre Epithel der Lunge (Lohi et al., J Biol Chem 277:14246-54, 2002).

Es wurde untersucht, ob SLC26A9 als Marker von Lungentumoren genutzt werden kann. Hierzu wurden Oligonukleotide (SEQ ID NO: 77, 78) verwendet, die eine spezifische Amplifikation von SLC26A9 ermöglichen. RT-PCR-Untersuchungen mit SLC26A9-spezifischen Primern (SEQ ID NO: 77, 78) zeigten eine selektive Expression in der Lunge und in allen (13/13) untersuchten Lungentumor-Proben (Abb. 12). Die übrigen Normalgewebe zeigten mit Ausnahme der Schilddrüse keine Expression. In quantitativen RT-PCR-Experimenten mit den Primern SEQ ID NO: 131 und 132 konnten diese Ergebnisse zum einen bestätigt sowie zusätzliche Erkenntnisse gewonnen werden. In gepoolten Proben von 4-5 Tumorgeweben konnten hohe Expressionsspiegel für SLC26A9-spezifische RNA in Lungen-, Kolon-, Pankreas- und Magentumoren detektiert werden. SLC26A9 ist Mitglied einer Familie von Transmembran-Anionentransportern. In der gesunden Lunge ist das Protein in Richtung Luftwege luminal gerichtet und damit IgG-Antikörpern aus dem Blut nicht direkt zugänglich. Dagegen ist die Polarität des Proteins in Tumoren aufgehoben. Erfindungsgemäß kann daher SLC26A9 in den definierten Tumoren, u.a. Lungen-, Magen- und Pankreastumore, als therapeutisches Target durch monoklonale Antikörper addressiert werden. Die ausgeprägte, hohe Expression und hohe Inzidenz von SLC26A9 für Lungen-, Magen-, Pankreas- und Ösophagustumore machen dieses Protein erfindungsgemäß zu einem exzellenten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR. Des weiteren können die extrazellulären Domänen von SLC26A9 erfindungsgemäß als Zielstruktur zur Immun-Diagnostik und Therapie mittels monoklonaler Antikörper verwendet werden. Des weiteren kann SLC26A9 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compounds", die die biologische Aktivität von SLC26A9 modulieren und zur Therapie von Lungentumoren und gastrointestinalen Tumoren eingesetzt werden können.

### Beispiel 12: Identifizierung von THC1005163 als diagnostisches und therapeutisches Krebs-Target

THC1005163 (SEQ ID NO: 57) ist ein Genfragment aus dem TIGR-Gen-Index. Das Gen ist nur im 3'-Bereich definiert, während ein ORF fehlt. RT-PCR-Untersuchungen erfolgten mit einem THC1005163-spezifischen Primer (SEQ ID NO: 79) und einem Oligo dT₁₈ -Primer, der am 5'-Ende ein spezifisches Tag von 21 spezifischen Basen hatte. Dieses Tag wurde mit Datenbank-Suchprogrammen auf Homologie mit bekannten Sequenzen überprüft. Dieser spezielle Primer wurde initial bei der cDNA-Synthese eingesetzt, um genomische DNA-Verunreinigungen auszuschließen. RT-PCR-Untersuchungen mit diesem Primer-Set zeigten eine Expression in Magen, Ovar, Lunge und in 5/9 Lungentumor-Biopsien (Abb. 13). Die übrigen Normalgewebe zeigten keine Expression.

### Beispiel 13: Identifizierung von LOC134288 als diagnostisches und therapeutisches Krebs-Target

LOC134288 (SEQ ID NO: 58) und sein prädiziertes Translationsprodukt (SEQ ID NO: 66) wurden in der Genbank unter der Zugangs-Nr. XM_059703 veröffentlicht.

Erfindungsgemäß wurde untersucht, ob LOC134288 als Marker von Nierentumoren genutzt werden kann. Hierzu wurden Oligonukleotide (SEQ ID NO: 80, 81) verwendet, die eine spezifische Amplifikation von LOC134288 ermöglichen. RT-PCR-Untersuchungen zeigten eine selektive Expression in der Niere und in 5/8 untersuchten Nierentumor-Biopsien (Abb. 14).

### Beispiel 14: Identifizierung von THC943866 als diagnostisches und therapeutisches Krebs-Target

THC943866 (SEQ ID NO: 59) ist ein Genfragment aus dem TIGR-Gen-Index. Es wurde untersucht, ob THC943866 als Marker von Nierentumoren genutzt werden kann. Hierzu wurden Oligonukleotide (SEQ ID NO: 82, 83) verwendet, die eine spezifische Amplifikation von THC943866 ermöglichen.

RT-PCR-Untersuchungen mit THC943866-spezifischen Primern (SEQ ID NO: 82, 83) zeigten eine selektive Expression in der Niere und in 4/8 untersuchten Nierentumor-Biopsien (Abb. 15).

### Beispiel 15: Identifizierung von FLJ21458 und B7h.4 als diagnostische und therapeutische Krebs-Targets

FLJ21458 (SEQ ID NO: 84) und B7h.4 (SEQ ID NO: 138) und ihre prädizierten Translationsprodukte (SEQ ID NO: 85, 139) stellen Spleissvarianten eines Gens dar und wurden in der Genbank unter der Zugangs-Nr. NM_034850 bzw. AY358523 veröffentlicht. Sequenzanalysen ergaben, dass die Proteine Mitglieder der Butyrophillin-Familie darstellen. Strukturanalysen ergaben, dass es sich um Typ-1-Transmembranproteine mit einer extrazellulären Immunglobulindomäne handelt. Zur Expressionsuntersuchung wurden Oligonukleotide (SEQ ID NO: 86, 87 bzw. SEQ ID NO: 140, 141) verwendet, die eine spezifische Amplifikation von FLJ21458 bzw. B7h.4 ermöglichen. RT-PCR-Untersuchungen mit FLJ21458-spezifischen Primern (SEQ ID NO: 86, 87) zeigten eine selektive Expression im Kolon und in 7/10 untersuchten Kolontumor-Biopsien (Abb. 16, Tab. 5). Eine quantitative RT-PCR mit spezifischen Primern (SEQ ID NO: 133, 134) bestätigte dieses selektive Expressionsprofil (Abb. 39). Darüberhinaus konnte in dem Experiment FLJ21458 gastrointestinal-spezifisch im Kolon sowie in Magen, im End- und Blinddarmund und in Testis nachgewiesen werden. Auch 7/11 Kolonmetastasen-Proben waren in der quantitativen PCR positiv. Die FLJ21458-spezifische Expression wurde auf andere Tumore erweitert und eine proteinspezifische Expression konnte in Magen-, Pankreas- und Lebertumoren nachgewiesen werden (Tab. 5). RT-PCR-Untersuchungen mit B7h.4-spezifischen Primern (SEQ ID NO: 140, 141) zeigten eine starke, selektive Expression im Lungentumoren, jedoch nicht im normalen Lungengewebe. Damit zeigen beide Spleissvarianten dieses Butyrophyllins eine tumorassoziierte Expression und können als diagnostische und therapeutische Tumortargets genutzt werden. Zum Nachweis von FLJ21458- und B7h.4-Proteins wurden Antikörper durch Immunisieren von Kaninchen hergestellt. Als Epitope zur Propagierung dieser Antikörper wurden Peptide genutzt, die in beiden Proteinen (FLJ21458 und B7h.4) enthalten sind.
SEQ ID NO: 135: QWQVFGPDKPVQAL
SEQ ID NO: 136: AKWKGPQGQDLSTDS

Eine FLJ21458- bzw. B7h.4-spezifische Reaktion konnte in der Immunfluoreszenz nachgewiesen werden (Abb. 40). Zur Spezifitätskontrolle der Antikörper wurden 293-Zellen mit einem Plasmid transfiziert, dass für ein FLJ21458-GFP-Fusionsprotein kodiert. Der Spezifitätsnachweis erfolgte durch Kolokalisationsuntersuchungen mit dem spezifischen Antikörper und mit dem autofluoreszierenden GFP. Eine Überlagerung beider Fluoreszenzbilder zeigte eindeutig, dass das Immunserum das FLJ21458-Protein erkennt (Abb. 40 oben). Aufgrund der identischen Epitope in B7h.4 können diese Antikörper auch zur Bindung und Erkennung des B7h.4 Proteins in Tumoren genutzt werden. Bedingt durch die Überexpression des Proteins ergab sich eine diffuse Zellfärbung, die keine eindeutige Proteinlokalisation zuließ. Aus diesem Grund wurde mit der magentumorspezifischen Zelllinie Snu16, die FLJ21458 endogen exprimiert, ein weiteres Immunfluoreszenzexperiment durchgeführt (Abb. 40 unten). Die Zellen wurden mit dem FLJ21458-spezifischen Antiserum sowie mit einem weiteren Antikörper angefärbt, der das Membranprotein E-Cadherin erkennt. Der FLJ21458-spezifische Antikörper färbt zumindest schwach die Zellmembranen an, was somit ein Beleg dafür ist, dass FLF21458 in der Zellmembran lokalisiert ist.

Bioinformatische Untersuchungen zeigten, dass das von FLJ21458 kodierte Protein ein Zelloberflächenmolekül darstellt und über eine Immunglobulinsupermolekül-Domäne verfügt. Die selektive Expression dieses Oberflächenmoleküls macht es zu einem guten Target für die Entwicklung von diagnostischen Verfahren zur Detektion von Tumorzellen und therapeutischen Verfahren zur Elimination von Tumorzellen.

Die ausgeprägte Expression und hohe Inzidenz von FLJ21458 für Magen- und Kolontumore machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin sowie die Detektion von Metastasen in anderen Organen mittels RT-PCR. Des Weiteren können die extrazellulären Domänen von FLJ21458 erfindungsgemäß als Zielstruktur zur Immun-Diagnostik und -Therapie mittels monoklonaler Antikörper verwendet werden. Des Weiteren kann FLJ21458 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compounds", die die biologische Aktivität von FLJ21458 modulieren und zur Therapie von gastrointestinalen Tumoren eingesetzt werden können.

**Tab. 5 FLJ21458 bzw. B7h.4*-Expression in Normal- und Tumorgewebe**

| Normalgewebe | Expression | | Tumortyp | Expression |
|---|---|---|---|---|
| Gehirn | - | | Kolon | 7/10 |
| Cerebellum (Kleinhirn) | - | | Pankreas | 5/6 |
| Myokard | nd | | Ösophagus | nd |
| Skelettmuskel | - | | Magen | 8/10 |
| Herzmuskel | - | | Lunge | 6/8* |
| Magen | ++ | | Brust | nd |
| Kolon | +++ | | Ovar | nd |
| Pankreas | - | | Endometrium | nd |
| Niere | - | | HNO | nd |
| Leber | - | | Niere | nd |
| Testis | ++ | | Prostata | nd |
| Thymus | nd | | Kolonmetastasen | 7/11 |
| Brust | nd | | Leber | 5/8 |
| Ovar | - | | | |
| Uterus | - | | | |
| Haut | - | | | |
| Lunge | - | | | |
| Schilddrüse | nd | | | |
| Lymphknoten | - | | | |
| Milz | - | | | |
| PBMC | - | | | |
| Nebenniere | nd | | | |
| Ösophagus | - | | | |
| Dünndarm | - | | | |
| Prostata | - | | | |

## Patentansprüche

1. Antikörper, der an ein Protein oder Polypeptid oder an einen Teil davon bindet, wobei das Protein oder Polypeptid von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 7, 8, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist, wobei der Antikörper unterschiedliche Glykosylierungsvarianten des Proteins oder Polypeptids erkennt.

2. Antikörper nach Anspruch 1, der selektiv an eine bestimmte Glykosylierungsform des Proteins oder Polypeptids, vorzugsweise eine Form, die an einer oder mehreren möglichen Glykosylierungsstellen nicht glykosyliert ist, bindet.

3. Antikörper nach Anspruch 1 oder 2, wobei die Glykosylierung eine N-Glykosylierung ist.

4. Antikörper nach einem der Ansprüche 1 bis 3, wobei die möglichen Glykosylierungsstellen die Aminosäurepositionen 37, 38, 45, 116, 141, 146 und 205 der in SEQ ID NO: 16 gezeigten Aminosäuresequenz betreffen.

5. Antikörper nach einem der Ansprüche 1 bis 4, der durch Immunisieren mit einem Peptid oder Polypeptid mit einer Aminosäuresequenz, die aus der Gruppe bestehend aus SEQ ID NOs: 16-19, 112, 113, 116, 137 und 142-150, einem Teil oder Derivat davon ausgewählt ist, erhältlich ist.

6. Antikörper nach einem der Ansprüche 1 bis 5, der durch Immunisieren mit einem Peptid oder Polypeptid mit einer Aminosäuresequenz, die aus der Gruppe bestehend aus SEQ ID NOs: 146-150, einem Teil oder Derivat davon ausgewählt ist, erhältlich ist.

7. Antikörper nach einem der Ansprüche 1 bis 6, wobei der Antikörper ein monoklonaler Antikörper ist.

8. Antikörper nach einem der Ansprüche 1 bis 7, wobei der Antikörper ein chimärer oder humanisierter Antikörper oder ein Fragment eines Antikörpers ist.

9. Antikörper nach einem der Ansprüche 1 bis 8, wobei der Antikörper mit einem therapeutischen oder diagnostischen Mittel gekoppelt ist.

10. Pharmazeutische Zusammensetzung, die einen Antikörper nach einem der Ansprüche 1 bis 9 umfasst.

11. Pharmazeutische Zusammensetzung nach Anspruch 10 zur Behandlung einer Erkrankung, die sich durch die Expression oder abnormale Expression eines Tumor-assoziierten Antigens auszeichnet.

12. Antikörper nach Anspruch 9 zur Verwendung in einem Verfahren zur Behandlung, Diagnose oder Überwachung einer Erkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 7, 8, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

13. Pharmazeutische Zusammensetzung nach Anspruch 11 oder Antikörper nach Anspruch 12, wobei die Erkrankung Krebs ist.

14. Pharmazeutische Zusammensetzung nach Anspruch 11 oder Antikörper nach Anspruch 12, wobei die Erkrankung ein Lungentumor, ein Brusttumor, ein Prostatatumor, ein Melanom, ein Kolontumor, ein Magentumor, ein Pankreastumor, ein HNO-Tumor, Nierenzellkarzinom oder ein Zervixkarzinom, ein Kolonkarzinom oder ein Mammakarzinom ist.

15. Antikörper nach einem der Ansprüche 1 bis 9 und 12 bis 14 oder pharmazeutische Zusammensetzung nach einem der Ansprüche 10, 11, 13 und 14, wobei das Protein oder Polypeptid oder das Tumor-assoziierte Antigen eine Aminosäuresequenz umfasst, die aus der Gruppe bestehend aus SEQ ID NOs: 16-19, 112, 113, 116, 137, und 142-150, einem Teil oder Derivat davon ausgewählt ist.

16. Nukleinsäure, die für ein Protein oder Polypeptid kodiert, das eine Aminosäuresequenz umfasst, ausgewählt aus der Gruppe, bestehend aus SEQ ID NOs: 146-150, einem Teil oder Derivat davon.

17. Protein oder Polypeptid, das eine Aminosäuresequenz umfasst, ausgewählt aus der Gruppe, bestehend aus SEQ ID NOs: 142-150, einem Teil oder Derivat davon, oder ein immunogenes Fragment des Proteins oder Polypeptids.


SEQUENZPROTOKOLL

<110>  Ganymed Pharmaceuticals AG

<120>  Differentiell in Tumoren exprimierte Genprodukte und deren Verwendung

<130>  342-23 EPT2

<150>  DE 10 2004 024 617.3
<151>  2004-05-18

<160>  150

<170>  PatentIn version 3.1

<210>  1
<211>  1875
<212>  DNA
<213>  Homo sapiens

<400>  1
caggccagag tcccagctgt cctggactct gctgtgggga agggctgatg caggtgtgga      60
gtcaaatgtg ggtgcctcct gcagccgggt gccaggaggg gtggaggggc caccctgggc     120
tttgtccggg agcctggtct tcccgtcctt gggctgacag gtgctgctgc ctctgagccc     180
tccctgctaa gagctgtgtg ctgggtaagg ctggtggccc tttgggctcc ctgtccagga     240
tttgtgctct ggagggtagg gcttgctggg ctggggactg gaggggaacg tggagctcct     300
tctgcctcct ttcctgcccc atgacagcag gcagatccca ggagagaaga gctcaggaga     360
tgggaagagg atctgtccag gggttagacc tcaagggtga cttggagttc tttacggcac     420
ccatgctttc tttgaggagt tttgtgtttg tgggtgtggg gtcggggctc acctcctccc     480
acatccctgc ccagaggtgg gcagagtggg ggcagtgcct tgctccccct gctcgctctc     540
tgctgacctc cggctccctg tgctgcccca ggaccatgaa tggcacctac aacacctgtg     600
gctccagcga cctcacctgg cccccagcga tcaagctggg cttctacgcc tacttgggcg     660
tcctgctggt gctaggcctg ctgctcaaca gcctggcgct ctgggtgttc tgctgccgca     720

tgcagcagtg gacggagacc cgcatctaca tgaccaacct ggcggtggcc gacctctgcc 780
tgctgtgcac cttgcccttc gtgctgcact ccctgcgaga cacctcagac acgccgctgt 840
gccagctctc ccagggcatc tacctgacca acaggtacat gagcatcagc ctggtcacgg 900
ccatcgccgt ggaccgctat gtggccgtgc ggcacccgct gcgtgcccgc gggctgcggt 960
cccccaggca ggctgcggcc gtgtgcgcgg tcctctgggt gctggtcatc ggctccctgg 1020
tggctcgctg gctcctgggg attcaggagg gcggcttctg cttcaggagc acccggcaca 1080
atttcaactc catggcgttc ccgctgctgg gattctacct gcccctggcc gtggtggtct 1140
tctgctccct gaaggtggtg actgccctgg cccagaggcc acccaccgac gtggggcagg 1200
cagaggccac ccgcaaggct gcccgcatgg tctgggccaa cctcctggtg ttcgtggtct 1260
gcttcctgcc cctgcacgtg gggctgacag tgcgcctcgc agtgggctgg aacgcctgtg 1320
ccctcctgga gacgatccgt cgcgccctgt acataaccag caagctctca gatgccaact 1380
gctgcctgga cgccatctgc tactactaca tggccaagga gttccaggag gcgtctgcac 1440
tggccgtggc tcccagtgct aaggcccaca aaagccagga ctctctgtgc gtgaccctcg 1500
cctaagaggc gtgctgtggg cgctgtgggc caggtctcgg gggctccggg aggtgctgcc 1560
tgccagggga agctggaacc agtagcaagg agcccgggat cagccctgaa ctcactgtgt 1620
attctcttgg agccttgggt gggcagggac ggcccaggta cctgctctct tgggaagaga 1680
gagggacagg gacaagggca agaggactga ggccagagca aggccaatgt cagagacccc 1740
cgggatgggg cctcacactt gccaccccca gaaccagctc acctggccag agtgggttcc 1800
tgctggccag ggtgcagcct tgatgacacc tgccgctgcc cctcggggct ggaataaaac 1860
tccccaccca gagtc 1875

<210> 2
<211> 3222
<212> DNA
<213> Homo sapiens

<400> 2
atgaagacgt tgctgttgga cttggctttg tggtcactgc tcttccagcc cgggtggctg 60
tcctttagtt cccaggtgag tcagaactgc cacaatggca gctatgaaat cagcgtcctg 120
atgatgggca actcagcctt tgcagagccc ctgaaaaact tggaagatgc ggtgaatgag 180
gggctggaaa tagtgagagg acgtctgcaa aatgctggcc taaatgtgac tgtgaacgct 240
actttcatgt attcggatgg tctgattcat aactcaggcg actgccggag tagcacctgt 300
gaaggcctcg acctactcag gaaaatttca aatgcacaac ggatgggctg tgtcctcata 360
gggccctcat gtacatactc caccttccag atgtaccttg acacagaatt gagctacccc 420
atgatctcag ctggaagttt tggattgtca tgtgactata aagaaacctt aaccaggctg 480

atgtctccag ctagaaagtt gatgtacttc ttggttaact tttggaaaac caacgatctg 540
cccttcaaaa cttattcctg gagcacttcg tatgtttaca agaatggtac agaaactgag 600
gactgtttct ggtaccttaa tgctctggag gctagcgttt cctatttctc ccacgaactc 660
ggctttaagg tggtgttaag acaagataag gagtttcagg atatcttaat ggaccacaac 720
aggaaaagca atgtgattat tatgtgtggt ggtccagagt tcctctacaa gctgaagggt 780
gaccgagcag tggctgaaga cattgtcatt attctagtgg atcttttcaa tgaccagtac 840
ttggaggaca atgtcacagc ccctgactat atgaaaaatg tccttgttct gacgctgtct 900
cctgggaatt cccttctaaa tagctctttc tccaggaatc tatcaccaac aaaacgagac 960
tttgctcttg cctatttgaa tggaatcctg ctctttggac atatgctgaa gatatttctt 1020
gaaaatggag aaaatattac cacccccaaa tttgctcatg ctttcaggaa tctcactttt 1080
gaagggtatg acggtccagt gaccttggat gactgggggg atgttgacag taccatggtg 1140
cttctgtata cctctgtgga caccaagaaa tacaaggttc ttttgaccta tgatacccac 1200
gtaaataaga cctatcctgt ggatatgagc cccacattca cttggaagaa ctctaaactt 1260
cctaatgata ttacaggccg gggccctcag atcctgatga ttgcagtctt caccctcact 1320
ggagctgtgg tgctgctcct gctcgtcgct ctcctgatgc tcagaaaata tagaaaagat 1380
tatgaacttc gtcagaaaaa atggtcccac attcctcctg aaaatatctt tcctctggag 1440
accaatgaga ccaatcatgt tagcctcaag atcgatgatg acaaaagacg agatacaatc 1500
cagagactac gacagtgcaa atacgacaaa aagcgagtga ttctcaaaga tctcaagcac 1560
aatgatggta atttcactga aaaacagaag atagaattga acaagttgct tcagattgac 1620
tattacaacc tgaccaagtt ctacggcaca gtgaaacttg ataccatgat cttcggggtg 1680
atagaatact gtgagagagg atccctccgg gaagttttaa atgacacaat ttcctaccct 1740
gatggcacat tcatggattg ggagtttaag atctctgtct tgtatgacat tgctaaggga 1800
atgtcatatc tgcactccag taagacagaa gtccatggtc gtctgaaatc taccaactgc 1860
gtagtggaca gtagaatggt ggtgaagatc actgattttg gctgcaattc cattttacct 1920
ccaaaaaagg acctgtggac agctccagag cacctccgcc aagccaacat ctctcagaaa 1980
ggagatgtgt acagctatgg gatcatcgca caggagatca ttctgcggaa agaaaccttc 2040
tacactttga gctgtcggga ccggaatgag aagattttca gagtggaaaa ttccaatgga 2100
atgaaaccct tccgcccaga tttattcttg gaaacagcag aggaaaaaga gctagaagtg 2160
tacctacttg taaaaaactg ttgggaggaa gatccagaaa agagaccaga tttcaaaaaa 2220
attgagacta cacttgccaa gatatttgga ctttttcatg accaaaaaaa tgaaagctat 2280
atggatacct tgatccgacg tctacagcta tattctcgaa acctggaaca tctggtagag 2340
gaaaggacac agctgtacaa ggcagagagg gacagggctg acagacttaa ctttatgttg 2400
cttccaaggc tagtggtaaa gtctctgaag gagaaaggct ttgtggagcc ggaactatat 2460
gaggaagtta caatctactt cagtgacatt gtaggtttca ctactatctg caaatacagc 2520

acccccatgg aagtggtgga catgcttaat gacatctata agagttttga ccacattgtt 2580

gatcatcatg atgtctacaa ggtggaaacc atcggtgatg cgtacatggt ggctagtggt 2640

ttgcctaaga gaaatggcaa tcggcatgca atagacattg ccaagatggc cttggaaatc 2700

ctcagcttca tggggacctt tgagctggag catcttcctg gcctcccaat atggattcgc 2760

attggagttc actctggtcc ctgtgctgct ggagttgtgg gaatcaagat gcctcgttat 2820

tgtctatttg gagatacggt caacacagcc tctaggatgg aatccactgg cctccctttg 2880

agaattcacg tgagtggctc caccatagcc atcctgaaga gaactgagtg ccagttcctt 2940

tatgaagtga gaggagaaac atacttaaag ggaagaggaa atgagactac ctactggctg 3000

actgggatga aggaccagaa attcaacctg ccaacccctc ctactgtgga gaatcaacag 3060

cgtttgcaag cagaattttc agacatgatt gccaactctt tacagaaaag acaggcagca 3120

gggataagaa gccaaaaacc cagacgggta gccagctata aaaaaggcac tctggaatac 3180

ttgcagctga ataccacaga caaggagagc acctattttt aa 3222

<210> 3

<211> 336

<212> DNA

<213> Homo sapiens

<400> 3
atgaagacgt tgctgttgga cttggctttg tggtcactgc tcttccagcc cgggtggctg 60

tcctttagtt cccaggtgag tcagaactgc cacaatggca gctatgaaat cagcgtcctg 120

atgatgggca actcagcctt tgcagagccc ctgaaaaact tggaagatgc ggtgaatgag 180

gggctggaaa tagtgagagg acgtctgcaa aatgctggcc taaatgtgac tgtgaacgct 240

actttcatgt attcggatgg tctgattcat aactcaggcg actgccggag tagcacctgt 300

gaaggcctcg acctactcag gaaaatttca ccttga 336

<210> 4

<211> 777

<212> DNA

<213> Homo sapiens

<400> 4
atgaagacgt tgctgttgga cttggctttg tggtcactgc tcttccagcc cgggtggctg 60

tcctttagtt cccaggtgag tcagaactgc cacaatggca gctatgaaat cagcgtcctg 120

atgatgggca actcagcctt tgcagagccc ctgaaaaact tggaagatgc ggtgaatgag 180

gggctggaaa tagtgagagg acgtctgcaa aatgctggcc taaatgtgac tgtgaacgct 240

actttcatgt attcggatgg tctgattcat aactcaggcg actgccggag tagcacctgt 300
gaaggcctcg acctactcag gaaaatttca aatgcacaac ggatgggctg tgtcctcata 360
gggccctcat gtacatactc caccttccag atgtaccttg acacagaatt gagctacccc 420
atgatctcag ctggaagttt tggattgtca tgtgactata aagaaacctt aaccaggctg 480
atgtctccag ctagaaagtt gatgtacttc ttggttaact tttggaaaac caacgatctg 540
cccttcaaaa cttattcctg gagcacttcg tatgtttaca agaatggtac agaaactgag 600
gactgtttct ggtaccttaa tgctctggag gctagcgttt cctatttctc ccacgaactc 660
ggctttaagg tggtgttaag acaagataag gagtttcagg atatcttaat ggaccacaac 720
aggaaaagca atgtgaccag tacttggagg acaatgtcac agcccctgac tatatga 777

<210> 5
<211> 3213
<212> DNA
<213> Homo sapiens

<400> 5
atgaagacgt tgctgttgga cttggctttg tggtcactgc tcttccagcc cgggtggctg 60
tcctttagtt cccaggtgag tcagaactgc cacaatggca gctatgaaat cagcgtcctg 120
atgatgggca actcagcctt tgcagagccc ctgaaaaact tggaagatgc ggtgaatgag 180
gggctggaaa tagtgagagg acgtctgcaa aatgctggcc taaatgtgac tgtgaacgct 240
actttcatgt attcggatgg tctgattcat aactcaggcg actgccggag tagcacctgt 300
gaaggcctcg acctactcag gaaaatttca aatgcacaac ggatgggctg tgtcctcata 360
gggccctcat gtacatactc caccttccag atgtaccttg acacagaatt gagctacccc 420
atgatctcag ctggaagttt tggattgtca tgtgactata aagaaacctt aaccaggctg 480
atgtctccag ctagaaagtt gatgtacttc ttggttaact tttggaaaac caacgatctg 540
cccttcaaaa cttattcctg gagcacttcg tatgtttaca agaatggtac agaaactgag 600
gactgtttct ggtaccttaa tgctctggag gctagcgttt cctatttctc ccacgaactc 660
ggctttaagg tggtgttaag acaagataag gagtttcagg atatcttaat ggaccacaac 720
aggaaaagca atgtgattat tatgtgtggt ggtccagagt tcctctacaa gctgaagggt 780
gaccgagcag tggctgaaga cattgtcatt attctagtgg atcttttcaa tgaccagtac 840
ttggaggaca atgtcacagc ccctgactat atgaaaaatg tccttgttct gacgctgtct 900
cctgggaatt cccttctaaa tagctctttc tccaggaatc tatcaccaac aaaacgagac 960
tttgctcttg cctatttgaa tggaatcctg ctctttggac atatgctgaa gatatttctt 1020
gaaaatggag aaaatattac cacccccaaa tttgctcatg ctttcaggaa tctcactttt 1080
gaagggtatg acggtccagt gaccttggat gactgggggg atgttgacag taccatggtg 1140

cttctgtata cctctgtgga caccaagaaa tacaaggttc ttttgaccta tgatacccac 1200
gtaaataaga cctatcctgt ggatatgagc cccacattca cttggaagaa ctctaaactt 1260
cctaatgata ttacaggccg gggccctcag atcctgatga ttgcagtctt caccctcact 1320
ggagctgtgg tgctgctcct gctcgtcgct ctcctgatgc tcagaaaata tagaaaagat 1380
tatgaacttc gtcagaaaaa atggtcccac attcctcctg aaaatatctt tcctctggag 1440
accaatgaga ccaatcatgt tagcctcaag atcgatgatg acaaaagacg agatacaatc 1500
cagagactac gacagtgcaa atacgacaaa aagcgagtga ttctcaaaga tctcaagcac 1560
aatgatggta atttcactga aaaacagaag atagaattga acaagattga ctattacaac 1620
ctgaccaagt tctacggcac agtgaaactt gataccatga tcttcggggt gatagaatac 1680
tgtgagagag gatccctccg ggaagtttta aatgacacaa tttcctaccc tgatggcaca 1740
ttcatggatt gggagtttaa gatctctgtc ttgtatgaca ttgctaaggg aatgtcatat 1800
ctgcactcca gtaagacaga agtccatggt cgtctgaaat ctaccaactg cgtagtggac 1860
agtagaatgg tggtgaagat cactgatttt ggctgcaatt ccattttacc tccaaaaaag 1920
gacctgtgga cagctccaga gcacctccgc caagccaaca tctctcagaa aggagatgtg 1980
tacagctatg ggatcatcgc acaggagatc attctgcgga aagaaacctt ctacactttg 2040
agctgtcggg accggaatga gaagattttc agagtggaaa attccaatgg aatgaaaccc 2100
ttccgcccag atttattctt ggaaacagca gaggaaaaag agctagaagt gtacctactt 2160
gtaaaaaact gttgggagga agatccagaa aagagaccag atttcaaaaa aattgagact 2220
acacttgcca agatatttgg actttttcat gaccaaaaaa atgaaagcta tatggatacc 2280
ttgatccgac gtctacagct atattctcga aacctggaac atctggtaga ggaaaggaca 2340
cagctgtaca aggcagagag ggacagggct gacagactta actttatgtt gcttccaagg 2400
ctagtggtaa agtctctgaa ggagaaaggc tttgtggagc cggaactata tgaggaagtt 2460
acaatctact tcagtgacat tgtaggtttc actactatct gcaaatacag cacccccatg 2520
gaagtggtgg acatgcttaa tgacatctat aagagttttg accacattgt tgatcatcat 2580
gatgtctaca aggtggaaac catcggtgat gcgtacatgg tggctagtgg tttgcctaag 2640
agaaatggca atcggcatgc aatagacatt gccaagatgg ccttggaaat cctcagcttc 2700
atggggacct ttgagctgga gcatcttcct ggcctcccaa tatggattcg cattggagtt 2760
cactctggtc cctgtgctgc tggagttgtg ggaatcaaga tgcctcgtta ttgtctattt 2820
ggagatacgg tcaacacagc ctctaggatg gaatccactg gcctcccttt gagaattcac 2880
gtgagtggct ccaccatagc catcctgaag agaactgagt gccagttcct ttatgaagtg 2940
agaggagaaa catacttaaa gggaagagga aatgagacta cctactggct gactgggatg 3000
aaggaccaga aattcaacct gccaacccct cctactgtgg agaatcaaca gcgtttgcaa 3060
gcagaatttt cagacatgat tgccaactct ttacagaaaa gacaggcagc agggataaga 3120
agccaaaaac ccagacgggt agccagctat aaaaaaggca ctctggaata cttgcagctg 3180

aataccacag acaaggagag cacctatttt taa 3213

<210> 6
<211> 550
<212> DNA
<213> Homo sapiens

<400> 6
ggggacactt tgtatggcaa gtggaaccac tggcttggtg gattttgcta gatttttctg 60

atttttaaac tcctgaaaaa tatcccagat aactgtcatg aagctggtaa ctatcttcct 120

gctggtgacc atcagccttt gtagttactc tgctactgcc ttcctcatca acaaagtgcc 180

ccttcctgtt gacaagttgg cacctttacc tctggacaac attcttccct ttatggatcc 240

attaaagctt cttctgaaaa ctctgggcat ttctgttgag caccttgtgg aggggctaag 300

gaagtgtgta aatgagctgg gaccagaggc ttctgaagct gtgaagaaac tgctggaggc 360

gctatcacac ttggtgtgac atcaagataa agagcggagg tggatgggga tggaagatga 420

tgctcctatc ctccctgcct gaaacctgtt ctaccaatta tagatcaaat gccctaaaat 480

gtagtgaccc gtgaaaagga caaataaagc aatgaatact aaaaaaaaaa aaaaaaaaaa 540

aaaaaaaaaa 550

<210> 7

<211> 786

<212> DNA

<213> Homo sapiens

<400> 7
atggccgtga ctgcctgtca gggcttgggg ttcgtggttt cactgattgg gattgcgggc 60

atcattgctg ccacctgcat ggaccagtgg agcacccaag acttgtacaa caaccccgta 120

acagctgttt tcaactacca ggggctgtgg cgctcctgtg tccgagagag ctctggcttc 180

accgagtgcc ggggctactt caccctgctg gggctgccag ccatgctgca ggcagtgcga 240

gccctgatga tcgtaggcat cgtcctgggt gccattggcc tcctggtatc catctttgcc 300

ctgaaatgca tccgcattgg cagcatggag gactctgcca aagccaacat gacactgacc 360

tccgggatca tgttcattgt ctcaggtctt tgtgcaattg ctggagtgtc tgtgtttgcc 420

aacatgctgg tgactaactt ctggatgtcc acagctaaca tgtacaccgg catgggtggg 480

atggtgcaga ctgttcagac caggtacaca tttggtgcgg ctctgttcgt gggctgggtc 540

gctggaggcc tcacactaat tgggggtgtg atgatgtgca tcgcctgccg gggcctggca 600

ccagaagaaa ccaactacaa agccgtttct tatcatgcct caggccacag tgttgcctac 660

aagcctggag gcttcaaggc cagcactggc tttgggtcca acaccaaaaa caagaagata 720

tacgatggag gtgcccgcac agaggacgag gtacaatctt atccttccaa gcacgactat 780

gtgtaa                                                                786

<210>  8

<211>  180

<212>  DNA

<213>  Homo sapiens


<400>  8
tgcgccacca tggccgtgac tgcctgtcag ggcttggggt tcgtggtttc actgattggg      60

attgcgggca tcattgctgc cacctgcatg gaccagtgga gcacccaaga cttgtacaac     120

aaccccgtaa cagctgtttt caactaccag gggctgtggc gctcctgtgt ccgagagagc     180

<210>  9

<211>  309

<212>  PRT

<213>  Homo sapiens


<400>  9

Met Asn Gly Thr Tyr Asn Thr Cys Gly Ser Ser Asp Leu Thr Trp Pro
1               5                   10                  15

Pro Ala Ile Lys Leu Gly Phe Tyr Ala Tyr Leu Gly Val Leu Leu Val
            20                  25                  30

Leu Gly Leu Leu Leu Asn Ser Leu Ala Leu Trp Val Phe Cys Cys Arg
        35                  40                  45

Met Gln Gln Trp Thr Glu Thr Arg Ile Tyr Met Thr Asn Leu Ala Val
    50                  55                  60

Ala Asp Leu Cys Leu Leu Cys Thr Leu Pro Phe Val Leu His Ser Leu
65                  70                  75                  80

Arg Asp Thr Ser Asp Thr Pro Leu Cys Gln Leu Ser Gln Gly Ile Tyr
                85                  90                  95

Leu Thr Asn Arg Tyr Met Ser Ile Ser Leu Val Thr Ala Ile Ala Val
            100                 105                 110

Asp Arg Tyr Val Ala Val Arg His Pro Leu Arg Ala Arg Gly Leu Arg
        115                 120                 125

Ser Pro Arg Gln Ala Ala Ala Val Cys Ala Val Leu Trp Val Leu Val
    130                 135                 140

Ile Gly Ser Leu Val Ala Arg Trp Leu Leu Gly Ile Gln Glu Gly Gly
145                 150                 155                 160

Phe Cys Phe Arg Ser Thr Arg His Asn Phe Asn Ser Met Arg Phe Pro
                165                 170                 175

Leu Leu Gly Phe Tyr Leu Pro Leu Ala Val Val Val Phe Cys Ser Leu
            180                 185                 190

Lys Val Val Thr Ala Leu Ala Gln Arg Pro Pro Thr Asp Val Gly Gln
        195                 200                 205

Ala Glu Ala Thr Arg Lys Ala Ala Arg Met Val Trp Ala Asn Leu Leu
    210                 215                 220

Val Phe Val Val Cys Phe Leu Pro Leu His Val Gly Leu Thr Val Arg
225                 230                 235                 240

Leu Ala Val Gly Trp Asn Ala Cys Ala Leu Leu Glu Thr Ile Arg Arg
                245                 250                 255

Ala Leu Tyr Ile Thr Ser Lys Leu Ser Asp Ala Asn Cys Cys Leu Asp
            260                 265                 270

Ala Ile Cys Tyr Tyr Tyr Met Ala Lys Glu Phe Gln Glu Ala Ser Ala
        275                 280                 285

Leu Ala Val Ala Pro Arg Ala Lys Ala His Lys Ser Gln Asp Ser Leu
    290                 295                 300

Cys Val Thr Leu Ala
305

<210>  10

<211>  394

<212>  PRT

<213>  Homo sapiens


<400>  10

Met Thr Ala Gly Arg Ser Gln Glu Arg Arg Ala Gln Glu Met Gly Arg
1               5                   10                  15

Gly Ser Val Gln Gly Leu Asp Leu Lys Gly Asp Leu Glu Phe Phe Thr
            20                  25                  30

Ala Pro Met Leu Ser Leu Arg Ser Phe Val Phe Val Gly Val Gly Ser
        35                  40                  45

Gly Leu Thr Ser Ser His Ile Pro Ala Gln Arg Trp Ala Glu Trp Gly
    50                  55                  60

Gln Cys Leu Ala Pro Pro Ala Arg Ser Leu Leu Thr Ser Gly Ser Leu
65                  70                  75                  80

Cys Cys Pro Arg Thr Met Asn Gly Thr Tyr Asn Thr Cys Gly Ser Ser
                85                  90                  95

Asp Leu Thr Trp Pro Pro Ala Ile Lys Leu Gly Phe Tyr Ala Tyr Leu
            100                 105                 110

Gly Val Leu Leu Val Leu Gly Leu Leu Leu Asn Ser Leu Ala Leu Trp
        115                 120                 125

Val Phe Cys Cys Arg Met Gln Gln Trp Thr Glu Thr Arg Ile Tyr Met
    130                 135                 140

Thr Asn Leu Ala Val Ala Asp Leu Cys Leu Leu Cys Thr Leu Pro Phe
145                 150                 155                 160

Val Leu His Ser Leu Arg Asp Thr Ser Asp Thr Pro Leu Cys Gln Leu
                165                 170                 175

Ser Gln Gly Ile Tyr Leu Thr Asn Arg Tyr Met Ser Ile Ser Leu Val
            180                 185                 190

Thr Ala Ile Ala Val Asp Arg Tyr Val Ala Val Arg His Pro Leu Arg
        195                 200                 205

Ala Arg Gly Leu Arg Ser Pro Arg Gln Ala Ala Ala Val Cys Ala Val
    210                 215                 220

Leu Trp Val Leu Val Ile Gly Ser Leu Val Ala Arg Trp Leu Leu Gly
225                 230                 235                 240

Ile Gln Glu Gly Gly Phe Cys Phe Arg Ser Thr Arg His Asn Phe Asn
                245                 250                 255

Ser Met Ala Phe Pro Leu Leu Gly Phe Tyr Leu Pro Leu Ala Val Val
            260                 265                 270

Val Phe Cys Ser Leu Lys Val Val Thr Ala Leu Ala Gln Arg Pro Pro
        275                 280                 285

Thr Asp Val Gly Gln Ala Glu Ala Thr Arg Lys Ala Ala Arg Met Val
    290                 295                 300

Trp Ala Asn Leu Leu Val Phe Val Val Cys Phe Leu Pro Leu His Val
305                 310                 315                 320

Gly Leu Thr Val Arg Leu Ala Val Gly Trp Asn Ala Cys Ala Leu Leu
                325                 330                 335

Glu Thr Ile Arg Arg Ala Leu Tyr Ile Thr Ser Lys Leu Ser Asp Ala
            340                 345                 350

Asn Cys Cys Leu Asp Ala Ile Cys Tyr Tyr Tyr Met Ala Lys Glu Phe
        355                 360                 365

Gln Glu Ala Ser Ala Leu Ala Val Ala Pro Ser Ala Lys Ala His Lys
    370                 375                 380

Ser Gln Asp Ser Leu Cys Val Thr Leu Ala
385                 390

<210>  11

<211>  1073

<212>  PRT

<213>  Homo sapiens

<400>  11

Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Asn Ala
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140

Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Ile Ile Met Cys Gly Gly Pro Glu Phe Leu Tyr
                245                 250                 255

Lys Leu Lys Gly Asp Arg Ala Val Ala Glu Asp Ile Val Ile Ile Leu
            260                 265                 270

Val Asp Leu Phe Asn Asp Gln Tyr Leu Glu Asp Asn Val Thr Ala Pro
        275                 280                 285

Asp Tyr Met Lys Asn Val Leu Val Leu Thr Leu Ser Pro Gly Asn Ser
    290                 295                 300

Leu Leu Asn Ser Ser Phe Ser Arg Asn Leu Ser Pro Thr Lys Arg Asp
305                 310                 315                 320

Phe Ala Leu Ala Tyr Leu Asn Gly Ile Leu Leu Phe Gly His Met Leu
                325                 330                 335

Lys Ile Phe Leu Glu Asn Gly Glu Asn Ile Thr Thr Pro Lys Phe Ala
            340                 345                 350

His Ala Phe Arg Asn Leu Thr Phe Glu Gly Tyr Asp Gly Pro Val Thr
        355                 360                 365

Leu Asp Asp Trp Gly Asp Val Asp Ser Thr Met Val Leu Leu Tyr Thr
    370                 375                 380

Ser Val Asp Thr Lys Lys Tyr Lys Val Leu Leu Thr Tyr Asp Thr His
385                 390                 395                 400

Val Asn Lys Thr Tyr Pro Val Asp Met Ser Pro Thr Phe Thr Trp Lys
                405                 410                 415

Asn Ser Lys Leu Pro Asn Asp Ile Thr Gly Arg Gly Pro Gln Ile Leu
            420                 425                 430

Met Ile Ala Val Phe Thr Leu Thr Gly Ala Val Val Leu Leu Leu Leu
        435                 440                 445

Val Ala Leu Leu Met Leu Arg Lys Tyr Arg Lys Asp Tyr Glu Leu Arg
    450                 455                 460

Gln Lys Lys Trp Ser His Ile Pro Pro Glu Asn Ile Phe Pro Leu Glu
465                 470                 475                 480

Thr Asn Glu Thr Asn His Val Ser Leu Lys Ile Asp Asp Asp Lys Arg
                485                 490                 495

Arg Asp Thr Ile Gln Arg Leu Arg Gln Cys Lys Tyr Asp Lys Lys Arg
            500                 505                 510

Val Ile Leu Lys Asp Leu Lys His Asn Asp Gly Asn Phe Thr Glu Lys
        515                 520                 525

Gln Lys Ile Glu Leu Asn Lys Leu Leu Gln Ile Asp Tyr Tyr Asn Leu
    530                 535                 540

Thr Lys Phe Tyr Gly Thr Val Lys Leu Asp Thr Met Ile Phe Gly Val
545                 550                 555                 560

Ile Glu Tyr Cys Glu Arg Gly Ser Leu Arg Glu Val Leu Asn Asp Thr
                565                 570                 575

Ile Ser Tyr Pro Asp Gly Thr Phe Met Asp Trp Glu Phe Lys Ile Ser
            580                 585                 590

Val Leu Tyr Asp Ile Ala Lys Gly Met Ser Tyr Leu His Ser Ser Lys
        595                 600                 605

Thr Glu Val His Gly Arg Leu Lys Ser Thr Asn Cys Val Val Asp Ser
    610                 615                 620

Arg Met Val Val Lys Ile Thr Asp Phe Gly Cys Asn Ser Ile Leu Pro
625                 630                 635                 640

Pro Lys Lys Asp Leu Trp Thr Ala Pro Glu His Leu Arg Gln Ala Asn
                645                 650                 655

Ile Ser Gln Lys Gly Asp Val Tyr Ser Tyr Gly Ile Ile Ala Gln Glu
            660                 665                 670

Ile Ile Leu Arg Lys Glu Thr Phe Tyr Thr Leu Ser Cys Arg Asp Arg
        675                 680                 685

Asn Glu Lys Ile Phe Arg Val Glu Asn Ser Asn Gly Met Lys Pro Phe
    690                 695                 700

Arg Pro Asp Leu Phe Leu Glu Thr Ala Glu Glu Lys Glu Leu Glu Val
705                 710                 715                 720

Tyr Leu Leu Val Lys Asn Cys Trp Glu Glu Asp Pro Glu Lys Arg Pro
                725                 730                 735

Asp Phe Lys Lys Ile Glu Thr Thr Leu Ala Lys Ile Phe Gly Leu Phe
            740                 745                 750

His Asp Gln Lys Asn Glu Ser Tyr Met Asp Thr Leu Ile Arg Arg Leu
        755                 760                 765

Gln Leu Tyr Ser Arg Asn Leu Glu His Leu Val Glu Glu Arg Thr Gln
    770                 775                 780

Leu Tyr Lys Ala Glu Arg Asp Arg Ala Asp Arg Leu Asn Phe Met Leu
785                 790                 795                 800

Leu Pro Arg Leu Val Val Lys Ser Leu Lys Glu Lys Gly Phe Val Glu
                805                 810                 815

Pro Glu Leu Tyr Glu Glu Val Thr Ile Tyr Phe Ser Asp Ile Val Gly
            820                 825                 830

Phe Thr Thr Ile Cys Lys Tyr Ser Thr Pro Met Glu Val Val Asp Met
        835                 840                 845

Leu Asn Asp Ile Tyr Lys Ser Phe Asp His Ile Val Asp His His Asp
    850                 855                 860

Val Tyr Lys Val Glu Thr Ile Gly Asp Ala Tyr Met Val Ala Ser Gly
865                 870                 875                 880

Leu Pro Lys Arg Asn Gly Asn Arg His Ala Ile Asp Ile Ala Lys Met
                885                 890                 895

Ala Leu Glu Ile Leu Ser Phe Met Gly Thr Phe Glu Leu Glu His Leu
            900                 905                 910

Pro Gly Leu Pro Ile Trp Ile Arg Ile Gly Val His Ser Gly Pro Cys
        915                 920                 925

Ala Ala Gly Val Val Gly Ile Lys Met Pro Arg Tyr Cys Leu Phe Gly
    930                 935                 940

Asp Thr Val Asn Thr Ala Ser Arg Met Glu Ser Thr Gly Leu Pro Leu
945                 950                 955                 960

Arg Ile His Val Ser Gly Ser Thr Ile Ala Ile Leu Lys Arg Thr Glu
                965                 970                 975

Cys Gln Phe Leu Tyr Glu Val Arg Gly Glu Thr Tyr Leu Lys Gly Arg
            980                 985                 990

Gly Asn Glu Thr Thr Tyr Trp Leu Thr Gly Met Lys Asp Gln Lys Phe
        995                 1000                1005

Asn Leu Pro Thr Pro Pro Thr Val Glu Asn Gln Gln Arg Leu Gln
    1010                1015                1020

Ala Glu Phe Ser Asp Met Ile Ala Asn Ser Leu Gln Lys Arg Gln
    1025                1030                1035

Ala Ala Gly Ile Arg Ser Gln Lys Pro Arg Arg Val Ala Ser Tyr
    1040                1045                1050

Lys Lys Gly Thr Leu Glu Tyr Leu Gln Leu Asn Thr Thr Asp Lys
    1055                1060                1065

Glu Ser Thr Tyr Phe
    1070

<210> 12

<211> 111

<212> PRT

<213> Homo sapiens

<400> 12

Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Pro
            100                 105                 110

<210> 13

<211> 258

<212> PRT

<213> Homo sapiens

<400> 13

Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Asn Ala
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140

Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Thr Ser Thr Trp Arg Thr Met Ser Gln Pro Leu
                245                 250                 255

Thr Ile

<210>  14
<211>  1070
<212>  PRT
<213>  Homo sapiens

<400>  14

Met Lys Thr Leu Leu Leu Asp Leu Ala Leu Trp Ser Leu Leu Phe Gln
1               5                   10                  15

Pro Gly Trp Leu Ser Phe Ser Ser Gln Val Ser Gln Asn Cys His Asn
            20                  25                  30

Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Asn Ala
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140

Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Ile Ile Met Cys Gly Gly Pro Glu Phe Leu Tyr
                245                 250                 255

Lys Leu Lys Gly Asp Arg Ala Val Ala Glu Asp Ile Val Ile Ile Leu
            260                 265                 270

Val Asp Leu Phe Asn Asp Gln Tyr Leu Glu Asp Asn Val Thr Ala Pro
        275                 280                 285

Asp Tyr Met Lys Asn Val Leu Val Leu Thr Leu Ser Pro Gly Asn Ser
    290                 295                 300

Leu Leu Asn Ser Ser Phe Ser Arg Asn Leu Ser Pro Thr Lys Arg Asp
305                 310                 315                 320

Phe Ala Leu Ala Tyr Leu Asn Gly Ile Leu Leu Phe Gly His Met Leu
                325                 330                 335

Lys Ile Phe Leu Glu Asn Gly Glu Asn Ile Thr Thr Pro Lys Phe Ala
            340                 345                 350

His Ala Phe Arg Asn Leu Thr Phe Glu Gly Tyr Asp Gly Pro Val Thr
        355                 360                 365

Leu Asp Asp Trp Gly Asp Val Asp Ser Thr Met Val Leu Leu Tyr Thr
    370                 375                 380

Ser Val Asp Thr Lys Lys Tyr Lys Val Leu Leu Thr Tyr Asp Thr His
385                 390                 395                 400

Val Asn Lys Thr Tyr Pro Val Asp Met Ser Pro Thr Phe Thr Trp Lys
                405                 410                 415

Asn Ser Lys Leu Pro Asn Asp Ile Thr Gly Arg Gly Pro Gln Ile Leu
            420                 425                 430

Met Ile Ala Val Phe Thr Leu Thr Gly Ala Val Val Leu Leu Leu Leu
        435                 440                 445

Val Ala Leu Leu Met Leu Arg Lys Tyr Arg Lys Asp Tyr Glu Leu Arg
    450                 455                 460

Gln Lys Lys Trp Ser His Ile Pro Pro Glu Asn Ile Phe Pro Leu Glu
465                 470                 475                 480

Thr Asn Glu Thr Asn His Val Ser Leu Lys Ile Asp Asp Asp Lys Arg
                485                 490                 495

Arg Asp Thr Ile Gln Arg Leu Arg Gln Cys Lys Tyr Asp Lys Lys Arg
            500                 505                 510

Val Ile Leu Lys Asp Leu Lys His Asn Asp Gly Asn Phe Thr Glu Lys
        515                 520                 525

Gln Lys Ile Glu Leu Asn Lys Ile Asp Tyr Tyr Asn Leu Thr Lys Phe
    530                 535                 540

Tyr Gly Thr Val Lys Leu Asp Thr Met Ile Phe Gly Val Ile Glu Tyr
545                 550                 555                 560

Cys Glu Arg Gly Ser Leu Arg Glu Val Leu Asn Asp Thr Ile Ser Tyr
                565                 570                 575

Pro Asp Gly Thr Phe Met Asp Trp Glu Phe Lys Ile Ser Val Leu Tyr
            580                 585                 590

Asp Ile Ala Lys Gly Met Ser Tyr Leu His Ser Ser Lys Thr Glu Val
        595                 600                 605

His Gly Arg Leu Lys Ser Thr Asn Cys Val Val Asp Ser Arg Met Val
    610                 615                 620

Val Lys Ile Thr Asp Phe Gly Cys Asn Ser Ile Leu Pro Pro Lys Lys
625                 630                 635                 640

Asp Leu Trp Thr Ala Pro Glu His Leu Arg Gln Ala Asn Ile Ser Gln
                645                 650                 655

Lys Gly Asp Val Tyr Ser Tyr Gly Ile Ile Ala Gln Glu Ile Ile Leu
            660                 665                 670

Arg Lys Glu Thr Phe Tyr Thr Leu Ser Cys Arg Asp Arg Asn Glu Lys
        675                 680                 685

Ile Phe Arg Val Glu Asn Ser Asn Gly Met Lys Pro Phe Arg Pro Asp
    690                 695                 700

Leu Phe Leu Glu Thr Ala Glu Glu Lys Glu Leu Glu Val Tyr Leu Leu
705                 710                 715                 720

Val Lys Asn Cys Trp Glu Glu Asp Pro Glu Lys Arg Pro Asp Phe Lys
                725                 730                 735

Lys Ile Glu Thr Thr Leu Ala Lys Ile Phe Gly Leu Phe His Asp Gln
            740                 745                 750

Lys Asn Glu Ser Tyr Met Asp Thr Leu Ile Arg Arg Leu Gln Leu Tyr
        755                 760                 765

Ser Arg Asn Leu Glu His Leu Val Glu Glu Arg Thr Gln Leu Tyr Lys
    770                 775                 780

Ala Glu Arg Asp Arg Ala Asp Arg Leu Asn Phe Met Leu Leu Pro Arg
785                 790                 795                 800

Leu Val Val Lys Ser Leu Lys Glu Lys Gly Phe Val Glu Pro Glu Leu
                805                 810                 815

Tyr Glu Glu Val Thr Ile Tyr Phe Ser Asp Ile Val Gly Phe Thr Thr
            820                 825                 830

Ile Cys Lys Tyr Ser Thr Pro Met Glu Val Val Asp Met Leu Asn Asp
        835                 840                 845

Ile Tyr Lys Ser Phe Asp His Ile Val Asp His His Asp Val Tyr Lys
    850                 855                 860

Val Glu Thr Ile Gly Asp Ala Tyr Met Val Ala Ser Gly Leu Pro Lys
865                 870                 875                 880

Arg Asn Gly Asn Arg His Ala Ile Asp Ile Ala Lys Met Ala Leu Glu
                885                 890                 895

Ile Leu Ser Phe Met Gly Thr Phe Glu Leu Glu His Leu Pro Gly Leu
            900                 905                 910

Pro Ile Trp Ile Arg Ile Gly Val His Ser Gly Pro Cys Ala Ala Gly
        915                 920                 925

Val Val Gly Ile Lys Met Pro Arg Tyr Cys Leu Phe Gly Asp Thr Val
    930                 935                 940

Asn Thr Ala Ser Arg Met Glu Ser Thr Gly Leu Pro Leu Arg Ile His
945                 950                 955                 960

Val Ser Gly Ser Thr Ile Ala Ile Leu Lys Arg Thr Glu Cys Gln Phe
                965                 970                 975

Leu Tyr Glu Val Arg Gly Glu Thr Tyr Leu Lys Gly Arg Gly Asn Glu
            980                 985                 990

Thr Thr Tyr Trp Leu Thr Gly Met Lys Asp Gln Lys Phe Asn Leu Pro
        995                 1000                1005

Thr Pro Pro Thr Val Glu Asn Gln Gln Arg Leu Gln Ala Glu Phe
    1010                1015                1020

Ser Asp Met Ile Ala Asn Ser Leu Gln Lys Arg Gln Ala Ala Gly
    1025                1030                1035

Ile Arg Ser Gln Lys Pro Arg Arg Val Ala Ser Tyr Lys Lys Gly
    1040                1045                1050

Thr Leu Glu Tyr Leu Gln Leu Asn Thr Thr Asp Lys Glu Ser Thr
    1055                1060                1065

Tyr Phe
    1070

<210>  15

<211>  93

<212>  PRT

<213>  Homo sapiens


<400>  15

Met Lys Leu Val Thr Ile Phe Leu Leu Val Thr Ile Ser Leu Cys Ser
1               5                   10                  15

Tyr Ser Ala Thr Ala Lys Leu Ile Asn Lys Cys Pro Leu Pro Val Asp
            20                  25                  30

Lys Leu Ala Pro Leu Pro Leu Asp Asn Ile Leu Pro Phe Met Asp Pro
        35                  40                  45

Leu Lys Leu Leu Leu Lys Thr Leu Gly Ile Ser Val Glu His Leu Val
    50                  55                  60

Glu Gly Leu Arg Lys Cys Val Asn Glu Leu Gly Pro Glu Ala Ser Glu
65                  70                  75                  80

Ala Val Lys Lys Leu Leu Glu Ala Leu Ser His Leu Val
                85                  90

<210>  16

<211>  261

<212>  PRT

<213>  Homo sapiens


<400>  16

Met Ala Val Thr Ala Cys Gln Gly Leu Gly Phe Val Val Ser Leu Ile
1               5                   10                  15

Gly Ile Ala Gly Ile Ile Ala Ala Thr Cys Met Asp Gln Trp Ser Thr
            20                  25                  30

Gln Asp Leu Tyr Asn Asn Pro Val Thr Ala Val Phe Asn Tyr Gln Gly
        35                  40                  45

Leu Trp Arg Ser Cys Val Arg Glu Ser Ser Gly Phe Thr Glu Cys Arg
    50                  55                  60

Gly Tyr Phe Thr Leu Leu Gly Leu Pro Ala Met Leu Gln Ala Val Arg
65                  70                  75                  80

Ala Leu Met Ile Val Gly Ile Val Leu Gly Ala Ile Gly Leu Leu Val
                85                  90                  95

Ser Ile Phe Ala Leu Lys Cys Ile Arg Ile Gly Ser Met Glu Asp Ser
            100                 105                 110

Ala Lys Ala Asn Met Thr Leu Thr Ser Gly Ile Met Phe Ile Val Ser
        115                 120                 125

Gly Leu Cys Ala Ile Ala Gly Val Ser Val Phe Ala Asn Met Leu Val
    130                 135                 140

Thr Asn Phe Trp Met Ser Thr Ala Asn Met Tyr Thr Gly Met Gly Gly
145                 150                 155                 160

Met Val Gln Thr Val Gln Thr Arg Tyr Thr Phe Gly Ala Ala Leu Phe
                165                 170                 175

Val Gly Trp Val Ala Gly Gly Leu Thr Leu Ile Gly Gly Val Met Met
            180                 185                 190

Cys Ile Ala Cys Arg Gly Leu Ala Pro Glu Glu Thr Asn Tyr Lys Ala
        195                 200                 205

Val Ser Tyr His Ala Ser Gly His Ser Val Ala Tyr Lys Pro Gly Gly
    210                 215                 220

Phe Lys Ala Ser Thr Gly Phe Gly Ser Asn Thr Lys Asn Lys Lys Ile
225                 230                 235                 240

Tyr Asp Gly Gly Ala Arg Thr Glu Asp Glu Val Gln Ser Tyr Pro Ser
                245                 250                 255

Lys His Asp Tyr Val
            260

<210>  17

<211>  10

<212>  PRT

<213>  Homo sapiens


<400>  17

Asp Gln Trp Ser Thr Gln Asp Leu Tyr Asn
1               5                   10

<210>  18

<211>  11

<212>  PRT

<213>  Homo sapiens


<400>  18

Asn Asn Pro Val Thr Ala Val Phe Asn Tyr Gln
1               5                   10

<210>  19

<211>  47

<212>  PRT

<213>  Homo sapiens


<400>  19

Met Ala Val Thr Ala Cys Gln Gly Leu Gly Phe Val Val Ser Leu Ile
1               5                   10                  15

Gly Ile Ala Gly Ile Ile Ala Ala Thr Cys Met Asp Gln Trp Ser Thr
            20                  25                  30

Gln Asp Leu Tyr Asn Asn Pro Val Thr Ala Val Phe Asn Tyr Gln
        35                  40                  45

<210>  20

<211>  21

<212>  DNA

<213>  Künstliche Sequenz

<220>
<223>  Beschreibung der künstlichen Sequenz: Oligonukleotid

<400>  20
aggtacatga gcatcagcct g                                                21

<210>  21

<211>  21

<212>  DNA

<213>  Künstliche Sequenz

<220>
<223>  Beschreibung der künstlichen Sequenz: Oligonukleotid

<400>  21
gcagcagttg gcatctgaga g                                                21

<210>  22

<211>  21

<212>  DNA

<213>  Künstliche Sequenz

<220>
<223>  Beschreibung der künstlichen Sequenz: Oligonukleotid

<400>  22
gcaatagaca ttgccaagat g                                                21

<210>  23

<211>  21

<212>  DNA

<213>  Künstliche Sequenz

<220>
<223>  Beschreibung der künstlichen Sequenz: Oligonukleotid

<400>  23
aacgctgttg attctccaca g                                                21

<210> 24

<211> 33

<212> DNA

<213> Künstliche Sequenz

<220>
<223> Beschreibung der künstlichen Sequenz: Oligonukleotid

<400> 24
ggatcctcct ttagttccca ggtgagtcag aac 33

<210> 25

<211> 21

<212> DNA

<213> Künstliche Sequenz

<220>
<223> Beschreibung der künstlichen Sequenz: Oligonukleotid

<400> 25
tgctctggag gctagcgttt c 21

<210> 26

<211> 21

<212> DNA

<213> Künstliche Sequenz

<220>
<223> Beschreibung der künstlichen Sequenz: Oligonukleotid

<400> 26
accaatcatg ttagcctcaa g 21

<210> 27

<211> 21

<212> DNA

<213> Künstliche Sequenz

<220>
<223> Beschreibung der künstlichen Sequenz: Oligonukleotid

<400>  27
agctatggga tcatcgcaca g                                                21

<210>  28
<211>  21
<212>  DNA
<213>  Künstliche Sequenz

<220>
<223>  Beschreibung der künstlichen Sequenz: Oligonukleotid

<400>  28
cctttgagct ggagcatctt c                                                21

<210>  29
<211>  21
<212>  DNA
<213>  Künstliche Sequenz

<220>
<223>  Beschreibung der künstlichen Sequenz: Oligonukleotid

<400>  29
ctttctagct ggagacatca g                                                21

<210>  30
<211>  21
<212>  DNA
<213>  Künstliche Sequenz

<220>
<223>  Beschreibung der künstlichen Sequenz: Oligonukleotid

<400>  30
caccatggta ctgtcaacat c                                                21

<210>  31
<211>  21
<212>  DNA
<213>  Künstliche Sequenz

<220>
<223>  Beschreibung der künstlichen Sequenz: Oligonukleotid

<400>  31
atgtcataca agacagagat c                                                21

<210>  32
<211>  21
<212>  DNA
<213>  Künstliche Sequenz

<220>
<223>  Beschreibung der künstlichen Sequenz: Oligonukleotid

<400>  32
tctgccttgt acagctgtgt c                                                21

<210>  33
<211>  21
<212>  DNA
<213>  Künstliche Sequenz

<220>
<223>  Beschreibung der künstlichen Sequenz: Oligonukleotid

<400>  33
tctgtggtat tcagctgcaa g                                                21

<210>  34
<211>  22
<212>  DNA
<213>  Künstliche Sequenz

<220>
<223>  Beschreibung der künstlichen Sequenz: Oligonukleotid

<400>  34
tactcaggaa aatttcacct tg                                               22

<210>  35
<211>  27

<212>  DNA

<213>  Künstliche Sequenz

<220>
<223>  Beschreibung der künstlichen Sequenz: Oligonukleotid

<400>  35
gaccacaaca ggaaaagcaa tgtgacc                                          27

<210>  36

<211>  22

<212>  DNA

<213>  Künstliche Sequenz

<220>
<223>  Beschreibung der künstlichen Sequenz: Oligonukleotid

<400>  36
gatagaattg aacaagattg ac                                               22

<210>  37

<211>  21

<212>  DNA

<213>  Künstliche Sequenz

<220>
<223>  Beschreibung der künstlichen Sequenz: Oligonukleotid

<400>  37
cagcctttgt agttactctg c                                                21

<210>  38

<211>  21

<212>  DNA

<213>  Künstliche Sequenz

<220>
<223>  Beschreibung der künstlichen Sequenz: Oligonukleotid

<400>  38
tgtcacacca agtgtgatag c                                                21

<210> 39

<211> 28

<212> DNA

<213> Künstliche Sequenz

<220>
<223> Beschreibung der künstlichen Sequenz: Oligonukleotid

<400> 39
ggttcgtggt ttcactgatt gggattgc 28

<210> 40

<211> 27

<212> DNA

<213> Künstliche Sequenz

<220>
<223> Beschreibung der künstlichen Sequenz: Oligonukleotid

<400> 40
cggctttgta gttggtttct tctggtg 27

<210> 41

<211> 3814

<212> DNA

<213> Homo sapiens

<400> 41
ctattgaagc cacctgctca ggacaatgaa attcttcagt tacattctgg tttatcgccg 60

atttctcttc gtggttttca ctgtgttggt tttactacct ctgcccatcg tcctccacac 120

caaggaagca gaatgtgcct acacactctt tgtggtcgcc acattttggc tcacagaagc 180

attgcctctg tcggtaacag ctttgctacc tagtttaatg ttacccatgt ttgggatcat 240

gccttctaag aaggtggcat ctgcttattt caaggatttt cacttactgc taattggagt 300

tatctgttta gcaacatcca tagaaaaatg gaatttgcac aagagaattg ctctgaaaat 360

ggtgatgatg gttggtgtaa atcctgcatg gctgacgctg gggttcatga gcagcactgc 420

ctttttgtct atgtggctca gcaacacctc gacggctgcc atggtgatgc ccattgcgga 480

ggctgtagtg cagcagatca tcaatgcaga agcagaggtc gaggccactc agatgactta 540

cttcaacgga tcaaccaacc acggactaga aattgatgaa agtgttaatg gacatgaaat 600

aaatgagagg aaagagaaaa caaaaccagt tccaggatac aataatgata cagggaaaat 660

ttcaagcaag gtggagttgg aaaagaactc aggcatgaga accaaatatc gaacaaagaa 720
gggccacgtg acacgtaaac ttacgtgttt gtgcattgcc tactcttcta ccattggtgg 780
actgacaaca atcactggta cctccaccaa cttgatcttt gcagagtatt tcaatacacg 840
ctatcctgac tgtcgttgcc tcaactttgg atcatggttt acgttttcct tcccagctgc 900
ccttatcatt ctactcttat cctggatctg gcttcagtgg cttttcctag gattcaattt 960
taaggagatg ttcaaatgtg gcaaaaccaa aacagtccaa caaaaagctt gtgctgaggt 1020
gattaagcaa gaataccaaa agcttgggcc aataaggtat caagaaattg tgaccttggt 1080
cctcttcatt ataatggctc tgctatggtt tagtcgagac cccggatttg ttcctggttg 1140
gtctgcactt ttttcagagt accctggttt tgctacagat tcaactgttg ctttacttat 1200
agggctgcta ttctttctta tcccagctaa gacactgact aaaactacac ctacaggaga 1260
aattgttgct tttgattact ctccactgat tacttggaaa gaattccagt cattcatgcc 1320
ctgggatata gccattcttg ttggtggagg gtttgccctg gcagatggtt gtgaggagtc 1380
tggattatct aagtggatag gaaataaatt atctcctctg ggttcattac cagcatggct 1440
aataattctg atatcttctt tgatggtgac atctttaact gaggtagcca gcaatccagc 1500
taccattaca ctctttctcc caatattatc tccattggcc gaagccattc atgtgaaccc 1560
tctttatatt ctgatacctt ctactctgtg tacttcattt gcattcctcc taccagtagc 1620
aaatccaccc aatgctattg tcttttcata tggtcatctg aaagtcattg acatggttaa 1680
agctggactt ggtgtcaaca ttgttggtgt tgctgtggtt atgcttggca tatgtacttg 1740
gattgtaccc atgtttgacc tctacactta cccttcgtgg gctcctgcta tgagtaatga 1800
gaccatgcca taataagcac aaaatttctg actatcttgc ggtaatttct ggaagacatt 1860
aatgattgac tgtaaaatgt ggctctaaat aactaatgac acacatttaa atcagttatg 1920
gtgtagctgc tgcaattccc gtgaataccc gaaacctgct ggtataactc agagtccata 1980
tttgttattg cagtgcaact aaagagcatc tatgtgcctt catcaagaag cccatgtttt 2040
gagattttgc tcatgaacca tctgcaactt gcttcatcat aagaataatt tataacttga 2100
ccttcaaaga gattagagca tttgtttcat cttacagttg gagttcaatg taacatttta 2160
aatgcaattt attatttcag aaatttccca tgaaactaaa aatagaaaat aagatataca 2220
agttaattcg gtacttggat aaatcatttc tgcattgttg ttccagagaa tttgctgaga 2280
aatcaaagcc atggtcatct ggtgatgaag agaaaaggtt aatctaaatg atatgtgcat 2340
ttcctcattt aaaaaatcca attggattat tcttaatata tacatgtaat atgaaaattg 2400
agattgaagc actaattcca aaattatggc tgaatatact aaataacaga aaagttacag 2460
ataagaattt atttctactg aactctatag ttagtgtaat ataattcata tttttatgat 2520
attggcacac tgagaaattc attttgtaga gctatggata aggcttgcta tgatttgcac 2580
tattagtaca gtatagttag aaaggaaagc tgaacactat aaaactatta acatattttc 2640
gtatatgagt aacaactttg cttaagtgtt tatcttagtt cagaaataca taatgtcata 2700

tgttaaaaat aaagagatgt agaaatctaa atgaattatc actgtgtata cagacagaaa 2760
aatcacataa ctctggtgtg ttaacattgc aatgaaaaaa tgaaaaaaag aaggaaaaaa 2820
gaataagaat gaaaactgct gacgtattac aaaacagaaa aataaatgat ttaaaatcaa 2880
atcaaaaaga aaaaaactaa acatttaaac aaaaatggga taagaatagt cttctagaag 2940
tgaggatgcg taaaagaatg agtttccaat taccctgatg tgacaattac acattgtaga 3000
caggtagcaa aatatcacat acacccccaa aatatgtaca aatattatat atcaataaat 3060
aaatttttaa agagtaagtg ctattggcat tccaaaattc agctaaagga aaaatgatca 3120
aaaacaaagt aaggtgcaca gttagcaaaa gatgcagatg ttatatcaca gcaattctca 3180
tgctaaaaat acaacaaaag acaaagcaaa aaataaacct ttgctttttt tttttttttt 3240
tttttttttt gagacggagt ctcgctctgt cgcccaggct ggagtgcagt ggcgggatct 3300
cggctcactg caagctccgc ctcccaggtt cacgccattc tcctgcctca gccaaacctt 3360
tgctattttt aatcttcgtt ggcactttcc agctgttact gaccttgtca ttttttgttc 3420
aaataagatt atttacaaac ttattcttga aactaaatat agtaaagagg gtttttaaaa 3480
taatatttaa catacgaatt attaattggc catgttcatt atttatctat gtttattaat 3540
gggccaatgc aaaaaatcat tttttcaaag aaaaatttgt ccatgtaaag cttaaattat 3600
aatattgctg ctttgtataa ctcttctatg tttattctat tcatttgttc ctttccctac 3660
catattttac acatgtattt ataatctgta gtatttatta catttctgct tttttctagt 3720
cattcaattt atcactgctg aattgcatca gatcatggat gcatttttat tatgaaaaaa 3780
taaaatgact tttcaaatta aaaaaaaaaa aaaa 3814

<210> 42
<211> 734
<212> DNA
<213> Homo sapiens

<400> 42
caggacaatg aaattcttca gttacattct ggtttatcgc cgatttctct tcgtggtttt 60
cactgtgttg gttttactac ctctgcccat cgtcctccac accaaggaag cagaatgtgc 120
ctacacactc tttgtggtcg ccacattttg gctcacagaa gcattgcctc tgtcggtaac 180
agctttgcta cctagtttaa tgttacccat gtttgggatc atgccttcta agaaggtggc 240
atctgcttat ttcaaggatt ttcacttact gctaattgga gttatctgtt tagcaacatc 300
catagaaaaa tggaatttgc acaagagaat tgctctgaaa atggtgatga tggttggtgt 360
aaatcctgca tggctgacgc tggggttcat gagcagcact gcctttttgt ctatgtggct 420
cagcaacacc tcgacggctg ccatggtgat gcccattgcg gaggctgtag tgcagcagat 480
catcaatgca gaagcagagg tcgaggccac tcagatgact tacttcaacg gatcaaccaa 540

ccacggacta gaaattgatg aaagtgttaa tggacatgaa ataaatgaga ggaaagagaa 600
aacaaaacca gttccaggat acaataatga tacagggaaa atttcaagca aggtggagtt 660
ggaaaagact gtttaactac tgaaatgaag ctattctcct gactaaacat aactgaaaaa 720
ccattcatta aatg 734

<210> 43
<211> 539
<212> DNA
<213> Homo sapiens

<400> 43
gccactcaga tgacttactt caacggatca accaaccacg gactagaaat tgatgaaagt 60
gttaatggac atgaaataaa tgagaggaaa gagaaaacaa aaccagttcc aggatacaat 120
aatgatacag ggaaaatttc aagcaaggtg gagttggaaa agcactggaa acttgcagtt 180
caagatggct ccccatctcc ctctgtccat tctgtatcgc agctagctgc tcaaggaaag 240
gagaaagtgg aaggcatatg tacttagaaa ttattctatt actttcctgg atttaagagt 300
attcagattt tctatttcaa catcaaacaa ttgcattttt aaaaagaaat ttatgtgttc 360
catgtcaaat ttagtagtgt gtggttgttt ataatatttt cttatatcta cttaatttct 420
atagtattta tagttatatg tctttatttc taacattttt cttgtgcttt taaagattat 480
ttaaagatta tttttaaata atctttattt catttaaata aaatatttta tttaagtct 539

<210> 44
<211> 556
<212> DNA
<213> Homo sapiens

<400> 44
cacggactag aaattgatga aagtgttaat ggacatgaaa taaatgagag gaaagagaaa 60
acaaaaccag ttccaggata caataatgat acagggaaaa tttcaagcaa ggtggagttg 120
gaaaagaact caggcatgag aaccaaatat cgaacaaaga agggccacgt gacacgtaaa 180
cttacgtgtt tgtgcattgc ctactcttct accattggtg gactgacaac aatcactggt 240
acctccacca acttgatctt tgcagagtat ttcaatacat tccatccaca cagaagagga 300
gatcgtacaa ggcatgtaca ccaggaggca gaaatttgag gcatatcttg gaactctgtc 360
taccacatcc tgaacatcac acagtttcca ctcttgttgc cttcaatcct gagaatgcat 420
ccaggagcca ttctgtttta tgtcaattac taattagatc atgtcacgtt actaacttac 480
tacgttccaa ttagtcctta ttgcatttgt aataaaatcc gcatactttc ggactggcta 540

caaggttata catgat                                                    556

<210>  45

<211>  595

<212>  PRT

<213>  Homo sapiens

<400>  45

Met Lys Phe Phe Ser Tyr Ile Leu Val Tyr Arg Arg Phe Leu Phe Val
1               5                   10                  15

Val Phe Thr Val Leu Val Leu Leu Pro Leu Pro Ile Val Leu His Thr
            20                  25                  30

Lys Glu Ala Glu Cys Ala Tyr Thr Leu Phe Val Val Ala Thr Phe Trp
        35                  40                  45

Leu Thr Glu Ala Leu Pro Leu Ser Val Thr Ala Leu Leu Pro Ser Leu
    50                  55                  60

Met Leu Pro Met Phe Gly Ile Met Pro Ser Lys Lys Val Ala Ser Ala
65                  70                  75                  80

Tyr Phe Lys Asp Phe His Leu Leu Leu Ile Gly Val Ile Cys Leu Ala
                85                  90                  95

Thr Ser Ile Glu Lys Trp Asn Leu His Lys Arg Ile Ala Leu Lys Met
            100                 105                 110

Val Met Met Val Gly Val Asn Pro Ala Trp Leu Thr Leu Gly Phe Met
        115                 120                 125

Ser Ser Thr Ala Phe Leu Ser Met Trp Leu Ser Asn Thr Ser Thr Ala
    130                 135                 140

Ala Met Val Met Pro Ile Ala Glu Ala Val Val Gln Gln Ile Ile Asn
145                 150                 155                 160

Ala Glu Ala Glu Val Glu Ala Thr Gln Met Thr Tyr Phe Asn Gly Ser
                165                 170                 175

Thr Asn His Gly Leu Glu Ile Asp Glu Ser Val Asn Gly His Glu Ile
            180                 185                 190

Asn Glu Arg Lys Glu Lys Thr Lys Pro Val Pro Gly Tyr Asn Asn Asp
        195                 200                 205

Thr Gly Lys Ile Ser Ser Lys Val Glu Leu Glu Lys Asn Ser Gly Met
    210                 215                 220

Arg Thr Lys Tyr Arg Thr Lys Lys Gly His Val Thr Arg Lys Leu Thr
225                 230                 235                 240

Cys Leu Cys Ile Ala Tyr Ser Ser Thr Ile Gly Gly Leu Thr Thr Ile
                245                 250                 255

Thr Gly Thr Ser Thr Asn Leu Ile Phe Ala Glu Tyr Phe Asn Thr Arg
            260                 265                 270

Tyr Pro Asp Cys Arg Cys Leu Asn Phe Gly Ser Trp Phe Thr Phe Ser
        275                 280                 285

Phe Pro Ala Ala Leu Ile Ile Leu Leu Leu Ser Trp Ile Trp Leu Gln
    290                 295                 300

Trp Leu Phe Leu Gly Phe Asn Phe Lys Glu Met Phe Lys Cys Gly Lys
305                 310                 315                 320

Thr Lys Thr Val Gln Gln Lys Ala Cys Ala Glu Val Ile Lys Gln Glu
                325                 330                 335

Tyr Gln Lys Leu Gly Pro Ile Arg Tyr Gln Glu Ile Val Thr Leu Val
            340                 345                 350

Leu Phe Ile Ile Met Ala Leu Leu Trp Phe Ser Arg Asp Pro Gly Phe
        355                 360                 365

Val Pro Gly Trp Ser Ala Leu Phe Ser Glu Tyr Pro Gly Phe Ala Thr
    370                 375                 380

Asp Ser Thr Val Ala Leu Leu Ile Gly Leu Leu Phe Phe Leu Ile Pro
385                 390                 395                 400

Ala Lys Thr Leu Thr Lys Thr Thr Pro Thr Gly Glu Ile Val Ala Phe
                405                 410                 415

Asp Tyr Ser Pro Leu Ile Thr Trp Lys Glu Phe Gln Ser Phe Met Pro
            420                 425                 430

Trp Asp Ile Ala Ile Leu Val Gly Gly Gly Phe Ala Leu Ala Asp Gly
        435                 440                 445

Cys Glu Glu Ser Gly Leu Ser Lys Trp Ile Gly Asn Lys Leu Ser Pro
    450                 455                 460

Leu Gly Ser Leu Pro Ala Trp Leu Ile Ile Leu Ile Ser Ser Leu Met
465                 470                 475                 480

Val Thr Ser Leu Thr Glu Val Ala Ser Asn Pro Ala Thr Ile Thr Leu
                485                 490                 495

Phe Leu Pro Ile Leu Ser Pro Leu Ala Glu Ala Ile His Val Asn Pro
            500                 505                 510

Leu Tyr Ile Leu Ile Pro Ser Thr Leu Cys Thr Ser Phe Ala Phe Leu
        515                 520                 525

Leu Pro Val Ala Asn Pro Pro Asn Ala Ile Val Phe Ser Tyr Gly His
    530                 535                 540

Leu Lys Val Ile Asp Met Val Lys Ala Gly Leu Gly Val Asn Ile Val
545                 550                 555                 560

Gly Val Ala Val Val Met Leu Gly Ile Cys Thr Trp Ile Val Pro Met
                565                 570                 575

Phe Asp Leu Tyr Thr Tyr Pro Ser Trp Ala Pro Ala Met Ser Asn Glu
            580                 585                 590

Thr Met Pro
        595

<210>  46

<211>  224

<212>  PRT

<213>  Homo sapiens


<400>  46

Arg Thr Met Lys Phe Phe Ser Tyr Ile Leu Val Tyr Arg Arg Phe Leu
1               5                   10                  15

Phe Val Val Phe Thr Val Leu Val Leu Leu Pro Leu Pro Ile Val Leu
            20                  25                  30

His Thr Lys Glu Ala Glu Cys Ala Tyr Thr Leu Phe Val Val Ala Thr
        35                  40                  45

Phe Trp Leu Thr Glu Ala Leu Pro Leu Ser Val Thr Ala Leu Leu Pro
    50                  55                  60

Ser Leu Met Leu Pro Met Phe Gly Ile Met Pro Ser Lys Lys Val Ala
65                  70                  75                  80

Ser Ala Tyr Phe Lys Asp Phe His Leu Leu Leu Ile Gly Val Ile Cys
                85                  90                  95

Leu Ala Thr Ser Ile Glu Lys Trp Asn Leu His Lys Arg Ile Ala Leu
            100                 105                 110

Lys Met Val Met Met Val Gly Val Asn Pro Ala Trp Leu Thr Leu Gly
        115                 120                 125

Phe Met Ser Ser Thr Ala Phe Leu Ser Met Trp Leu Ser Asn Thr Ser
    130                 135                 140

Thr Ala Ala Met Val Met Pro Ile Ala Glu Ala Val Val Gln Gln Ile
145                 150                 155                 160

Ile Asn Ala Glu Ala Glu Val Glu Ala Thr Gln Met Thr Tyr Phe Asn
                165                 170                 175

Gly Ser Thr Asn His Gly Leu Glu Ile Asp Glu Ser Val Asn Gly His
            180                 185                 190

Glu Ile Asn Glu Arg Lys Glu Lys Thr Lys Pro Val Pro Gly Tyr Asn
        195                 200                 205

Asn Asp Thr Gly Lys Ile Ser Ser Lys Val Glu Leu Glu Lys Thr Val
    210                 215                 220

<210>  47

<211>  88

<212>  PRT

<213>  Homo sapiens


<400>  47

Ala Thr Gln Met Thr Tyr Phe Asn Gly Ser Thr Asn His Gly Leu Glu
1               5                   10                  15

Ile Asp Glu Ser Val Asn Gly His Glu Ile Asn Glu Arg Lys Glu Lys
            20                  25                  30

Thr Lys Pro Val Pro Gly Tyr Asn Asn Asp Thr Gly Lys Ile Ser Ser
        35                  40                  45

Lys Val Glu Leu Glu Lys His Trp Lys Leu Ala Val Gln Asp Gly Ser
    50                  55                  60

Pro Ser Pro Ser Val His Ser Val Ser Gln Leu Ala Ala Gln Gly Lys
65                  70                  75                  80

Glu Lys Val Glu Gly Ile Cys Thr
                85

<210> 48

<211> 112

<212> PRT

<213> Homo sapiens

<400> 48

His Gly Leu Glu Ile Asp Glu Ser Val Asn Gly His Glu Ile Asn Glu
1               5                   10                  15

Arg Lys Glu Lys Thr Lys Pro Val Pro Gly Tyr Asn Asn Asp Thr Gly
            20                  25                  30

Lys Ile Ser Ser Lys Val Glu Leu Glu Lys Asn Ser Gly Met Arg Thr
        35                  40                  45

Lys Tyr Arg Thr Lys Lys Gly His Val Thr Arg Lys Leu Thr Cys Leu
    50                  55                  60

Cys Ile Ala Tyr Ser Ser Thr Ile Gly Gly Leu Thr Thr Ile Thr Gly
65                  70                  75                  80

Thr Ser Thr Asn Leu Ile Phe Ala Glu Tyr Phe Asn Thr Phe His Pro
                85                  90                  95

His Arg Arg Gly Asp Arg Thr Arg His Val His Gln Glu Ala Glu Ile
            100                 105                 110

<210> 49

<211> 21

<212> DNA

<213> Künstliche Sequenz

<220>
<223> Beschreibung der künstlichen Sequenz: Oligonukleotid

<400> 49
ccagctttaa ccatgtcaat g                                              21

<210> 50

<211> 21

<212> DNA

<213> Künstliche Sequenz

<220>

<223>  Beschreibung der künstlichen Sequenz: Oligonukleotid

<400>  50
cagatggttg tgaggagtct g                                                21

<210>  51
<211>  3311
<212>  DNA
<213>  Homo sapiens

<400>  51
tgctaatgct tttggtacaa atggatgtgg aatataattg aatattttct tgtttaaggg      60
gagcatgaag aggtgttgag gttatgtcaa gcatctggca cagctgaagg cagatggaaa     120
tatttacaag tacgcaattt gagactaaga tattgttatc attctcctat tgaagacaag     180
agcaatagta aaacacatca ggtcaggggg ttaaagacct gtgataaacc acttccgata     240
agttggaaac gtgtgtctat attttcatat ctgtatatat ataatggtaa agaaagacac     300
cttcgtaacc cgcattttcc aaagagagga atcacaggga gatgtacagc aatggggcca     360
tttaagagtt ctgtgttcat cttgattctt caccttctag aaggggccct gagtaattca     420
ctcattcagc tgaacaacaa tggctatgaa ggcattgtcg ttgcaatcga ccccaatgtg     480
ccagaagatg aaacactcat tcaacaaata aaggacatgg tgacccaggc atctctgtat     540
ctgtttgaag ctacaggaaa gcgattttat ttcaaaaatg ttgccatttt gattcctgaa     600
acatggaaga caaaggctga ctatgtgaga ccaaaacttg agacctacaa aaatgctgat     660
gttctggttg ctgagtctac tcctccaggt aatgatgaac cctacactga gcagatgggc     720
aactgtggag agaagggtga aaggatccac ctcactcctg atttcattgc aggaaaaaag     780
ttagctgaat atggaccaca aggtaaggca tttgtccatg agtgggctca tctacgatgg     840
ggagtatttg acgagtacaa taatgatgag aaattctact tatccaatgg aagaatacaa     900
gcagtaagat gttcagcagg tattactggt acaaatgtag taaagaagtg tcagggaggc     960
agctgttaca ccaaaagatg cacattcaat aaagttacag gactctatga aaaaggatgt    1020
gagtttgttc tccaatcccg ccagacggag aaggcttcta taatgtttgc acaacatgtt    1080
gattctatag ttgaattctg tacagaacaa aaccacaaca aagaagctcc aaacaagcaa    1140
aatcaaaaat gcaatctccg aagcacatgg gaagtgatcc gtgattctga ggactttaag    1200
aaaaccactc ctatgacaac acagccacca aatcccacct tctcattgct gcagattgga    1260
caaagaattg tgtgtttagt ccttgacaaa tctggaagca tggcgactgg taaccgcctc    1320
aatcgactga atcaagcagg ccagcttttc ctgctgcaga cagttgagct ggggtcctgg    1380
gttgggatgg tgacatttga cagtgctgcc catgtacaaa gtgaactcat acagataaac    1440
agtggcagtg acagggacac actcgccaaa agattacctg cagcagcttc aggagggacg    1500

tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580
aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880
attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaattttct aaaatgtatt ttagacttcc 3180
tgtagggggc gatatactaa atgtatatag tacatttata ctaaatgtat tcctgtaggg 3240
ggcgatatac taaatgtatt ttagacttcc tgtagggggc gataaaataa aatgctaaac 3300
aactgggtaa a 3311

<210> 52
<211> 3067

<212>  DNA
<213>  Homo sapiens

<400>  52
aattaaatta tgagaattaa aaagacaaca ttgagcagag atgaaaaagg aagggaggaa      60
aaggtggaaa agaaaagaag acaagaagcg agtagtggtc tctaacttgc tctttgaagg     120
atggtctcac aaagagaacc ccaacagaca tcatcgtggg aatcaaatca agaccagcaa     180
gtacaccgtg ttgtccttcg tccccaaaaa catttttgag cagctacacc ggtttgccaa     240
tctctatttt gtgggcattg cggttctgaa ttttatccct gtggtcaatg ctttccagcc     300
tgaggtgagc atgataccaa tctgtgttat cctggcagtc actgccatca aggacgcttg     360
ggaagacctc cggaggtaca aatcggataa agtcatcaat aaccgagagt gcctcatcta     420
cagcagaaaa gagcagacct atgtgcagaa gtgctggaag gatgtgcgtg tgggagactt     480
catccaaatg aaatgcaatg agattgtccc agcagacata ctcctccttt tttcctctga     540
ccccaatggg atatgccatc tggaaactgc cagcttggat ggagagacaa acctcaagca     600
aagacgtgtc gtgaagggct tctcacagca ggaggtacag ttcgaaccag agcttttcca     660
caataccatc gtgtgtgaga aacccaacaa ccacctcaac aaatttaagg gttatatgga     720
gcatcctgac cagaccagga ctggctttgg ctgtgagagt cttctgcttc gaggctgcac     780
catcagaaac accgagatgg ctgttggcat tgtcatctat gcaggccatg agacgaaagc     840
catgctgaac aacagtggcc cccggtacaa acgcagcaag attgagcggc gcatgaatat     900
agacatcttc ttctgcattg ggatcctcat cctcatgtgc cttattggag ctgtaggtca     960
cagcatctgg aatgggacct ttgaagaaca ccctcccttc gatgtgccag atgccaatgg    1020
cagcttcctt cccagtgccc ttgggggctt ctacatgttc ctcacaatga tcatcctgct    1080
ccaggtgctg atccccatct ctttgtatgt ctccattgag ctggtgaagc tcgggcaagt    1140
gttcttcttg agcaatgacc ttgacctgta tgatgaagag accgatttat ccattcaatg    1200
tcgagccctc aacatcgcag aggacttggg ccagatccag tacatcttct ccgataagac    1260
ggggaccctg acagagaaca agatggtgtt ccgacgttgc accatcatgg gcagcgagta    1320
ttctcaccaa gaaaatggta tagaagctcc caagggctcc atccctcttt ctaaaaggaa    1380
ataccctgct ctcctaagaa acgaggagat aaaagacatt ctcctggctc tcttagaggc    1440
tgtgtggcat ttccacaagt tgcttcctgt atccctgtgg tcttccttgt cacagatcag    1500
ggctgttcca attacttgta aactttcatt tgtttacaaa ggttagaagt tatcccatat    1560
gtggttcccc ttcagctgat ctttgtctgg tgccagacaa agcactttat gagacgagtt    1620
ttttatctgt cagcaatgga ttggagacat ttcccaattg tgtgccagtc acacaaccaa    1680
ggcttaggaa tttctcaggc caccttacct gacatgtcag ggcaggtctg tgtctaggtg    1740
catggtcaga tttaatacat ccagaagatg tcttctattc taacagatct cttagcttgt    1800
cactgaggca aagttttgat ttaggagata gggctataaa atgcctggac tgttaccttg    1860

catggactga atatgactca taaaactgat ctgattcctt cagccatcat ctgcccaact 1920
tggttcccct ccccaccccc ccacaacaca cacacacact ttctaagaaa agaaaagaaa 1980
ttcttttttt tcaatacttt aagttctggg atacatgtgc agaatgtgca ggtttgttac 2040
ataggtatac atgtgtcatg gtggtttgca gcacccacca acccatcatc taccttaggt 2100
atttctccta atgctatccc tcccctagcc cccaaccccc cgatgggctc cagtgtgtga 2160
tgttcccctc catgtccatg tgttctcatt gttcaattcc cacttatgag tgagaacatg 2220
cagtatttgg ttttctgttc ttgtgttagt ttgctgatgg tttcctgttc atccgtgtcc 2280
ctgcaaagga catgaactca tcctttttta tggctgcata atattccatg gtgtatatgt 2340
gccacatttt ctttatccag tctatcgctg atgggcactg gggttggttc caagtctttg 2400
ctattgtgaa cagtgctgca ataaacttac atgtgcatgt gtctttagta gaatgattta 2460
taatcctttg ggtatatacc cagtaatggg attgctggtc aaatggtatt tctggttcta 2520
gatccttgag gaatctttgt cttccacaat ggttgaacta atttgtactc ccaccaacag 2580
tgtaaaagta ttcctgtttc tctacatcct cttcagcatc tgttgtgtcc tgacatttta 2640
atgatcacta ttctcactgg cgtgagatgt tatctcattg tggttttgat ttgcatttct 2700
ctaatgacca gtaatgatga gctttttttc atatgtttgt tggctgcata aatgtcttct 2760
tttgagaagt gtctgttcat atccttcacc cattttttga agaaaacaaa ctcttaagag 2820
agcagtattc attcttttga gtgtgaggga tggagaaaga gaaagatgga gagagtatta 2880
taagcagctg tatccccttt gccatggtga tagcagacca ttcacatggg agcttctggt 2940
ctctttgtaa taataataag agccacatta ccagtactta gagtatgcta gttattttaa 3000
cacattgtat cattaaatct tcaaaacatc cctatgagtt agaaacctaa aaaaaaaaaa 3060
aaaaaaa 3067

<210> 53
<211> 2778
<212> DNA
<213> Homo sapiens

<400> 53
ctcattttga tgtctagaat caggggatcc aggatcatca ccaaggtcat tttcccaggt 60
atggaggggt ctttctgctt ctttcttgtc atgcacagct gctgaggaag gggctgggag 120
taaagacagt gaaatgggga ggaggagtcc attcaaaccg agaaacaaag tgtttggttt 180
ttcttacccc tggtgtagaa gctaccaacc ttttccaaga aagagggcct ggcccccttc 240
tcgggtctgg ctgggtgcct gctgtgcctc tctggcctcc cctccgaagg gcaccattcc 300
ctcgggtgag tactaccggc ctgcaccgtc ttccagtggg gacagcctga gaagagagtc 360
tggggcctta cttcagtacc ttccttcact ggcctcaccc tgtgcaaatc atgccacacg 420

ctgcagcctc cttttcccta tctataaaat aaaaatgacc ctgctctatc tcactgggct 480
ggcaagaaca cactgttgtt gccttgcaga cagatgtgct gaggctgtag aaagtgcttt 540
ttatttggtt gggagcttgt gcataaatgc gagaggggct gcacatctga cggactagag 600
gtgactcatg gctgaaccgg aacaggacat cggggagaag ccagcagcca tgctgaactc 660
tccacagggc cctgtgaaaa gctcttcacc tcctctgccc tctggatcta gtgaagccta 720
ttcatccttc agatgtcagc tcaaataatc aaccttcatg gaggcctccc ttgaccccta 780
acatgctttc aaagtactgt gtatttcaca ttcatcatgc cccgacaact gtgatttccc 840
atttattaat atctgtctct tctgctggcc tgcaaactcc aggagcacag agacatcttt 900
gggatttttg aacatgattt ccccagggct tagcccagtg cctggtgcaa agcaggcttt 960
caacatgttc agtggatatt gtaagaaaga aagaaataca caaaaggcct ggcatatgca 1020
aagcactcta aatattcact cctttccctt ccctctgggt gagaaaattt ctccttataa 1080
agacaccctc ctaactgtat ctctgctaga gaactgaaga cataaagcac tctgtgccaa 1140
aaatatttaa gtaaaaactt gagctaagca cagagattat aaatatttct tccccagatt 1200
acgcaccatt taaaaatact gtctcagctc cttttcatga tttgggtggt gattaaagaa 1260
aattactctt caagactgaa agtcattact gcccttttcc tgacttgcct tttcccttga 1320
gaaggggagg ataagctgca gggcaggaag tggaagtggg gcatccttgt cctttgtctg 1380
gcagacagcc aactggtcag gtactgctcc ttctcaactc tttcctgatt cccaggtgaa 1440
tataaacaag aaggcacaaa tccacacttg ccaacaacgg acccaagtga taacaagaaa 1500
cccagtgaca cctgtctagg tgaagactca gcccctatgt gaccaggttg caaagccaaa 1560
ctgaccatct gctttccatt tggactttta gttcatactg tatcttctca ggacagttaa 1620
gttggaatac aatgccactg tcctgaaaga tggtagaatt atcctatttc tggaggagtg 1680
ggggtggtgg gtaggaatct caagagcgat ttgctcctct gcacaatagc ttctttaagg 1740
acaccagggc ccccagggct atacatttcc ctgaagcttt ccagataagc aacaaggtat 1800
gagcacctgc tatgtattgc ccaagggtga tgtgtttaaa tatccattgc atattttaaa 1860
tccttggctg gcttaaagct gcaagctttc tgtcttcagt ggatataatg gggcatacaa 1920
tcccagagct tgcccaacac tccaagaaaa gaaccctcag ctaatgcaaa gtgtgtatgt 1980
gcccatgaaa gctccatgtc tacttaacat tcagttttta ggattattta tgctgtaata 2040
atagatatga aaatctctga caggtatttt gtttccttta caaactgtat ttgaatttat 2100
gggtgattta gagcttgtgt ttaaagtcag aattcagaac cccaaagaaa atgacttcat 2160
tgaaattgaa ctgaagagac aagaactgag ttaccaaaac ctactaaacg tgagttgctg 2220
tgaactgggg attaaaccag aacgagtgga gaagatcaga aagctaccaa acacactgct 2280
cagaaaggac aaagacattc gaagactgcg ggactttcag gaagtggaac tcattttaat 2340
gaaaaatgga agctccagat tgacagaata tgtgccatct ctgacagaaa ggccctgcta 2400
tgatagcaaa gctgcaaaaa tgacttatta aatactccca ggaatggccg cgcatggtgg 2460

ctcaccccct gtaatcccag cactttggga agccaaggtg ggcggatcac ctgaggtcag 2520
gagttctaga ccagcctggc caacatatag tgaaacccag tctctactaa aaaaaataca 2580
aaaattagct aggtgtggtg gcgcacacct gtagtagtcc cagctacatg ggaagctgag 2640
gcaggagaat cacctgaacc caggaggcag aggttgcagt gagctgagat tgcgccactg 2700
cactccagcc tggcgacaga gcaagactct gtctctcaaa ataaataaat aaataaataa 2760
ataaataaat aaataatc 2778

<210> 54
<211> 1646
<212> DNA
<213> Homo sapiens

<400> 54
gcccgggaga ggagaggagc gggccgagga ctccagcgtg cccaggtctg gcatcctgca 60
cttgctgccc tctgacacct gggaagatgg ccggcccgtg gaccttcacc cttctctgtg 120
gtttgctggc agccaccttg atccaagcca ccctcagtcc cactgcagtt ctcatcctcg 180
gcccaaaagt catcaaagaa aagctgacac aggagctgaa ggaccacaac gccaccagca 240
tcctgcagca gctgccgctg ctcagtgcca tgcgggaaaa gccagccgga ggcatccctg 300
tgctgggcag cctggtgaac accgtcctga agcacatcat ctggctgaag gtcatcacag 360
ctaacatcct ccagctgcag gtgaagccct cggccaatga ccaggagctg ctagtcaaga 420
tccccctgga catggtggct ggattcaaca cgcccctggt caagaccatc gtggagttcc 480
acatgacgac tgaggcccaa gccaccatcc gcatggacac cagtgcaagt ggccccaccc 540
gcctggtcct cagtgactgt gccaccagcc atgggagcct gcgcatccaa ctgctgcata 600
agctctcctt cctggtgaac gccttagcta agcaggtcat gaacctccta gtgccatccc 660
tgcccaatct agtgaaaaac cagctgtgtc ccgtgatcga ggcttccttc aatggcatgt 720
atgcagacct cctgcagctg gtgaaggtgc ccatttccct cagcattgac cgtctggagt 780
ttgaccttct gtatcctgcc atcaagggtg acaccattca gctctacctg ggggccaagt 840
tgttggactc acagggaaag gtgaccaagt ggttcaataa ctctgcagct tccctgacaa 900
tgcccaccct ggacaacatc ccgttcagcc tcatcgtgag tcaggacgtg gtgaaagctg 960
cagtggctgc tgtgctctct ccagaagaat tcatggtcct gttggactct gtgcttcctg 1020
agagtgccca tcggctgaag tcaagcatcg ggctgatcaa tgaaaaggct gcagataagc 1080
tgggatctac ccagatcgtg aagatcctaa ctcaggacac tcccgagttt tttatagacc 1140
aaggccatgc caaggtggcc caactgatcg tgctggaagt gtttccctcc agtgaagccc 1200
tccgcccttt gttcaccctg ggcatcgaag ccagctcgga agctcagttt tacaccaaag 1260
gtgaccaact tatactcaac ttgaataaca tcagctctga tcggatccag ctgatgaact 1320

ctgggattgg ctggttccaa cctgatgttc tgaaaaacat catcactgag atcatccact 1380
ccatcctgct gccgaaccag aatggcaaat taagatctgg ggtcccagtg tcattggtga 1440
aggccttggg attcgaggca gctgagtcct cactgaccaa ggatgccctt gtgcttactc 1500
cagcctcctt gtggaaaccc agctctcctg tctcccagtg aagacttgga tggcagccat 1560
cagggaaggc tgggtcccag ctgggagtat gggtgtgagc tctatagacc atccctctct 1620
gcaatcaata aacacttgcc tgtgat 1646

<210> 55
<211> 1049
<212> DNA
<213> Homo sapiens

<400> 55
ggagtggggg agagagagga gaccaggaca gctgctgaga cctctaagaa gtccagatac 60
taagagcaaa gatgtttcaa actgggggcc tcattgtctt ctacgggctg ttagcccaga 120
ccatggccca gtttggaggc ctgcccgtgc ccctggacca gaccctgccc ttgaatgtga 180
atccagccct gcccttgagt cccacaggtc ttgcaggaag cttgacaaat gccctcagca 240
atggcctgct gtctgggggc ctgttgggca ttctggaaaa ccttccgctc ctggacatcc 300
tgaagcctgg aggaggtact tctggtggcc tccttggggg actgcttgga aaagtgacgt 360
cagtgattcc tggcctgaac aacatcattg acataaaggt cactgacccc cagctgctgg 420
aacttggcct tgtgcagagc cctgatggcc accgtctcta tgtcaccatc cctctcggca 480
taaagctcca agtgaatacg cccctggtcg gtgcaagtct gttgaggctg gctgtgaagc 540
tggacatcac tgcagaaatc ttagctgtga gagataagca ggagaggatc cacctggtcc 600
ttggtgactg cacccattcc cctggaagcc tgcaaatttc tctgcttgat ggacttggcc 660
ccctccccat tcaaggtctt ctggacagcc tcacagggat cttgaataaa gtcctgcctg 720
agttggttca gggcaacgtg tgccctctgg tcaatgaggt tctcagaggc ttggacatca 780
ccctggtgca tgacattgtt aacatgctga tccacggact acagtttgtc atcaaggtct 840
aagccttcca ggaaggggct ggcctctgct gagctgcttc ccagtgctca cagatggctg 900
gcccatgtgc tggaagatga cacagttgcc ttctctccga ggaacctgcc ccctctcctt 960
tcccaccagg cgtgtgtaac atcccatgtg cctcacctaa taaaatggct cttcttctgc 1020
aaaaaaaaaa aaaaaaaaaa aaaaaaaaa 1049

<210> 56
<211> 4815
<212> DNA

<213>  Homo sapiens

<400>  56
gagcagagcc ctttcacaca cctcaggaac acctttcggc tgcccgctcc ccagacacac      60
ctgcagccct gcccagccgg ctttgctcac ccactgcttg taaatgcccc agatatgagc     120
cagcccaggc cccgctacgt ggtagacaga gccgcatact cccttaccct cttcgacgat     180
gagtttgaga agaaggaccg gacataccca gtgggagaga aacttcgcaa tgccttcaga     240
tgttcctcag ccaagatcaa agctgtggtg tttgggctgc tgcctgtgct ctcctggctc     300
cccaagtaca agattaaaga ctacatcatt cctgacctgc tcggtggact cagcggggga     360
tccatccagg tcccacaagg catggcattt gctctgctgg ccaaccttcc tgcagtcaat     420
ggcctctact cctccttctt ccccctcctg acctacttct tcctgggggg tgttcaccag     480
atggtgccag gtacctttgc cgttatcagc atcctggtgg gtaacatctg tctgcagctg     540
gccccagagt cgaaattcca ggtcttcaac aatgccacca atgagagcta tgtggacaca     600
gcagccatgg aggctgagag gctgcacgtg tcagctacgc tagcctgcct caccgccatc     660
atccagatgg gtctgggctt catgcagttt ggctttgtgg ccatctacct ctccgagtcc     720
ttcatccggg gcttcatgac ggccgccggc ctgcagatcc tgatttcggt gctcaagtac     780
atcttcggac tgaccatccc ctcctacaca ggcccagggt ccatcgtctt taccttcatt     840
gacatttgca aaaacctccc ccacaccaac atcgcctcgc tcatcttcgc tctcatcagc     900
ggtgccttcc tggtgctggt gaaggagctc aatgctcgct acatgcacaa gattcgcttc     960
cccatcccta cagagatgat tgtggtggtg gtggcaacag ctatctccgg gggctgtaag    1020
atgcccaaaa agtatcacat gcagatcgtg ggagaaatcc aacgcgggtt ccccaccccg    1080
gtgtcgcctg tggtctcaca gtggaaggac atgataggca cagccttctc cctagccatc    1140
gtgagctacg tcatcaacct ggctatgggc cggaccctgg ccaacaagca cggctacgac    1200
gtggattcga accaggagat gatcgctctc ggctgcagca acttctttgg ctccttcttt    1260
aaaattcatg tcatttgctg tgcgctttct gtcactctgg ctgtggatgg agctggagga    1320
aaatcccagg tggccagcct gtgtgtgtct ctggtggtga tgatcaccat gctggtcctg    1380
gggatctatc tgtatcctct ccctaagtct gtgctaggag ccctgatcgc tgtcaatctc    1440
aagaactccc tcaagcaact caccgacccc tactacctgt ggaggaagag caagctggac    1500
tgttgcatct gggtagtgag cttcctctcc tccttcttcc tcagcctgcc ctatggtgtg    1560
gcagtgggtg tcgccttctc cgtcctggtc gtggtcttcc agactcagtt tcgaaatggc    1620
tatgcactgg cccaggtcat ggacactgac atttatgtga atcccaagac ctataatagg    1680
gcccaggata tccaggggat taaaatcatc acgtactgct cccctctcta ctttgccaac    1740
tcagagatct tcaggcaaaa ggtcatcgcc aagacaggca tggaccccca gaaagtatta    1800
ctagccaagc aaaaatacct caagaagcag gagaagcgga gaatgaggcc cacacaacag    1860
aggaggtctc tattcatgaa aaccaagact gtctccctgc aggagctgca gcaggacttt    1920

gagaatgcgc cccccaccga ccccaacaac aaccagaccc cggctaacgg caccagcgtg 1980
tcctatatca ccttcagccc tgacagctcc tcacctgccc agagtgagcc accagcctcc 2040
gctgaggccc ccggcgagcc cagtgacatg ctggccagcg tcccaccctt cgtcaccttc 2100
cacaccctca tcctggacat gagtggagtc agcttcgtgg acttgatggg catcaaggcc 2160
ctggccaagc tgagctccac ctatgggaag atcggcgtga aggtcttctt ggtgaacatc 2220
catgcccagg tgtacaatga cattagccat ggaggcgtct ttgaggatgg gagtctagaa 2280
tgcaagcacg tctttcccag catacatgac gcagtcctct ttgcccaggc aaatgctaga 2340
gacgtgaccc caggacacaa cttccaaggg gctccagggg atgctgagct ctccttgtac 2400
gactcagagg aggacattcg cagctactgg gacttagagc aggagatgtt cgggagcatg 2460
tttcacgcag agaccctgac cgccctgtga gggctcagcc agtcctcatg ctgcctacag 2520
agtgcctggc acttgggact tccataaagg atgagcctgg ggtcacaggg ggtgtcgggc 2580
ggaggaaagt gcatccccca gagcttgggt tcctctctcc tctccccctc tctcctccct 2640
tccttccctc cccgcatctc cagagagagc ctctcagcag caggggggtg ctacccttac 2700
gggagtgaga gtctggtgag cccactcttc acccgtcagg ccctggccgc aatggacaag 2760
cctcctgctc actccacccc acccacatct gccctgtcct tggcagctga aggacacctt 2820
gacttccagc ttttacgagt gagccaaaaa cagaaggaca agtacaactg tgctggcctg 2880
ctgtacaagc ttcaaaaagt gtcccagagc ccgcacggct cggtgtcaga tggtgtcagg 2940
ctgtcacgga catagggata aacttggtta ggactctggc ttgccttccc cagctgcctc 3000
aactctgtct ctggcagctc tgcacccagg gaccatgtgc tctccacacc caggagtcta 3060
ggccttggta actatgcgcc ccccctccat catccccaag gctgcccaaa ccaccactgc 3120
tgtcagcaag cacatcagac tctagcctgg acagtggcca ggaccgtcga gaccaccaga 3180
gctacctccc cggggacagc ccactaaggt tctgcctcag cctcctgaaa catcactgcc 3240
ctcagaggct gctcccttcc cctggaggct ggctagaaac cccaaagagg gggatgggta 3300
gctggcagaa tcatctggca tcctagtaat agataccagt tattctgcac aaaacttttg 3360
ggaattcctc tttgcaccca gagactcaga ggggaagagg gtgctagtac caacacaggg 3420
aaaacggatg ggacctgggc ccagacagtc ccccttgacc ccagggccca tcagggaaat 3480
gcctcccttt ggtaaatctg ccttatcctt ctttacctgg caaagagcca atcatgttaa 3540
ctcttcctta tcagcctgtg gcccagagac acaatggggt ccttctgtag gcaaaggtgg 3600
aagtcctcca gggatccgct acatccccta actgcatgca gatgtggaaa ggggctgatc 3660
cagattgggt cttcctgcac aggaagactc tttaacaccc ttaggacctc aggccatctt 3720
ctcctatgaa gatgaaaata ggggttaagt tttccatatg tacaaggagg tattgagagg 3780
aaccctactg ttgacttgaa aataaatagg ttccatgtgt aagtgttttg taaaatttca 3840
gtggaaatgc acagaaaatc ttctggcctc tcatcactgc ttttctcaag cttcttcagc 3900
ttaacaaccc cttccctaac aggttgggct ggcccagcct aggaaaacat ccccatttct 3960

aacttcagcc agacctgcgt tgtgtgtctg tgtgttgagt gagctggtca gctaacaagt 4020
cttcttagag ttaaaggagg gggtgctggc caagagccaa cacattcttg gcccaggagc 4080
attgcttttc tgtgaattca ttatgccatc tggctgccaa tggaactcaa aacttggaag 4140
gcgaaggaca atgttatctg ggattcaccg tgcccagcac ccgaagtgcc aaattccagg 4200
aggacaagag ccttagccaa tgacaactca ctctccccta ctccacctcc ttccaagtcc 4260
agctcaggcc caggaggtgg gagaaggtca cagagcctca ggaatttcca agtcagagtc 4320
ccctttgaac caagtatcta gatcccctga ggacttgatg aagtgatcct taacccccaa 4380
gtaatcatta acccccagac cagcctcaga actgaaggag attgttgacc cagtgacctg 4440
gagttgaggc tcagggagag atctgccaca tgtctgaggg ttgcagagcc cgctgtggag 4500
gtaagattgg aaacacatga ggcagaggga agacattgaa gaaaacatct ctgctggaat 4560
atttggaaaa gaacactctt ctggacctgg ttgaagcagg aaagatggag gcaaagtagt 4620
gaaataatcc agaatttcaa tgcttttgaa tgttcttagt gatactgacc tgtgataata 4680
taattcccag ggaggactgg gaaccttatc tcttgagata tttgcataat ttatttaatt 4740
taagcctcat tctccttttg ttcattttgg taataaactg gatttgaatt gtgaacaaaa 4800
aaaaaaaaaa aaaaa 4815

<210> 57
<211> 2572
<212> DNA
<213> Homo sapiens

<400> 57
aatgctctaa gacctctcag cacgggcgga agaaactccc ggagagctca cccaaaaaac 60
aaggagatcc catctagatt tcttcttgct tttgactcac agctggaagt tagaaaagcc 120
tcgatttcat ctttggagag gccaaatggt cttagcctca gtctctgtct ctaaatattc 180
caccataaaa cagctgagtt atttatgaat tagaggctat agctcacatt ttcaatcctc 240
tatttctttt tttaaatata actttctact ctgatgagag aatgtggttt taatctctct 300
ctcacatttt gatgatttag acagactccc cctcttcctc ctagtcaata aacccattga 360
tgatctattt cccagcttat ccccaagaaa acttttgaaa ggaaagagta gacccaaaga 420
tgttattttc tgctgtttga attttgtctc cccaccccca acttggctag taataaacac 480
ttactgaaga agaagcaata agagaaagat atttgtaatc tctccagccc atgatctcgg 540
ttttcttaca ctgtgatctt aaaagttacc aaaccaaagt cattttcagt ttgaggcaac 600
caaacctttc tactgctgtt gacatcttct tattacagca acaccattct aggagtttcc 660
tgagctctcc actggagtcc tctttctgtc gcgggtcaga aattgtccct agatgaatga 720
gaaaattatt ttttttaatt taagtcctaa atatagttaa aataaataat gttttagtaa 780

aatgatacac tatctctgtg aaatagcctc acccctacat gtggatagaa ggaaatgaaa 840
aaataattgc tttgacattg tctatatggt actttgtaaa gtcatgctta agtacaaatt 900
ccatgaaaag ctcactgatc ctaattcttt ccctttgagg tctctatggc tctgattgta 960
catgatagta agtgtaagcc atgtaaaaag taaataatgt ctgggcacag tggctcacgc 1020
ctgtaatcct agcactttgg gaggctgagg aggaaggatc acttgagccc agaagttcga 1080
gactagcctg ggcaacatgg agaagccctg tctctacaaa atacagagag aaaaaatcag 1140
ccagtcatgg tggcatacac ctgtagtccc agcattccgg gaggctgagg tgggaggatc 1200
acttgagccc agggaggttg gggctgcagt gagccatgat cacaccactg cactccagcc 1260
aggtgacata gcgagatcct gtctaaaaaa ataaaaaata aataatggaa cacagcaagt 1320
cctaggaagt aggttaaaac taattcttta aaaaaaaaaa aaagttgagc ctgaattaaa 1380
tgtaatgttt ccaagtgaca ggtatccaca tttgcatggt tacaagccac tgccagttgg 1440
cagtagcact ttcctggcac tgtggtcggt tttgttttgt tttgctttgt ttagagacgg 1500
ggtctcactt tccaggctgg cctcaaactc ctgcactcaa gcaattcttc taccctggcc 1560
tcccaagtag ctggaattac aggtgtgcgc catcacaact agctggtggt cagttttgtt 1620
actctgagag ctgttcactt ctctgaattc acctagagtg gttggaccat cagatgtttg 1680
ggcaaaactg aaagctcttt gcaaccacac accttccctg agcttacatc actgcccttt 1740
tgagcagaaa gtctaaattc cttccaagac agtagaattc catcccagta ccaaagccag 1800
ataggccccc taggaaactg aggtaagagc agtctctaaa aactacccac agcagcattg 1860
gtgcagggga acttggccat taggttatta tttgagagga aagtcctcac atcaatagta 1920
catatgaaag tgacctccaa ggggattggt gaatactcat aaggatcttc aggctgaaca 1980
gactatgtct ggggaaagaa cggattatgc cccattaaat aacaagttgt gttcaagagt 2040
cagagcagtg agctcagagg cccttctcac tgagacagca acatttaaac caaaccagag 2100
gaagtatttg tggaactcac tgcctcagtt tgggtaaagg atgagcagac aagtcaacta 2160
aagaaaaaag aaaagcaagg aggagggttg agcaatctag agcatggagt ttgttaagtg 2220
ctctctggat ttgagttgaa gagcatccat ttgagttgaa ggccacaggg cacaatgagc 2280
tctcccttct accaccagaa agtccctggt caggtctcag gtagtgcggt gtggctcagc 2340
tgggttttta attagcgcat tctctatcca acatttaatt gtttgaaagc ctccatatag 2400
ttagattgtg ctttgtaatt ttgttgttgt tgctctatct tattgtatat gcattgagta 2460
ttaacctgaa tgttttgtta cttaaatatt aaaaacactg ttatcctaca aaaaaaccct 2520
caaaggctga aaataaagaa ggaagatgga gacaccctct gggggtcctc tc 2572

<210> 58
<211> 1324
<212> DNA

<213>  Homo sapiens

<400>  58
ctttgcagtg gatgcccttg gcagggtgag cccacaagga gcaatggagc agggcagcgg      60
ccgcttggag gacttccctg tcaatgtgtt ctccgtcact ccttacacac ccagcaccgc     120
tgacatccag gtgtccgatg atgacaaggc gggggccacc ttgctcttct caggcatctt     180
tctgggactg gtggggatca cattcactgt catgggctgg atcaaatacc aaggtgtctc     240
ccactttgaa tggacccagc tccttgggcc cgtcctgctg tcagttgggg tgacattcat     300
cctgattgct gtgtgcaagt tcaaaatgct ctcctgccag ttgtgcaaag aaagtgagga     360
aagggtcccg gactcggaac agacaccagg aggaccatca tttgttttca ctggcatcaa     420
ccaacccatc accttccatg gggccactgt ggtgcagtac atccctcctc cttatggttc     480
tccagagcct atggggataa ataccagcta cctgcagtct gtggtgagcc cctgcggcct     540
cataacctct ggaggggcag cagccgccat gtcaagtcct cctcaatact acaccatcta     600
ccctcaagat aactctgcat ttgtggttga tgagggctgc ctttctttca cggacggtgg     660
aaatcacagg cccaatcctg atgttgacca gctagaagag acacagctgg aagaggaggc     720
ctgtgcctgc ttctctcctc ccccttatga agaaatatac tctctccctc gctagaggct     780
attctgatat aataacacaa tgctcagctc agggagcaag tgtttccgtc attgttacct     840
gacaaccgtg gtgttctatg ttgtaacctt cagaagttac agcagcgccc aggcagcctg     900
acagagatca ttcaaggggg gaaaggggaa gtgggaggtg caatttctca gattggtaaa     960
aattaggctg ggctggggaa attctcctcc ggaacagttt caaattccct cgggtaagaa    1020
atctcctgta taaggttcag gagcaggaat ttcacttttt catccaccac cctccccctt    1080
ctctgtagga aggcattggt ggctcaattt taaccccagc agccaatgga aaaatcacga    1140
cttctgagac tttgggagtt tccacagagg tgagagtcgg gtgggaagga agcagggaag    1200
agaaagcagg cccagctgga gatttcctgg tggctgtcct tggccccaaa gcagactcac    1260
taatcccaaa caactcagct gccatctggc ctctctgagg actctgggta ccttaaagac    1320
tata                                                                  1324

<210>  59
<211>  683
<212>  DNA
<213>  Homo sapiens

<400>  59
caggaaagtt cgtgctgcta ggcagaggaa ctgcagcttg ttggcaggtg aagggagcct      60
gtttagctgt gtccagcaac aacttacgtg gtcctgcttg tgttccaggt gaagcgtctg     120
gccgccgagc agaggaatca agacctgctc attctttcct cgggggatcc atccagcaat     180

gacatcatct catgctgcca caaggacccc aagtctgggc tgctggggac cagccacgct 240
ccccactgct cattccttca tcctagagac attctgactc tcctccgact gcgctgtgca 300
caggcgtgac aagctctttt acatctcagt ctgcacaact tcaggcactt agcagattga 360
tatgcatcca acaaatattg attgaatatc tgctaaatac ccagtaatgt ttcatgagtg 420
attgggtgaa taaaggaatg ctggttcctt ctggccatat taactcctgc acaatactaa 480
gaaaaataaa ttgcactagc tgtggaataa tgtgaatccc aatgtcatct attgaaatat 540
tacctgacta ttaagaggta tttatttttg tatcttttct agcaaagtaa ataaaattct 600
taatacagca tatcccctta ttcacggggg gtatgttcca agacccccgg tggatgcctg 660
aaactatgga taataccaga tcc 683

<210> 60
<211> 914
<212> PRT
<213> Homo sapiens

<400> 60

Met Gly Pro Phe Lys Ser Ser Val Phe Ile Leu Ile Leu His Leu Leu
1               5                   10                  15

Glu Gly Ala Leu Ser Asn Ser Leu Ile Gln Leu Asn Asn Asn Gly Tyr
            20                  25                  30

Glu Gly Ile Val Val Ala Ile Asp Pro Asn Val Pro Glu Asp Glu Thr
        35                  40                  45

Leu Ile Gln Gln Ile Lys Asp Met Val Thr Gln Ala Ser Leu Tyr Leu
    50                  55                  60

Phe Glu Ala Thr Gly Lys Arg Phe Tyr Phe Lys Asn Val Ala Ile Leu
65                  70                  75                  80

Ile Pro Glu Thr Trp Lys Thr Lys Ala Asp Tyr Val Arg Pro Lys Leu
                85                  90                  95

Glu Thr Tyr Lys Asn Ala Asp Val Leu Val Ala Glu Ser Thr Pro Pro
            100                 105                 110

Gly Asn Asp Glu Pro Tyr Thr Glu Gln Met Gly Asn Cys Gly Glu Lys
        115                 120                 125

Gly Glu Arg Ile His Leu Thr Pro Asp Phe Ile Ala Gly Lys Lys Leu
    130                 135                 140

Ala Glu Tyr Gly Pro Gln Gly Lys Ala Phe Val His Glu Trp Ala His
145                 150                 155                 160

Leu Arg Trp Gly Val Phe Asp Glu Tyr Asn Asn Asp Glu Lys Phe Tyr
                165                 170                 175

Leu Ser Asn Gly Arg Ile Gln Ala Val Arg Cys Ser Ala Gly Ile Thr
            180                 185                 190

Gly Thr Asn Val Val Lys Lys Cys Gln Gly Gly Ser Cys Tyr Thr Lys
        195                 200                 205

Arg Cys Thr Phe Asn Lys Val Thr Gly Leu Tyr Glu Lys Gly Cys Glu
    210                 215                 220

Phe Val Leu Gln Ser Arg Gln Thr Glu Lys Ala Ser Ile Met Phe Ala
225                 230                 235                 240

Gln His Val Asp Ser Ile Val Glu Phe Cys Thr Glu Gln Asn His Asn
                245                 250                 255

Lys Glu Ala Pro Asn Lys Gln Asn Gln Lys Cys Asn Leu Arg Ser Thr
            260                 265                 270

Trp Glu Val Ile Arg Asp Ser Glu Asp Phe Lys Lys Thr Thr Pro Met
        275                 280                 285

Thr Thr Gln Pro Pro Asn Pro Thr Phe Ser Leu Leu Gln Ile Gly Gln
    290                 295                 300

Arg Ile Val Cys Leu Val Leu Asp Lys Ser Gly Ser Met Ala Thr Gly
305                 310                 315                 320

Asn Arg Leu Asn Arg Leu Asn Gln Ala Gly Gln Leu Phe Leu Leu Gln
                325                 330                 335

Thr Val Glu Leu Gly Ser Trp Val Gly Met Val Thr Phe Asp Ser Ala
            340                 345                 350

Ala His Val Gln Ser Glu Leu Ile Gln Ile Asn Ser Gly Ser Asp Arg
        355                 360                 365

Asp Thr Leu Ala Lys Arg Leu Pro Ala Ala Ala Ser Gly Gly Thr Ser
    370                 375                 380

Ile Cys Ser Gly Leu Arg Ser Ala Phe Thr Val Ile Arg Lys Lys Tyr
385                 390                 395                 400

Pro Thr Asp Gly Ser Glu Ile Val Leu Leu Thr Asp Gly Glu Asp Asn
                405                 410                 415

Thr Ile Ser Gly Cys Phe Asn Glu Val Lys Gln Ser Gly Ala Ile Ile
            420                 425                 430

His Thr Val Ala Leu Gly Pro Ser Ala Ala Gln Glu Leu Glu Glu Leu
        435                 440                 445

Ser Lys Met Thr Gly Gly Leu Gln Thr Tyr Ala Ser Asp Gln Val Gln
    450                 455                 460

Asn Asn Gly Leu Ile Asp Ala Phe Gly Ala Leu Ser Ser Gly Asn Gly
465                 470                 475                 480

Ala Val Ser Gln Arg Ser Ile Gln Leu Glu Ser Lys Gly Leu Thr Leu
                485                 490                 495

Gln Asn Ser Gln Trp Met Asn Gly Thr Val Ile Val Asp Ser Thr Val
            500                 505                 510

Gly Lys Asp Thr Leu Phe Leu Ile Thr Trp Thr Thr Gln Pro Pro Gln
        515                 520                 525

Ile Leu Leu Trp Asp Pro Ser Gly Gln Lys Gln Gly Gly Phe Val Val
    530                 535                 540

Asp Lys Asn Thr Lys Met Ala Tyr Leu Gln Ile Pro Gly Ile Ala Lys
545                 550                 555                 560

Val Gly Thr Trp Lys Tyr Ser Leu Gln Ala Ser Ser Gln Thr Leu Thr
                565                 570                 575

Leu Thr Val Thr Ser Arg Ala Ser Asn Ala Thr Leu Pro Pro Ile Thr
            580                 585                 590

Val Thr Ser Lys Thr Asn Lys Asp Thr Ser Lys Phe Pro Ser Pro Leu
        595                 600                 605

Val Val Tyr Ala Asn Ile Arg Gln Gly Ala Ser Pro Ile Leu Arg Ala
    610                 615                 620

Ser Val Thr Ala Leu Ile Glu Ser Val Asn Gly Lys Thr Val Thr Leu
625                 630                 635                 640

Glu Leu Leu Asp Asn Gly Ala Gly Ala Asp Ala Thr Lys Asp Asp Gly
                645                 650                 655

Val Tyr Ser Arg Tyr Phe Thr Thr Tyr Asp Thr Asn Gly Arg Tyr Ser
            660                 665                 670

Val Lys Val Arg Ala Leu Gly Gly Val Asn Ala Ala Arg Arg Arg Val
        675                 680                 685

Ile Pro Gln Gln Ser Gly Ala Leu Tyr Ile Pro Gly Trp Ile Glu Asn
    690                 695                 700

Asp Glu Ile Gln Trp Asn Pro Pro Arg Pro Glu Ile Asn Lys Asp Asp
705                 710                 715                 720

Val Gln His Lys Gln Val Cys Phe Ser Arg Thr Ser Ser Gly Gly Ser
                725                 730                 735

Phe Val Ala Ser Asp Val Pro Asn Ala Pro Ile Pro Asp Leu Phe Pro
            740                 745                 750

Pro Gly Gln Ile Thr Asp Leu Lys Ala Glu Ile His Gly Gly Ser Leu
        755                 760                 765

Ile Asn Leu Thr Trp Thr Ala Pro Gly Asp Asp Tyr Asp His Gly Thr
    770                 775                 780

Ala His Lys Tyr Ile Ile Arg Ile Ser Thr Ser Ile Leu Asp Leu Arg
785                 790                 795                 800

Asp Lys Phe Asn Glu Ser Leu Gln Val Asn Thr Thr Ala Leu Ile Pro
                805                 810                 815

Lys Glu Ala Asn Ser Glu Glu Val Phe Leu Phe Lys Pro Glu Asn Ile
            820                 825                 830

Thr Phe Glu Asn Gly Thr Asp Leu Phe Ile Ala Ile Gln Ala Val Asp
        835                 840                 845

Lys Val Asp Leu Lys Ser Glu Ile Ser Asn Ile Ala Arg Val Ser Leu
    850                 855                 860

Phe Ile Pro Pro Gln Thr Pro Pro Glu Thr Pro Ser Pro Asp Glu Thr
865                 870                 875                 880

Ser Ala Pro Cys Pro Asn Ile His Ile Asn Ser Thr Ile Pro Gly Ile
                885                 890                 895

His Ile Leu Lys Ile Met Trp Lys Trp Ile Gly Glu Leu Gln Leu Ser
            900                 905                 910

Ile Ala

<210>  61

<211>  501

<212>  PRT

<213>  Homo sapiens

<400> 61

Met Lys Lys Glu Gly Arg Lys Arg Trp Lys Arg Lys Glu Asp Lys Lys
1               5                   10                  15

Arg Val Val Val Ser Asn Leu Leu Phe Glu Gly Trp Ser His Lys Glu
            20                  25                  30

Asn Pro Asn Arg His His Arg Gly Asn Gln Ile Lys Thr Ser Lys Tyr
        35                  40                  45

Thr Val Leu Ser Phe Val Pro Lys Asn Ile Phe Glu Gln Leu His Arg
    50                  55                  60

Phe Ala Asn Leu Tyr Phe Val Gly Ile Ala Val Leu Asn Phe Ile Pro
65                  70                  75                  80

Val Val Asn Ala Phe Gln Pro Glu Val Ser Met Ile Pro Ile Cys Val
                85                  90                  95

Ile Leu Ala Val Thr Ala Ile Lys Asp Ala Trp Glu Asp Leu Arg Arg
            100                 105                 110

Tyr Lys Ser Asp Lys Val Ile Asn Asn Arg Glu Cys Leu Ile Tyr Ser
        115                 120                 125

Arg Lys Glu Gln Thr Tyr Val Gln Lys Cys Trp Lys Asp Val Arg Val
    130                 135                 140

Gly Asp Phe Ile Gln Met Lys Cys Asn Glu Ile Val Pro Ala Asp Ile
145                 150                 155                 160

Leu Leu Leu Phe Ser Ser Asp Pro Asn Gly Ile Cys His Leu Glu Thr
                165                 170                 175

Ala Ser Leu Asp Gly Glu Thr Asn Leu Lys Gln Arg Arg Val Val Lys
            180                 185                 190

Gly Phe Ser Gln Gln Glu Val Gln Phe Glu Pro Glu Leu Phe His Asn
        195                 200                 205

Thr Ile Val Cys Glu Lys Pro Asn Asn His Leu Asn Lys Phe Lys Gly
    210                 215                 220

Tyr Met Glu His Pro Asp Gln Thr Arg Thr Gly Phe Gly Cys Glu Ser
225                 230                 235                 240

Leu Leu Leu Arg Gly Cys Thr Ile Arg Asn Thr Glu Met Ala Val Gly
                245                 250                 255

Ile Val Ile Tyr Ala Gly His Glu Thr Lys Ala Met Leu Asn Asn Ser
            260                 265                 270

Gly Pro Arg Tyr Lys Arg Ser Lys Ile Glu Arg Arg Met Asn Ile Asp
        275                 280                 285

Ile Phe Phe Cys Ile Gly Ile Leu Ile Leu Met Cys Leu Ile Gly Ala
    290                 295                 300

Val Gly His Ser Ile Trp Asn Gly Thr Phe Glu Glu His Pro Pro Phe
305                 310                 315                 320

Asp Val Pro Asp Ala Asn Gly Ser Phe Leu Pro Ser Ala Leu Gly Gly
                325                 330                 335

Phe Tyr Met Phe Leu Thr Met Ile Ile Leu Leu Gln Val Leu Ile Pro
            340                 345                 350

Ile Ser Leu Tyr Val Ser Ile Glu Leu Val Lys Leu Gly Gln Val Phe
        355                 360                 365

Phe Leu Ser Asn Asp Leu Asp Leu Tyr Asp Glu Glu Thr Asp Leu Ser
    370                 375                 380

Ile Gln Cys Arg Ala Leu Asn Ile Ala Glu Asp Leu Gly Gln Ile Gln
385                 390                 395                 400

Tyr Ile Phe Ser Asp Lys Thr Gly Thr Leu Thr Glu Asn Lys Met Val
                405                 410                 415

Phe Arg Arg Cys Thr Ile Met Gly Ser Glu Tyr Ser His Gln Glu Asn
            420                 425                 430

Gly Ile Glu Ala Pro Lys Gly Ser Ile Pro Leu Ser Lys Arg Lys Tyr
        435                 440                 445

Pro Ala Leu Leu Arg Asn Glu Glu Ile Lys Asp Ile Leu Leu Ala Leu
    450                 455                 460

Leu Glu Ala Val Trp His Phe His Lys Leu Leu Pro Val Ser Leu Trp
465                 470                 475                 480

Ser Ser Leu Ser Gln Ile Arg Ala Val Pro Ile Thr Cys Lys Leu Ser
                485                 490                 495

Phe Val Tyr Lys Gly
            500

<210> 62

<211> 154

<212>   PRT

<213>   Homo sapiens

<400>   62

Met Gly Arg Arg Ser Pro Phe Lys Pro Arg Asn Lys Val Phe Gly Phe
1               5                   10                  15

Ser Tyr Pro Trp Cys Arg Ser Tyr Gln Pro Phe Pro Arg Lys Arg Ala
            20                  25                  30

Trp Pro Pro Ser Arg Val Trp Leu Gly Ala Cys Cys Ala Ser Leu Ala
        35                  40                  45

Ser Pro Pro Lys Gly Thr Ile Pro Ser Gly Glu Tyr Tyr Arg Pro Ala
    50                  55                  60

Pro Ser Ser Ser Gly Asp Ser Leu Arg Arg Glu Ser Gly Ala Leu Leu
65                  70                  75                  80

Gln Tyr Leu Pro Ser Leu Ala Ser Pro Cys Ala Asn His Ala Thr Arg
                85                  90                  95

Cys Ser Leu Leu Phe Pro Ile Tyr Lys Ile Lys Met Thr Leu Leu Tyr
            100                 105                 110

Leu Thr Gly Leu Ala Arg Thr His Cys Cys Cys Leu Ala Asp Arg Cys
        115                 120                 125

Ala Glu Ala Val Glu Ser Ala Phe Tyr Leu Val Gly Ser Leu Cys Ile
    130                 135                 140

Asn Ala Arg Gly Ala Ala His Leu Thr Asp
145                 150

<210>   63

<211>   484

<212>   PRT

<213>   Homo sapiens

<400>   63

Met Ala Gly Pro Trp Thr Phe Thr Leu Leu Cys Gly Leu Leu Ala Ala
1               5                   10                  15

Thr Leu Ile Gln Ala Thr Leu Ser Pro Thr Ala Val Leu Ile Leu Gly
            20                  25                  30

Pro Lys Val Ile Lys Glu Lys Leu Thr Gln Glu Leu Lys Asp His Asn
        35                  40                  45

Ala Thr Ser Ile Leu Gln Gln Leu Pro Leu Leu Ser Ala Met Arg Glu
    50                  55                  60

Lys Pro Ala Gly Gly Ile Pro Val Leu Gly Ser Leu Val Asn Thr Val
65                  70                  75                  80

Leu Lys His Ile Ile Trp Leu Lys Val Ile Thr Ala Asn Ile Leu Gln
                85                  90                  95

Leu Gln Val Lys Pro Ser Ala Asn Asp Gln Glu Leu Leu Val Lys Ile
            100                 105                 110

Pro Leu Asp Met Val Ala Gly Phe Asn Thr Pro Leu Val Lys Thr Ile
        115                 120                 125

Val Glu Phe His Met Thr Thr Glu Ala Gln Ala Thr Ile Arg Met Asp
    130                 135                 140

Thr Ser Ala Ser Gly Pro Thr Arg Leu Val Leu Ser Asp Cys Ala Thr
145                 150                 155                 160

Ser His Gly Ser Leu Arg Ile Gln Leu Leu His Lys Leu Ser Phe Leu
                165                 170                 175

Val Asn Ala Leu Ala Lys Gln Val Met Asn Leu Leu Val Pro Ser Leu
            180                 185                 190

Pro Asn Leu Val Lys Asn Gln Leu Cys Pro Val Ile Glu Ala Ser Phe
        195                 200                 205

Asn Gly Met Tyr Ala Asp Leu Leu Gln Leu Val Lys Val Pro Ile Ser
    210                 215                 220

Leu Ser Ile Asp Arg Leu Glu Phe Asp Leu Leu Tyr Pro Ala Ile Lys
225                 230                 235                 240

Gly Asp Thr Ile Gln Leu Tyr Leu Gly Ala Lys Leu Leu Asp Ser Gln
                245                 250                 255

Gly Lys Val Thr Lys Trp Phe Asn Asn Ser Ala Ala Ser Leu Thr Met
            260                 265                 270

Pro Thr Leu Asp Asn Ile Pro Phe Ser Leu Ile Val Ser Gln Asp Val
        275                 280                 285

Val Lys Ala Ala Val Ala Ala Val Leu Ser Pro Glu Glu Phe Met Val
    290                 295                 300

Leu Leu Asp Ser Val Leu Pro Glu Ser Ala His Arg Leu Lys Ser Ser
305                 310                 315                 320

Ile Gly Leu Ile Asn Glu Lys Ala Ala Asp Lys Leu Gly Ser Thr Gln
                325                 330                 335

Ile Val Lys Ile Leu Thr Gln Asp Thr Pro Glu Phe Phe Ile Asp Gln
            340                 345                 350

Gly His Ala Lys Val Ala Gln Leu Ile Val Leu Glu Val Phe Pro Ser
        355                 360                 365

Ser Glu Ala Leu Arg Pro Leu Phe Thr Leu Gly Ile Glu Ala Ser Ser
    370                 375                 380

Glu Ala Gln Phe Tyr Thr Lys Gly Asp Gln Leu Ile Leu Asn Leu Asn
385                 390                 395                 400

Asn Ile Ser Ser Asp Arg Ile Gln Leu Met Asn Ser Gly Ile Gly Trp
                405                 410                 415

Phe Gln Pro Asp Val Leu Lys Asn Ile Ile Thr Glu Ile Ile His Ser
            420                 425                 430

Ile Leu Leu Pro Asn Gln Asn Gly Lys Leu Arg Ser Gly Val Pro Val
        435                 440                 445

Ser Leu Val Lys Ala Leu Gly Phe Glu Ala Ala Glu Ser Ser Leu Thr
    450                 455                 460

Lys Asp Ala Leu Val Leu Thr Pro Ala Ser Leu Trp Lys Pro Ser Ser
465                 470                 475                 480

Pro Val Ser Gln

<210>  64

<211>  256

<212>  PRT

<213>  Homo sapiens

<400>  64

Met Phe Gln Thr Gly Gly Leu Ile Val Phe Tyr Gly Leu Leu Ala Gln
1               5                   10                  15

Thr Met Ala Gln Phe Gly Gly Leu Pro Val Pro Leu Asp Gln Thr Leu
            20                  25                  30

Pro Leu Asn Val Asn Pro Ala Leu Pro Leu Ser Pro Thr Gly Leu Ala
        35                  40                  45

Gly Ser Leu Thr Asn Ala Leu Ser Asn Gly Leu Leu Ser Gly Gly Leu
    50                  55                  60

Leu Gly Ile Leu Glu Asn Leu Pro Leu Leu Asp Ile Leu Lys Pro Gly
65                  70                  75                  80

Gly Gly Thr Ser Gly Gly Leu Leu Gly Gly Leu Leu Gly Lys Val Thr
                85                  90                  95

Ser Val Ile Pro Gly Leu Asn Asn Ile Ile Asp Ile Lys Val Thr Asp
            100                 105                 110

Pro Gln Leu Leu Glu Leu Gly Leu Val Gln Ser Pro Asp Gly His Arg
        115                 120                 125

Leu Tyr Val Thr Ile Pro Leu Gly Ile Lys Leu Gln Val Asn Thr Pro
    130                 135                 140

Leu Val Gly Ala Ser Leu Leu Arg Leu Ala Val Lys Leu Asp Ile Thr
145                 150                 155                 160

Ala Glu Ile Leu Ala Val Arg Asp Lys Gln Glu Arg Ile His Leu Val
                165                 170                 175

Leu Gly Asp Cys Thr His Ser Pro Gly Ser Leu Gln Ile Ser Leu Leu
            180                 185                 190

Asp Gly Leu Gly Pro Leu Pro Ile Gln Gly Leu Leu Asp Ser Leu Thr
        195                 200                 205

Gly Ile Leu Asn Lys Val Leu Pro Glu Leu Val Gln Gly Asn Val Cys
    210                 215                 220

Pro Leu Val Asn Glu Val Leu Arg Gly Leu Asp Ile Thr Leu Val His
225                 230                 235                 240

Asp Ile Val Asn Met Leu Ile His Gly Leu Gln Phe Val Ile Lys Val
                245                 250                 255

<210>  65

<211>  791

<212>  PRT

<213>  Homo sapiens


<400>  65

Met Ser Gln Pro Arg Pro Arg Tyr Val Val Asp Arg Ala Ala Tyr Ser
1               5                   10                  15

Leu Thr Leu Phe Asp Asp Glu Phe Glu Lys Lys Asp Arg Thr Tyr Pro
            20                  25                  30

Val Gly Glu Lys Leu Arg Asn Ala Phe Arg Cys Ser Ser Ala Lys Ile
        35                  40                  45

Lys Ala Val Val Phe Gly Leu Leu Pro Val Leu Ser Trp Leu Pro Lys
    50                  55                  60

Tyr Lys Ile Lys Asp Tyr Ile Ile Pro Asp Leu Leu Gly Gly Leu Ser
65                  70                  75                  80

Gly Gly Ser Ile Gln Val Pro Gln Gly Met Ala Phe Ala Leu Leu Ala
                85                  90                  95

Asn Leu Pro Ala Val Asn Gly Leu Tyr Ser Ser Phe Phe Pro Leu Leu
            100                 105                 110

Thr Tyr Phe Phe Leu Gly Gly Val His Gln Met Val Pro Gly Thr Phe
        115                 120                 125

Ala Val Ile Ser Ile Leu Val Gly Asn Ile Cys Leu Gln Leu Ala Pro
    130                 135                 140

Glu Ser Lys Phe Gln Val Phe Asn Asn Ala Thr Asn Glu Ser Tyr Val
145                 150                 155                 160

Asp Thr Ala Ala Met Glu Ala Glu Arg Leu His Val Ser Ala Thr Leu
                165                 170                 175

Ala Cys Leu Thr Ala Ile Ile Gln Met Gly Leu Gly Phe Met Gln Phe
            180                 185                 190

Gly Phe Val Ala Ile Tyr Leu Ser Glu Ser Phe Ile Arg Gly Phe Met
        195                 200                 205

Thr Ala Ala Gly Leu Gln Ile Leu Ile Ser Val Leu Lys Tyr Ile Phe
    210                 215                 220

Gly Leu Thr Ile Pro Ser Tyr Thr Gly Pro Gly Ser Ile Val Phe Thr
225                 230                 235                 240

Phe Ile Asp Ile Cys Lys Asn Leu Pro His Thr Asn Ile Ala Ser Leu
                245                 250                 255

Ile Phe Ala Leu Ile Ser Gly Ala Phe Leu Val Leu Val Lys Glu Leu
            260                 265                 270

Asn Ala Arg Tyr Met His Lys Ile Arg Phe Pro Ile Pro Thr Glu Met
        275                 280                 285

Ile Val Val Val Val Ala Thr Ala Ile Ser Gly Gly Cys Lys Met Pro
    290                 295                 300

Lys Lys Tyr His Met Gln Ile Val Gly Glu Ile Gln Arg Gly Phe Pro
305                 310                 315                 320

Thr Pro Val Ser Pro Val Val Ser Gln Trp Lys Asp Met Ile Gly Thr
                325                 330                 335

Ala Phe Ser Leu Ala Ile Val Ser Tyr Val Ile Asn Leu Ala Met Gly
            340                 345                 350

Arg Thr Leu Ala Asn Lys His Gly Tyr Asp Val Asp Ser Asn Gln Glu
        355                 360                 365

Met Ile Ala Leu Gly Cys Ser Asn Phe Phe Gly Ser Phe Phe Lys Ile
    370                 375                 380

His Val Ile Cys Cys Ala Leu Ser Val Thr Leu Ala Val Asp Gly Ala
385                 390                 395                 400

Gly Gly Lys Ser Gln Val Ala Ser Leu Cys Val Ser Leu Val Val Met
                405                 410                 415

Ile Thr Met Leu Val Leu Gly Ile Tyr Leu Tyr Pro Leu Pro Lys Ser
            420                 425                 430

Val Leu Gly Ala Leu Ile Ala Val Asn Leu Lys Asn Ser Leu Lys Gln
        435                 440                 445

Leu Thr Asp Pro Tyr Tyr Leu Trp Arg Lys Ser Lys Leu Asp Cys Cys
    450                 455                 460

Ile Trp Val Val Ser Phe Leu Ser Ser Phe Phe Leu Ser Leu Pro Tyr
465                 470                 475                 480

Gly Val Ala Val Gly Val Ala Phe Ser Val Leu Val Val Val Phe Gln
                485                 490                 495

Thr Gln Phe Arg Asn Gly Tyr Ala Leu Ala Gln Val Met Asp Thr Asp
            500                 505                 510

Ile Tyr Val Asn Pro Lys Thr Tyr Asn Arg Ala Gln Asp Ile Gln Gly
        515                 520                 525

Ile Lys Ile Ile Thr Tyr Cys Ser Pro Leu Tyr Phe Ala Asn Ser Glu
    530                 535                 540

Ile Phe Arg Gln Lys Val Ile Ala Lys Thr Gly Met Asp Pro Gln Lys
545                 550                 555                 560

Val Leu Leu Ala Lys Gln Lys Tyr Leu Lys Lys Gln Glu Lys Arg Arg
                565                 570                 575

Met Arg Pro Thr Gln Gln Arg Arg Ser Leu Phe Met Lys Thr Lys Thr
            580                 585                 590

Val Ser Leu Gln Glu Leu Gln Gln Asp Phe Glu Asn Ala Pro Pro Thr
        595                 600                 605

Asp Pro Asn Asn Asn Gln Thr Pro Ala Asn Gly Thr Ser Val Ser Tyr
    610                 615                 620

Ile Thr Phe Ser Pro Asp Ser Ser Ser Pro Ala Gln Ser Glu Pro Pro
625                 630                 635                 640

Ala Ser Ala Glu Ala Pro Gly Glu Pro Ser Asp Met Leu Ala Ser Val
                645                 650                 655

Pro Pro Phe Val Thr Phe His Thr Leu Ile Leu Asp Met Ser Gly Val
            660                 665                 670

Ser Phe Val Asp Leu Met Gly Ile Lys Ala Leu Ala Lys Leu Ser Ser
        675                 680                 685

Thr Tyr Gly Lys Ile Gly Val Lys Val Phe Leu Val Asn Ile His Ala
    690                 695                 700

Gln Val Tyr Asn Asp Ile Ser His Gly Gly Val Phe Glu Asp Gly Ser
705                 710                 715                 720

Leu Glu Cys Lys His Val Phe Pro Ser Ile His Asp Ala Val Leu Phe
                725                 730                 735

Ala Gln Ala Asn Ala Arg Asp Val Thr Pro Gly His Asn Phe Gln Gly
            740                 745                 750

Ala Pro Gly Asp Ala Glu Leu Ser Leu Tyr Asp Ser Glu Glu Asp Ile
        755                 760                 765

Arg Ser Tyr Trp Asp Leu Glu Gln Glu Met Phe Gly Ser Met Phe His
    770                 775                 780

Ala Glu Thr Leu Thr Ala Leu
785                 790

<210>  66

<211>  243

<212>  PRT
<213>  Homo sapiens

<400>  66

Met Glu Gln Gly Ser Gly Arg Leu Glu Asp Phe Pro Val Asn Val Phe
1               5                   10                  15

Ser Val Thr Pro Tyr Thr Pro Ser Thr Ala Asp Ile Gln Val Ser Asp
            20                  25                  30

Asp Asp Lys Ala Gly Ala Thr Leu Leu Phe Ser Gly Ile Phe Leu Gly
        35                  40                  45

Leu Val Gly Ile Thr Phe Thr Val Met Gly Trp Ile Lys Tyr Gln Gly
    50                  55                  60

Val Ser His Phe Glu Trp Thr Gln Leu Leu Gly Pro Val Leu Leu Ser
65                  70                  75                  80

Val Gly Val Thr Phe Ile Leu Ile Ala Val Cys Lys Phe Lys Met Leu
                85                  90                  95

Ser Cys Gln Leu Cys Lys Glu Ser Glu Glu Arg Val Pro Asp Ser Glu
            100                 105                 110

Gln Thr Pro Gly Gly Pro Ser Phe Val Phe Thr Gly Ile Asn Gln Pro
        115                 120                 125

Ile Thr Phe His Gly Ala Thr Val Val Gln Tyr Ile Pro Pro Pro Tyr
    130                 135                 140

Gly Ser Pro Glu Pro Met Gly Ile Asn Thr Ser Tyr Leu Gln Ser Val
145                 150                 155                 160

Val Ser Pro Cys Gly Leu Ile Thr Ser Gly Gly Ala Ala Ala Ala Met
                165                 170                 175

Ser Ser Pro Pro Gln Tyr Tyr Thr Ile Tyr Pro Gln Asp Asn Ser Ala
            180                 185                 190

Phe Val Val Asp Glu Gly Cys Leu Ser Phe Thr Asp Gly Gly Asn His
        195                 200                 205

Arg Pro Asn Pro Asp Val Asp Gln Leu Glu Glu Thr Gln Leu Glu Glu
    210                 215                 220

Glu Ala Cys Ala Cys Phe Ser Pro Pro Pro Tyr Glu Glu Ile Tyr Ser
225                 230                 235                 240

Leu Pro Arg

<210> 67
<211> 21
<212> DNA
<213> Künstliche Sequenz

<220>
<223> Beschreibung der künstlichen Sequenz: Oligonukleotid

<400> 67
acacgaatgg tagatacagt g 21

<210> 68
<211> 21
<212> DNA
<213> Künstliche Sequenz

<220>
<223> Beschreibung der künstlichen Sequenz: Oligonukleotid

<400> 68
atacttgtga gctgttccat g 21

<210> 69
<211> 21
<212> DNA
<213> Künstliche Sequenz

<220>
<223> Beschreibung der künstlichen Sequenz: Oligonukleotid

<400> 69
actgttacct tgcatggact g 21

<210> 70
<211> 21
<212> DNA
<213> Künstliche Sequenz

<220>

<223>  Beschreibung der künstlichen Sequenz: Oligonukleotid

<400>  70
caatgagaac acatggacat g                                                21

<210>  71
<211>  21
<212>  DNA
<213>  Künstliche Sequenz

<220>
<223>  Beschreibung der künstlichen Sequenz: Oligonukleotid

<400>  71
ccatgaaagc tccatgtcta c                                                21

<210>  72
<211>  21
<212>  DNA
<213>  Künstliche Sequenz

<220>
<223>  Beschreibung der künstlichen Sequenz: Oligonukleotid

<400>  72
agagatggca catattctgt c                                                21

<210>  73
<211>  21
<212>  DNA
<213>  Künstliche Sequenz

<220>
<223>  Beschreibung der künstlichen Sequenz: Oligonukleotid

<400>  73
atcggctgaa gtcaagcatc g                                                21

<210>  74
<211>  21
<212>  DNA

<213>  Künstliche Sequenz

<220>
<223>  Beschreibung der künstlichen Sequenz: Oligonukleotid

<400>  74
tggtcagtga ggactcagct g                                                21

<210>  75
<211>  21
<212>  DNA
<213>  Künstliche Sequenz

<220>
<223>  Beschreibung der künstlichen Sequenz: Oligonukleotid

<400>  75
tttctctgct tgatgcactt g                                                21

<210>  76
<211>  21
<212>  DNA
<213>  Künstliche Sequenz

<220>
<223>  Beschreibung der künstlichen Sequenz: Oligonukleotid

<400>  76
gtgagcactg ggaagcagct c                                                21

<210>  77
<211>  21
<212>  DNA
<213>  Künstliche Sequenz

<220>
<223>  Beschreibung der künstlichen Sequenz: Oligonukleotid

<400>  77
ggcaaatgct agagacgtga c                                                21

<210>  78

<211>  21

<212>  DNA

<213>  Künstliche Sequenz

<220>
<223>  Beschreibung der künstlichen Sequenz: Oligonukleotid

<400>  78
aggtgtcctt cagctgccaa g                                                21

<210>  79

<211>  21

<212>  DNA

<213>  Künstliche Sequenz

<220>
<223>  Beschreibung der künstlichen Sequenz: Oligonukleotid

<400>  79
gttaagtgct ctctggattt g                                                21

<210>  80

<211>  21

<212>  DNA

<213>  Künstliche Sequenz

<220>
<223>  Beschreibung der künstlichen Sequenz: Oligonukleotid

<400>  80
atcctgattg ctgtgtgcaa g                                                21

<210>  81

<211>  21

<212>  DNA

<213>  Künstliche Sequenz

<220>
<223>  Beschreibung der künstlichen Sequenz: Oligonukleotid

<400>  81
ctcttctagc tggtcaacat c                                                21

<210> 82

<211> 21

<212> DNA

<213> Künstliche Sequenz

<220>
<223> Beschreibung der künstlichen Sequenz: Oligonukleotid

<400> 82
ccagcaacaa cttacgtggt c 21

<210> 83

<211> 21

<212> DNA

<213> Künstliche Sequenz

<220>
<223> Beschreibung der künstlichen Sequenz: Oligonukleotid

<400> 83
cctttattca cccaatcact c 21

<210> 84

<211> 2165

<212> DNA

<213> Homo sapiens

<400> 84
agaacagcgc agtttgccct ccgctcacgc agagcctctc cgtggcctcc gcaccttgag 60

cattaggcca gttctcctct tctctctaat ccatccgtca cctctcctgt catccgtttc 120

catgccgtga ggtccattca cagaacacat ccatggctct catgctcagt ttggttctga 180

gtctcctcaa gctgggatca gggcagtggc aggtgtttgg gccagacaag cctgtccagg 240

ccttggtggg ggaggacgca gcattctcct gtttcctgtc tcctaagacc aatgcagagg 300

ccatggaagt gcggttcttc aggggccagt tctctagcgt ggtccacctc tacagggacg 360

ggaaggacca gccatttatg cagatgccac agtatcaagg caggacaaaa ctggtgaagg 420

attctattgc ggaggggcgc atctctctga ggctggaaaa cattactgtg ttggatgctg 480

gcctctatgg gtgcaggatt agttcccagt cttactacca gaaggccatc tgggagctac 540

aggtgtcagc actgggctca gttcctctca tttccatcac gggatatgtt gatagagaca 600

tccagctact ctgtcagtcc tcgggctggt tcccccggcc cacagcgaag tggaaaggtc 660
cacaaggaca ggatttgtcc acagactcca ggacaaacag agacatgcat ggcctgtttg 720
atgtggagat ctctctgacc gtccaagaga acgccgggag catatcctgt tccatgcggc 780
atgctcatct gagccgagag gtggaatcca gggtacagat aggagatacc tttttcgagc 840
ctatatcgtg gcacctggct accaaagtac tgggaatact ctgctgtggc ctattttttg 900
gcattgttgg actgaagatt ttcttctcca aattccagtg taagcgagag agagaagcat 960
gggccggtgc cttattcatg gttccagcag ggacaggatc agagatgctc ccacatccag 1020
ctgcttctct tcttctagtc ctagcctcca ggggcccagg cccaaaaaag gaaaatccag 1080
gcggaactgg actggagaag aaagcacgga caggcagaat tgagagacgc ccggaaacac 1140
gcagtggagg tgactctgga tccagagacg gctcacccga agctctgcgt ttctgatctg 1200
aaaactgtaa cccatagaaa agctccccag gaggtgcctc actctgagaa gagatttaca 1260
aggaagagtg tggtggcttc tcagagtttc caagcaggga aacattactg ggaggtggac 1320
ggaggacaca ataaaaggtg gcgcgtggga gtgtgccggg atgatgtgga caggaggaag 1380
gagtacgtga ctttgtctcc cgatcatggg tactgggtcc tcagactgaa tggagaacat 1440
ttgtatttca cattaaatcc ccgttttatc agcgtcttcc ccaggacccc acctacaaaa 1500
ataggggtct tcctggacta tgagtgtggg accatctcct tcttcaacat aaatgaccag 1560
tcccttattt ataccctgac atgtcggttt gaaggcttat tgaggcccta cattgagtat 1620
ccgtcctata atgagcaaaa tggaactccc atagtcatct gcccagtcac ccaggaatca 1680
gagaaagagg cctcttggca aagggcctct gcaatcccag agacaagcaa cagtgagtcc 1740
tcctcacagg caaccacgcc cttcctcccc aggggtgaaa tgtaggatga atcacatccc 1800
acattcttct ttagggatat taaggtctct ctcccagatc caaagtcccg cagcagccgg 1860
ccaaggtggc ttccagatga agggggactg gcctgtccac atgggagtca ggtgtcatgg 1920
ctgccctgag ctgggaggga agaaggctga cattacattt agtttgctct cactccatct 1980
ggctaagtga tcttgaaata ccacctctca ggtgaagaac cgtcaggaat tcccatctca 2040
caggctgtgg tgtagattaa gtagacaagg aatgtgaata atgcttagat cttattgatg 2100
acagagtgta tcctaatggt ttgttcatta tattacactt tcagtaaaaa aaaaaaaaaa 2160
aaaaa 2165

<210> 85
<211> 347
<212> PRT
<213> Homo sapiens

<400> 85

Met Ala Leu Met Leu Ser Leu Val Leu Ser Leu Leu Lys Leu Gly Ser
1               5                   10                  15

Gly Gln Trp Gln Val Phe Gly Pro Asp Lys Pro Val Gln Ala Leu Val
            20                  25                  30

Gly Glu Asp Ala Ala Phe Ser Cys Phe Leu Ser Pro Lys Thr Asn Ala
        35                  40                  45

Glu Ala Met Glu Val Arg Phe Phe Arg Gly Gln Phe Ser Ser Val Val
    50                  55                  60

His Leu Tyr Arg Asp Gly Lys Asp Gln Pro Phe Met Gln Met Pro Gln
65                  70                  75                  80

Tyr Gln Gly Arg Thr Lys Leu Val Lys Asp Ser Ile Ala Glu Gly Arg
                85                  90                  95

Ile Ser Leu Arg Leu Glu Asn Ile Thr Val Leu Asp Ala Gly Leu Tyr
            100                 105                 110

Gly Cys Arg Ile Ser Ser Gln Ser Tyr Tyr Gln Lys Ala Ile Trp Glu
        115                 120                 125

Leu Gln Val Ser Ala Leu Gly Ser Val Pro Leu Ile Ser Ile Thr Gly
    130                 135                 140

Tyr Val Asp Arg Asp Ile Gln Leu Leu Cys Gln Ser Ser Gly Trp Phe
145                 150                 155                 160

Pro Arg Pro Thr Ala Lys Trp Lys Gly Pro Gln Gly Gln Asp Leu Ser
                165                 170                 175

Thr Asp Ser Arg Thr Asn Arg Asp Met His Gly Leu Phe Asp Val Glu
            180                 185                 190

Ile Ser Leu Thr Val Gln Glu Asn Ala Gly Ser Ile Ser Cys Ser Met
        195                 200                 205

Arg His Ala His Leu Ser Arg Glu Val Glu Ser Arg Val Gln Ile Gly
    210                 215                 220

Asp Thr Phe Phe Glu Pro Ile Ser Trp His Leu Ala Thr Lys Val Leu
225                 230                 235                 240

Gly Ile Leu Cys Cys Gly Leu Phe Phe Gly Ile Val Gly Leu Lys Ile
                245                 250                 255

Phe Phe Ser Lys Phe Gln Cys Lys Arg Glu Arg Glu Ala Trp Ala Gly
            260                 265                 270

Ala Leu Phe Met Val Pro Ala Gly Thr Gly Ser Glu Met Leu Pro His
      275                 280                 285

Pro Ala Ala Ser Leu Leu Leu Val Leu Ala Ser Arg Gly Pro Gly Pro
    290                 295                 300

Lys Lys Glu Asn Pro Gly Gly Thr Gly Leu Glu Lys Lys Ala Arg Thr
305                 310                 315                 320

Gly Arg Ile Glu Arg Arg Pro Glu Thr Arg Ser Gly Gly Asp Ser Gly
                325                 330                 335

Ser Arg Asp Gly Ser Pro Glu Ala Leu Arg Phe
            340                 345

<210>  86

<211>  21

<212>  DNA

<213>  Künstliche Sequenz

<220>
<223>  Beschreibung der künstlichen Sequenz: Oligonukleotid

<400>  86
attcatggtt ccagcaggga c                                                21

<210>  87

<211>  21

<212>  DNA

<213>  Künstliche Sequenz

<220>
<223>  Beschreibung der künstlichen Sequenz: Oligonukleotid

<400>  87
gggagacaaa gtcacgtact c                                                21

<210>  88
<211>  22
<212>  DNA
<213>  künstliche Sequenz

<220>
<223>  Beschreibung der künstlichen Sequenz: Oligonukleotid

<400> 88
tcctggtgtt cgtggtctgc tt 22

<210> 89
<211> 22
<212> DNA
<213> künstliche Sequenz

<220>
<223> Beschreibung der künstlichen Sequenz: Oligonukleotid

<400> 89
gagagtcctg gcttttgtgg gc 22

<210> 90
<211> 15
<212> PRT
<213> Homo sapiens

<400> 90

Gly Ser Ser Asp Leu Thr Trp Pro Pro Ala Ile Lys Leu Gly Cys
1               5                   10                  15

<210> 91
<211> 16
<212> PRT
<213> Homo sapiens

<400> 91

Asp Arg Tyr Val Ala Val Arg His Pro Leu Arg Ala Arg Gly Leu Arg
1               5                   10                  15

<210> 92
<211> 15
<212> PRT
<213> Homo sapiens

<400> 92

Val Ala Pro Arg Ala Lys Ala His Lys Ser Gln Asp Ser Leu Cys
1               5                   10                  15

<210> 93
<211> 13
<212> PRT
<213> Homo sapiens

<400> 93

Cys Phe Arg Ser Thr Arg His Asn Phe Asn Ser Met Arg
1               5                   10

<210> 94
<211> 22
<212> PRT
<213> Homo sapiens

<400> 94

Met Asn Gly Thr Tyr Asn Thr Cys Gly Ser Ser Asp Leu Thr Trp Pro
1               5                   10                  15

Pro Ala Ile Lys Leu Gly
            20

<210>  95
<211>  14
<212>  PRT
<213>  Homo sapiens

<400>  95

Arg Asp Thr Ser Asp Thr Pro Leu Cys Gln Leu Ser Gln Gly
1               5                   10

<210>  96
<211>  22
<212>  PRT
<213>  Homo sapiens

<400>  96

Gly Ile Gln Glu Gly Gly Phe Cys Phe Arg Ser Thr Arg His Asn Phe
1               5                   10                  15

Asn Ser Met Arg Phe Pro
            20

<210>  97
<211>  30
<212>  PRT
<213>  Homo sapiens

<400>  97

Ala Lys Glu Phe Gln Glu Ala Ser Ala Leu Ala Val Ala Pro Arg Ala
1               5                   10                  15

Lys Ala His Lys Ser Gln Asp Ser Leu Cys Val Thr Leu Ala
            20                  25                  30

<210>  98
<211>  22
<212>  DNA
<213>  künstliche Sequenz

<220>
<223>  Beschreibung der künstlichen Sequenz: Oligonukleotid

<400>  98
tcctgctcgt cgctctcctg at                                          22

<210>  99
<211>  20
<212>  DNA
<213>  künstliche Sequenz

<220>

<223>  Beschreibung der künstlichen Sequenz: Oligonukleotid

<400>  99
tcgcttttg tcgtatttgc                                                    20


<210>  100
<211>  15
<212>  PRT
<213>  Homo sapiens

<400>  100

His Asn Gly Ser Tyr Glu Ile Ser Val Leu Met Met Gly Asn Ser
1               5                   10                  15


<210>  101
<211>  15
<212>  PRT
<213>  Homo sapiens

<400>  101

Asn Leu Pro Thr Pro Pro Thr Val Glu Asn Gln Gln Arg Leu Ala
1               5                   10                  15


<210>  102
<211>  619
<212>  PRT
<213>  Homo sapiens

<400>  102

Arg Lys Tyr Arg Lys Asp Tyr Glu Leu Arg Gln Lys Lys Trp Ser His
1               5                   10                  15


Ile Pro Pro Glu Asn Ile Phe Pro Leu Glu Thr Asn Glu Thr Asn His
            20                  25                  30


Val Ser Leu Lys Ile Asp Asp Asp Lys Arg Arg Asp Thr Ile Gln Arg
        35                  40                  45


Leu Arg Gln Cys Lys Tyr Asp Lys Lys Arg Val Ile Leu Lys Asp Leu
    50                  55                  60


Lys His Asn Asp Gly Asn Phe Thr Glu Lys Gln Lys Ile Glu Leu Asn
65                  70                  75                  80


Lys Leu Leu Gln Ile Asp Tyr Tyr Asn Leu Thr Lys Phe Tyr Gly Thr
                85                  90                  95


Val Lys Leu Asp Thr Met Ile Phe Gly Val Ile Glu Tyr Cys Glu Arg
            100                 105                 110


Gly Ser Leu Arg Glu Val Leu Asn Asp Thr Ile Ser Tyr Pro Asp Gly
        115                 120                 125

Thr Phe Met Asp Trp Glu Phe Lys Ile Ser Val Leu Tyr Asp Ile Ala
    130                 135                 140

Lys Gly Met Ser Tyr Leu His Ser Ser Lys Thr Glu Val His Gly Arg
145                 150                 155                 160

Leu Lys Ser Thr Asn Cys Val Val Asp Ser Arg Met Val Val Lys Ile
                165                 170                 175

Thr Asp Phe Gly Cys Asn Ser Ile Leu Pro Pro Lys Lys Asp Leu Trp
            180                 185                 190

Thr Ala Pro Glu His Leu Arg Gln Ala Asn Ile Ser Gln Lys Gly Asp
        195                 200                 205

Val Tyr Ser Tyr Gly Ile Ile Ala Gln Glu Ile Ile Leu Arg Lys Glu
    210                 215                 220

Thr Phe Tyr Thr Leu Ser Cys Arg Asp Arg Asn Glu Lys Ile Phe Arg
225                 230                 235                 240

Val Glu Asn Ser Asn Gly Met Lys Pro Phe Arg Pro Asp Leu Phe Leu
                245                 250                 255

Glu Thr Ala Glu Glu Lys Glu Leu Glu Val Tyr Leu Leu Val Lys Asn
            260                 265                 270

Cys Trp Glu Glu Asp Pro Glu Lys Arg Pro Asp Phe Lys Lys Ile Glu
        275                 280                 285

Thr Thr Leu Ala Lys Ile Phe Gly Leu Phe His Asp Gln Lys Asn Glu
    290                 295                 300

Ser Tyr Met Asp Thr Leu Ile Arg Arg Leu Gln Leu Tyr Ser Arg Asn
305                 310                 315                 320

Leu Glu His Leu Val Glu Glu Arg Thr Gln Leu Tyr Lys Ala Glu Arg
                325                 330                 335

Asp Arg Ala Asp Arg Leu Asn Phe Met Leu Leu Pro Arg Leu Val Val
            340                 345                 350

Lys Ser Leu Lys Glu Lys Gly Phe Val Glu Pro Glu Leu Tyr Glu Glu
        355                 360                 365

Val Thr Ile Tyr Phe Ser Asp Ile Val Gly Phe Thr Thr Ile Cys Lys
    370                 375                 380

Tyr Ser Thr Pro Met Glu Val Val Asp Met Leu Asn Asp Ile Tyr Lys
385                 390                 395                 400

Ser Phe Asp His Ile Val Asp His His Asp Val Tyr Lys Val Glu Thr
                405                 410                 415

Ile Gly Asp Ala Tyr Met Val Ala Ser Gly Leu Pro Lys Arg Asn Gly
            420                 425                 430

Asn Arg His Ala Ile Asp Ile Ala Lys Met Ala Leu Glu Ile Leu Ser
        435                 440                 445

Phe Met Gly Thr Phe Glu Leu Glu His Leu Pro Gly Leu Pro Ile Trp
    450                 455                 460

Ile Arg Ile Gly Val His Ser Gly Pro Cys Ala Ala Gly Val Val Gly
465                 470                 475                 480

Ile Lys Met Pro Arg Tyr Cys Leu Phe Gly Asp Thr Val Asn Thr Ala
                485                 490                 495

Ser Arg Met Glu Ser Thr Gly Leu Pro Leu Arg Ile His Val Ser Gly
            500                 505                 510

Ser Thr Ile Ala Ile Leu Lys Arg Thr Glu Cys Gln Phe Leu Tyr Glu
        515                 520                 525

Val Arg Gly Glu Thr Tyr Leu Lys Gly Arg Gly Asn Glu Thr Thr Tyr
    530                 535                 540

Trp Leu Thr Gly Met Lys Asp Gln Lys Phe Asn Leu Pro Thr Pro Pro
545                 550                 555                 560

Thr Val Glu Asn Gln Gln Arg Leu Gln Ala Glu Phe Ser Asp Met Ile
                565                 570                 575

Ala Asn Ser Leu Gln Lys Arg Gln Ala Ala Gly Ile Arg Ser Gln Lys
            580                 585                 590

Pro Arg Arg Val Ala Ser Tyr Lys Lys Gly Thr Leu Glu Tyr Leu Gln
        595                 600                 605

Leu Asn Thr Thr Asp Lys Glu Ser Thr Tyr Phe
    610                 615

<210>  103
<211>  20
<212>  DNA
<213>  künstliche Sequenz

<220>
<223>  Beschreibung der künstlichen Sequenz: Oligonukleotid

<400>  103
gctggtaact atcttcctgc                                                  20

<210>  104
<211>  20
<212>  DNA
<213>  künstliche Sequenz

<220>
<223>  Beschreibung der künstlichen Sequenz: Oligonukleotid


<400>  104
gaagaatgtt gtccagaggt                                                    20


<210>  105
<211>  15
<212>  PRT
<213>  Homo sapiens

<400>  105

Leu Ile Asn Lys Val Pro Leu Pro Val Asp Lys Leu Ala Pro Leu
1               5                   10                  15


<210>  106
<211>  15
<212>  PRT
<213>  Homo sapiens

<400>  106

Ser Glu Ala Val Lys Lys Leu Leu Glu Ala Leu Ser His Leu Val
1               5                   10                  15


<210>  107
<211>  20
<212>  DNA
<213>  künstliche Sequenz

<220>
<223>  Beschreibung der künstlichen Sequenz: Oligonukleotid


<400>  107
tgttttcaac taccaggggc                                                    20


<210>  108
<211>  20
<212>  DNA
<213>  künstliche Sequenz

<220>
<223>  Beschreibung der künstlichen Sequenz: Oligonukleotid


<400>  108
tgttggcttt ggcagagtcc                                                    20


<210>  109
<211>  24
<212>  DNA
<213>  künstliche Sequenz

<220>

<223>  Beschreibung der künstlichen Sequenz: Oligonukleotid

<400>  109
gaggcagagt tcaggcttca ccga                                              24

<210>  110
<211>  20
<212>  DNA
<213>  künstliche Sequenz

<220>
<223>  Beschreibung der künstlichen Sequenz: Oligonukleotid

<400>  110
tgttggcttt ggcagagtcc                                                   20

<210>  111
<211>  56
<212>  PRT
<213>  Homo sapiens

<400>  111

Thr Gly Met Asp Met Trp Ser Thr Gln Asp Leu Tyr Asp Asn Pro Val
1               5                   10                  15

Thr Ser Val Phe Gln Tyr Glu Gly Leu Trp Arg Ser Cys Val Arg Gln
            20                  25                  30

Ser Ser Gly Phe Thr Glu Cys Arg Pro Tyr Phe Thr Ile Leu Gly Leu
        35                  40                  45

Pro Ala Met Leu Gln Ala Val Arg
    50                  55

<210>  112
<211>  53
<212>  PRT
<213>  Homo sapiens

<400>  112

Asp Gln Trp Ser Thr Gln Asp Leu Tyr Asn Asn Pro Val Thr Ala Val
1               5                   10                  15

Phe Asn Tyr Gln Gly Leu Trp Arg Ser Cys Val Arg Glu Ser Ser Gly
            20                  25                  30

Phe Thr Glu Cys Arg Gly Tyr Phe Thr Leu Leu Gly Leu Pro Ala Met
        35                  40                  45

Leu Gln Ala Val Arg
    50

<210>  113
<211>  14

<212>  PRT
<213>  Homo sapiens

<400>  113

Ser Thr Gln Asp Leu Tyr Asn Asn Pro Val Thr Ala Val Phe
1               5                   10


<210>  114
<211>  12
<212>  PRT
<213>  Homo sapiens

<400>  114

Asp Met Trp Ser Thr Gln Asp Leu Tyr Asp Asn Pro
1               5                   10


<210>  115
<211>  12
<212>  PRT
<213>  Homo sapiens

<400>  115

Cys Arg Pro Tyr Phe Thr Ile Leu Gly Leu Pro Ala
1               5                   10


<210>  116
<211>  13
<212>  PRT
<213>  Homo sapiens

<400>  116

Thr Asn Phe Trp Met Ser Thr Ala Asn Met Tyr Thr Gly
1               5                   10


<210>  117
<211>  816
<212>  DNA
<213>  Homo sapiens

<400>  117
gccaggatca tgtccaccac cacatgccaa gtggtggcgt tcctcctgtc catcctgggg      60

ctggccggct gcatcgcggc caccgggatg gacatgtgga gcacccagga cctgtacgac     120

aaccccgtca cctccgtgtt ccagtacgaa gggctctgga ggagctgcgt gaggcagagt     180

tcaggcttca ccgaatgcag gccctatttc accatcctgg gacttccagc catgctgcag     240

gcagtgcgag ccctgatgat cgtaggcatc gtcctgggtg ccattggcct cctggtatcc     300

atctttgccc tgaaatgcat ccgcattggc agcatggagg actctgccaa agccaacatg     360

acactgacct ccgggatcat gttcattgtc tcaggtcttt gtgcaattgc tggagtgtct     420

gtgtttgcca acatgctggt gactaacttc tggatgtcca cagctaacat gtacaccggc     480

atgggtggga tggtgcagac tgttcagacc aggtacacat ttggtgcggc tctgttcgtg     540

ggctgggtcg ctggaggcct cacactaatt gggggtgtga tgatgtgcat cgcctgccgg     600

ggcctggcac cagaagaaac caactacaaa gccgtttctt atcatgcctc aggccacagt 660
gttgcctaca agcctggagg cttcaaggcc agcactggct ttgggtccaa caccaaaaac 720
aagaagatat acgatggagg tgcccgcaca gaggacgagg tacaatctta tccttccaag 780
cacgactatg tgtaatgctc taagacctct cagcac 816

<210> 118
<211> 261
<212> PRT
<213> Homo sapiens

<400> 118

Met Ser Thr Thr Thr Cys Gln Val Val Ala Phe Leu Leu Ser Ile Leu
1               5                   10                  15

Gly Leu Ala Gly Cys Ile Ala Ala Thr Gly Met Asp Met Trp Ser Thr
            20                  25                  30

Gln Asp Leu Tyr Asp Asn Pro Val Thr Ser Val Phe Gln Tyr Glu Gly
        35                  40                  45

Leu Trp Arg Ser Cys Val Arg Gln Ser Ser Gly Phe Thr Glu Cys Arg
    50                  55                  60

Pro Tyr Phe Thr Ile Leu Gly Leu Pro Ala Met Leu Gln Ala Val Arg
65                  70                  75                  80

Ala Leu Met Ile Val Gly Ile Val Leu Gly Ala Ile Gly Leu Leu Val
                85                  90                  95

Ser Ile Phe Ala Leu Lys Cys Ile Arg Ile Gly Ser Met Glu Asp Ser
            100                 105                 110

Ala Lys Ala Asn Met Thr Leu Thr Ser Gly Ile Met Phe Ile Val Ser
        115                 120                 125

Gly Leu Cys Ala Ile Ala Gly Val Ser Val Phe Ala Asn Met Leu Val
    130                 135                 140

Thr Asn Phe Trp Met Ser Thr Ala Asn Met Tyr Thr Gly Met Gly Gly
145                 150                 155                 160

Met Val Gln Thr Val Gln Thr Arg Tyr Thr Phe Gly Ala Ala Leu Phe
                165                 170                 175

Val Gly Trp Val Ala Gly Gly Leu Thr Leu Ile Gly Gly Val Met Met
            180                 185                 190

Cys Ile Ala Cys Arg Gly Leu Ala Pro Glu Glu Thr Asn Tyr Lys Ala
        195                 200                 205

Val Ser Tyr His Ala Ser Gly His Ser Val Ala Tyr Lys Pro Gly Gly
    210                 215                 220

Phe Lys Ala Ser Thr Gly Phe Gly Ser Asn Thr Lys Asn Lys Lys Ile
225                 230                 235                 240

Tyr Asp Gly Gly Ala Arg Thr Glu Asp Glu Val Gln Ser Tyr Pro Ser
                245                 250                 255

Lys His Asp Tyr Val
            260

<210>  119
<211>  227
<212>  DNA
<213>  Homo sapiens

<400>  119
gccaggatca tgtccaccac cacatgccaa gtggtggcgt tcctcctgtc catcctgggg      60

ctggccggct gcatcgcggc caccgggatg gacatgtgga gcacccagga cctgtacgac     120

aaccccgtca cctccgtgtt ccagtacgaa gggctctgga ggagctgcgt gaggcagagt     180

tcaggcttca ccgaatgcag gccctatttc accatcctgg gacttcc                   227

<210>  120
<211>  69
<212>  PRT
<213>  Homo sapiens

<400>  120

Met Ser Thr Thr Thr Cys Gln Val Val Ala Phe Leu Leu Ser Ile Leu
1               5                   10                  15

Gly Leu Ala Gly Cys Ile Ala Ala Thr Gly Met Asp Met Trp Ser Thr
            20                  25                  30

Gln Asp Leu Tyr Asp Asn Pro Val Thr Ser Val Phe Gln Tyr Glu Gly
        35                  40                  45

Leu Trp Arg Ser Cys Val Arg Gln Ser Ser Gly Phe Thr Glu Cys Arg
    50                  55                  60

Pro Tyr Phe Thr Ile
65

<210>  121
<211>  20
<212>  DNA
<213>  künstliche Sequenz

<220>
<223>  Beschreibung der künstlichen Sequenz: Oligonukleotid

<400>  121

aatgagagga aagagaaaac                                                20

<210>  122
<211>  20
<212>  DNA
<213>  künstliche Sequenz

<220>
<223>  Beschreibung der künstlichen Sequenz: Oligonukleotid

<400>  122
atggtagaag agtaggcaat                                                20

<210>  123
<211>  15
<212>  PRT
<213>  Homo sapiens

<400>  123

Glu Lys Trp Asn Leu His Lys Arg Ile Ala Leu Lys Met Val Cys
1               5                   10                  15

<210>  124
<211>  11
<212>  PRT
<213>  Homo sapiens

<400>  124

Cys Leu Gly Phe Asn Phe Lys Glu Met Phe Lys
1               5                   10

<210>  125
<211>  23
<212>  DNA
<213>  künstliche Sequenz

<220>
<223>  Beschreibung der künstlichen Sequenz: Oligonukleotid

<400>  125
taatgatgaa ccctacactg agc                                            23

<210>  126
<211>  20
<212>  DNA
<213>  künstliche Sequenz

<220>
<223>  Beschreibung der künstlichen Sequenz: Oligonukleotid

<400>  126
atggacaaat gccctacctt                                                20

<210>  127
<211>  22
<212>  DNA
<213>  künstliche Sequenz

<220>
<223>  Beschreibung der künstlichen Sequenz: Oligonukleotid

<400>  127
agtgctggaa ggatgtgcgt gt                                              22

<210>  128
<211>  20
<212>  DNA
<213>  künstliche Sequenz

<220>
<223>  Beschreibung der künstlichen Sequenz: Oligonukleotid

<400>  128
ttgaggtggt tgttgggttt                                                 20

<210>  129
<211>  20
<212>  DNA
<213>  künstliche Sequenz

<220>
<223>  Beschreibung der künstlichen Sequenz: Oligonukleotid

<400>  129
agatgtgctg aggctgtaga                                                 20

<210>  130
<211>  20
<212>  DNA
<213>  künstliche Sequenz

<220>
<223>  Beschreibung der künstlichen Sequenz: Oligonukleotid

<400>  130
atgaaggttg attatttgag                                                 20

<210>  131
<211>  23
<212>  DNA
<213>  künstliche Sequenz

<220>
<223>  Beschreibung der künstlichen Sequenz: Oligonukleotid

<400>  131
agccgcatac tcccttaccc tct                                             23

<210>  132
<211>  20
<212>  DNA
<213>  künstliche Sequenz

<220>
<223>  Beschreibung der künstlichen Sequenz: Oligonukleotid

<400> 132
gcagcagccc aaacaccaca                                                    20


<210> 133
<211> 20
<212> DNA
<213> künstliche Sequenz

<220>
<223> Beschreibung der künstlichen Sequenz: Oligonukleotid


<400> 133
ctgagccgag aggtggaatc                                                    20


<210> 134
<211> 20
<212> DNA
<213> künstliche Sequenz

<220>
<223> Beschreibung der künstlichen Sequenz: Oligonukleotid


<400> 134
ctctctcgct tacactggaa                                                    20


<210> 135
<211> 14
<212> PRT
<213> Homo sapiens

<400> 135

Gln Trp Gln Val Phe Gly Pro Asp Lys Pro Val Gln Ala Leu
1               5                   10


<210> 136
<211> 15
<212> PRT
<213> Homo sapiens

<400> 136

Ala Lys Trp Lys Gly Pro Gln Gly Gln Asp Leu Ser Thr Asp Ser
1               5                   10                  15


<210> 137
<211> 32
<212> PRT
<213> Homo sapiens

<400> 137

Asn Met Leu Val Thr Asn Phe Trp Met Ser Thr Ala Asn Met Tyr Thr
1               5                   10                  15


Gly Met Gly Gly Met Val Gln Thr Val Gln Thr Arg Tyr Thr Phe Gly
            20                  25                  30

<210> 138
<211> 2052
<212> DNA
<213> Homo sapiens

<400> 138
gacagctgtg tctcgatgga gtagactctc agaacagcgc agtttgccct ccgctcacgc 60

agagcctctc cgtggcttcc gcaccttgag cattaggcca gttctcctct tctctctaat 120

ccatccgtca cctctcctgt catccgtttc catgccgtga ggtccattca cagaacacat 180

ccatggctct catgctcagt ttggttctga gtctcctcaa gctgggatca gggcagtggc 240

aggtgtttgg gccagacaag cctgtccagg ccttggtggg ggaggacgca gcattctcct 300

gtttcctgtc tcctaagacc aatgcagagg ccatggaagt gcggttcttc aggggccagt 360

tctctagcgt ggtccacctc tacagggacg ggaaggacca gccatttatg cagatgccac 420

agtatcaagg caggacaaaa ctggtgaagg attctattgc ggaggggcgc atctctctga 480

ggctggaaaa cattactgtg ttggatgctg gcctctatgg gtgcaggatt agttcccagt 540

cttactacca gaaggccatc tgggagctac aggtgtcagc actgggctca gttcctctca 600

tttccatcac gggatatgtt gatagagaca tccagctact ctgtcagtcc tcgggctggt 660

tcccccggcc cacagcgaag tggaaaggtc cacaaggaca ggatttgtcc acagactcca 720

ggacaaacag agacatgcat ggcctgtttg atgtggagat ctctctgacc gtccaagaga 780

acgccgggag catatcctgt tccatgcggc atgctcatct gagccgagag gtggaatcca 840

gggtacagat aggagatacc tttttcgagc ctatatcgtg gcacctggct accaaagtac 900

tgggaatact ctgctgtggc ctattttttg gcattgttgg actgaagatt ttcttctcca 960

aattccagtg gaaaatccag gcggaactgg actggagaag aaagcacgga caggcagaat 1020

tgagagacgc ccggaaacac gcagtggagg tgactctgga tccagagacg gctcacccga 1080

agctctgcgt ttctgatctg aaaactgtaa cccatagaaa agctccccag gaggtgcctc 1140

actctgagaa gagatttaca aggaagagtg tggtggcttc tcagagtttc caagcaggga 1200

aacattactg ggaggtggac ggaggacaca ataaaaggtg gcgcgtggga gtgtgccggg 1260

atgatgtgga caggaggaag gagtacgtga ctttgtctcc cgatcatggg tactgggtcc 1320

tcagactgaa tggagaacat ttgtatttca cattaaatcc ccgttttatc agcgtcttcc 1380

ccaggacccc acctacaaaa ataggggtct tcctggacta tgagtgtggg accatctcct 1440

tcttcaacat aaatgaccag tcccttattt ataccctgac atgtcggttt gaaggcttat 1500

tgaggcccta cattgagtat ccgtcctata atgagcaaaa tggaactccc atagtcatct 1560

gcccagtcac ccaggaatca gagaaagagg cctcttggca aagggcctct gcaatcccag 1620

agacaagcaa cagtgagtcc tcctcacagg caaccacgcc cttcctcccc aggggtgaaa 1680

tgtaggatga atcacatccc acattcttct ttagggatat taaggtctct ctcccagatc 1740

caaagtcccg cagcagccgg ccaaggtggc ttccagatga agggggactg gcctgtccac 1800

atgggagtca ggtgtcatgg ctgccctgag ctgggaggga agaaggctga cattacattt 1860
agtttgctct cactccatct ggctaagtga tcttgaaata ccacctctca ggtgaagaac 1920
cgtcaggaat tcccatctca caggctgtgg tgtagattaa gtagacaagg aatgtgaata 1980
atgcttagat cttattgatg acagagtgta tcctaatggt ttgttcatta tattacactt 2040
tcagtaaaaa aa 2052

<210> 139
<211> 500
<212> PRT
<213> Homo sapiens

<400> 139

Met Ala Leu Met Leu Ser Leu Val Leu Ser Leu Leu Lys Leu Gly Ser
1               5                   10                  15

Gly Gln Trp Gln Val Phe Gly Pro Asp Lys Pro Val Gln Ala Leu Val
            20                  25                  30

Gly Glu Asp Ala Ala Phe Ser Cys Phe Leu Ser Pro Lys Thr Asn Ala
        35                  40                  45

Glu Ala Met Glu Val Arg Phe Phe Arg Gly Gln Phe Ser Ser Val Val
    50                  55                  60

His Leu Tyr Arg Asp Gly Lys Asp Gln Pro Phe Met Gln Met Pro Gln
65                  70                  75                  80

Tyr Gln Gly Arg Thr Lys Leu Val Lys Asp Ser Ile Ala Glu Gly Arg
                85                  90                  95

Ile Ser Leu Arg Leu Glu Asn Ile Thr Val Leu Asp Ala Gly Leu Tyr
            100                 105                 110

Gly Cys Arg Ile Ser Ser Gln Ser Tyr Tyr Gln Lys Ala Ile Trp Glu
        115                 120                 125

Leu Gln Val Ser Ala Leu Gly Ser Val Pro Leu Ile Ser Ile Thr Gly
    130                 135                 140

Tyr Val Asp Arg Asp Ile Gln Leu Leu Cys Gln Ser Ser Gly Trp Phe
145                 150                 155                 160

Pro Arg Pro Thr Ala Lys Trp Lys Gly Pro Gln Gly Gln Asp Leu Ser
                165                 170                 175

Thr Asp Ser Arg Thr Asn Arg Asp Met His Gly Leu Phe Asp Val Glu
            180                 185                 190

Ile Ser Leu Thr Val Gln Glu Asn Ala Gly Ser Ile Ser Cys Ser Met

        195                 200                 205

Arg His Ala His Leu Ser Arg Glu Val Glu Ser Arg Val Gln Ile Gly
    210                 215                 220

Asp Thr Phe Phe Glu Pro Ile Ser Trp His Leu Ala Thr Lys Val Leu
225                 230                 235                 240

Gly Ile Leu Cys Cys Gly Leu Phe Phe Gly Ile Val Gly Leu Lys Ile
                245                 250                 255

Phe Phe Ser Lys Phe Gln Trp Lys Ile Gln Ala Glu Leu Asp Trp Arg
            260                 265                 270

Arg Lys His Gly Gln Ala Glu Leu Arg Asp Ala Arg Lys His Ala Val
        275                 280                 285

Glu Val Thr Leu Asp Pro Glu Thr Ala His Pro Lys Leu Cys Val Ser
    290                 295                 300

Asp Leu Lys Thr Val Thr His Arg Lys Ala Pro Gln Glu Val Pro His
305                 310                 315                 320

Ser Glu Lys Arg Phe Thr Arg Lys Ser Val Val Ala Ser Gln Ser Phe
                325                 330                 335

Gln Ala Gly Lys His Tyr Trp Glu Val Asp Gly Gly His Asn Lys Arg
            340                 345                 350

Trp Arg Val Gly Val Cys Arg Asp Asp Val Asp Arg Arg Lys Glu Tyr
        355                 360                 365

Val Thr Leu Ser Pro Asp His Gly Tyr Trp Val Leu Arg Leu Asn Gly
    370                 375                 380

Glu His Leu Tyr Phe Thr Leu Asn Pro Arg Phe Ile Ser Val Phe Pro
385                 390                 395                 400

Arg Thr Pro Pro Thr Lys Ile Gly Val Phe Leu Asp Tyr Glu Cys Gly
                405                 410                 415

Thr Ile Ser Phe Phe Asn Ile Asn Asp Gln Ser Leu Ile Tyr Thr Leu
            420                 425                 430

Thr Cys Arg Phe Glu Gly Leu Leu Arg Pro Tyr Ile Glu Tyr Pro Ser
        435                 440                 445

Tyr Asn Glu Gln Asn Gly Thr Pro Ile Val Ile Cys Pro Val Thr Gln
    450                 455                 460

Glu Ser Glu Lys Glu Ala Ser Trp Gln Arg Ala Ser Ala Ile Pro Glu

465                 470                 475                 480

Thr Ser Asn Ser Glu Ser Ser Ser Gln Ala Thr Thr Pro Phe Leu Pro
                485                 490                 495

Arg Gly Glu Met
            500

<210>  140
<211>  21
<212>  DNA
<213>  künstliche Sequenz

<220>
<223>  Beschreibung der künstlichen Sequenz: Oligonukleotid

<400>  140
tccaaattcc agtggaaaat c                                                21

<210>  141
<211>  21
<212>  DNA
<213>  künstliche Sequenz

<220>
<223>  Beschreibung der künstlichen Sequenz: Oligonukleotid

<400>  141
ccacactcat agtccaggaa g                                                21

<210>  142
<211>  116
<212>  PRT
<213>  Homo sapiens

<400>  142

Ala Leu Met Ile Val Gly Ile Val Leu Gly Ala Ile Gly Leu Leu Val
1               5                   10                  15

Ser Ile Phe Ala Leu Lys Cys Ile Arg Ile Gly Ser Met Glu Asp Ser
            20                  25                  30

Ala Lys Ala Asn Met Thr Leu Thr Ser Gly Ile Met Phe Ile Val Ser
        35                  40                  45

Gly Leu Cys Ala Ile Ala Gly Val Ser Val Phe Ala Asn Met Leu Val
    50                  55                  60

Thr Asn Phe Trp Met Ser Thr Ala Asn Met Tyr Thr Gly Met Gly Gly
65                  70                  75                  80

Met Val Gln Thr Val Gln Thr Arg Tyr Thr Phe Gly Ala Ala Leu Phe
                85                  90                  95

Val Gly Trp Val Ala Gly Gly Leu Thr Leu Ile Gly Gly Val Met Met
            100                 105                 110

Cys Ile Ala Cys
        115

<210> 143
<211> 24
<212> PRT
<213> Homo sapiens

<400> 143

Arg Ile Gly Ser Met Glu Asp Ser Ala Lys Ala Asn Met Thr Leu Thr
1               5                   10                  15

Ser Gly Ile Met Phe Ile Val Ser
            20

<210> 144
<211> 8
<212> PRT
<213> Homo sapiens

<400> 144

Ala Lys Ala Asn Met Thr Leu Thr
1               5

<210> 145
<211> 14
<212> PRT
<213> Homo sapiens

<400> 145

Met Glu Asp Ser Ala Lys Ala Asn Met Thr Leu Thr Ser Gly
1               5                   10

<210> 146
<211> 14
<212> PRT
<213> Homo sapiens

<400> 146

Met Glu Asp Ser Ala Lys Ala Asp Met Thr Leu Thr Ser Gly
1               5                   10

<210> 147
<211> 9
<212> PRT
<213> Homo sapiens

<400> 147

Ser Ala Lys Ala Asp Met Thr Leu Thr
1               5

<210> 148

<211>  9
<212>  PRT
<213>  Homo sapiens

<400>  148

Ala Lys Ala Asp Met Thr Leu Thr Leu
1               5


<210>  149
<211>  53
<212>  PRT
<213>  Homo sapiens

<400>  149

Asp Gln Trp Ser Thr Gln Asp Leu Tyr Asp Asn Pro Val Thr Ala Val
1               5                   10                  15


Phe Asn Tyr Gln Gly Leu Trp Arg Ser Cys Val Arg Glu Ser Ser Gly
            20                  25                  30


Phe Thr Glu Cys Arg Gly Tyr Phe Thr Leu Leu Gly Leu Pro Ala Met
        35                  40                  45


Leu Gln Ala Val Arg
    50


<210>  150
<211>  14
<212>  PRT
<213>  Homo sapiens

<400>  150

Ser Thr Gln Asp Leu Tyr Asp Asn Pro Val Thr Ala Val Phe
1               5                   10